(19) Europäisches Patentamt
European Patent Office
Office européen des brevets

(11) Publication number : **0 515 041 A2**

(12) **EUROPEAN PATENT APPLICATION**

(21) Application number : **92303669.3**

(22) Date of filing : **23.04.92**

(51) Int. Cl.$^5$ : **C07D 403/04,** A01N 43/54, A01N 43/58, C07D 401/04, C07D 401/14

(30) Priority : **24.04.91 US 690744**

(43) Date of publication of application : **25.11.92 Bulletin 92/48**

(84) Designated Contracting States : **PT**

(71) Applicant : **E.I. DU PONT DE NEMOURS AND COMPANY**
**1007 Market Street**
**Wilmington Delaware 19898 (US)**

(72) Inventor : **Chang, Zen-Yu**
**23 Staten Drive**
**Hockessin, Delaware 19707 (US)**
Inventor : **Hanagan, Mary Ann**
**108 Country Flower Road**
**Newark, Delaware 19711 (US)**
Inventor : **Selby, Thomas Paul**
**116 Hunter Court**
**Wilmington, Delaware 19808 (US)**
Inventor : **Frasier, Deborah A.**
**109 Deerfield Road, Apartment B**
**Elkton, Maryland (US)**

(74) Representative : **Hildyard, Edward Martin et al**
**Frank B. Dehn & Co. European Patent Attorneys Imperial House 15-19 Kingsway**
**London WC2B 6UZ (GB)**

(54) **Fungicidal pyrazoles, pyrazolines and tetrahydropyridazines.**

(57) Fungicidal compounds useful e.g. for plant protection have the formula :

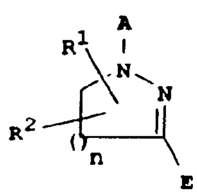
I

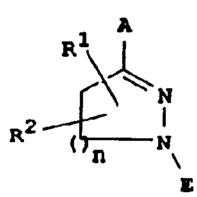
II

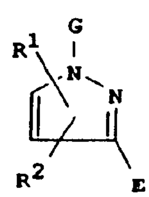
III

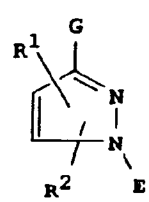
IV

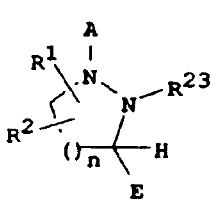
V

or

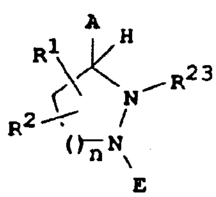
VI

wherein :

A is a heterocyclic group ;
G is optionally substituted quinazolinyl ;
E is H ; halogen, or an organic group ;
n is 1, 2 or 3
$R^1$ is H, halogen, cyano or an organic group ;
$R^2$ is H, cyano or an organic group ; and
$R^{23}$ is H or an organic group.

EP 0 515 041 A2

## FIELD OF THE INVENTION

The present invention relates to novel fungicides, their salts and metal complexes thereof, processes for their production, fungicidal compositions containing them, and a fungicidal method for applying them.

## BACKGROUND OF THE INVENTION

New fungicides for controlling fungus growth on vegetation are in constant demand. In the most common situation, such compounds are sought to selectively control fungus growth on useful crops such as cotton, rice, corn, wheat and soybeans, to name a few. Unchecked fungus growth in such crops can cause significant losses, reducing profit to the farmer and increasing costs to the consumer. There are many products commercially available for these purposes, but the search continues for products which are more effective, less costly and environmentally safe.

A number of fungicides have been developed and employed. For example, U.S. 3,920,646 discloses the compound

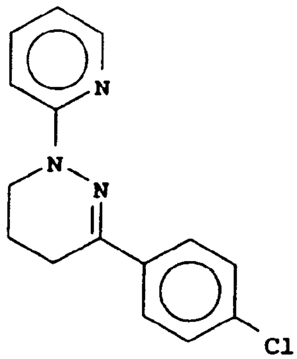

as an anti-inflammatory agent.

Konishi et al. (J. Pest. Sci. **1990**, 15, 13-22) disclose fungicidal pyazolylpyrimidines of the formula

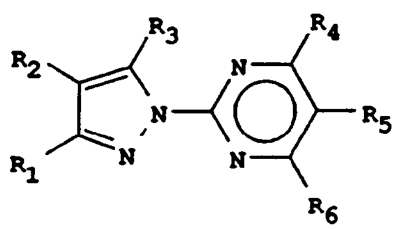

wherein $R_1$-$R_6$ are H, alkyl, aryl or alkenyl. Alkyl substitution enhanced the fungicidal activity in both pyrazole and pyrimidine rings. The activity was impaired by introduction of a phenyl group on the pyrazole ring.

## SUMMARY OF THE INVENTION

This invention pertains to compounds of Formulae I, II, III, IV, V, or VI including all geometric and stereoisomers, their salts, metal complexes thereof and agricultural compositions containing them and their use as fungicides.

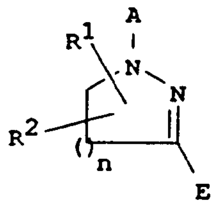

I

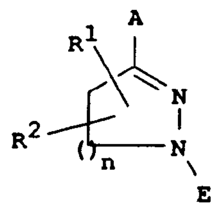

II

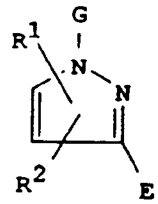

III

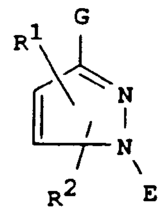

IV

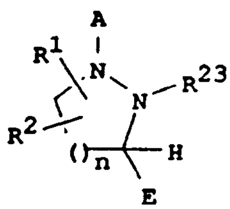

V

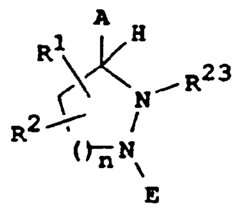

VI

.

wherein:

A is 2-pyrimidinyl, 2-pyridyl, 2-quinolinyl, 2-quinazolinyl, 1-isoquinolinyl or 3 isoquinolinyl each optionally substituted with $R^3$, $R^4$ and $R^{18}$; or s-triazinyl optionally substituted with $R^3$ and $R^4$; provided that $R^3$, $R^4$ and $R^{18}$ only substitute carbon atoms of the heterocycles;

G is 2-quinazolinyl optionally substituted with $R^3$, $R^4$ and $R^{18}$;

E is H; halogen; $C_1$-$C_6$ alkyl; $C_3$-$C_7$ cycloalkyl optionally substituted with 1-2 methyl; $C_1$-$C_6$ haloalkyl; $C_1$-$C_6$ alkylthio; $C_1$-$C_6$ alkoxy; $C_1$-$C_6$ haloalkoxy; or phenyl, phenoxy, phenylthio, phenylamino, phenylmethyl, indanyl, tetrahydronaphthalenyl, 1-naphthalenyl, 2-naphthalenyl, thienyl, furanyl or pyridyl each optionally substituted with $R^5$, $R^6$ and $R^7$;

n is 1, 2 or 3;

$R^1$ is H; halogen; cyano; hydroxy, $C_1$-$C_4$ alkoxy, -OC(=O)$R^{19}$, -OC(=O)NH$R^{20}$ $C_1$-$C_4$ alkyl; $C_1$-$C_4$ haloalkyl; $C_2$-$C_3$ alkylcarbonyl; $C_2$-$C_4$ alkenyl; $C_2$-$C_6$ alkoxyalkyl; $C_2$-$C_4$ alkynyl; $C_2$-$C_3$ alkoxycarbonyl; or phenyl, phenylmethyl, 1-naphthalenyl, 2-naphthalenyl, thienyl, furanyl or pyridyl each optionally substituted with $R^8$, $R^9$ and $R^{10}$;

$R^2$ is H, cyano, $C_1$-$C_4$ alkyl or $C_1$-$C_4$ haloalkyl;

$R^3$, $R^4$ and $R^{18}$ are independently halogen; cyano; hydroxy; ($C_1$-$C_4$ alkyl)$_3$silylmethyl; phenyl optionally substituted with $R^{21}$; $C_1$-$C_6$ alkyl; cyclopropyl; $C_1$-$C_6$ haloalkyl; $C_1$-$C_6$ alkylthio; $C_2$-$C_4$ alkenyl; $C_2$-$C_4$ alkynyl; $C_1$-$C_4$ alkoxy; $C_1$-$C_4$ haloalkoxy; $C_2$-$C_4$ alkenyloxy; $C_2$-$C_4$ alkynyloxy; $C_2$-$C_4$ alkoxyalkyl; $NR^{11}R^{12}$; or when $R^3$ and $R^4$, $R^3$ and $R^{18}$ or $R^4$ and $R^{18}$ substitute adjacent carbon atoms, then $R^3$ and $R^4$, $R^3$ and $R^{18}$ or $R^4$ and $R^{18}$ may together be $-(CH_2)_3-$ or $-(CH_2)_4-$ each optionally substituted with 1-2 methyl;

$R^5$ and $R^8$ are independently halogen; cyano; nitro; hydroxy, hydroxycarbonyl; $C_1$-$C_6$ alkyl; $C_3$-$C_6$ cycloalkyl, $C_1$-$C_6$ haloalkyl; $C_1$-$C_4$ alkylthio; $C_1$-$C_4$ alkylsulfinyl; $C_1$-$C_4$ alkylsulfonyl; ($C_1$-$C_4$ alkyl)$_3$silyl; $C_2$-$C_5$ alkylcarbonyl; $C_2$-$C_4$ alkenyl; $C_2$-$C_4$ alkenyloxy; $C_2$-$C_4$ alkynyl; $C_2$-$C_4$ alkynyloxy; $C_1$-$C_4$ alkoxy; $C_1$-$C_4$ haloalkoxy; $C_2$-$C_4$ alkoxyalkyl; $C_2$-$C_5$ alkoxycarbonyl; $C_2$-$C_4$ alkoxyalkoxy; $NR^{13}R^{14}$; C(=O)$NR^{15}R^{16}$; or phenyl, phenoxy or phenylthio each optionally substituted with $R^{17}$;

$R^6$, $R^7$, $R^9$, $R^{10}$, $R^{17}$, $R^{21}$, $R^{22}$, and $R^{24}$ are independently halogen, $C_1$-$C_4$ alkyl, $C_1$-$C_4$ haloalkyl, $C_1$-$C_4$ alkoxy or $C_1$-$C_4$ haloalkoxy;

$R^{11}$, $R^{12}$, $R^{13}$, $R^{14}$, $R^{15}$, $R^{16}$ are independently H; $C_1$-$C_2$ alkyl; or $R^{11}$ and $R^{12}$, $R^{13}$ and $R^{14}$ or $R^{15}$ and $R^{16}$ can be taken together with the nitrogen to which they attached to form a morpholino, pyrrolidino or piperidino group.

$R^{19}$ and $R^{25}$ are H or $C_1$-$C_3$ alkyl;

$R^{20}$ and $R^{26}$ are $C_1$-$C_4$ alkyl; or phenyl optionally substituted with $R^{22}$;

$R^{23}$ is H, $C_1$-$C_4$ alkyl, $C_1$-$C_4$ haloalkyl, $C_2$-$C_5$ alkylcarbonyl, phenylcarbonyl optionally substituted with $R^{24}$, $C_3$-$C_4$ alkenyl, $C_3$-$C_4$ alkynyl, phenylmethyl optionally substituted with $R^{24}$ on the phenyl ring. $C_1$-$C_4$ alkylsulfinyl, $C_1$-$C_4$ alkylsulfonyl, phenylsulfinyl, $C_1$-$C_4$ alkylsulfonyl, phenylsulfinyl optionally substituted with $R^{24}$, phenylsulfonyl optionally substituted with $R^{24}$, $C_2$-$C_4$ alkoxycarbonyl, phenoxycarbonyl optionally substituted with $R^{24}$, C (=O) $NR^{25}R^{26}$, C(=S) $NHR^{26}$ P (=S) $(OR^{26})_2$, P(=O) $(OR^{26})_2$, or S $(=O)_2NR^{25}R^{26}$;

provided that

i) when E is halogen, $C_1$-$C_6$ alkylthio, $C_1$-$C_6$ alkoxy, $C_1$-$C_6$ haloalkoxy, phenoxy, phenylthio or phenylamino, then E may only substitute compounds of Formula I and III;

ii) for compounds of Formula I, when A is 2-pyridyl, n is 2, and $R^1$ and $R^2$ are H, then E is not phenyl substituted with 1 to 2 fluorine, chlorine, trifluoromethyl, $C_1$-$C_4$ alkyl, $C_1$-$C_4$ alkoxy, or E is not thienyl or furanyl;

iii) for compounds of Formula III, either E is phenyl, phenoxy, phenylthio, phenylmethyl, 1-naphthalenyl, 2-naphthalenyl, thienyl, furanyl, pyridyl each optionally substituted with $R^5$, $R^6$ and $R^7$; or $R^1$ is phenyl, phenylmethyl, 1-naphthalenyl, 2-naphthalenyl, thienyl, furanyl or pyridyl each optionally substituted with $R^8$, $R^9$ and $R^{10}$; and $R^1$ must be in the 4-position;

iv) for compounds of Formula III, $R^5$ is not $NR^{13}R^{14}$;

v) for compounds of Formulae I and II, when n is 1, $R^1$ and $R^2$ do not occupy the 5-position of the pyrazoline ring;

vi) for compounds of Formula I, when A is s-triazinyl, then $R^3$ or $R^4$ are not $NH_2$;

vii) for compounds of Formula I, when A is 2-pyridyl optionally substituted with $R^3$, $R^{18}$ and $R^4$, and n is 1, then E is not phenylamino optionally substituted with $R^5$, $R^6$ and $R^7$;

viii) for compounds of Formulae I and III, when A is 2-pyridyl, n is 1, and $R^1$ and $R^2$ are H, then E is not phenyl, 9-bromophenyl, 4-methoxyphenyl, 4-nitrophenyl or 4-hydroxyphenyl;

ix) for compounds of Formula II, when n is 3, E is not H or $C_1$-$C_5$ alkyl;

x) for compounds of Formula II, when n is 1, then E is not H;

xi) for compounds of Formula I, when n is 1, and A is 6-methoxypyridine, then E is not 4-*N*,*N*-diethylaminophenyl;

xii) for compounds of Formula II, when A is 2-pyridyl, n is 2, and $R^1$ and $R^2$ are H, then E is not $C_1$-$C_4$ alkyl or pyridyl.

Preferred for reasons of greatest fungicidal activity and/or ease of synthesis are

1. Compounds of Formula I and V wherein:

A is 2-pyrimidinyl or 2-quinazolinyl optionally substituted with $R^3$, $R^4$ and $R^{18}$; and

$R^1$ is H; hydroxy, $C_1$-$C_4$ alkoxy, $C_1$-$C_4$ alkyl; $C_1$-$C_4$ haloalkyl; $C_2$-$C_3$ alkylcarbonyl; $C_2$-$C_4$ alkenyl; $C_2$-$C_4$ alkynyl; $C_2$-$C_3$ alkoxycarbonyl; or phenyl, phenylmethyl, 1-naphthalenyl, 2-naphthalenyl, thienyl, furanyl or pyridyl each optionally substituted with $R^8$, $R^9$ and $R^{10}$;

$R^3$, $R^4$ and $R^{18}$ are independently halogen, $C_1$-$C_4$ alkyl, cyclopropyl, $C_1$-$C_4$ haloalkyl, allyl, $C_2$-$C_3$ alkynyl, $C_1$-$C_4$ alkoxy or $C_1$-$C_4$ haloalkoxy;

$R^{23}$ is H, C(=O)$NHR^{26}$, or $C_2$-$C_4$ alkoxycarbonyl;

and metal complexes thereof.

2. Compounds of Preferred 1 wherein:

A is 2-pyrimidinyl optionally substituted with $R^3$, $R^4$ and $R^{18}$;

n is 1 or 2;

E is phenyl, indanyl, tetrahydronaphthalenyl, 1-naphthalenyl, thienyl, or pyridyl each optionally substituted with $R^5$, $R^6$ and $R^7$;

$R^1$ is H; hydroxy, $C_1$-$C_4$ alkoxy, or $C_1$-$C_4$ alkyl;

$R^5$ is halogen; cyano; $C_1$-$C_4$ alkyl; $C_1$-$C_4$ haloalkyl; allyl; propargyl; $C_1$-$C_4$ alkoxy; $C_1$-$C_4$ haloalkoxy; or phenyl or phenoxy each optionally substituted with $R^{17}$; and

$R^6$, $R^7$, $R^9$, $R^{10}$ and $R^{17}$ are independently H, F, Cl, methyl, trifluoromethyl, methoxy or trifluoromethoxy;

and metal complexes thereof.

3. Compounds of Preferred 2 wherein

E is phenyl, indanyl or tetrahydronaphthalenyl each optionally substituted with $R^5$, $R^6$ and $R^7$; and

$R^2$ is H or $C_1$-$C_4$ alkyl.

and metal complexes thereof

Specifically preferred for greatest fungicidal activity and/or ease of synthesis are:

1-(4,6-dimethyl-2-pyrimidinyl)-3-(3,4-dimethylphenyl)-1,4,5,6-tetrahydropyridazine;

1-(4,6-dimethyl-2-pyrimidinyl)-3-(4-ethylphenyl)-1,4,5,6-tetrahydropyridazine;

1-(4,6-dimethyl-2-pyrimidinyl)-1,4,5,6-tetrahydro-3-(4-methylphenyl)pyridazine;

1-(4,6-dimethyl-2-pyrimidinyl)-1,4,5,6-tetrahydro-3-(4-(1-methylethyl)phenyl)pyridazine;

1-(4,6-dimethyl-2-pyrimidinyl)-4-ethyl-1,4,5,6-tetrahydro-3-phenylpyridazine;

1-(4,6-dimethyl-2-pyrimidinyl)-1,4,5,6-tetrahydro-4-methyl-3-phenylpyridazine.

This invention further comprises a method for controlling fungus disease in plants comprising applying to the locus to be protected an effective amount of a compound of Formulae I, II, III, IV, V or VI wherein:

A and G are 2-pyrimidinyl, 2-pyridyl, 2-quinolinyl, 2-quinazolinyl, 1-isoquinolinyl or 3 isoquinolinyl each

optionally substituted with $R^3$, $R^4$ and $R^{18}$; or s-triazinyl optionally substituted with $R^3$ and $R^4$; provided that $R^3$, $R^4$ and $R^{18}$ only substitute carbon atoms of the heterocycles;

E is H; halogen; $C_1$-$C_6$ alkyl; $C_3$-$C_7$ cycloalkyl optionally substituted with 1-2 methyl; $C_1$-$C_6$ haloalkyl; $C_1$-$C_6$ alkylthio; $C_1$-$C_6$ alkoxy; $C_1$-$C_6$ haloalkoxy; or phenyl, phenoxy, phenylthio, phenylamino, phenylmethyl, indanyl, tetrahydronaphthalenyl, 1-naphthalenyl, 2-naphthalenyl, thienyl, furanyl or pyridyl each optionally substituted with $R^5$, $R^6$ and $R^7$;

n is 1, 2 or 3;

$R^1$ is H; halogen; cyano; hydroxy, $C_1$-$C_4$ alkoxy, -OC(=O)$R^{19}$, -OC(=O)NH$R^{20}$ $C_1$-$C_4$ alkyl; $C_1$-$C_4$ haloalkyl; $C_2$-$C_3$ alkylcarbonyl; $C_2$-$C_4$ alkenyl; $C_2$-$C_6$ alkoxyalkyl; $C_2$-$C_4$ alkynyl; $C_2$-$C_3$ alkoxycarbonyl; or phenyl, phenylmethyl, 1-naphthalenyl, 2-naphthalenyl, thienyl, furanyl or pyridyl each optionally substituted with $R^8$, $R^9$ and $R^{10}$;

$R^2$ is H, cyano, $C_1$-$C_4$ alkyl or $C_1$-$C_4$ haloalkyl;

$R^3$, $R^4$ and $R^{18}$ are independently halogen; cyano; hydroxy; ($C_1$-$C_4$ alkyl)$_3$silylmethyl; phenyl optionally substituted with $R^{21}$; $C_1$-$C_6$ alkyl; cyclopropyl; $C_1$-$C_6$ haloalkyl; $C_1$-$C_6$ alkylthio; $C_2$-$C_4$ alkenyl; $C_2$-$C_4$ alkynyl; $C_1$-$C_4$ alkoxy; $C_1$-$C_4$ haloalkoxy; $C_2$-$C_4$ alkenyloxy; $C_2$-$C_4$ alkynyloxy; $C_2$-$C_4$ alkoxyalkyl; $NR^{11}R^{12}$; or when $R^3$ and $R^4$, $R^3$ and $R^{18}$ or $R^4$ and $R^{18}$ substitute adjacent carbon atoms, then $R^3$ and $R^4$, $R^3$ and $R^{18}$ or $R^4$ and $R^{18}$ may together be $-(CH_2)_3-$ or $-(CH_2)_4-$ each optionally substituted with 1-2 methyl;

$R^5$ and $R^8$ are independently halogen; cyano; nitro; hydroxy, hydroxycarbonyl; $C_1$-$C_6$ alkyl; $C_3$-$C_6$ cycloalkyl, $C_1$-$C_6$ haloalkyl; $C_1$-$C_4$ alkylthio; $C_1$-$C_4$ alkylsulfinyl; $C_1$-$C_4$ alkylsulfonyl; ($C_1$-$C_4$ alkyl)$_3$silyl; $C_2$-$C_5$ alkylcarbonyl; $C_2$-$C_4$ alkenyl; $C_2$-$C_4$ alkenyloxy; $C_2$-$C_4$ alkynyl; $C_2$-$C_4$ alkynyloxy; $C_1$-$C_4$ alkoxy; $C_1$-$C_4$ haloalkoxy; $C_2$-$C_4$ alkoxyalkyl; $C_2$-$C_5$ alkoxycarbonyl; $C_2$-$C_4$ alkoxyalkoxy; $NR^{13}R^{14}$; C(=O)$NR^{15}R^{16}$; or phenyl, phenoxy or phenylthio each optionally substituted with $R^{17}$;

$R^6$, $R^7$, $R^9$, $R^{10}$, $R^{17}$, $R^{21}$, $R^{22}$, and $R^{24}$ are independently halogen, $C_1$-$C_4$ alkyl, $C_1$-$C_4$ haloalkyl, $C_1$-$C_4$ alkoxy or $C_1$-$C_4$ haloalkoxy;

$R^{11}$, $R^{12}$, $R^{13}$, $R^{14}$, $R^{15}$, $R^{16}$ are independently H; $C_1$-$C_2$ alkyl; or $R^{11}$ and $R^{12}$, $R^{13}$ and $R^{14}$ or $R^{15}$ and $R^{16}$ can be taken together with the nitrogen to which they attached to form a morpholino, pyrrolidino or piperidino group;

$R^{19}$ and $R^{25}$ are H or $C_1$-$C_3$ alkyl;

$R^{20}$ and $R^{26}$ are $C_1$-$C_4$ alkyl; or phenyl optionally substituted with $R^{22}$; and

$R^{23}$ is H, $C_1$-$C_4$ alkyl, $C_1$-$C_4$ haloalkyl, $C_2$-$C_5$ alkylcarbonyl, phenylcarbonyl optionally substituted with $R^{24}$, $C_3$-$C_4$ alkenyl, $C_3$-$C_4$ alkynyl, phenylmethyl optionally substituted with $R^{24}$ on the phenyl ring. $C_1$-$C_4$ alkylsulfinyl, $C_1$-$C_4$ alkylsulfonyl, phenylsulfinyl, $C_1$-$C_4$ alkylsulfonyl, phenylsulfinyl optionally substituted with $R^{24}$, phenylsulfonyl optionally substituted with $R^{24}$, $C_2$-$C_4$ alkoxycarbonyl, phenoxycarbonyl optionally substituted with $R^{24}$, C(=O)$NR^{25}R^{26}$, C(=S)NH$R^{26}$ P(=S)(O$R^{26}$)$_2$, P(=O)(O$R^{26}$)$_2$, or S(=O)$_2$$NR^{25}R^{26}$;

or their agriculturally suitable salts or metal complexes thereof;

provided that

i) when E is halogen, $C_1$-$C_6$ alkylthio, $C_1$-$C_6$ alkoxy, $C_1$-$C_6$ haloalkoxy, phenoxy, phenylthio or phenylamino, then E may only substitute compounds of Formula I and III;

ii) for compounds of Formula III, either E is phenyl, phenoxy, phenylthio, phenylamino, phenylmethyl, 1-naph-thalenyl, 2-naphthalenyl, thienyl, furanyl, pyridyl each optionally substituted with $R^5$, $R^6$ and $R^7$; or $R^1$ is phenyl, benzyl, 1-naphthalenyl, 2-naphthalenyl, thienyl, furanyl or pyridyl each optionally substituted with $R^8$, $R^9$ and $R^{10}$; and $R^1$ must be in the 4-position; and

iii) for compounds of Formula I, when E is H, n is 1, $R^1$ is 5-methyl, and $R^2$ is H, then A is not s-triazinyl optionally substituted with $R^3$ and $R^4$.

## PREFERRED METHODS

Preferred for reasons of greatest fungicidal activity and/or ease of synthesis are

1. Methods employing compounds of Formula I and V and metal complexes thereof wherein:

A and G are 2-pyrimidinyl or 2-quinazolinyl optionally substituted with $R^3$, $R^4$ and $R^{18}$; and

$R^1$ is H; hydroxy, $C_1$-$C_4$ alkoxy, $C_1$-$C_4$ alkyl; $C_1$-$C_4$ haloalkyl; $C_2$-$C_3$ alkylcarbonyl; $C_2$-$C_4$ alkenyl; $C_2$-$C_4$ alkynyl; $C_2$-$C_3$ alkoxycarbonyl; or phenyl, phenylmethyl, 1-naphthalenyl, 2-naphthalenyl, thienyl, furanyl or pyridyl each optionally substituted with $R^8$, $R^9$ and $R^{10}$;

$R^3$, $R^4$ and $R^{18}$ are independently halogen,$C_1$-$C_4$ alkyl, cyclopropyl, $C_1$-$C_4$ haloalkyl, allyl, $C_2$-$C_3$ alkynyl, $C_1$-$C_4$ alkoxy or $C_1$-$C_4$ haloalkoxy; and

$R^{23}$ is H, C(=O)NH$R^{26}$, or $C_2$-$C_4$ alkoxycarbonyl.

2. A method according to Preferred 1 wherein:

A is 2-pyrimidinyl optionally substituted with $R^3$, $R^4$ and $R^{18}$;

n is 1 or 2;

E is phenyl, indanyl, tetrahydronaphthalenyl, 1-naphthalenyl, thienyl, or pyridyl each optionally substituted with $R^5$, $R^6$ and $R^7$;

$R^1$ is H; hydroxy, $C_1$-$C_4$ alkoxy, or $C_1$-$C_4$ alkyl;

$R^5$ is halogen; cyano; $C_1$-$C_4$ alkyl; $C_1$-$C_4$ haloalkyl; allyl; propargyl; $C_1$-$C_4$ alkoxy; $C_1$-$C_4$ haloalkoxy; or phenyl or phenoxy each optionally substituted with $R^{17}$; and

$R^6$, $R^7$, $R^9$, $R^{10}$ and $R^{17}$ are independently H, F, Cl, methyl, trifluoromethyl, methoxy or trifluoromethoxy.

3. A method according to Preferred 2 wherein

E is phenyl, indanyl or tetrahydronaphthalenyl each optionally substituted with $R^5$, $R^6$ and $R^7$; and

$R^2$ is H or $C_1$-$C_4$ alkyl.

Specifically preferred for greatest fungicidal activity and/or ease of synthesis are methods employing:

1-(4,6-dimethyl-2-pyrimidinyl)-3-(3,4-dimethylphenyl)-1,4,5,6-tetrahydropyridazine;
1-(4,6-dimethyl-2-pyrimidinyl)-3-(4-ethylphenyl)-1,4,5,6-tetrahydropyridazine;
1-(4,6-dimethyl-2-pyrimidinyl)-1,4,5,6-tetrahydro-3-(4-methylphenyl)pyridazine;
1-(4,6-dimethyl-2-pyrimidinyl)-1,4,5,6-tetrahydro-3-(4-(1-methylethyl)phenyl)pyridazine;
1-(4,6-dimethyl-2-pyrimidinyl)-4-ethyl-1,4,5,6-tetrahydro-3-phenylpyridazine;
1-(4,6-dimethyl-2-pyrimidinyl)-1,4,5,6-tetrahydro-4-methyl-3-phenylpyridazine.

## DETAILED DESCRIPTION OF THE INVENTION

### Synthesis

Compounds of Formula I where E is as described previously with the exception of halogen, phenoxy, phenylthio, phenylamino, $C_1$-$C_6$ alkoxy, $C_1$-$C_6$ alkylthio and $C_1$-$C_6$ haloalkoxy, and $R^1$ and $R^2$ are as described previously, can be prepared by one or more of the methods described in Equations 1 to 14.

As shown in Equation 1 below, compounds of Formula Ia can be prepared by deprotonation of compounds of Formula Ib with a strong base such as lithium diisopropyl amide (LDA) followed by addition of $R^2$-L where L is a leaving group such as Cl, Br, I, $OSO_2CH_3$ or $OSO_2C_6H_4CH_3$. The reaction is carried out at about -78° to about 100°C in an inert, aprotic solvent such as tetrahydrofuran (THF) or dimethoxyethane (DME).

Equation 1

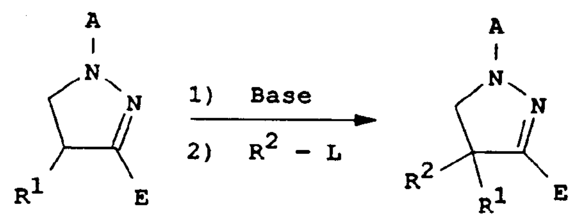


Ib    Ia

Compounds of Formula Ib can be prepared by reacting hydrazine 1 with 2 as shown below in Equation 2. The reaction is carried out at about 50° to about 100°C in a lower alcohol solvent such as ethanol or 2-propanol.

Equation 2

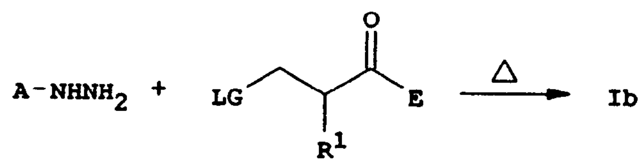


1    2

LG = Cl, $NMe_2$, $NMe_2 \bullet HCl$

A base such as sodium hydroxide is added if necessary. The hydrazines 1 can be prepared by treating a compound of Formula 3 with hydrazine monohydrate as taught in EP293743-A and by Naito et al. (Chem. Pharm. Bull. 1969, 17, 1467-1478). Compounds of Formula 2 are either commercially available or can be prepared by methods described in Carey, F.A.; Sundberg, R.J. Advanced Organic Chemistry; plenum:New York, 1983; Part B, pp. 58-62:

$$A{-}X + NH_2NH_2 \bullet H_2O \longrightarrow \underline{1}$$

3

X = Cl, SH

Compounds of Formulae Ic, Id and Ie can be prepared by reacting 1 with α,β-unsaturated ketones 4, 5 or 6 as shown below in Equations 3, 4 and 5. The reaction is carried out at 50°C to 100°C in a lower alcohol solvent such as ethanol or 2-propanol in the presence of a catalytic amount of an acid, such as hydrochloric acid. Compounds of Formulae 4, 5 and 6 are well known in the literature and can be prepared by methods known to one skilled in the art.

Equation 3

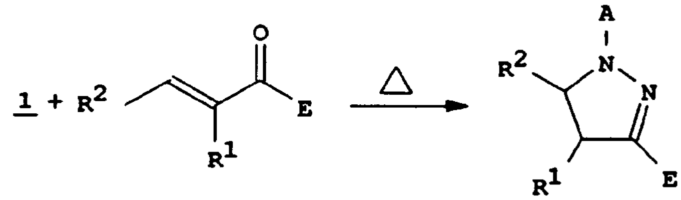

4 Ic

Equation 4

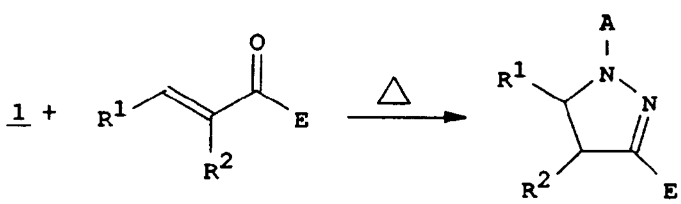

5 Id

Equation 5

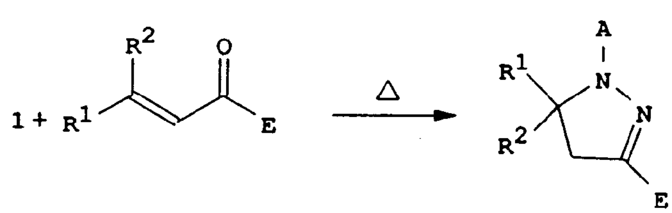

6 Ie

As shown below in Equation 6, compounds of Formula If can be prepared from compounds of Formula Ig according to the procedure described previously for Formula Ib.

Equation 6

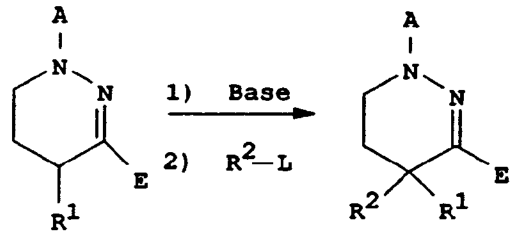


Ig .If

Compounds of Formula Ig can be prepared by reacting 1 and 7 as shown below in Equation 7. The reaction is carried out at 25° to 100°C in an organic solvent such as ethanol, 2-propanol, acetonitrile or *N,N*-dimethylformamide in the presence of a catalytic amount of an acid such as toluenesulfonic acid and a drying agent such as molecular sieves (3Å). This reaction can also be carried out in two steps. The first step involves the formation of the hydrazone from ketone 7 and hydrazine 1 in an organic solvent such as acetic acid or acetonitrile. The hydrazone product is isolated and dissolved in an inert solvent such as THF. Treatment with sodium hydride provides Ig. If acetonitrile is used as the solvent, potassium carbonate can be used as the base instead of sodium hydride.

Equation 7

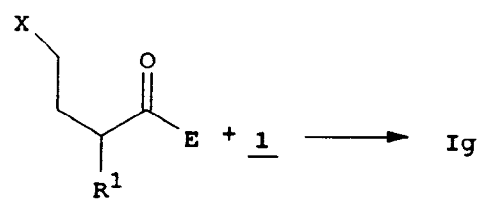


7

X = Cl, Br

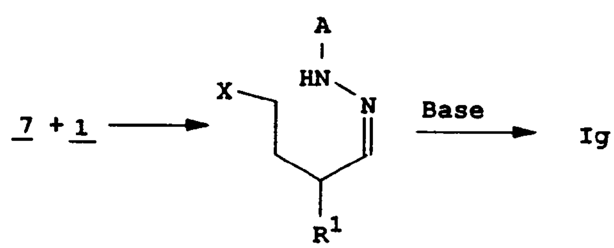


Compounds of Formula 7 can be prepared from keto esters 8 and ethylene oxide using the general method described by Cannon et al. (Org. Syn. , Coll. Vol. IV, 1963, 597-600).

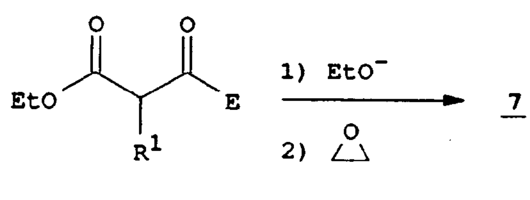


8

Compounds of Formula 7a, wherein E is an aromatic group optionally substituted with $R^5$, $R^6$ and $R^7$, and

$R^1$ is H, alkyl, halogen, or haloalkyl, can be prepared by Friedel-Crafts acylation of the parent compound E-H with an $R^1$-substituted 4-chlorobutyryl chloride according to the procedure set out in the literature (for example, see Close; J. Am. Chem. Soc., **1957**, *79*, 1455) and illustrated below.

The corresponding chlorobutyryl chloride can be prepared by reacting γ-butyrolactone with thionyl chloride in the presence of zinc chloride according to the procedure taught by Goel et al. (Synthesis, 1973, 538; see Equation below).

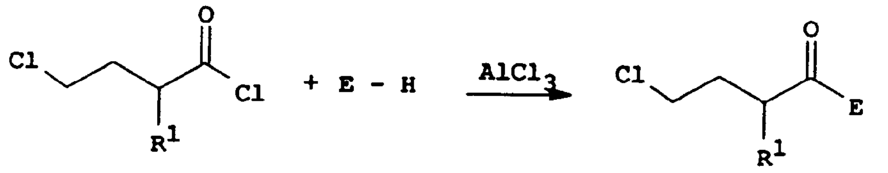


7a

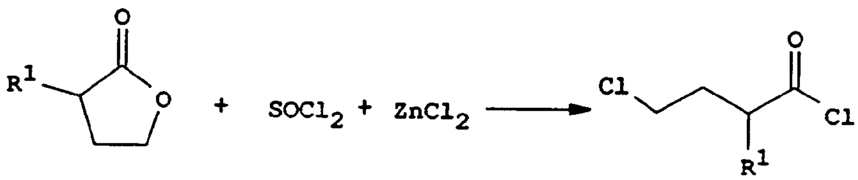


Compounds of Formula 7a can also be prepared by condensing γ-butyrolactone with an ester followed by alkylation with $R^1X$ and treatment of the alkylated product with hydrochloric acid.

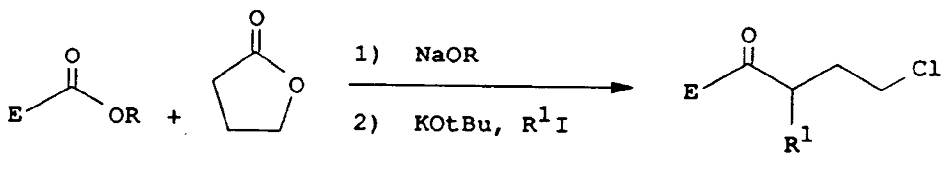


7a

Similarly, compounds of Formulae Ih, Ii, Ij, and Ik can be prepared by the same method from the corresponding keto esters and oxiranes as shown below in Equations 8, 9 and 10. The stereoisomers obtained in the reactions can be separated by chromatography.

Equation 8

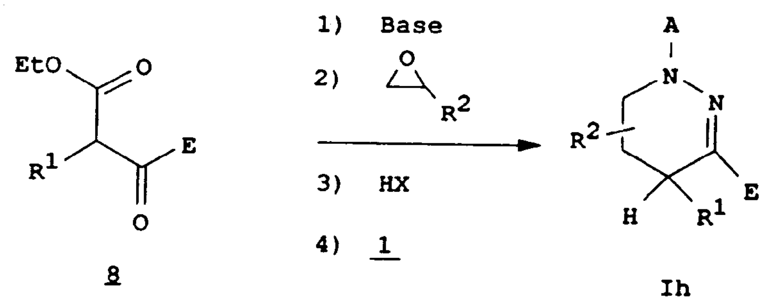


8

Ih

Equation 9

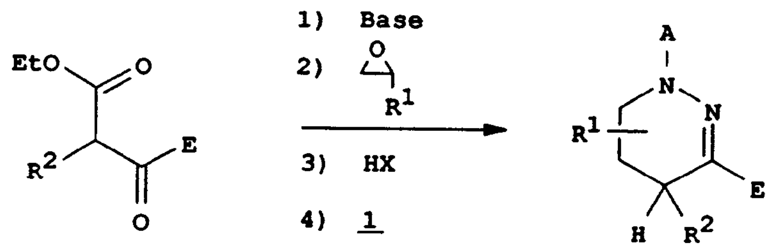


Ii

Equation 10

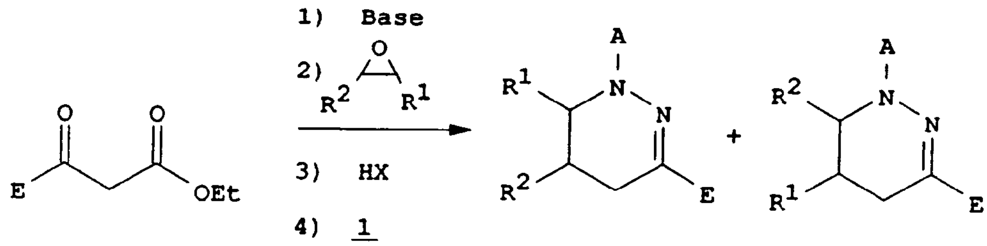


Ij

Ik

Methods to prepare β-keto esters and oxiranes are well known in the literature and can be prepared by methods known to one skilled in the art. For example, keto esters 8 can be prepared by treating ketones of Formula 9 with a base such as sodium hydride in an aprotic solvent such as DMF followed by addition of diethyl carbonate.

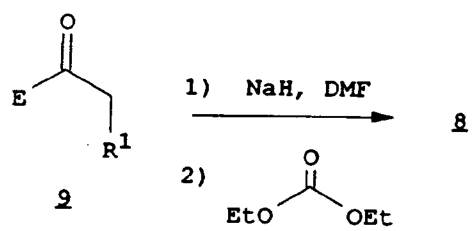


Compounds of Formula Ij and Ik can also be prepared using malonate as the starting material as shown below in Equation 11. The intermediate lactones 10 and 11 are condensed with an ester ECOOR, decarboxylated and cyclized with hydrazine 1 to form Ij and Ik.

Equation 11

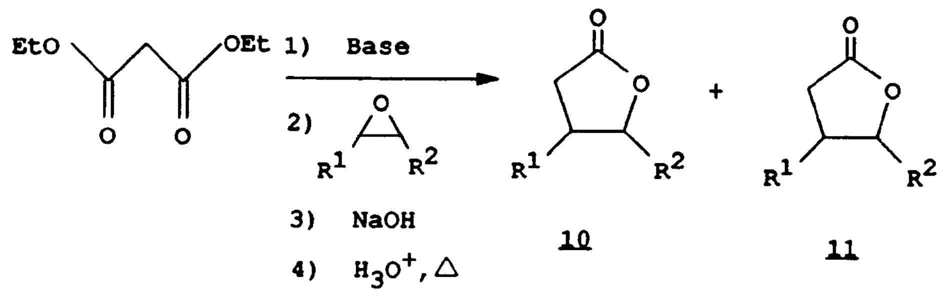


10 + 11

1) NaOMe
2) ECOOR
3) HX
4) 1

→ Ij + Ik

As shown below in Equation 12, compounds of Formula In can be prepared by standard alkylation of compounds of Formula Io with $R^2$-L as described previously.

Equation 12

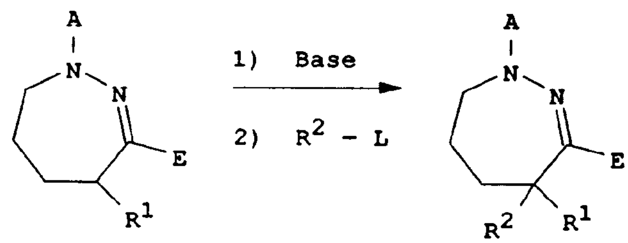


Io    In

Compounds of Formula Io are prepared from 1 and bromoketone 12 as shown below in Equation 13 according to the method described for the preparation of compounds of Formula Ib. Methods to prepare compounds of Formula 12 are well known to one skilled in the art.

Equation 13

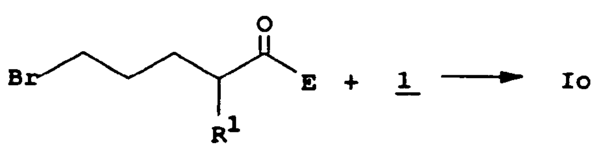


12

Those skilled in the art will recognize that compounds of Formula Ip can be prepared from appropriately substituted bromoketones by the same method described above.

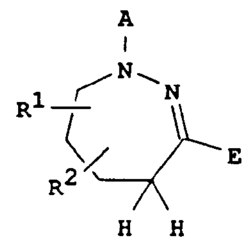

Ip

Compounds of Formula II can be prepared by one or more of the methods shown below in Equations 14, 15, and 16.

As shown in Equation 14, compounds of Formula IIa, a subset of Formula II, can be prepared by reacting hydrazine 13 and α,β-unsaturated ketone 14. The reaction is carried out at 50° to 100°C in a lower alcohol solvent such as ethanol or 2-propanol in the presence of an acid catalyst such as hydrochloric acid.

Equation 14

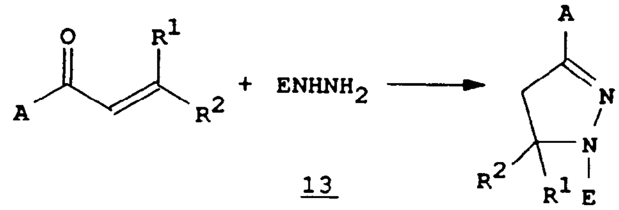

13

14

IIa

As shown in Equation 15, compounds of Formula IIb, where $R^1$ and $R^2$ are substituted at different carbons, can be prepared by reacting compounds of Formula 13 with ketone 15a or 15b according to the method described for Formula IIa.

Equation 15

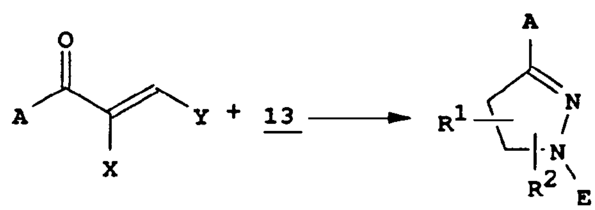

15a, X=$R^1$, Y=$R^2$

15b, X=$R^2$, Y=$R^1$

IIb

As shown in Equation 16, compounds of Formula IIc can be prepared from compounds of Formula 13 and ketone 16 according to the procedure described for Formula IIa. Deprotonation of Formula IIc with a base such as LDA followed by alkylation with $R^2$-L provides compounds of Formula IId.

Equation 16

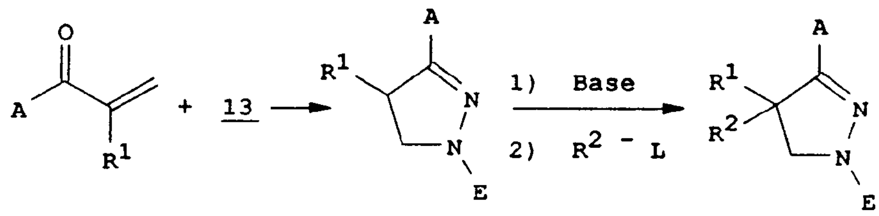


16 IIc IId

Methods to prepare ketones 14, 15a, 15b and 16 from ketone 17 and carbonyl compounds of Formula 18 are well known to one skilled in the art.

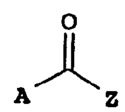

17

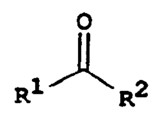

18

Z = H, $R^1$, or $R^2$

Methods to prepare heteroaryl carbonyl compound 17 and carbonyl compound 18 are well known to one skilled in the art.

Compounds of Formula II where n=2 (IIe) and n=3 (IIf) are prepared by a variety of methods described for compounds of Formulae If to Ip.

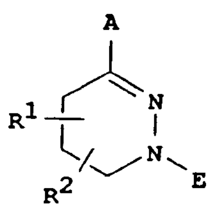

IIe

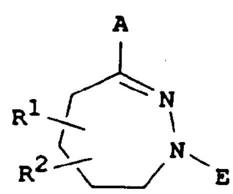

IIf

The appropriate starting ketones, epoxides, bromoketones and alkenes can be prepared by one skilled in the art.

Pyrazoles of Formula III can be prepared as shown below in Equation 17 from a pyrazole salt 19a such as the sodium salt, with a heterocycle 20 containing an activated leaving group such as a halogen in an organic solvent such as THF. This method allows the preparation of pyrazoles III with large substituents E in the 3-position.

The salt 19a is prepared from the pyrazole 19b and an organometallic such as sodium hydride.

Equation 17

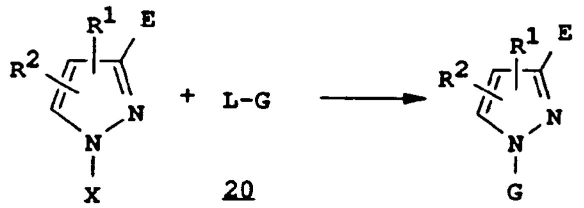


20

19

III

| | | | |
|---|---|---|---|
| 19a | X=Li, Na or K | L=Leaving group; such as Cl, Br, I, $MeSO_2$, and the like. |
| 19b | X=H | |

Pyrazoles of the Formula IIIa also may be prepared from dicarbonyl compounds. As set forth below in Equation 18, keto aldehydes such as 21 can be condensed with a heterocyclic hydrazine 1a in an alcoholic solvent such as ethanol with an acid to provide pyrazoles as a mixture of 3,4- and 4,5-isomers which can be separated by chromatography.

Equation 18

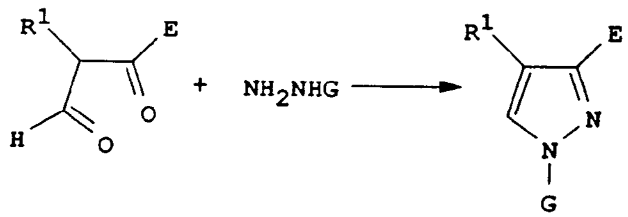


21 1a IIIa

The reaction of diketones 22 as set forth in Equation 19 below, under the same conditions, gives pyrazoles 23 as a mixture of isomers which can be separated by chromatography.

Equation 19

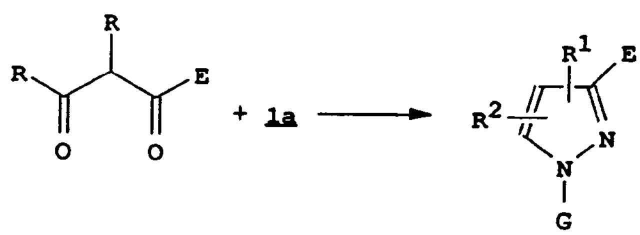


22

R=$R^1$ or $R^2$

23

Pyrazoles 24 also may be prepared by heating a mixture of keto aldehydes such as 21 and hydrazine in an alcoholic solvent such as ethanol with a trace of an acid catalyst such as hydrochloric acid as shown below in Equation 20.

Equation 20

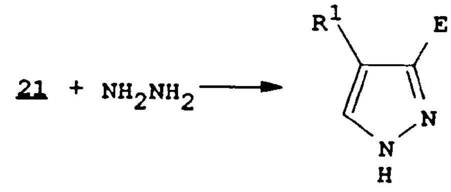


24

The reaction of diketones 22 with hydrazine under the same conditions, as shown in Equation 21 below, gives pyrazoles 25 as a mixture of isomers.

Equation 21

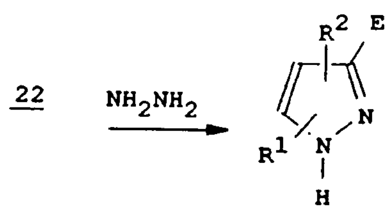


25

Several other methods to prepare pyrazoles are described in the literature (Kost, A.N.; Grandberg, I.I., Advan. Heterocycl. Chem. 1966, 6, 347-429).

When A=G, compounds of Formula III can also prepared by oxidation of compounds of Formulae Ic and Id with nickel peroxide ($NiO_2$) or manganese dioxide ($MnO_2$) as shown below in Equation 22 according to the procedure taught by Evans et al. (J. Org. Chem. 1979, 44, 497-501).

Equation 22

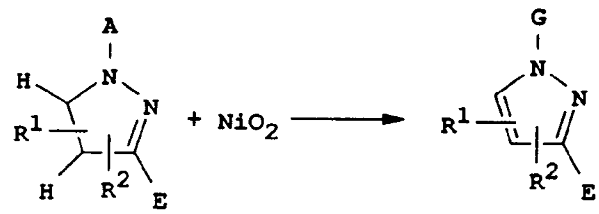


Ic and Id

III

When A=G compounds of Formula IV, as shown below in Equation 23 are similarly prepared by oxidation of IIb with nickel peroxide.

Equation 23

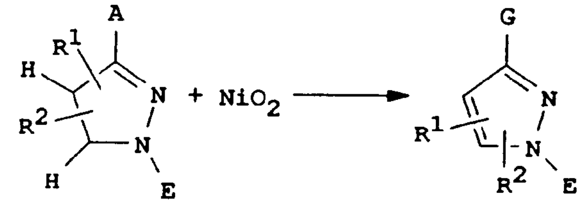


IIb IV

Compounds of Formula Va, a subset of V wherein $R^{23}$ is H, can be prepared by reduction of compounds of Formula I with sodium borohydride/titanium (IV) chloride according to the procedure taught by Kano et. al. (Synthesis, 1980, 695) as set forth in Equation 24. One skilled in the art will recognize that some substituents in Compounds of Formula I are not compatible with the reduction conditions and therefore protection and deprotection techniques are necessary in these cases.

Equation 24

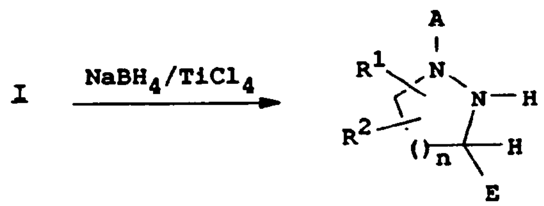


Va

Compounds of Formula Vb wherein $R^{27}$ is $C_1$-$C_4$ alkyl, $C_1$-$C_4$ haloalkyl, optionally substituted phenylmethyl, $C_3$-$C_4$ alkenyl, or $C_3$-$C_4$ alkynyl, can be prepared by treating compounds of Formula Va with the appropriate alkylating agent of Formula 26 as set forth in Equation 25 below.

Equation 25

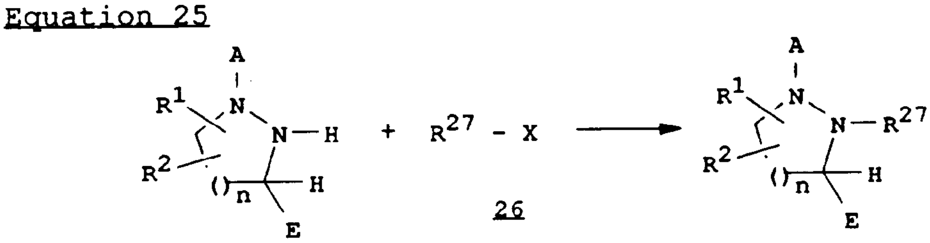


Va Vb

The leaving group X in the compound of Formula 26 may be a halogen, acetate or another moiety used by those skilled in the art for alkylating. Iodine and bromine are commonly used leaving groups X.

The compounds of Formula Va are dissolved in an inert solvent such as methylene chloride, tetrahydrofuran (THF) or benzene and treated with the compound of Formula 26 and a base at a temperature ranging from 0° to 100°C. Triethylamine, *N,N*-diisopropylethylamine, and other tertiary-amine bases are preferred.

The product of Formula Vb can be isolated by evaporation of the solvent, dissolving the residue in a water immiscible solvent such as ether. This solution may be washed with dilute aqueous mineral acid, water, and brine, and dried. Evaporation of the solvent followed by crystallization or chromatography affords the purified product.

Compounds of Formula Vc where $R^{28}$ is $C_1$-$C_4$ alkylsulfinyl, optionally-substituted phenylsulfinyl, $C_1$-$C_4$ alkylsulfonyl, optionally substituted phenylsulfonyl, $C_1$-$C_4$ alkylcarbonyl, optionally substituted phenyl carbonyl, $C(=O)NR^{25}R^{26}$, $P(=S)(OR^{26})_2$, $P(=O)(OR^{26})_2$, or $S(O)_2NR^{25}R^{26}$ can be prepared by treating compounds of Formula Va with the appropriate acylating, sulfinylating, sulfonylating, or phosphonating agent of Formula 27 as set forth in Equation 26.

Equation 26

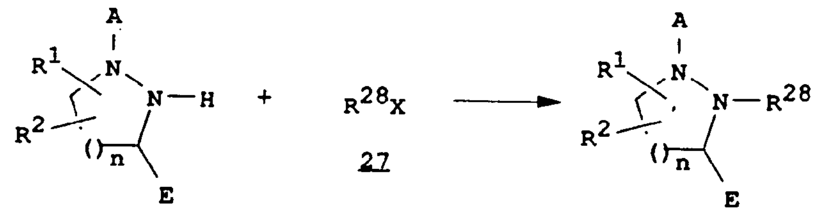


Va Vc

In Equation 26, the leaving group X in the compound of Formula 27 may be a halogen, acetate or another moiety used by those skilled in the art for acylating, sulfinylating, sulfonylating or phosphonating. Chlorine is the most commonly used leaving group X. In those cases, the compounds of Formula 27 can be an acid chloride, chloroformate, sulfinyl chloride, sulfonyl chloride, chlorophosphate or carbamoyl chloride.

The compound of Formula Va is dissolved in an inert solvent such as methylene chloride, tetrahydrofuran (THF), or benzene arid treated with the compound of Formula 27 and a base at a temperature ranging from 0°C to 100°C. Triethylamine, *N,N*-diisopropylethylamine, and other tertiary-amine bases are preferred.

The product of Formula Vc can be isolated by evaporation of the solvent and dissolving the residue in a water immiscible solvent such as ether. This solution may be washed with a dilute aqueous mineral acid, water, and brine, and dried. Evaporation of the solvent followed by crystallization or chromatography affords the purified product.

In cases where $R^{23}$ is $C(=O)NR^{25}R^{26}$ and $R^{25}$ is H, or $C(=S)NHR^{26}$. The compounds of Formula Vd can be prepared by treating the compound of Formula Va with an isocyanate or an isothiocyanate as set forth in Equation 27 below.

Equation 27

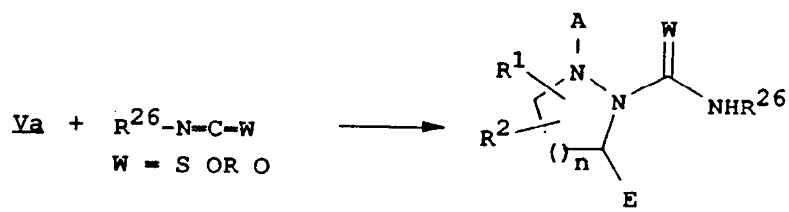


Vd

The compound of Formula Va is dissolved in an inert solvent such as toluene, THF, acetonitrile, or 1,2-dichloroethane and treated with the isocyanate or isothiocyanate, at a temperature ranging from 0° to 50°C. The product of Formula Vd can be isolated by evaporation of the solvent followed by crystallization or chromatography.

Compounds of Formula VI can be similarly prepared from compounds of Formula II according to the procedures described for the preparation of the compounds of Formula V.

The metal complexes of the compounds I-VI of the invention include complexes with copper, zinc, iron, magnesium or manganese cations. These complexes can be made by combining the compound with the metal salt, either in aprotic solvents such as ether or tetrahydrofuran or they can be generated in protic solvents such as methanol. The complex may crystallize and precipitate from solution or the complex is crystallized as the solvent is removed.

Those skilled in the art will recognize that Formulae I, II, V and VI can contain two or more asymmetric carbon atoms. The stereoisomers that result can be separated using standard methods known in the art if desired.

Without further elaboration, it is believed that one skilled in the art can, using the preceding description, utilize the present invention to its fullest extent. The following preferred specific embodiments are, therefore, to be construed as merely illustrative, and not limiting of the disclosure in any way whatsoever. In the following examples, all temperatures are set forth in degrees Celsius; unless otherwise indicated, all parts and percentages are by weight.

Compounds of Formula I wherein E is $C_1$-$C_6$ alkylthio, $C_1$-$C_6$ alkoxy, phenylthio, phenoxy of phenylamino (Iq), as shown below in Equation 28, are prepared by the displacement of the methylthio group in compounds

of Formula 28 by various nucleophiles in the presence of a base. Suitable nucleophiles can be optionally substituted phenols, thiophenols, or anilines, $C_1$-$C_6$ alkylthiols, $C_1$-$C_6$ alcohols and $C_1$-$C_6$ halo-substituted alcohols.

Equation 28

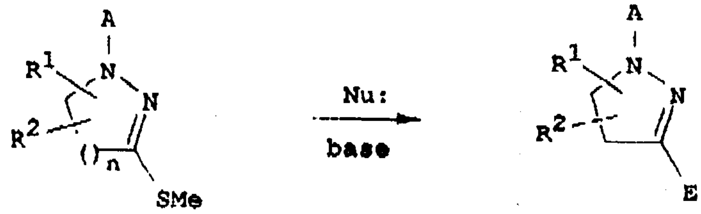


28 Iq

Nu:= optionally substituted phenol; thiophenol, or aniline; $C_1$-$C_6$ alcohol, $C_1$-$C_6$ alkylthiol, $C_1$-$C_6$ halo-substituted alcohol.

n= 1-3

Compounds of Formula 28 can be prepared by treating hydrazides of Formula 29 with $P_2S_5$ in pyridine at reflux followed by alkylating the resulting thio derivative with iodomethane in the presence of a base such as triethylamine as shown in Equation 29.

Equation 29

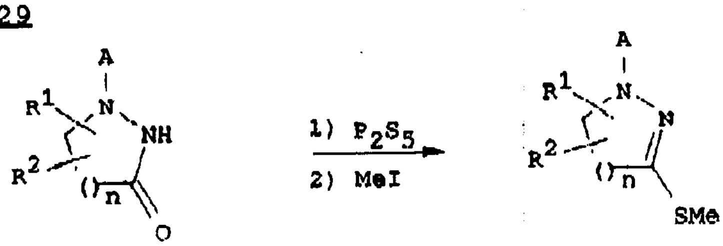


29 28

Compounds of Formula 29 can be prepared by treating compounds of Formula 3a, with compounds of Formula of 30 in the presence of a base such as triethylamine. (Equation 30)

Equation 30

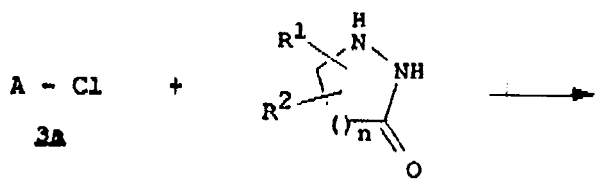

3a 30 29

Compounds of Formula 30 can be prepared from the reaction of acid chloride 31. (Equation 31)

Equation 31

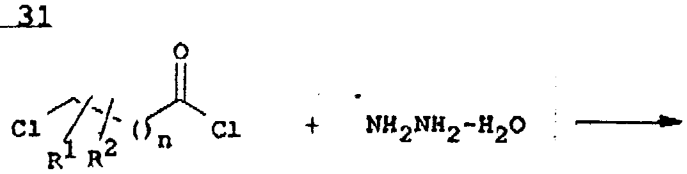

n=1, 2, 3 30

Compounds of Formula I wherein E is chlorine and bromine (Ir) can be prepared from halogenation of compounds of Formula 29 with halogenating reagents such as phosphorus bromide or phosphorus chloride according to the standard procedures set out in the literature.

Equation 32

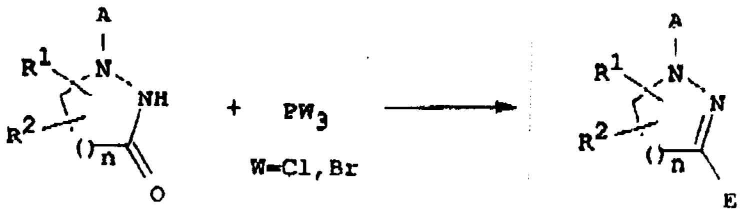


29

Ir

E=Cl,Br

EXAMPLE 1

Synthesis of 2-[3-(2-chlorophenyl)-4,5-dihydro-1H-pyrazol-1-yl]-4,6-dimethylpyrimidine

Paraformaldehyde (7.20 g, 240 mmol), 1-(2-chlorophenyl)ethanone (23.2 g, 150 mmol), dimethylamine hydrochloride (14.7 g, 180 mmol), and hydrochloric acid (12M, 7.2 mL) are combined in 180 mL of ethanol. The suspension which becomes a solution upon heating is heated at reflux for 4 days and then cooled in an ice bath. The solution is evaporated in a rotary evaporator under reduced pressure. As soon as precipitate appears in the flask, the evaporation is stopped. The suspension is cooled in an ice bath and filtered to give 13.6 g of 1-(2-chlorophenyl)-3-(dimethylamino)-1-propanone hydrochloride as a white solid: mp 168-170°C. $^1$H NMR (DMSO-$d_6$) δ 2.75 (s, 6H), 3.40 (t, 2H), 3.57 (t, 2H), 7.50 (m, 3H), 7.81 (d, 1H), 11.10 (bs, 1H).

To a suspension of the preceding compound (1.76 g, 7.09 mmol) and 4,6-dimethyl-2-hydrazinylpyrimidine (0.98 g, 7.09 mmol) in 2-propanol (40 mL) is added 50% sodium hydroxide solution (1.2 mL). The suspension is heated at reflux for 7 h and stirred at room temperature overnight. The solvent is removed and the residue is partitioned between 50 mL of water and 60 mL of chloroform. The organic portion is separated and the aqueous portion is extracted with chloroform (60 mL). The two organic portions are combined and dried ($MgSO_4$). Solvent is removed and the residue is purified by flash chromatography to give 0.35 g of the title compound as a solid: mp 116-118°C. $^1$H NMR ($CDCl_3$) δ 2.40 (s, 6H), 3.47 (t, 2H), 4.21 (t, 2H), 6.47 (s, 1H), 7.40 (m, 3H), 7.89 (m, 1H).

EXAMPLE 2

Synthesis of 1-(4,6-dimethyl-2-pyrimidinyl)-1,4,5,6-tetrahydro-3-phenylpyridazine

4-Chloro-1-phenyl-1-butanone (2.00 g, 11.0 mmol), 4,6-dimethyl-2-hydrazinylpyrimidine (1.50 g, 10.9 mmol) and triethylamine (3 mL) are combined in 60 mL of 2-propanol. The solution is heated at reflux overnight. The solvent is removed and the residue is partitioned between 75 mL of 5% sodium bicarbonate solution and 75 mL of ethyl acetate. The organic portion is separated and the aqueous portion is extracted with ethyl acetate (75 mL). The two organic portions are combined, washed with 50 mL brine, dried ($MgSO_4$) and the solvent is removed. The residue is purified by chromatography to give 0.58 g of 1-(4,6-dimethyl-2-pyrimidinyl)-1,4,5,6-tetrahydro-3-phenyl-pyridazine as a solid: mp 95-97°C. $^1$H NMR ($CDCl_3$) δ 2.11 (m, 2H), 2.42 (s, 6H), 2.71 (t, 2H), 4.10 (t, 2H), 6.50 (s, 1H), 7.30 (m, 3H), 7.90 (m, 2H).

EXAMPLE 3

Synthesis of 4-methyl-2-(4-methyl-3-phenyl-1H-pyrazol-1-yl)pyrimidine

Under nitrogen, 0.35 g (8.86 mmol) of sodium hydride is washed with hexane. To this, 40 mL THF is added and the reaction is cooled to 0°C. A solution of 1.00 g (6.33 mmol) of 4-methyl-3-phenyl-1H-pyrazole (Matsukawa, T.; Ohta, B., J. Pharm. Soc. Japn., **1950**, 70, 134) in 10 mL THF is added dropwise. After gas evolution ceases, 0.85 g (6.64 mmol) of 2-chloro-4-methylpyrimidine (Moon, M.W. et al.; J. Agric. Food Chem., **1977**, 25(5), 1039-49) in 10 mL THF is added and the reaction is heated at reflux overnight. Water (150 mL) is added

and the mixture is extracted with ethyl acetate (2X50 mL). The organic portions are washed with water, then brine, and dried ($MgSO_4$) and concentrated to yield 1.6 g of a brown oil.

This oil is purified by chromatography on silica gel to give an oil which solidifies on standing to give 1.02 g of the title compound of this example as a solid. $^1H$ NMR ($CDCl_3$) δ 2.3 (s, 3H), 2.6 (s, 3H), 7.0 (d, 1H), 7.3-7.5 (m, 3H), 7.8 (m, 2H), 8.4 (s, 1H), 8.6 (d, 1H).

EXAMPLE 4

Synthesis of 4,6-dimethyl-2-(5-methyl-4-phenyl-1H-pyrazol-1-yl)-pyrimidine and 4,6-dimethyl-2-(3-methyl-4-phenyl-1H-pyrazol-1-yl)-pyrimidine

To a mixture of 2.0 g (12.3 mmol) of 2-phenyl-3-oxobutanal and 1.7 g (12.3 mmol) of 2-hydrazino-4,6-dimethylpyrimidine (Graf, H. et al., EP293743), and 100 mL methanol, 3 drops of concentrated hydrochloric acid are added. The reaction is heated at reflux for 4 h. The methanol is removed under reduced pressure to leave an oil which crystallizes on standing. This is triturated with hexane to give 2.42 g of pyrazol pyrimidine as a mixture of 68% 4,6-dimethyl-2-(5-methyl-4-phenyl-1H-pyrazol-1-yl)pyrimidine and 32% 4,6-dimethyl-2-(3-methyl-4-phenyl-1H-pyrazol-1-yl)-pyrimidine.

Chromatography of a 1.17 g portion of the pyrazolyl pyrimidines on 120 mL of silica gel eluting with 1:2 ethyl acetate:hexane affords first 0.160 g of 4,6-dimethyl-2-(3-methyl-4-phenyl-1H-pyrazol-1-yl)pyrimidine as a solid with a melting point of 123-124.5°C. $^1H$-NMR ($CDCl_3$) δ 2.56 (s, 9H), 6.92 (s, 1H), 7.3-7.55 (m, 5H), 8.70 (s, 1H).

Also eluting is 0.782 g of a mixture of the two title compounds of this example in a 70:30 ratio, respectively, and finally 0.175 g of 4,6-dimethyl-2-(5-methyl-4-phenyl-1H-pyrazol-1-yl)pyridine as a solid melting at 93.5-94°C. $^1H$-NMR ($CDCl_3$) δ 2.58 (s, 6H), 2.75 (s, 3H), 6.98 (s, 1H), 7.3-7.45 (m, 5H), 7.85 (s, 1H).

EXAMPLE 5

Synthesis of 3-(4-chlorophenyl)-1,4,5,6-tetrahydro-1-[4-methyl-6-trifluoromethyl)-2-pyrimidinyl]pyridazine

4-chloro-1-(4-chlorophenyl)-1-butanone (690 mg, 3.16 mmol), 4-methyl-6-trifluoromethyl-2-hydrazinopyrimidine (500 mg, 2.87 mmol), butanesulfonic acid (5 drops) and 3Å molecular sieves (1 scoop) are combined in 14 mL of anhydrous acetonitrile. The mixture is stirred overnight at room temperature, diluted with dichloromethane and filtered. The filtrate is washed with saturated sodium bicarbonate, dried ($Na_2SO_4$), filtered and concentrated. The residue is passed through a plug of silica gel using 30% of ethyl acetate/hexane. The filtrate is concentrated, dissolved in 14 mL of anhydrous THF. Sodium hydride (130 mg of 60% dispersion, 3.16 mmol) is added and the mixture is stirred for 10 min at 25°C. Saturated ammonium chloride solution and ether are added. The ether layer is separated, washed with saturated sodium chloride solution dried ($Na_2SO_4$), filtered, and concentrated. The residue is purified by chromatography to give 580 mg (60%) of the title compound as a solid:mp 150-152°C. $^1H$ NMR ($CDCl_3$) δ 2.1(m, 2H), 2.6(s, 3H), 2.7(m, 2H), 4.1(m, 2H), 6.9(s, 1H), 7.4(m, 2H), 7.8(m, 2H).

EXAMPLE 6

Synthesis of 3-(3,4-dimethylphenyl)-1-(4,6-dimethyl-2-pyrimidinyl)-1,4,5,6-tetrahydropyridazine

To a stirred solution of 4,6-dimethyl-2-hydrazinopyrimidine (500 mg, 3.62 mmol) in 7.2 mL of acetic acid under nitrogen is added 4-chloro-1-(3,4-dimethyl-phenyl)-1-butanone (763 mg, 3.62 mmol). The solution is stirred at 25°C overnight. Acetic acid is removed. The residue is taken up in dilute sodium bicarbonate solution, extracted with dichloromethane twice, dried ($MgSO_4$) and concentrated to give the intermediate hydrazone as a brown oily solid (1.21 g). A portion of this solid (200 mg, 0.60 mmol) is dissolved in 3 mL of anhydrous THF and stirred under nitrogen. Sodium hydride (29 mg of 60% dispersion, 0.72 mmol) is added in 3 portions. After 25 minutes, 2 drops of water is added. The mixture is diluted with 20 mL of water, extract with dichloromethane (4 x 5 mL), and extracted with 10 mL of ethyl acetate. The organic extracts are combined, dried ($MgSO_4$) and concentrated. The residue is purified by chromatography to give 115 mg (65% yield over 2 steps) of the title compound as a solid: mp 119-120°C. $^1H$ NMR ($CDCl_3$) δ 2.1 (m, 2H), 2.27(s, 3H), 2.30(s, 3H), 2.42(s, 6H), 2.7 (t, 2H), 4.1 (dd, 2H), 6.49(s, 1H), 7.1 (d, 1H), 7.55(dd, 1H), 7.7 (d, 1H).

EXAMPLE 7

Synthesis of 2-((3-(3,4-dimethylphenyl)-5,6-dihydro-1(4H)-pyridazinyl))-4-methylquinazoline

To a solution of 2-hydrazino-4-methylquinazoline (500 mg, 3.34 mmol) in 18 mL of anhydrous acetonitrile under nitrogen is added 4-chloro-1-(3,4-dimethylphenyl)-1-butanone (770 mg, 3.67 mmol), butanesulfonic acid (5 drops), and 3Å molecular sieves (1 scoop). The mixture is stirred at 25°C overnight. An excess amount of potassium carbonate is added and the mixture is stirred over a weekend. Dichloromethane and water are added. The organic layer is separated and washed with saturated sodium chloride solution dried ($Na_2SO_4$) and concentrated. The residue is purified by chromatography to give 670 mg (62%) of the title compound as a yellow solid: mp 159-161°C. $^1$H NMR ($CDCl_3$) δ 2.18(m, 2H), 2.29(s, 3H), 2.33(s, 3H), 2.75(t, 2H), 2.93(s, 3H), 4.2 (m, 2H), 7.15 (d, 1H), 7.3 (m, 1H), 7.6-7.8(m, 4H), 7.9 (d, 1H)

EXAMPLE 8

Synthesis of 2-[3-(4-chlorophenyl)-5,6-dihydro-1(4H)-pyridazinyl]-4-methylquinazoline

To a solution of 2-hydrazino-4-methylquinazoline (300 mg, 2.0 mmol) in 15 mL of anhydrous acetonitrile under nitrogen is added 4-chloro-1-(4-chlorophenyl)-1-butanone (0.48 g, 2.2 mmol) and butanesulfonic acid (3 drops). The reaction mixture is stirred at 25°C overnight. The mixture is filtered and the solid washed with hexane to yield 0.35 g (53%) of the title compound: mp 248-252°C. $^1$H NMR ($CDCl_3$) δ 2.22(m, 2H), 2.9 (t, 2H), 2.99(s, 3H), 4.3 (m, 2H), 7.5 (m, 4H), 7.95(d, 2H), 8.45(d, 2H).

EXAMPLE 9

Synthesis of 3-(3,4-dimethylphenyl)-1-(4,6-dimethyl-2-pyrimidinyl)hexahydropyridazine

A solution of 1-(4,6-dimethyl-2-pyrimidinyl)-3-(3,4-dimethylphenyl)-1,4,5,6-tetrahydropyridazine (0.30 g, 1.02 mmol) in anhydrous 1,2-dimethoxyethane (5 mL) is added dropwise to a mixture of titanium (IV) chloride (1,5 mmol, 1.5 mL) and sodium borohydride (3.06 mmol, 0.12 g) at 0°C in 10 mL of 1,2-dimethoxyethane. The reaction mixture is allowed to warm to room temperature and is stirred for 16 h. The reaction is then quenched with water, basified with saturated aqueous sodium bicarbonate and extracted three times with dichloromethane. The combined organic extracts are washed with brine, dried over sodium sulfate and concentrated. Flash chromatography on silica gel affords 210 mg of the desired product as an oil. $^1$H NMR ($CDCl_3$) δ 7.25 (s, 1H), 7.17 (m, 2H), 6.4 (bs, 1H), 6.22 (s, 1H), 4.8 (m, 1H), 3.7 (m, 1H), 3.2 (m, 1H), 2.28 (s, 3H), 2.27 (s, 9H), 1.9 (m, 2H), 1.8 (m, 1H), 1.7 (m, 1H).

EXAMPLE 10

Synthesis of 3-(4-chlorophenyl)-1-(4,6-dimethyl-2-pyrimidinyl)-1,4,5,6-tetrahydropyridazine, complex with zinc chloride

A solution of 302 mg (1.00 mmol) of 3-(4-chlorophenyl)-1-(4,6-dimethyl-2-pyrimidinyl)-1,4,5,6-tetrahydropyridazine in 5 mL of ether and 5 mL of tetrahydrofuran is treated with 1.0 mL of 1.0M $ZnCl_2$ in ether at room temperature. As the addition proceeds, a white crystalline precipitate begins to form. The reaction mixture is stirred at room temperature for 18 h and then concentrated *in vacuo* to yield 0.46 g of a white crystalline solid, mp 231-232°C. This material is crystallized from dichloromethane to yield white needles. $^1$H NMR ($CDCl_3$, 400 MHz): 7.78 (d, 8.5 Hz, 2H); 7.52 (d, 8.5 Hz, 2H); 6.71 (s, 1H); 4.31-4.25 (m, 2H); 2.92 (t, 6.4 Hz, 2H); 2.66 (s, 3H); 2.48 (s, 3H); 2.26-2.16 (m, 2H).

EXAMPLE 11

Synthesis of 3-(4-chlorophenyl)-1-(4,6-dimethyl-2-pyrimidinyl)-1,4,5,6-tetrahydropyridazine, complex with copper (II) chloride

A solution of 401 mg (1.33 mmol) of 3-(4-chlorophenyl)-1-(4,6-dimethyl-2-pyrimidinyl)-1,4,5,6-tetrahydropyridazine in 8 ml of tetrahydrofuran is treated with 179 mg of anhydrous $CuCl_2$ dissolved in 4 ml of absolute methanol. The reaction mixture immediately acquires a dark olive-green color and is stirred at room temperature

for 18 h. After concentration *in vacuo*, the resulting residue is triturated with ether several times, concentrating *in vacuo* each time. A total of 0.55 g of a free-flowing emerald green solid is thus obtained, mp 135-138°C. Crystallization from dichloromethane results in emerald green prisms.

Examples of compounds of the invention are shown in Tables 1-35. One skilled in the art will recognize that these compounds can readily be converted to their conjugate acid salts. The compounds of Tables 1-35 exemplify the limits of the broadest method claim. Some of the compounds listed are outside the scope of the compound claims. Abbreviations employed in Tables 1-35 are as follows:

t - is tertiary
s - is secondary
n - is normal
i - is iso
c - is cyclo
Me - is methyl
Et - is ethyl
Pr - is propyl
Bu - is butyl
Hex - is hexyl
Ph - is phenyl
Bzl - is benzyl
i-Pr - is isopropyl
t-Bu - is tertiary-butyl
n-Bu - is normal-butyl
c-Pr - is cyclopropyl
c-Hex - is cyclohexyl
sec-Bu - is secondary-butyl
MeO - is methoxy
i-PrO - is isopropoxy
EtS - is ethylthio
sec-BuS - is secondary-butylthio
CN - is cyano
TMS - is trimethylsilyl
Ac - is acetyl
MeS(O) - is methylsulfinyl
$MeS(O)_2$ - is methylsulfonyl

**TABLE 1**

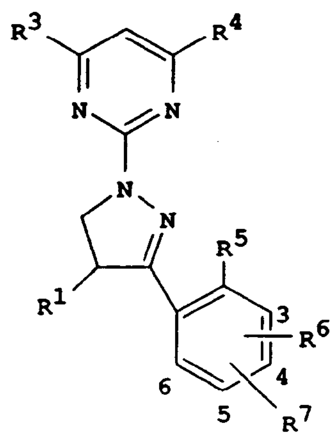

$R^7$ is H; $R^3$ is Me; $R^4$ is Me

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |

$R^7$ is H; $R^3$ is Me; $R^4$ is Me

| $R^1$ | $R^5$ | $R^6$ | $R^1$ | $R^5$ | $R^6$ |
|---|---|---|---|---|---|
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $HCF_2O$ | H | H | $HCF_2O$ | 6-$HCF_2O$ |
| H | Br | H | H | I | H |
| H | t-BuO | H | H | EtO | H |
| H | H | 4-$NMe_2$ | Me | H | 4-$NEt_2$ |
| H | H | 4-piperidino | Me | H | 4-pyrolidino |

$R^7$ is H; $R^3$ is Me; $R^4$ is H

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |

$R^7$ is H; $R^3$ is Me; $R^4$ is H

| $R^1$ | $R^5$ | $R^6$ | $R^1$ | $R^5$ | $R^6$ |
|---|---|---|---|---|---|
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $NO_2$ | 6-Cl | Me | CN | 6-CN |
| H | Br | 6-Br | Me | $MeS(O)_2$ | 4-F |
| H | $HCF_2O$ | 4-MeO | Me | i-Pr | H |

$R^7$ is H; $R^3$ is H; $R^4$ is H

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |

$R^7$ is H; $R^3$ is H; $R^4$ is H

| $R^1$ | $R^5$ | $R^6$ | $R^1$ | $R^5$ | $R^6$ |
|---|---|---|---|---|---|
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| Me | t-Bu | H | H | TMS | 6-Me |
| Me | i-PrO | H | H | TMS | 4-F |
| Me | $CF_3CF_2CF_2$ | H | H | TMS | 5-$CF_3$ |

$R^1$ is H; $R^3$ and $R^4$ are Me

| $R^5$ | $R^6$ | $R^7$ |
|---|---|---|
| H | 4-Cl | 5-Cl |
| H | 4-F | 6-sec-Bu |
| H | 4-Et | 5-I |

$R^1$, $R^3$ and $R^4$ are Me

| $R^5$ | $R^6$ | $R^7$ |
|---|---|---|
| Cl | 4-Cl | 6-Cl |
| Cl | 4-Cl | 6-MeO |
| Cl | 3-Me | 4-Cl |

| $R^1$ is H; $R^3$ and $R^4$ are Me | | |
|---|---|---|
| $R^5$ | $R^6$ | $R^7$ |
| H | 3-F | 6-$CF_3CH_2O$ |
| H | 4-Me | 6-$CF_3CF_2$ |
| H | 4-Br | 6-n-BuO |
| Me | 4-Me | 6-Me |
| Me | 4-F | 6-Me |
| Me | 4-t-Bu | 6-t-Bu |
| Me | 4-$CF_3$ | 6-Cl |
| Me | 3-Me | 5-Br |
| Me | 5-i-Pr | 6-MeO |
| t-Bu | 6-t-Bu | H |
| t-Bu | 4-t-BuO | H |
| t-Bu | H | H |
| $CF_3(CH_2)_3O$ | H | H |
| $CF_3(CF_2)_2$ | H | H |
| $(CF_3)_2CH$ | H | H |
| sec-BuS | H | H |
| MeS | 6-MeS | H |
| EtS | 4-F | H |
| MeS(O) | H | H |
| i-Prs(O) | H | H |
| t-BuS$(O)_2$ | H | H |
| MeS$(O)_2$ | H | H |
| $CH_2$=CH | H | H |
| $CH_2$=C$(CH_3)CH_2$ | H | H |
| $CH_2$=$CHCH_2O$ | H | H |
| $MeOCH_2CH_2$ | H | H |
| $MeO_2C$ | H | H |
| $MeOCH_2O$ | H | H |

| $R^1$, $R^3$ and $R^4$ are Me | | |
|---|---|---|
| $R^5$ | $R^6$ | $R^7$ |
| Cl | 3-$CF_3$ | 5-$CF_3$ |
| Cl | 4-MeO | 5-t-BuO |
| Cl | 3-n-Bu | 4-Me |
| TMS | H | H |
| TMS | H | 4-F |
| TMS | H | 6-Me |
| TMS | H | 6-MeO |
| TMS | H | 6-Cl |
| TMS | H | 6-$HCF_2O$ |
| Br | 6-Br | H |
| $NMe_2$ | H | H |
| CONHEt | H | H |
| CN | H | H |
| 4-F-Ph | H | H |
| 2-MePh | H | H |
| $NO_2$ | 6-Me | H |
| 4-Me-PhO | H | H |
| PhS | H | H |
| $CO_2H$ | 3-MeO | H |
| $CO_2H$ | H | H |
| HC≡C | H | H |
| MeC≡C | H | H |
| MeC≡$CCH_2O$ | 4-F | H |
| t-BuO | H | H |
| n-PrO | H | H |
| EtO | 5-EtO | H |
| Ac | H | H |
| sec-BuCO | H | H |

$R^4$ is Me; $R^6$ and $R^7$ are H

| $R^1$ | $R^3$ | $R^5$ |
|---|---|---|
| H | c-Pr | H |
| H | c-Pr | F |
| H | c-Pr | Cl |
| H | c-Pr | Me |
| H | c-Pr | $CF_3CH_2O$ |
| H | c-Pr | $CF_3$ |
| H | c-Pr | MeO |
| Me | $MeC\equiv C$ | H |
| Me | $MeC\equiv C$ | F |
| Me | $MeC\equiv C$ | Cl |
| Me | $MeC\equiv C$ | Me |
| Me | $MeC\equiv C$ | $CF_3CH_2O$ |
| Me | Cl | $CF_3$ |
| Me | $CF_2Cl$ | MeO |
| i-Pr | $CF_3$ | H |
| i-Pr | sec-Bu | F |
| i-Pr | $CF_3$ | Cl |
| i-Pr | $CF_3$ | Me |
| i-Pr | $CF_3$ | $CF_3CH_2O$ |
| i-Pr | Et | $CF_3$ |
| i-Pr | MeO | MeO |
| Et | c-Pr | H |
| Et | $MeC\equiv C$ | F |
| Et | $CH_2F$ | Cl |
| Et | $CF_3CH_2O$ | Me |
| Et | i-Pr | $CF_3CH_2O$ |
| Et | n-Bu | $CF_3$ |
| Et | $HC\equiv CCH_2O$ | MeO |
| t-Bu | Br | Cl |
| Ph | $CF_3(CF_2)_3$ | Me |
| Bzl | sec-BuS | $CF_3CH_2O$ |
| Me | $NH_2$ | H |
| Me | $NMe_2$ | H |
| Me | $4\text{-}NEt_2$ | H |
| Me | 4-piperidino | H |

$R^1$, $R^6$ and $R^7$ are H

| $R^3$ | $R^4$ | $R^5$ |
|---|---|---|
| c-Pr | c-Pr | H |
| c-Pr | c-Pr | F |
| c-Pr | c-Pr | Cl |
| c-Pr | c-Pr | Me |
| c-Pr | $CH_3C\equiv C$ | $CF_3CH_2O$ |
| c-Pr | $CH_3C\equiv C$ | $CF_3$ |
| c-Pr | $CH_3C\equiv C$ | MeO |
| c-Pr | $CF_3$ | H |
| c-Pr | $CF_3$ | F |
| c-Pr | $CF_3$ | Cl |
| c-Pr | $CH_3OCH_2$ | Me |
| c-Pr | $CF_3CH_2O$ | $CF_3CH_2O$ |
| c-Pr | MeS | $CF_3$ |
| c-Pr | $CH_2{=}C(Et)$ | MeO |
| c-Pr | $CH_2{=}CHCH_2$ | H |
| c-Pr | t-BuO | F |
| c-Pr | $HCF_2O$ | Cl |
| c-Pr | $CH_2{=}CHCH_2O$ | Me |
| c-Pr | $MeC\equiv CCH_2O$ | $CF_3CH_2O$ |
| c-Pr | $NMe_2$ | $CF_3$ |
| c-Pr | NHEt | MeO |
| Cl | Cl | H |
| Cl | Cl | F |
| Cl | Cl | Cl |
| Cl | Cl | Me |
| $CH_3C\equiv C$ | Cl | $CF_3CH_2O$ |
| $CH_3C\equiv C$ | F | $CF_3$ |
| $CH_3C\equiv C$ | $CH_3OCH_2$ | MeO |
| $OCF_3$ | sec-Bu | Cl |
| $OCF_3$ | Br | Me |
| $OCF_3$ | i-Pr | $CF_3CH_2O$ |
| $NH_2$ | $NH_2$ | H |
| $NMe_2$ | $NMe_2$ | H |
| Me | $NH_2$ | H |
| Me | $NEt_2$ | H |

TABLE 2

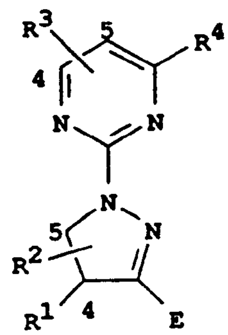

| $R^1$, $R^2$, and $R^3$ are H; $R^4$ is Me | $R^1$ and $R^2$ are H; $R^3$ is 4-Me; $R^4$ is Me |
|---|---|
| E | E |
| 1-naphthalenyl | 1-naphthalenyl |
| 2-furanyl | 2-furanyl |
| 2-naphthalenyl | 2-naphthalenyl |
| 3-thienyl | 3-thienyl |
| 2,5-dimethyl-3-furanyl | 2,5-dimethyl-3-furanyl |
| 2,5-dimethyl-3-thienyl | 2,5-dimethyl-3-thienyl |
| 4-methylphenoxy | 4-methylphenoxy |
| 2-chlorophenoxy | 2-chlorophenoxy |
| 2,6-dimethylphenoxy | 2,6-dimethylphenoxy |
| 3-methylphenylthio | 4-cyanophenylthio |
| phenylamino | 4-methylphenylamino |
| benzyl | Cl |
| Et | *n*-hex |
| *sec*-Bu | Me |
| *c*-propyl | *c*-hexyl |
| *cis*-2-methylcycloheptyl | $CF_3CH_2CH_2$ |
| *sec*-butylthio | *n*-butoxy |
| $CF_3CH_2O$ | $Cl(CH_2)_5O$ |
| 5-methyl-2-thienyl | 4-methyl-3-furanyl |
| 5-methyl-2-pyridyl | 2-methyl-3-pyridyl |

$R^1$, $R^2$ and $R^3$ are H; $R^4$ is Me

| E |
|---|
| 4-pyridyl |
| 2-indanyl |
| 2-tetrahydronaphthalenyl |

$R^1$, $R^2$, $R^3$ and $R^4$ are H

| E |
|---|
| 1-naphthalenyl |
| 2-furanyl |
| 3-thienyl |
| 3-pyridyl |

$R^3$ is 4-Me; $R^4$ is Me

| $R^1$ | $R^2$ | E |
|---|---|---|
| H | 5-Me | Ph |
| H | 5-*i*-Pr | 2-Me-Ph |
| H | 5-*n*-Bu | 2-Cl-Ph |
| H | 5-CN | 2-MeO-Ph |
| H | 5-$CF_3$ | 2-$CF_3CH_2O$-Ph |
| H | 5-$CF_3CH_2$ | 1-naphthalenyl |
| *i*-Pr | 5-Me | Ph |
| *i*-Pr | 5-Me | 2-Me-Ph |

$R^1$ and $R^2$ are H; $R^3$ is 4-Me; $R^4$ is Me

| E |
|---|
| 4-chloro-3-pyridyl |
| 2-indanyl |
| 2-tetrahydronaphthalenyl |

$R^1$ and $R^4$ are Me; $R^3$ is 4-Me; $R^2$ is H

| E |
|---|
| 1-naphthalenyl |
| 2-furanyl |
| 3-thienyl |
| 3-pyridyl |

$R^3$ is H; $R^4$ is Me

| $R^1$ | $R^2$ | E |
|---|---|---|
| H | 5-Et | Ph |
| H | 5-*sec*-Bu | 2-Me-Ph |
| H | 5-$CF_3(CF_2)_3$ | 2-Cl-Ph |
| H | 5-*t*-Bu | 2-MeO-Ph |
| H | 5-$FCH_2$ | 2-$CF_3CH_2O$-Ph |
| H | 5-*n*-Pr | 1-naphthalenyl |
| Me | 4-Me | Ph |
| Me | 4-Me | 2-Me-Ph |

$R^3$ is 4-Me; $R^4$ is Me

| $R^1$ | $R^2$ | E |
|---|---|---|
| *i*-Pr | 5-Me | 2-Cl-Ph |
| *i*-Pr | 5-Me | 2-MeO-Ph |
| *i*-Pr | 5-Me | 2-$CF_3CH_2O$-Ph |
| Cl | H | Ph |
| F | H | 2-Me-Ph |
| $CF_3CF_2$ | H | 2-Cl-Ph |
| $CH_2$=$CHCH_2$ | H | 2-MeO-Ph |
| $CO_2Me$ | H | 2-$CF_3CH_2O$-Ph |
| 2-Me-Ph | H | Me |
| Bzl | H | Ph |
| 2-naphthalenyl | H | *n*-Bu |
| 3-thienyl | H | $CF_3CF_2$ |
| 3-pyridyl | H | Me |
| CN | 5-Me | Ph |
| *t*-Bu | 5-Me | 2-Me-Ph |
| $ClCH_2$ | 5-Me | 2-Cl-Ph |
| Et | 5-Me | 2-MeO-Ph |
| *n*-Pr | 5-Me | 2-$CF_3CH_2O$-Ph |
| Me | 4-Me | 2-$CF_3$-Ph |
| *i*-Pr | 4-Me | 2-$CF_3$-Ph |
| $CF_3$ | 4-$CF_3$ | 2-$CF_3$-Ph |
| Me | 4-Me | 2-TMS-Ph |
| H | 5-OH | Ph |
| H | 5-MeO | 4-Me-Ph |
| H | 5-OC(O)Me | 4-Cl-Ph |
| H | 5-OC(O)NHMe | Ph |

$R^3$ is H; $R^4$ is Me

| $R^1$ | $R^2$ | E |
|---|---|---|
| Me | 4-Me | 2-Cl-Ph |
| Me | 4-Me | 2-MeO-Ph |
| Me | 4-Me | $2-CF_3CH_2O-Ph$ |
| Br | H | Ph |
| CN | H | 2-Me-Ph |
| Ac | H | 2-Cl-Ph |
| $CH_3C{\equiv}CCH_2$ | H | 2-MeO-Ph |
| $CO_2Et$ | H | $2-CF_3CH_2O-Ph$ |
| 4-Cl-Ph | H | Ph |
| 5-Me-3-furyl | H | i-Pr |
| EtCO | H | 2-Cl-Ph |
| 2-furyl | 4-Me | $CF_3$ |
| Ph | 5-Me | Me |
| CN | 4-Me | Ph |
| t-Bu | 4-Me | 2-Me-Ph |
| $FCH_2$ | 4-Me | 2-Cl-Ph |
| Et | 4-Me | 2-MeO-Ph |
| $Cl(CH_2)_4$ | 4-Me | $2-CF_3CH_2O-Ph$ |
| Me | 4-Me | $2-CF_3-Ph$ |
| i-Pr | 5-CN | $2-CF_3-Ph$ |
| $CF_3$ | 5-Me | $2-CF_3-Ph$ |
| i-Pr | 4-Me | 2-TMS-Ph |
| H | 5-OH | Ph |
| H | 5-MeO | 4-Me-Ph |
| H | 5-OC(O)Me | 4-Cl-Ph |
| H | 5-OC(O)NHEt | Ph |

TABLE 3

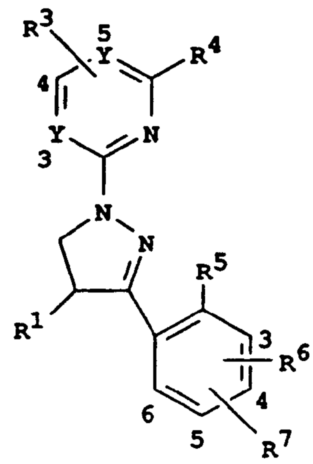

$R^7$ is H; $R^3$ is H; $R^4$ is H; Y is CH

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |

$R^7$ is H; $R^3$ is H; $R^4$ is H; Y is CH

| $R^1$ | $R^5$ | $R^6$ | $R^1$ | $R^5$ | $R^6$ |
|---|---|---|---|---|---|
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $HCF_2O$ | H | H | $HCF_2O$ | 6-$HCF_2O$ |
| H | Br | H | H | I | H |
| H | t-BuO | H | H | EtO | H |

$R^7$ is H; $R^3$ is H; $R^4$ is Me; Y is CH

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |

$R^7$ is H; $R^3$ is H; $R^4$ is Me; Y is CH

| $R^1$ | $R^5$ | $R^6$ | $R^1$ | $R^5$ | $R^6$ |
|---|---|---|---|---|---|
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $HCF_2O$ | H | H | $HCF_2O$ | 6-$HCF_2O$ |
| H | Br | H | H | I | H |
| H | t-BuO | H | H | EtO | H |

$R^7$ is H; $R^3$ is 4-Me; $R^4$ is Me; Y is N

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |

$R^7$ is H; $R^3$ is 4-Me; $R^4$ is Me; Y is N

| $R^1$ | $R^5$ | $R^6$ | $R^1$ | $R^5$ | $R^6$ |
|---|---|---|---|---|---|
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $HCF_2O$ | H | H | $HCF_2O$ | 6-$HCF_2O$ |
| H | Br | H | H | Br | H |
| H | t-BuO | H | H | t-BuO | H |

$R^4$ is Me; $R^6$ and $R^7$ are H
Y is CH

| $R^1$ | $R^3$ | $R^5$ |
|---|---|---|
| H | 4-c-Pr | H |
| H | 4-c-Pr | F |
| H | 4-c-Pr | Cl |
| H | 4-c-Pr | Me |
| H | 4-c-Pr | $CF_3CH_2O$ |
| H | 4-c-Pr | $CF_3$ |
| H | 4-c-Pr | MeO |

$R^1$, $R^6$, and $R^7$ are H; Y is N

| $R^3$ | $R^4$ | $R^5$ |
|---|---|---|
| 4-c-Pr | c-Pr | H |
| 4-c-Pr | c-Pr | F |
| 4-c-Pr | c-Pr | Cl |
| 4-c-Pr | c-Pr | Me |
| 4-c-Pr | $CH_3C{\equiv}C$ | $CF_3CH_2O$ |
| 4-c-Pr | $CH_3C{\equiv}C$ | $CF_3$ |
| 4-c-Pr | $CH_3C{\equiv}C$ | MeO |

$R^4$ is Me; $R^6$ and $R^7$ are H
Y is CH

| $R^1$ | $R^3$ | $R^5$ |
|---|---|---|
| Me | 4-MeC≡C | H |
| Me | 4-MeC≡C | F |
| Me | 4-MeC≡C | Cl |
| Me | 4-MeC≡C | Me |
| Me | 4-MeC≡C | $CF_3CH_2O$ |
| Me | 5-Cl | $CF_3$ |
| Me | 4-$CF_2Cl$ | MeO |
| i-Pr | 5-$CF_3$ | H |
| i-Pr | 4-sec-Bu | F |
| i-Pr | 4-$CF_3$ | Cl |
| i-Pr | 4-$CF_3$ | Me |
| i-Pr | 4-$CF_3$ | $CF_3CH_2O$ |
| i-Pr | 5-Et | $CF_3$ |
| i-Pr | 4-MeO | MeO |
| Et | 4-c-Pr | H |
| Et | 3-MeC≡C | F |
| Et | 4-$CH_2F$ | Cl |
| Et | 4-$CF_3CH_2O$ | Me |
| Et | 4-i-Pr | $CF_3CH_2O$ |
| Et | 4-n-Bu | $CF_3$ |
| Et | 4-HC≡$CCH_2O$ | MeO |
| t-Bu | 3-Br | Cl |
| Ph | 4-$CF_3(CF_2)_3$ | Me |
| Bzl | 4-sec-BuS | $CF_3CH_2O$ |

$R^1$, $R^6$, and $R^7$ are H; Y is N

| $R^3$ | $R^4$ | $R^5$ |
|---|---|---|
| 4-c-Pr | $CF_3$ | H |
| 4-c-Pr | $CF_3$ | F |
| 4-c-Pr | $CF_3$ | Cl |
| 4-c-Pr | $CH_3OCH_2$ | Me |
| 4-c-Pr | $CF_3CH_2O$ | $CF_3CH_2O$ |
| 4-c-Pr | MeS | $CF_3$ |
| 4-c-Pr | $CH_2$=C(Et) | MeO |
| 4-c-Pr | $CH_2$=$CHCH_2$ | H |
| 4-c-Pr | t-BuO | F |
| 4-c-Pr | $HCF_2O$ | Cl |
| 4-c-Pr | $CH_2$=$CHCH_2O$ | Me |
| 4-c-Pr | MeC≡$CCH_2O$ | $CF_3CH_2O$ |
| 4-c-Pr | $NMe_2$ | $CF_3$ |
| 4-c-Pr | NHEt | MeO |
| 4-Cl | Cl | H |
| 4-Cl | Cl | F |
| 4-Cl | Cl | Cl |
| 4-Cl | Cl | Me |
| 4-$CH_3$C≡C | Cl | $CF_3CH_2O$ |
| 4-$CH_3$C≡C | F | $CF_3$ |
| 4-$CH_3$C≡C | $CH_3OCH_2$ | MeO |
| 4-$OCF_3$ | sec-Bu | Cl |
| 4-$OCF_3$ | Br | Me |
| 4-$OCF_3$ | i-Pr | $CF_3CH_2O$ |

TABLE 4

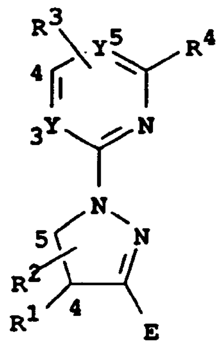

| $R^1$, $R^2$, and $R^3$ are H; $R^4$ is Me; Y is CH | $R^1$ and $R^2$ are H; $R^3$ is 4-Me; $R^4$ is Me; Y is N |
|---|---|
| E | E |
| 1-naphthalenyl | 1-naphthalenyl |
| 2-furanyl | 2-furanyl |
| 2-naphthalenyl | 2-naphthalenyl |
| 3-thienyl | 3-thienyl |
| 2,5-dimethyl-3-furanyl | 2,5-dimethyl-3-furanyl |
| 2,5-dimethyl-3-thienyl | 2,5-dimethyl-3-thienyl |
| 4-methylphenoxy | 4-methylphenoxy |
| 2-chlorophenoxy | 2-chlorophenoxy |
| 2,6-dimethylphenoxy | 2,6-dimethylphenoxy |
| 3-methylphenylthio | 4-cyanophenylthio |
| phenylamino | 4-methylphenylamino |
| benzyl | Cl |
| Et | n-hex |
| sec-Bu | Me |
| c-propyl | c-hexyl |
| cis-2-methylcycloheptyl | $CF_3CH_2CH_2$ |
| sec-butylthio | n-BuO |
| $CF_3CH_2O$ | $Cl(CH_2)_5O$ |
| 5-methyl-2-thienyl | 4-methyl-3-furanyl |
| 5-methyl-2-pyridyl | 2-methyl-3-pyridyl |

$R^1$, $R^2$, $R^3$ and $R^4$ are H;
Y is CH

**E**

4-pyridyl
2-indanyl
2-tetrahydronaphthalenyl

$R^1$, $R^2$, $R^3$ and $R^4$ are H;
Y is CH

**E**

1-naphthalenyl
2-furanyl
3-thienyl
3-pyridyl

$R^1$ and $R^4$ are Me; $R^3$ is 4-Me;
$R^2$ is H; Y is N

**E**

4-chloro-3-pyridyl
2-indanyl
2-tetrahydronaphthalenyl

$R^1$ and $R^4$ are Me; $R^3$ is 4-Me;
$R^2$ is H; Y is N

**E**

1-naphthalenyl
2-furanyl
3-thienyl
3-pyridyl

TABLE 5

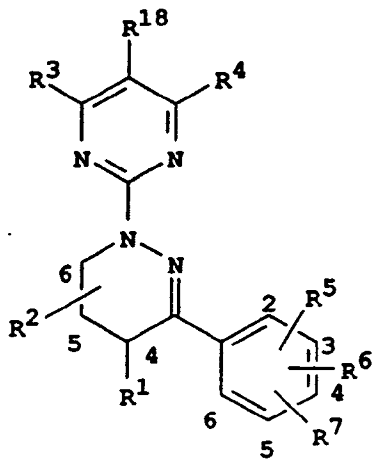

$R^2$ is H; $R^3$ is Me; $R^4$ is Me; $R^7$ is H; $R^{18}$ is H

| $R^1$ | $R^5$ | $R^6$ | $R^1$ | $R^5$ | $R^6$ |
|---|---|---|---|---|---|
| H | H | H | Me | 4-Et | H |
| H | 4-$NMe_2$ | H | Me | 4-i-Pr | H |
| H | 4-Me | H | Me | 4-Cl | H |
| H | 4-Et | H | Me | 4-MeO | H |
| H | 4-n-Pr | H | Me | 4-EtO | H |
| H | 4-i-Pr | H | Me | 4-$CF_3$ | H |
| H | 4-n-Bu | H | Et | H | H |
| H | 4-sec-Bu | H | H | 3-$NMe_2$ | H |
| H | 4-i-Bu | H | H | 3-Me | H |
| H | 4-t-Bu | H | H | 3-Et | H |
| H | 4-Cl | H | H | 3-n-Pr | H |
| H | 4-Br | H | H | 3-i-Pr | H |
| H | 4-F | H | H | 3-n-Bu | H |
| H | 4-OH | H | H | 3-Cl | H |
| H | 4-MeO | H | H | 3-Br | H |
| H | 4-EtO | H | H | 3-F | H |
| H | 4-$CF_3$ | H | H | 3-OH | H |
| H | 4-$CF_3CH_2O$ | H | H | 3-MeO | H |
| Me | H | H | H | 3-EtO | H |
| Me | 4-Me | H | H | 3-$CF_3$ | H |

$R^2$ is H; $R^3$ is Me; $R^4$ is Me; $R^7$ is H; $R^{18}$ is H

| $R^1$ | $R^5$ | $R^6$ | $R^1$ | $R^5$ | $R^6$ |
|---|---|---|---|---|---|
| H | 3-$CF_3CH_2O$ | H | H | 2-Me | 5-Me |
| Me | 3-Me | H | H | 3-Me | 4-Me |
| Me | 3-Et | H | H | 2-Et | 4-Et |
| Me | 3-_i_-Pr | H | H | 2-Et | 5-Et |
| Me | 3-Cl | H | H | 3-Et | 4-Et |
| Me | 3-MeO | H | H | 2-Me | 5-_t_-Bu |
| Me | 3-EtO | H | H | 2-Cl | 4-Cl |
| Me | 3-$CF_3$ | H | H | 2-Cl | 5-Cl |
| Et | 3-Me | H | Et | 3-MeO | H |
| Et | 3-Et | H | Et | 3-EtO | H |
| Et | 3-_i_-Pr | H | Et | $CF_3$ | H |
| Et | 3-Cl | H | Me | 2-Me | 4-Me |
| Et | 4-Me | H | Me | 2-Me | 5-Me |
| Et | 4-Et | H | Me | 3-Me | 4-Me |
| Et | 4-_i_-Pr | H | Me | 2-Et | 4-Et |
| Et | 4-Cl | H | Me | 2-Et | 5-Et |
| Et | 4-MeO | H | Me | 3-Et | 4-Et |
| Et | 4-EtO | H | Me | 2-Me | 5-_t_-Bu |
| Et | 4-$CF_3$ | H | Et | 2-Me | 4-Me |
| H | 2-Me | H | Et | 2-Me | 5-Me |
| H | 2-Et | H | Et | 3-Me | 4-Me |
| H | 2-Cl | H | Et | 2-Et | 4-Et |
| H | 2-F | H | Et | 2-Et | 5-Et |
| H | 2-OH | H | Et | 3-Et | 4-Et |
| Me | 2-Me | H | H | 4-Ph | H |
| Me | 2-Cl | H | H | 4-PhO | H |
| Me | 2-F | H | H | 4-_c_-Hex | H |
| Et | 2-Me | H | H | 4-Hex | H |
| Et | 2-Cl | H | H | 4-_n_-Amyl | H |
| Et | 2-F | H | Me | 4-Ph | H |
| H | 2-Me | 4-Me | Me | 4-PhO | H |

$R^2$ is H; $R^3$ is Me; $R^4$ is Me; $R^7$ is H; $R^{18}$ is H

| $R^1$ | $R^5$ | $R^6$ | $R^1$ | $R^5$ | $R^6$ |
|---|---|---|---|---|---|
| Me | 4-c-Hex | H | H | 3-$NH_2$ | H |
| Me | 4-Hex | H | H | 4-$NH_2$ | H |
| Me | 4-n-Amyl | H | Me | 3-$NH_2$ | H |
| H | 3-Cl | 4-Cl | Me | 4-$NH_2$ | H |
| Me | 2-Cl | 4-Cl | Et | 3-$NH_2$ | H |
| Me | 2-Cl | 5-Cl | Et | 4-$NH_2$ | H |
| Me | 3-Cl | 4-Cl | n-Pr | 4-$NMe_2$ | H |
| Et | 2-Cl | 4-Cl | n-Pr | 4-Me | H |
| Et | 2-Cl | 5-Cl | n-Pr | 4-Et | H |
| Et | 3-Cl | 4-Cl | n-Pr | 4-n-Pr | H |
| H | 2-MeO | 4-MeO | n-Pr | 4-Cl | H |
| H | 3-MeO | 5-MeO | n-Pr | 4-F | H |
| H | 3-MeO | 4-MeO | n-Pr | 4-Br | H |
| Me | 2-MeO | 4-MeO | n-Pr | 4-MeO | H |
| Me | 3-MeO | 5-MeO | n-Pr | 4-EtO | H |
| Me | 3-MeO | 4-MeO | n-Pr | 4-CF3 | H |
| Et | 2-MeO | 4-MeO | n-Pr | 4-$CF_3CH_2O$ | H |
| Et | 3-MeO | 5-MeO | n-Pr | 3-$NMe_2$ | H |
| Et | 3-MeO | 4-MeO | n-Pr | 3-Me | H |
| H | 3-Br | 5-Br | n-Pr | 3-Et | H |
| Me | 3-Br | 5-Br | n-Pr | 3-n-Pr | H |
| Et | 3-Br | 5-Br | n-Pr | 3-Cl | H |
| H | 3-Me | 5-Me | n-Pr | 3-F | H |
| Me | 3-Me | 5-Me | n-Pr | 3-Br | H |
| Et | 3-Me | 5-Me | n-Pr | 3-MeO | H |
| H | 3-Cl | 4-MeO | n-Pr | 3-EtO | H |
| Me | 3-Cl | 4-MeO | n-Pr | 3-$CF_3$ | H |
| Et | 3-Cl | 4-MeO | n-Pr | 3-$CF_3CH_2O$ | H |
| Me | 4-$NMe_2$ | H | n-Pr | 3-Me | 4-Me |
| Me | 3-$NMe_2$ | H | n-Pr | 3-Me | 5-Me |
| Et | 4-$NMe_2$ | H | n-Pr | 3-Cl | 4-Cl |
| Et | 3-$NMe_2$ | H | n-Pr | 3-MeO | 4-MeO |

$R^2$ is H; $R^3$ is Me; $R^4$ is Me; $R^7$ is H; $R^{18}$ is H

| $R^1$ | $R^5$ | $R^6$ | $R^1$ | $R^5$ | $R^6$ |
|---|---|---|---|---|---|
| n-Pr | 3-MeO | 5-MeO | i-Pr | 4-MeO | H |
| n-Pr | H | H | i-Pr | 4-EtO | H |
| n-Bu | H | H | i-Pr | 4-$CF_3$ | H |
| n-Bu | 4-Me | H | i-Pr | 4-$CF_3CH_2O$ | H |
| n-Bu | 4-Et | H | i-Pr | 3-Me | 4-Me |
| n-Bu | 4-n-Pr | H | i-Pr | 3-Me | 5-Me |
| n-Bu | 4-i-Pr | H | i-Pr | 3-Cl | 4-Cl |
| n-Bu | 4-Cl | H | i-Pr | 3-MeO | 4-MeO |
| n-Bu | 4-F | H | i-Pr | 3-MeO | 5-MeO |
| n-Bu | 4-Br | H | H | 4-TMS | H |
| n-Bu | 4-MeO | H | H | 4-I | H |
| n-Bu | 4-EtO | H | H | 4-t-BuO | H |
| n-Bu | 4-$CF_3$ | H | H | 4-$CF_3(CH_2)_3O$ | H |
| n-Bu | 4-$CF_3CH_2O$ | H | H | 4-$CF_3(CF_2)_2$ | H |
| n-Bu | 3-Me | H | H | 4-$(CF_3)_2CH$ | H |
| n-Bu | 3-Et | H | H | 4-$CH_3CHClCH$ | H |
| n-Bu | 3-n-Pr | H | Me | 4-TMS | H |
| n-Bu | 3-Cl | H | Me | 4-I | H |
| n-Bu | 3-F | H | Me | 4-t-BuO | H |
| n-Bu | 3-MeO | H | Me | 4-$CF_3(CH_2)_3O$ | H |
| n-Bu | 3-EtO | H | H | 4-MeS | H |
| n-Bu | 3-$CF_3$ | H | H | 4-EtS | H |
| n-Bu | 3-$CF_3CH_2O$ | H | H | 4-MeS(O) | H |
| i-Pr | H | H | H | 4-i-PrS(O) | H |
| i-Pr | 4-Me | H | H | 4-$MeS(O)_2$ | H |
| i-Pr | 4-Et | H | H | 4-$CH_2$=CH | H |
| i-Pr | 4-n-Pr | H | H | 4-$CH_2$=C$(CH_3)CH_2)$ | H |
| i-Pr | 4-i-Pr | H | H | 4-$CH_2$=$CHCH_2O$ | H |
| i-Pr | 4-Cl | H | H | 4-$MeOCH_2CH_2$ | H |
| i-Pr | 4-F | H | H | 4-$MeOCH_2O$ | H |
| i-Pr | 4-Br | H | | | |

$R^2$ is H; $R^3$ is Me; $R^4$ is c-Pr; $R^7$ is H; $R^{18}$ is H

| $R^1$ | $R^5$ | $R^6$ | $R^1$ | $R^5$ | $R^6$ |
|---|---|---|---|---|---|
| H | H | H | H | 3-Cl | H |
| H | 4-$NMe_2$ | H | H | 3-Br | H |
| H | 4-Me | H | H | 3-F | H |
| H | 4-Et | H | H | 3-OH | H |
| H | 4-n-Pr | H | H | 3-MeO | H |
| H | 4-i-Pr | H | H | 3-EtO | H |
| H | 4-n-Bu | H | H | 3-$CF_3$ | H |
| H | 4-sec-Bu | H | H | 3-$CF_3CH_2O$ | H |
| H | 4-i-Bu | H | Me | 3-Me | H |
| H | 4-t-Bu | H | Me | 3-Et | H |
| H | 4-Cl | H | Me | 3-i-Pr | H |
| H | 4-Br | H | Me | 3-Cl | H |
| H | 4-F | H | Me | 3-MeO | H |
| H | 4-OH | H | Me | 3-EtO | H |
| H | 4-MeO | H | Me | 3-$CF_3$ | H |
| H | 4-EtO | H | Et | 3-Me | H |
| H | 4-$CF_3$ | H | Et | 3-Et | H |
| H | 4-$CF_3CH_2O$ | H | Et | 3-i-Pr | H |
| Me | H | H | Et | 3-Cl | H |
| Me | 4-Me | H | Et | 4-Me | H |
| Me | 4-Et | H | Et | 4-Et | H |
| Me | 4-i-Pr | H | Et | 4-i-Pr | H |
| Me | 4-Cl | H | Et | 4-Cl | H |
| Me | 4-MeO | H | Et | 4-MeO | H |
| Me | 4-EtO | H | Et | 4-EtO | H |
| Me | 4-$CF_3$ | H | Et | 4-$CF_3$ | H |
| Et | H | H | H | 2-Me | H |
| H | 3-$NMe_2$ | H | H | 2-Et | H |
| H | 3-Me | H | H | 2-Cl | H |
| H | 3-Et | H | H | 2-F | H |
| H | 3-n-Pr | H | H | 2-OH | H |
| H | 3-i-Pr | H | Me | 2-Me | H |

$R^2$ is H; $R^3$ is Me; $R^4$ is c-Pr; $R^7$ is H; $R^{18}$ is H

| $R^1$ | $R^5$ | $R^6$ | $R^1$ | $R^5$ | $R^6$ |
|---|---|---|---|---|---|
| H | 3-n-Bu | H | Me | 2-Cl | H |
| Me | 2-F | H | H | 4-Hex | H |
| Et | 2-Me | H | H | 4-n-Amyl | H |
| Et | 2-Cl | H | Me | 4-Ph | H |
| Et | 2-F | H | Me | 4-PhO | H |
| H | 2-Me | 4-Me | Me | 4-c-Hex | H |
| H | 2-Me | 5-Me | Me | 4-Hex | H |
| H | 3-Me | 4-Me | Me | 4-n-Amyl | H |
| H | 2-Et | 4-Et | H | 3-Cl | 4-Cl |
| H | 2-Et | 5-Et | Me | 2-Cl | 4-Cl |
| H | 3-Et | 4-Et | Me | 2-Cl | 5-Cl |
| H | 2-Me | 5-t-Bu | Me | 3-Cl | 4-Cl |
| H | 2-Cl | 4-Cl | Et | 2-Cl | 4-Cl |
| H | 2-Cl | 5-Cl | Et | 2-Cl | 5-Cl |
| Et | 3-MeO | H | Et | 3-Cl | 4-Cl |
| Et | 3-EtO | H | H | 2-MeO | 4-MeO |
| Et | 3-$CF_3$ | H | H | 3-MeO | 5-MeO |
| Me | 2-Me | 4-Me | H | 3-MeO | 4-MeO |
| Me | 2-Me | 5-Me | Me | 2-MeO | 4-MeO |
| Me | 3-Me | 4-Me | Me | 3-MeO | 5-MeO |
| Me | 2-Et | 4-Et | Me | 3-MeO | 4-MeO |
| Me | 2-Et | 5-Et | Et | 2-MeO | 4-MeO |
| Me | 3-Et | 4-Et | Et | 3-MeO | 5-MeO |
| Me | 2-Me | 5-t-Bu | Et | 3-MeO | 4-MeO |
| Et | 2-Me | 4-Me | H | 3-Br | 5-Br |
| Et | 2-Me | 5-Me | Me | 3-Br | 5-Br |
| Et | 3-Me | 4-Me | Et | 3-Br | 5-Br |
| Et | 2-Et | 4-Et | H | 3-Me | 5-Me |
| Et | 2-Et | 5-Et | Me | 3-Me | 5-Me |
| Et | 3-Et | 4-Et | Et | 3-Me | 5-Me |
| H | 4-Ph | H | H | 3-Cl | 4-MeO |
| H | 4-PhO | H | Me | 3-Cl | 4-MeO |
| H | 4-c-Hex | H | Et | 3-Cl | 4-MeO |
| Me | 4-$NMe_2$ | H | n-Pr | 3-Me | 5-Me |

$R^2$ is H; $R^3$ is Me; $R^4$ is c-Pr; $R^7$ is H; $R^{18}$ is H

| $R^1$ | $R^5$ | $R^6$ | $R^1$ | $R^5$ | $R^6$ |
|---|---|---|---|---|---|
| Me | 3-$NMe_2$ | H | n-Pr | 3-Cl | 4-Cl |
| Et | 4-$NMe_2$ | H | n-Pr | 3-MeO | 4-MeO |
| Et | 3-$NMe_2$ | H | n-Pr | 3-MeO | 5-MeO |
| H | 3-$NH_2$ | H | n-Pr | H | H |
| H | 4-$NH_2$ | H | n-Bu | H | H |
| Me | 3-$NH_2$ | H | n-Bu | 4-Me | H |
| Me | 4-$NH_2$ | H | n-Bu | 4-Et | H |
| Et | 3-$NH_2$ | H | n-Bu | 4-n-Pr | H |
| Et | 4-$NH_2$ | H | n-Bu | 4-i-Pr | H |
| n-Pr | 4-$NMe_2$ | H | n-Bu | 4-Cl | H |
| n-Pr | 4-Me | H | n-Bu | 4-F | H |
| n-Pr | 4-Et | H | n-Bu | 4-Br | H |
| n-Pr | 4-n-Pr | H | n-Bu | 4-MeO | H |
| n-Pr | 4-Cl | H | n-Bu | 4-EtO | H |
| n-Pr | 4-F | H | n-Bu | 4-$CF_3$ | H |
| n-Pr | 4-Br | H | n-Bu | 4-$CF_3CH_2O$ | H |
| n-Pr | 4-MeO | H | n-Bu | 3-Me | H |
| n-Pr | 4-EtO | H | n-Bu | 3-Et | H |
| n-Pr | 4-CF3 | H | n-Bu | 3-n-Pr | H |
| n-Pr | 4-$CF_3CH_2O$ | H | n-Bu | 3-Cl | H |
| n-Pr | 3-$NMe_2$ | H | n-Bu | 3-F | H |
| n-Pr | 3-Me | H | n-Bu | 3-MeO | H |
| n-Pr | 3-Et | H | n-Bu | 3-EtO | H |
| n-Pr | 3-n-Pr | H | n-Bu | 3-$CF_3$ | H |
| n-Pr | 3-Cl | H | n-Bu | 3-$CF_3CH_2O$ | H |
| n-Pr | 3-F | H | i-Pr | H | H |
| n-Pr | 3-Br | H | i-Pr | 4-Me | H |
| n-Pr | 3-MeO | H | i-Pr | 4-Et | H |
| n-Pr | 3-EtO | H | i-Pr | 4-n-Pr | H |
| n-Pr | 3-$CF_3$ | H | i-Pr | 4-i-Pr | H |
| n-Pr | 3-$CF_3CH_2O$ | H | i-Pr | 4-Cl | H |
| n-Pr | 3-Me | 4-Me | i-Pr | 4-F | H |

$R^2$ is H; $R^3$ is Me; $R^4$ is c-Pr; $R^7$ is H; $R^{18}$ is H

| $R^1$ | $R^5$ | $R^6$ | $R^1$ | $R^5$ | $R^6$ |
|---|---|---|---|---|---|
| i-Pr | 4-Br | H | H | $CH_3CHClCH$ | H |
| i-Pr | 4-MeO | H | Me | 4-TMS | H |
| i-Pr | 4-EtO | H | Me | 4-I | H |
| i-Pr | 4-$CF_3$ | H | Me | 4-t-BuO | H |
| i-Pr | 4-$CF_3CH_2O$ | H | Me | 4-$CF_3(CH_2)_3O$ | H |
| i-Pr | 3-Me | 4-Me | H | 4-MeS | H |
| i-Pr | 3-Me | 5-Me | H | 4-EtS | H |
| i-Pr | 3-Cl | 4-Cl | H | 4-MeS(O) | H |
| i-Pr | 3-MeO | 4-MeO | H | 4-i-PrS(O) | H |
| i-Pr | 3-MeO | 5-MeO | H | 4-MeS$(O)_2$ | H |
| H | 4-TMS | H | H | 4-$CH_2$=CH | H |
| H | 4-I | H | H | 4-$CH_2$=C$(CH_3)CH_2$) | H |
| H | 4-t-BuO | H | H | 4-$CH_2$=$CHCH_2O$ | H |
| H | 4-$CF_3(CH_2)_3O$ | H | H | 4-$MeOCH_2CH_2$ | H |
| H | 4-$CF_3(CF_2)_2$ | H | H | 4-$MeOCH_2O$ | H |
| H | 4-$(CF_3)_2CH$ | H | | | |

$R^2$ is H; $R^3$ is Me; $R^4$ is Et; $R^7$ is H; $R^{18}$ is H

| $R^1$ | $R^5$ | $R^6$ | $R^1$ | $R^5$ | $R^6$ |
|---|---|---|---|---|---|
| H | H | H | H | 4-F | H |
| H | 4-$NMe_2$ | H | H | 4-OH | H |
| H | 4-Me | H | H | 4-MeO | H |
| H | 4-Et | H | H | 4-EtO | H |
| H | 4-n-Pr | H | H | 4-$CF_3$ | H |
| H | 4-i-Pr | H | H | 4-$CF_3CH_2O$ | H |
| H | 4-n-Bu | H | Me | H | H |
| H | 4-sec-Bu | H | Me | 4-Me | H |
| H | 4-i-Bu | H | Me | 4-Et | H |
| H | 4-t-Bu | H | Me | 4-i-Pr | H |
| H | 4-Cl | H | Me | 4-Cl | H |
| H | 4-Br | H | Me | 4-MeO | H |
| Me | 4-EtO | H | Et | 4-$CF_3$ | H |

$R^2$ is H; $R^3$ is Me; $R^4$ is Et; $R^7$ is H; $R^{18}$ is H

| $R^1$ | $R^5$ | $R^6$ | $R^1$ | $R^5$ | $R^6$ |
|---|---|---|---|---|---|
| Me | 4-$CF_3$ | H | H | 2-Me | H |
| Et | H | H | H | 2-Et | H |
| H | 3-$NMe_2$ | H | H | 2-Cl | H |
| H | 3-Me | H | H | 2-F | H |
| H | 3-Et | H | H | 2-OH | H |
| H | 3-n-Pr | H | Me | 2-Me | H |
| H | 3-i-Pr | H | Me | 2-Cl | H |
| H | 3-n-Bu | H | Me | 2-F | H |
| H | 3-Cl | H | Et | 2-Me | H |
| H | 3-Br | H | Et | 2-Cl | H |
| H | 3-F | H | Et | 2-F | H |
| H | 3-OH | H | H | 2-Me | 4-Me |
| H | 3-MeO | H | H | 2-Me | 5-Me |
| H | 3-EtO | H | H | 3-Me | 4-Me |
| H | 3-$CF_3$ | H | H | 2-Et | 4-Et |
| H | 3-$CF_3CH_2O$ | H | H | 2-Et | 5-Et |
| Me | 3-Me | H | H | 3-Et | 4-Et |
| Me | 3-Et | H | H | 2-Me | 5-t-Bu |
| Me | 3-i-Pr | H | H | 2-Cl | 4-Cl |
| Me | 3-Cl | H | H | 2-Cl | 5-Cl |
| Me | 3-MeO | H | Et | 3-MeO | H |
| Me | 3-EtO | H | Et | 3-EtO | H |
| Me | 3-$CF_3$ | H | Et | $CF_3$ | H |
| Et | 3-Me | H | Me | 2-Me | 4-Me |
| Et | 3-Et | H | Me | 2-Me | 5-Me |
| Et | 3-i-Pr | H | Me | 3-Me | 4-Me |
| Et | 3-Cl | H | Me | 2-Et | 4-Et |
| Et | 4-Me | H | Me | 2-Et | 5-Et |
| Et | 4-Et | H | Me | 3-Et | 4-Et |
| Et | 4-i-Pr | H | Me | 2-Me | 5-t-Bu |
| Et | 4-Cl | H | Et | 2-Me | 4-Me |
| Et | 4-MeO | H | Et | 2-Me | 5-Me |

$R^2$ is H; $R^3$ is Me; $R^4$ is Et; $R^7$ is H; $R^{18}$ is H

| $R^1$ | $R^5$ | $R^6$ | $R^1$ | $R^5$ | $R^6$ |
|---|---|---|---|---|---|
| Et | 4-EtO | H | Et | 3-Me | 4-Me |
| Et | 2-Et | 4-Et | Me | 3-Me | 5-Me |
| Et | 2-Et | 5-Et | Et | 3-Me | 5-Me |
| Et | 3-Et | 4-Et | H | 3-Cl | 4-MeO |
| H | 4-Ph | H | Me | 3-Cl | 4-MeO |
| H | 4-PhO | H | Et | 3-Cl | 4-MeO |
| H | 4-c-Hex | H | Me | 4-$NMe_2$ | H |
| H | 4-Hex | H | Me | 3-$NMe_2$ | H |
| H | 4-n-Amyl | H | Et | 4-$NMe_2$ | H |
| Me | 4-Ph | H | Et | 3-$NMe_2$ | H |
| Me | 4-PhO | H | H | 3-$NH_2$ | H |
| Me | 4-c-Hex | H | H | 4-$NH_2$ | H |
| Me | 4-Hex | H | Me | 3-$NH_2$ | H |
| Me | 4-n-Amyl | H | Me | 4-$NH_2$ | H |
| H | 3-Cl | 4-Cl | Et | 3-$NH_2$ | H |
| Me | 2-Cl | 4-Cl | Et | 4-$NH_2$ | H |
| Me | 2-Cl | 5-Cl | n-Pr | 4-$NMe_2$ | H |
| Me | 3-Cl | 4-Cl | n-Pr | 4-Me | H |
| Et | 2-Cl | 4-Cl | n-Pr | 4-Et | H |
| Et | 2-Cl | 5-Cl | n-Pr | 4-n-Pr | H |
| Et | 3-Cl | 4-Cl | n-Pr | 4-Cl | H |
| H | 2-MeO | 4-MeO | n-Pr | 4-F | H |
| H | 3-MeO | 5-MeO | n-Pr | 4-Br | H |
| H | 3-MeO | 4-MeO | n-Pr | 4-MeO | H |
| Me | 2-MeO | 4-MeO | n-Pr | 4-EtO | H |
| Me | 3-MeO | 5-MeO | n-Pr | 4-CF3 | H |
| Me | 3-MeO | 4-MeO | n-Pr | 4-$CF_3CH_2O$ | H |
| Et | 2-MeO | 4-MeO | n-Pr | 3-$NMe_2$ | H |
| Et | 3-MeO | 5-MeO | n-Pr | 3-Me | H |
| Et | 3-MeO | 4-MeO | n-Pr | 3-Et | H |
| H | 3-Br | 5-Br | n-Pr | 3-n-Pr | H |
| Me | 3-Br | 5-Br | n-Pr | 3-Cl | H |
| Et | 3-Br | 5-Br | n-Pr | 3-F | H |
| H | 3-Me | 5-Me | n-Pr | 3-Br | H |

$R^2$ is H; $R^3$ is Me; $R^4$ is Et; $R^7$ is H; $R^{18}$ is H

| $R^1$ | $R^5$ | $R^6$ | $R^1$ | $R^5$ | $R^6$ |
|---|---|---|---|---|---|
| n-Pr | 3-MeO | H | i-Pr | 4-i-Pr | H |
| n-Pr | 3-EtO | H | i-Pr | 4-Cl | H |
| n-Pr | 3-$CF_3$ | H | i-Pr | 4-F | H |
| n-Pr | 3-$CF_3CH_2O$ | H | i-Pr | 4-Br | H |
| n-Pr | 3-Me | 4-Me | i-Pr | 4-MeO | H |
| n-Pr | 3-Me | 5-Me | i-Pr | 4-EtO | H |
| n-Pr | 3-Cl | 4-Cl | i-Pr | 4-$CF_3$ | H |
| n-Pr | 3-MeO | 4-MeO | i-Pr | 4-$CF_3CH_2O$ | H |
| n-Pr | 3-MeO | 5-MeO | i-Pr | 3-Me | 4-Me |
| n-Pr | H | H | i-Pr | 3-Me | 5-Me |
| n-Bu | H | H | i-Pr | 3-Cl | 4-Cl |
| n-Bu | 4-Me | H | i-Pr | 3-MeO | 4-MeO |
| n-Bu | 4-Et | H | i-Pr | 3-MeO | 5-MeO |
| n-Bu | 4-n-Pr | H | H | 4-TMS | H |
| n-Bu | 4-i-Pr | H | H | 4-I | H |
| n-Bu | 4-Cl | H | H | 4-t-BuO | H |
| n-Bu | 4-F | H | H | 4-$CF_3(CH_2)_3O$ | H |
| n-Bu | 4-Br | H | H | 4-$CF_3(CF_2)_2$ | H |
| n-Bu | 4-MeO | H | H | 4-$(CF_3)_2CH$ | H |
| n-Bu | 4-EtO | H | H | 4-$CH_3CHClCH$ | H |
| n-Bu | 4-$CF_3$ | H | Me | 4-TMS | H |
| n-Bu | 4-$CF_3CH_2O$ | H | Me | 4-I | H |
| n-Bu | 3-Me | H | Me | 4-t-BuO | H |
| n-Bu | 3-Et | H | Me | 4-$CF_3(CH_2)_3O$ | H |
| n-Bu | 3-n-Pr | H | H | 4-MeS | H |
| n-Bu | 3-Cl | H | H | 4-EtS | H |
| n-Bu | 3-F | H | H | 4-MeS(O) | H |
| n-Bu | 3-MeO | H | H | 4-i-PrS(O) | H |
| n-Bu | 3-EtO | H | H | 4-$MeS(O)_2$ | H |
| n-Bu | 3-$CF_3$ | H | H | 4-$CH_2$=CH | H |
| n-Bu | 3-$CF_3CH_2O$ | H | H | 4-$CH_2$=C($CH_3$)$CH_2$) | H |
| i-Pr | H | H | H | 4-$CH_2$=$CHCH_2O$ | H |
| i-Pr | 4-Me | H | H | 4-$MeOCH_2CH_2$ | H |
| i-Pr | 4-Et | H | H | 4-$MeOCH_2O$ | H |
| i-Pr | 4-n-Pr | H | | | |

$R^2$ is H; $R^3$ is Et; $R^4$ is Et; $R^7$ is H; $R^{18}$ is H

| $R^1$ | $R^5$ | $R^6$ | $R^1$ | $R^5$ | $R^6$ |
|---|---|---|---|---|---|
| H | H | H | H | 3-n-Bu | H |
| H | 4-$NMe_2$ | H | H | 3-Cl | H |
| H | 4-Me | H | H | 3-Br | H |
| H | 4-Et | H | H | 3-F | H |
| H | 4-n-Pr | H | H | 3-OH | H |
| H | 4-i-Pr | H | H | 3-MeO | H |
| H | 4-n-Bu | H | H | 3-EtO | H |
| H | 4-sec-Bu | H | H | 3-$CF_3$ | H |
| H | 4-i-Bu | H | H | 3-$CF_3CH_2O$ | H |
| H | 4-t-Bu | H | Me | 3-Me | H |
| H | 4-Cl | H | Me | 3-Et | H |
| H | 4-Br | H | Me | 3-i-Pr | H |
| H | 4-F | H | Me | 3-Cl | H |
| H | 4-OH | H | Me | 3-MeO | H |
| H | 4-MeO | H | Me | 3-EtO | H |
| H | 4-EtO | H | Me | 3-$CF_3$ | H |
| H | 4-$CF_3$ | H | Et | 3-Me | H |
| H | 4-$CF_3CH_2O$ | H | Et | 3-Et | H |
| Me | H | H | Et | 3-i-Pr | H |
| Me | 4-Me | H | Et | 3-Cl | H |
| Me | 4-Et | H | Et | 4-Me | H |
| Me | 4-i-Pr | H | Et | 4-Et | H |
| Me | 4-Cl | H | Et | 4-i-Pr | H |
| Me | 4-MeO | H | Et | 4-Cl | H |
| Me | 4-EtO | H | Et | 4-MeO | H |
| Me | 4-$CF_3$ | H | Et | 4-EtO | H |
| Et | H | H | Et | 4-$CF_3$ | H |
| H | 3-$NMe_2$ | H | H | 2-Me | H |
| H | 3-Me | H | H | 2-Et | H |
| H | 3-Et | H | H | 2-Cl | H |
| H | 3-n-Pr | H | H | 2-F | H |
| H | 3-i-Pr | H | H | 2-OH | H |

$R^2$ is H; $R^3$ is Et; $R^4$ is Et; $R^7$ is H; $R^{18}$ is H

| $R^1$ | $R^5$ | $R^6$ | $R^1$ | $R^5$ | $R^6$ |
|---|---|---|---|---|---|
| Me | 2-Me | H | H | 4-Ph | H |
| Me | 2-Cl | H | H | 4-PhO | H |
| Me | 2-F | H | H | 4-c-Hex | H |
| Et | 2-Me | H | H | 4-Hex | H |
| Et | 2-Cl | H | H | 4-n-Amyl | H |
| Et | 2-F | H | Me | 4-Ph | H |
| H | 2-Me | 4-Me | Me | 4-PhO | H |
| H | 2-Me | 5-Me | Me | 4-c-Hex | H |
| H | 3-Me | 4-Me | Me | 4-Hex | H |
| H | 2-Et | 4-Et | Me | 4-n-Amyl | H |
| H | 2-Et | 5-Et | H | 3-Cl | 4-Cl |
| H | 3-Et | 4-Et | Me | 2-Cl | 4-Cl |
| H | 2-Me | 5-t-Bu | Me | 2-Cl | 5-Cl |
| H | 2-Cl | 4-Cl | Me | 3-Cl | 4-Cl |
| H | 2-Cl | 5-Cl | Et | 2-Cl | 4-Cl |
| Et | 3-MeO | H | Et | 2-Cl | 5-Cl |
| Et | 3-EtO | H | Et | 3-Cl | 4-Cl |
| Et | 3-$CF_3$ | H | H | 2-MeO | 4-MeO |
| Me | 2-Me | 4-Me | H | 3-MeO | 5-MeO |
| Me | 2-Me | 5-Me | H | 3-MeO | 4-MeO |
| Me | 3-Me | 4-Me | Me | 2-MeO | 4-MeO |
| Me | 2-Et | 4-Et | Me | 3-MeO | 5-MeO |
| Me | 2-Et | 5-Et | Me | 3-MeO | 4-MeO |
| Me | 3-Et | 4-Et | Et | 2-MeO | 4-MeO |
| Me | 2-Me | 5-t-Bu | Et | 3-MeO | 5-MeO |
| Et | 2-Me | 4-Me | Et | 3-MeO | 4-MeO |
| Et | 2-Me | 5-Me | H | 3-Br | 5-Br |
| Et | 3-Me | 4-Me | Me | 3-Br | 5-Br |
| Et | 2-Et | 4-Et | Et | 3-Br | 5-Br |
| Et | 2-Et | 5-Et | H | 3-Me | 5-Me |
| Et | 3-Et | 4-Et | Me | 3-Me | 5-Me |

$R^2$ is H; $R^3$ is Et; $R^4$ is Et; $R^7$ is H; $R^{18}$ is H

| $R^1$ | $R^5$ | $R^6$ | $R^1$ | $R^5$ | $R^6$ |
|---|---|---|---|---|---|
| Et | 3-Me | 5-Me | n-Pr | 3-MeO | H |
| H | 3-Cl | 4-MeO | n-Pr | 3-EtO | H |
| Me | 3-Cl | 4-MeO | n-Pr | 3-$CF_3$ | H |
| Et | 3-Cl | 4-MeO | n-Pr | 3-$CF_3CH_2O$ | H |
| Me | 4-$NMe_2$ | H | n-Pr | 3-Me | 4-Me |
| Me | 3-$NMe_2$ | H | n-Pr | 3-Me | 5-Me |
| Et | 4-$NMe_2$ | H | n-Pr | 3-Cl | 4-Cl |
| Et | 3-$NMe_2$ | H | n-Pr | 3-MeO | 4-MeO |
| H | 3-$NH_2$ | H | n-Pr | 3-MeO | 5-MeO |
| H | 4-$NH_2$ | H | n-Pr | H | H |
| Me | 3-$NH_2$ | H | n-Bu | H | H |
| Me | 4-$NH_2$ | H | n-Bu | 4-Me | H |
| Et | 3-$NH_2$ | H | n-Bu | 4-Et | H |
| Et | 4-$NH_2$ | H | n-Bu | 4-n-Pr | H |
| n-Pr | 4-$NMe_2$ | H | n-Bu | 4-i-Pr | H |
| n-Pr | 4-Me | H | n-Bu | 4-Cl | H |
| n-Pr | 4-Et | H | n-Bu | 4-F | H |
| n-Pr | 4-n-Pr | H | n-Bu | 4-Br | H |
| n-Pr | 4-Cl | H | n-Bu | 4-MeO | H |
| n-Pr | 4-F | H | n-Bu | 4-EtO | H |
| n-Pr | 4-Br | H | n-Bu | 4-$CF_3$ | H |
| n-Pr | 4-MeO | H | n-Bu | 4-$CF_3CH_2O$ | H |
| n-Pr | 4-EtO | H | n-Bu | 3-Me | H |
| n-Pr | 4-CF3 | H | n-Bu | 3-Et | H |
| n-Pr | 4-$CF_3CH_2O$ | H | n-Bu | 3-n-Pr | H |
| n-Pr | 3-$NMe_2$ | H | n-Bu | 3-Cl | H |
| n-Pr | 3-Me | H | n-Bu | 3-F | H |
| n-Pr | 3-Et | H | n-Bu | 3-MeO | H |
| n-Pr | 3-n-Pr | H | n-Bu | 3-EtO | H |
| n-Pr | 3-Cl | H | n-Bu | 3-$CF_3$ | H |
| n-Pr | 3-F | H | n-Bu | 3-$CF_3CH_2O$ | H |
| n-Pr | 3-Br | H | i-Pr | H | H |

$R^2$ is H; $R^3$ is Et; $R^4$ is Et; $R^7$ is H; $R^{18}$ is H

| $R^1$ | $R^5$ | $R^6$ | $R^1$ | $R^5$ | $R^6$ |
|---|---|---|---|---|---|
| i-Pr | 4-Me | H | H | 4-$CF_3(CH_2)_3O$ | H |
| i-Pr | 4-Et | H | H | 4-$CF_3(CF_2)_2$ | H |
| i-Pr | 4-n-Pr | H | H | 4-$(CF_3)_2CH$ | H |
| i-Pr | 4-i-Pr | H | H | 4-$CH_3CHClCH$ | H |
| i-Pr | 4-Cl | H | Me | 4-TMS | H |
| i-Pr | 4-F | H | Me | 4-I | H |
| i-Pr | 4-Br | H | Me | 4-t-BuO | H |
| i-Pr | 4-MeO | H | Me | 4-$CF_3(CH_2)_3O$ | H |
| i-Pr | 4-EtO | H | H | 4-MeS | H |
| i-Pr | 4-$CF_3$ | H | H | 4-EtS | H |
| i-Pr | 4-$CF_3CH_2O$ | H | H | 4-MeS(O) | H |
| i-Pr | 3-Me | 4-Me | H | 4-i-PrS(O) | H |
| i-Pr | 3-Me | 5-Me | H | 4-$MeS(O)_2$ | H |
| i-Pr | 3-Cl | 4-Cl | H | 4-$CH_2$=CH | H |
| i-Pr | 3-MeO | 4-MeO | H | 4-$CH_2$=C$(CH_3)CH_2)$ | H |
| i-Pr | 3-MeO | 5-MeO | H | 4-$CH_2$=$CHCH_2O$ | H |
| H | 4-TMS | H | H | 4-$MeOCH_2CH_2$ | H |
| H | 4-I | H | H | 4-$MeOCH_2O$ | H |
| H | 4-t-BuO | H | | | |

$R^2$ is H; $R^3$ is Me; $R^4$ is i-Pr; $R^7$ is H; $R^{18}$ is H

| $R^1$ | $R^5$ | $R^6$ | $R^1$ | $R^5$ | $R^6$ |
|---|---|---|---|---|---|
| H | H | H | H | 4-i-Bu | H |
| H | 4-$NMe_2$ | H | H | 4-t-Bu | H |
| H | 4-Me | H | H | 4-Cl | H |
| H | 4-Et | H | H | 4-Br | H |
| H | 4-n-Pr | H | H | 4-F | H |
| H | 4-i-Pr | H | H | 4-OH | H |
| H | 4-n-Bu | H | H | 4-MeO | H |
| H | 4-sec-Bu | H | H | 4-EtO | H |

$R^2$ is H; $R^3$ is Me; $R^4$ is i-Pr; $R^7$ is H; $R^{18}$ is H

| $R^1$ | $R^5$ | $R^6$ | $R^1$ | $R^5$ | $R^6$ |
|---|---|---|---|---|---|
| H | 4-$CF_3$ | H | Et | 3-Me | H |
| H | 4-$CF_3CH_2O$ | H | Et | 3-Et | H |
| Me | H | H | Et | 3-i-Pr | H |
| Me | 4-Me | H | Et | 3-Cl | H |
| Me | 4-Et | H | Et | 4-Me | H |
| Me | 4-i-Pr | H | Et | 4-Et | H |
| Me | 4-Cl | H | Et | 4-i-Pr | H |
| Me | 4-MeO | H | Et | 4-Cl | H |
| Me | 4-EtO | H | Et | 4-MeO | H |
| Me | 4-$CF_3$ | H | Et | 4-EtO | H |
| Et | H | H | Et | 4-$CF_3$ | H |
| H | 3-$NMe_2$ | H | H | 2-Me | H |
| H | 3-Me | H | H | 2-Et | H |
| H | 3-Et | H | H | 2-Cl | H |
| H | 3-n-Pr | H | H | 2-F | H |
| H | 3-i-Pr | H | H | 2-OH | H |
| H | 3-n-Bu | H | Me | 2-Me | H |
| H | 3-Cl | H | Me | 2-Cl | H |
| H | 3-Br | H | Me | 2-F | H |
| H | 3-F | H | Et | 2-Me | H |
| H | 3-OH | H | Et | 2-Cl | H |
| H | 3-MeO | H | Et | 2-F | H |
| H | 3-EtO | H | H | 2-Me | 4-Me |
| H | 3-$CF_3$ | H | H | 2-Me | 5-Me |
| H | 3-$CF_3CH_2O$ | H | H | 3-Me | 4-Me |
| Me | 3-Me | H | H | 2-Et | 4-Et |
| Me | 3-Et | H | H | 2-Et | 5-Et |
| Me | 3-i-Pr | H | H | 3-Et | 4-Et |
| Me | 3-Cl | H | H | 2-Me | 5-t-Bu |
| Me | 3-MeO | H | H | 2-Cl | 4-Cl |
| Me | 3-EtO | H | H | 2-Cl | 5-Cl |
| Me | 3-$CF_3$ | H | Et | 3-MeO | H |

$R^2$ is H; $R^3$ is Me; $R^4$ is i-Pr; $R^7$ is H; $R^{18}$ is H

| $R^1$ | $R^5$ | $R^6$ | $R^1$ | $R^5$ | $R^6$ |
|---|---|---|---|---|---|
| Et | 3-EtO | H | H | 2-MeO | 4-MeO |
| Et | $CF_3$ | H | H | 3-MeO | 5-MeO |
| Me | 2-Me | 4-Me | H | 3-MeO | 4-MeO |
| Me | 2-Me | 5-Me | Me | 2-MeO | 4-MeO |
| Me | 3-Me | 4-Me | Me | 3-MeO | 5-MeO |
| Me | 2-Et | 4-Et | Me | 3-MeO | 4-MeO |
| Me | 2-Et | 5-Et | Et | 2-MeO | 4-MeO |
| Me | 3-Et | 4-Et | Et | 3-MeO | 5-MeO |
| Me | 2-Me | 5-t-Bu | Et | 3-MeO | 4-MeO |
| Et | 2-Me | 4-Me | H | 3-Br | 5-Br |
| Et | 2-Me | 5-Me | Me | 3-Br | 5-Br |
| Et | 3-Me | 4-Me | Et | 3-Br | 5-Br |
| Et | 2-Et | 4-Et | H | 3-Me | 5-Me |
| Et | 2-Et | 5-Et | Me | 3-Me | 5-Me |
| Et | 3-Et | 4-Et | Et | 3-Me | 5-Me |
| H | 4-Ph | H | H | 3-Cl | 4-MeO |
| H | 4-PhO | H | Me | 3-Cl | 4-MeO |
| H | 4-c-Hex | H | Et | 3-Cl | 4-MeO |
| H | 4-Hex | H | Me | 4-$NMe_2$ | H |
| H | 4-n-Amyl | H | Me | 3-$NMe_2$ | H |
| Me | 4-Ph | H | Et | 4-$NMe_2$ | H |
| Me | 4-PhO | H | Et | 3-$NMe_2$ | H |
| Me | 4-c-Hex | H | H | 3-$NH_2$ | H |
| Me | 4-Hex | H | H | 4-$NH_2$ | H |
| Me | 4-n-Amyl | H | Me | 3-$NH_2$ | H |
| H | 3-Cl | 4-Cl | Me | 4-$NH_2$ | H |
| Me | 2-Cl | 4-Cl | Et | 3-$NH_2$ | H |
| Me | 2-Cl | 5-Cl | Et | 4-$NH_2$ | H |
| Me | 3-Cl | 4-Cl | n-Pr | 4-$NMe_2$ | H |
| Et | 2-Cl | 4-Cl | n-Pr | 4-Me | H |
| Et | 2-Cl | 5-Cl | n-Pr | 4-Et | H |
| Et | 3-Cl | 4-Cl | n-Pr | 4-n-Pr | H |

$R^2$ is H; $R^3$ is Me; $R^4$ is i-Pr; $R^7$ is H; $R^{18}$ is H

| $R^1$ | $R^5$ | $R^6$ | $R^1$ | $R^5$ | $R^6$ |
|---|---|---|---|---|---|
| n-Pr | 4-Cl | H | n-Bu | 4-MeO | H |
| n-Pr | 4-F | H | n-Bu | 4-EtO | H |
| n-Pr | 4-Br | H | n-Bu | 4-$CF_3$ | H |
| n-Pr | 4-MeO | H | n-Bu | 4-$CF_3CH_2O$ | H |
| n-Pr | 4-EtO | H | n-Bu | 3-Me | H |
| n-Pr | 4-CF3 | H | n-Bu | 3-Et | H |
| n-Pr | 4-$CF_3CH_2O$ | H | n-Bu | 3-n-Pr | H |
| n-Pr | 3-$NMe_2$ | H | n-Bu | 3-Cl | H |
| n-Pr | 3-Me | H | n-Bu | 3-F | H |
| n-Pr | 3-Et | H | n-Bu | 3-MeO | H |
| n-Pr | 3-n-Pr | H | n-Bu | 3-EtO | H |
| n-Pr | 3-Cl | H | n-Bu | 3-$CF_3$ | H |
| n-Pr | 3-F | H | n-Bu | 3-$CF_3CH_2O$ | H |
| n-Pr | 3-Br | H | i-Pr | H | H |
| n-Pr | 3-MeO | H | i-Pr | 4-Me | H |
| n-Pr | 3-EtO | H | i-Pr | 4-Et | H |
| n-Pr | 3-$CF_3$ | H | i-Pr | 4-n-Pr | H |
| n-Pr | 3-$CF_3CH_2O$ | H | i-Pr | 4-i-Pr | H |
| n-Pr | 3-Me | 4-Me | i-Pr | 4-Cl | H |
| n-Pr | 3-Me | 5-Me | i-Pr | 4-F | H |
| n-Pr | 3-Cl | 4-Cl | i-Pr | 4-Br | H |
| n-Pr | 3-MeO | 4-MeO | i-Pr | 4-MeO | H |
| n-Pr | 3-MeO | 5-MeO | i-Pr | 4-EtO | H |
| n-Pr | H | H | i-Pr | 4-$CF_3$ | H |
| n-Bu | H | H | i-Pr | 4-$CF_3CH_2O$ | H |
| n-Bu | 4-Me | H | i-Pr | 3-Me | 4-Me |
| n-Bu | 4-Et | H | i-Pr | 3-Me | 5-Me |
| n-Bu | 4-n-Pr | H | i-Pr | 3-Cl | 4-Cl |
| n-Bu | 4-i-Pr | H | i-Pr | 3-MeO | 4-MeO |
| n-Bu | 4-Cl | H | i-Pr | 3-MeO | 5-MeO |
| n-Bu | 4-F | H | H | 4-TMS | H |
| n-Bu | 4-Br | H | H | 4-I | H |

$R^2$ is H; $R^3$ is Me; $R^4$ is i-Pr; $R^7$ is H; $R^{18}$ is H

| $R^1$ | $R^5$ | $R^6$ | $R^1$ | $R^5$ | $R^6$ |
|---|---|---|---|---|---|
| H | 4-t-BuO | H | H | 4-EtS | H |
| H | 4-$CF_3(CH_2)_3O$ | H | H | 4-MeS(O) | H |
| H | 4-$CF_3(CF_2)_2$ | H | H | 4-i-PrS(O) | H |
| H | 4-$(CF_3)_2CH$ | H | H | 4-MeS$(O)_2$ | H |
| H | 4-$CH_3CHClCH$ | H | H | 4-$CH_2$=CH | H |
| Me | 4-TMS | H | H | 4-$CH_2$=C$(CH_3)CH_2$) | H |
| Me | 4-I | H | H | 4-$CH_2$=$CHCH_2O$ | H |
| Me | 4-t-BuO | H | H | 4-$MeOCH_2CH_2$ | H |
| Me | 4-$CF_3(CH_2)_3O$ | H | H | 4-$MeOCH_2O$ | H |
| H | 4-MeS | H | | | |

$R^2$ is H; $R^3$ is Me; $R^4$ is H; $R^7$ is H; $R^{18}$ is H

| $R^1$ | $R^5$ | $R^6$ | $R^1$ | $R^5$ | $R^6$ |
|---|---|---|---|---|---|
| H | H | H | H | 4-$CF_3CH_2O$ | H |
| H | 4-$NMe_2$ | H | Me | H | H |
| H | 4-Me | H | Me | 4-Me | H |
| H | 4-Et | H | Me | 4-Et | H |
| H | 4-n-Pr | H | Me | 4-i-Pr | H |
| H | 4-i-Pr | H | Me | 4-Cl | H |
| H | 4-n-Bu | H | Me | 4-MeO | H |
| H | 4-sec-Bu | H | Me | 4-EtO | H |
| H | 4-i-Bu | H | Me | 4-$CF_3$ | H |
| H | 4-t-Bu | H | Et | H | H |
| H | 4-Cl | H | H | 3-$NMe_2$ | H |
| H | 4-Br | H | H | 3-Me | H |
| H | 4-F | H | H | 3-Et | H |
| H | 4-OH | H | H | 3-n-Pr | H |
| H | 4-MeO | H | H | 3-i-Pr | H |
| H | 4-EtO | H | H | 3-n-Bu | H |
| H | 4-$CF_3$ | H | H | 3-Cl | H |

$R^2$ is H; $R^3$ is Me; $R^4$ is H; $R^7$ is H; $R^{18}$ is H

| $R^1$ | $R^5$ | $R^6$ | $R^1$ | $R^5$ | $R^6$ |
|---|---|---|---|---|---|
| H | 3-Br | H | Me | 2-Me | H |
| H | 3-F | H | Me | 2-Cl | H |
| H | 3-OH | H | Me | 2-F | H |
| H | 3-MeO | H | Et | 2-Me | H |
| H | 3-EtO | H | Et | 2-Cl | H |
| H | 3-$CF_3$ | H | Et | 2-F | H |
| H | 3-$CF_3CH_2O$ | H | H | 2-Me | 4-Me |
| Me | 3-Me | H | H | 2-Me | 5-Me |
| Me | 3-Et | H | H | 3-Me | 4-Me |
| Me | 3-i-Pr | H | H | 2-Et | 4-Et |
| Me | 3-Cl | H | H | 2-Et | 5-Et |
| Me | 3-MeO | H | H | 3-Et | 4-Et |
| Me | 3-EtO | H | H | 2-Me | 5-t-Bu |
| Me | 3-$CF_3$ | H | H | 2-Cl | 4-Cl |
| Et | 3-Me | H | H | 2-Cl | 5-Cl |
| Et | 3-Et | H | Et | 3-MeO | H |
| Et | 3-i-Pr | H | Et | 3-EtO | H |
| Et | 3-Cl | H | Et | 3-$CF_3$ | H |
| Et | 4-Me | H | Me | 2-Me | 4-Me |
| Et | 4-Et | H | Me | 2-Me | 5-Me |
| Et | 4-i-Pr | H | Me | 3-Me | 4-Me |
| Et | 4-Cl | H | Me | 2-Et | 4-Et |
| Et | 4-MeO | H | Me | 2-Et | 5-Et |
| Et | 4-EtO | H | Me | 3-Et | 4-Et |
| Et | 4-$CF_3$ | H | Me | 2-Me | 5-t-Bu |
| H | 2-Me | H | Et | 2-Me | 4-Me |
| H | 2-Et | H | Et | 2-Me | 5-Me |
| H | 2-Cl | H | Et | 3-Me | 4-Me |
| H | 2-F | H | Et | 2-Et | 4-Et |
| H | 2-OH | H | Et | 2-Et | 5-Et |

$R^2$ is H; $R^3$ is Me; $R^4$ is H; $R^7$ is H; $R^{18}$ is H

| $R^1$ | $R^5$ | $R^6$ | $R^1$ | $R^5$ | $R^6$ |
|---|---|---|---|---|---|
| Et | 3-Et | 4-Et | H | 3-Cl | 4-MeO |
| H | 4-Ph | H | Me | 3-Cl | 4-MeO |
| H | 4-PhO | H | Et | 3-Cl | 4-MeO |
| H | 4-c-Hex | H | Me | 4-$NMe_2$ | H |
| H | 4-Hex | H | Me | 3-$NMe_2$ | H |
| H | 4-n-Amyl | H | Et | 4-$NMe_2$ | H |
| Me | 4-Ph | H | Et | 3-$NMe_2$ | H |
| Me | 4-PhO | H | H | 3-$NH_2$ | H |
| Me | 4-c-Hex | H | H | 4-$NH_2$ | H |
| Me | 4-Hex | H | Me | 3-$NH_2$ | H |
| Me | 4-n-Amyl | H | Me | 4-$NH_2$ | H |
| H | 3-Cl | 4-Cl | Et | 3-$NH_2$ | H |
| Me | 2-Cl | 4-Cl | Et | 4-$NH_2$ | H |
| Me | 2-Cl | 5-Cl | n-Pr | 4-$NMe_2$ | H |
| Me | 3-Cl | 4-Cl | n-Pr | 4-Me | H |
| Et | 2-Cl | 4-Cl | n-Pr | 4-Et | H |
| Et | 2-Cl | 5-Cl | n-Pr | 4-n-Pr | H |
| Et | 3-Cl | 4-Cl | n-Pr | 4-Cl | H |
| H | 2-MeO | 4-MeO | n-Pr | 4-F | H |
| H | 3-MeO | 5-MeO | n-Pr | 4-Br | H |
| H | 3-MeO | 4-MeO | n-Pr | 4-MeO | H |
| Me | 2-MeO | 4-MeO | n-Pr | 4-EtO | H |
| Me | 3-MeO | 5-MeO | n-Pr | 4-CF3 | H |
| Me | 3-MeO | 4-MeO | n-Pr | 4-$CF_3CH_2O$ | H |
| Et | 2-MeO | 4-MeO | n-Pr | 3-$NMe_2$ | H |
| Et | 3-MeO | 5-MeO | n-Pr | 3-Me | H |
| Et | 3-MeO | 4-MeO | n-Pr | 3-Et | H |
| H | 3-Br | 5-Br | n-Pr | 3-n-Pr | H |
| Me | 3-Br | 5-Br | n-Pr | 3-Cl | H |
| Et | 3-Br | 5-Br | n-Pr | 3-F | H |
| H | 3-Me | 5-Me | n-Pr | 3-Br | H |
| Me | 3-Me | 5-Me | n-Pr | 3-MeO | H |

$R^2$ is H; $R^3$ is Me; $R^4$ is H; $R^7$ is H; $R^{18}$ is H

| $R^1$ | $R^5$ | $R^6$ | $R^1$ | $R^5$ | $R^6$ |
|---|---|---|---|---|---|
| Et | 3-Me | 5-Me | n-Pr | 3-EtO | H |
| n-Pr | 3-$CF_3$ | H | i-Pr | 4-i-Pr | H |
| n-Pr | 3-$CF_3CH_2O$ | H | i-Pr | 4-Cl | H |
| n-Pr | 3-Me | 4-Me | i-Pr | 4-F | H |
| n-Pr | 3-Me | 5-Me | i-Pr | 4-Br | H |
| n-Pr | 3-Cl | 4-Cl | i-Pr | 4-MeO | H |
| n-Pr | 3-MeO | 4-MeO | i-Pr | 4-EtO | H |
| n-Pr | 3-MeO | 5-MeO | i-Pr | 4-$CF_3$ | H |
| n-Pr | H | H | i-Pr | 4-$CF_3CH_2O$ | H |
| n-Bu | H | H | i-Pr | 3-Me | 4-Me |
| n-Bu | 4-Me | H | i-Pr | 3-Me | 5-Me |
| n-Bu | 4-Et | H | i-Pr | 3-Cl | 4-Cl |
| n-Bu | 4-n-Pr | H | i-Pr | 3-MeO | 4-MeO |
| n-Bu | 4-i-Pr | H | i-Pr | 3-MeO | 5-MeO |
| n-Bu | 4-Cl | H | H | 4-TMS | H |
| n-Bu | 4-F | H | H | 4-I | H |
| n-Bu | 4-Br | H | H | 4-t-BuO | H |
| n-Bu | 4-MeO | H | H | 4-$CF_3(CH_2)_3O$ | H |
| n-Bu | 4-EtO | H | n-Bu | 4-$CF_3$ | H |
| n-Bu | 4-$CF_3$ | H | n-Bu | 4-$CF_3CH_2O$ | H |
| n-Bu | 4-$CF_3CH_2O$ | H | n-Bu | 3-Me | H |
| n-Bu | 3-Me | H | n-Bu | 3-Et | H |
| n-Bu | 3-Et | H | n-Bu | 3-n-Pr | H |
| n-Bu | 3-n-Pr | H | n-Bu | 3-Cl | H |
| n-Bu | 3-Cl | H | n-Bu | 3-F | H |
| n-Bu | 3-F | H | n-Bu | 3-MeO | H |
| n-Bu | 3-MeO | H | n-Bu | 3-EtO | H |
| n-Bu | 3-EtO | H | n-Bu | 3-$CF_3$ | H |
| n-Bu | 3-$CF_3$ | H | n-Bu | 3-$CF_3CH_2O$ | H |
| n-Bu | 3-$CF_3CH_2O$ | H | i-Pr | H | H |
| i-Pr | H | H | i-Pr | 4-Me | H |
| i-Pr | 4-Me | H | i-Pr | 4-Et | H |
| i-Pr | 4-Et | H | i-Pr | 4-n-Pr | H |
| i-Pr | 4-n-Pr | H | i-Pr | 4-i-Pr | H |

$R^2$ is H; $R^3$ is Me; $R^4$ is H; $R^7$ is H; $R^{18}$ is H

| $R^1$ | $R^5$ | $R^6$ | $R^1$ | $R^5$ | $R^6$ |
|---|---|---|---|---|---|
| i-Pr | 4-Cl | H | H | 4-$(CF_3)_2CH$ | H |
| i-Pr | 4-F | H | H | 4-$CH_3CHClCH$ | H |
| i-Pr | 4-Br | H | Me | 4-TMS | H |
| i-Pr | 4-MeO | H | Me | 4-I | H |
| i-Pr | 4-EtO | H | Me | 4-t-BuO | H |
| i-Pr | 4-$CF_3$ | H | Me | 4-$CF_3(CH_2)_3O$ | H |
| i-Pr | 4-$CF_3CH_2O$ | H | H | 4-MeS | H |
| i-Pr | 3-Me | 4-Me | H | 4-EtS | H |
| i-Pr | 3-Me | 5-Me | H | 4-MeS(O) | H |
| i-Pr | 3-Cl | 4-Cl | H | 4-i-PrS(O) | H |
| i-Pr | 3-MeO | 4-MeO | H | 4-MeS$(O)_2$ | H |
| i-Pr | 3-MeO | 5-MeO | H | 4-$CH_2$=CH | H |
| H | 4-TMS | H | H | 4-$CH_2$=C$(CH_3)CH_2$) | H |
| H | 4-I | H | H | 4-$CH_2$=$CHCH_2O$ | H |
| H | 4-t-BuO | H | H | 4-$MeOCH_2CH_2$ | H |
| H | 4-$CF_3(CH_2)_3O$ | H | H | 4-$MeOCH_2O$ | H |
| H | 4-$CF_3(CF_2)_2$ | H | | | |

$R^2$ is H; $R^3$ is H; $R^4$ is H; $R^7$ is H; $R^{18}$ is H

| $R^1$ | $R^5$ | $R^6$ | $R^1$ | $R^5$ | $R^6$ |
|---|---|---|---|---|---|
| H | H | H | H | 4-Br | H |
| H | 4-$NMe_2$ | H | H | 4-F | H |
| H | 4-Me | H | H | 4-OH | H |
| H | 4-Et | H | H | 4-MeO | H |
| H | 4-n-Pr | H | H | 4-EtO | H |
| H | 4-i-Pr | H | H | 4-$CF_3$ | H |
| H | 4-n-Bu | H | H | 4-$CF_3CH_2O$ | H |
| H | 4-sec-Bu | H | Me | H | H |
| H | 4-i-Bu | H | Me | 4-Me | H |
| H | 4-t-Bu | H | Me | 4-Et | H |
| H | 4-Cl | H | Me | 4-i-Pr | H |

$R^2$ is H; $R^3$ is H; $R^4$ is H; $R^7$ is H; $R^{18}$ is H

| $R^1$ | $R^5$ | $R^6$ | $R^1$ | $R^5$ | $R^6$ |
|---|---|---|---|---|---|
| Me | 4-Cl | H | Et | 4-i-Pr | H |
| Me | 4-MeO | H | Et | 4-Cl | H |
| Me | 4-EtO | H | Et | 4-MeO | H |
| Me | 4-$CF_3$ | H | Et | 4-EtO | H |
| Et | H | H | Et | 4-$CF_3$ | H |
| H | 3-$NMe_2$ | H | H | 2-Me | H |
| H | 3-Me | H | H | 2-Et | H |
| H | 3-Et | H | H | 2-Cl | H |
| H | 3-n-Pr | H | H | 2-F | H |
| H | 3-i-Pr | H | H | 2-OH | H |
| H | 3-n-Bu | H | Me | 2-Me | H |
| H | 3-Cl | H | Me | 2-Cl | H |
| H | 3-Br | H | Me | 2-F | H |
| H | 3-F | H | Et | 2-Me | H |
| H | 3-OH | H | Et | 2-Cl | H |
| H | 3-MeO | H | Et | 2-F | H |
| H | 3-EtO | H | H | 2-Me | 4-Me |
| H | 3-$CF_3$ | H | H | 2-Me | 5-Me |
| H | 3-$CF_3CH_2O$ | H | H | 3-Me | 4-Me |
| Me | 3-Me | H | H | 2-Et | 4-Et |
| Me | 3-Et | H | H | 2-Et | 5-Et |
| Me | 3-i-Pr | H | H | 3-Et | 4-Et |
| Me | 3-Cl | H | H | 2-Me | 5-t-Bu |
| Me | 3-MeO | H | H | 2-Cl | 4-Cl |
| Me | 3-EtO | H | H | 2-Cl | 5-Cl |
| Me | 3-$CF_3$ | H | Et | 3-MeO | H |
| Et | 3-Me | H | Et | 3-EtO | H |
| Et | 3-Et | H | Et | 3-$CF_3$ | H |
| Et | 3-i-Pr | H | Me | 2-Me | 4-Me |
| Et | 3-Cl | H | Me | 2-Me | 5-Me |
| Et | 4-Me | H | Me | 3-Me | 4-Me |
| Et | 4-Et | H | Me | 2-Et | 4-Et |

$R^2$ is H; $R^3$ is H; $R^4$ is H; $R^7$ is H; $R^{18}$ is H

| $R^1$ | $R^5$ | $R^6$ | $R^1$ | $R^5$ | $R^6$ |
|---|---|---|---|---|---|
| Me | 2-Et | 5-Et | Et | 2-MeO | 4-MeO |
| Me | 3-Et | 4-Et | Et | 3-MeO | 5-MeO |
| Me | 2-Me | 5-t-Bu | Et | 3-MeO | 4-MeO |
| Et | 2-Me | 4-Me | H | 3-Br | 5-Br |
| Et | 2-Me | 5-Me | Me | 3-Br | 5-Br |
| Et | 3-Me | 4-Me | Et | 3-Br | 5-Br |
| Et | 2-Et | 4-Et | H | 3-Me | 5-Me |
| Et | 2-Et | 5-Et | Me | 3-Me | 5-Me |
| Et | 3-Et | 4-Et | Et | 3-Me | 5-Me |
| H | 4-Ph | H | H | 3-Cl | 4-MeO |
| H | 4-PhO | H | Me | 3-Cl | 4-MeO |
| H | 4-c-Hex | H | Et | 3-Cl | 4-MeO |
| H | 4-Hex | H | Me | 4-$NMe_2$ | H |
| H | 4-n-Amyl | H | Me | 3-$NMe_2$ | H |
| Me | 4-Ph | H | Et | 4-$NMe_2$ | H |
| Me | 4-PhO | H | Et | 3-$NMe_2$ | H |
| Me | 4-c-Hex | H | H | 3-$NH_2$ | H |
| Me | 4-Hex | H | H | 4-$NH_2$ | H |
| Me | 4-n-Amyl | H | Me | 3-$NH_2$ | H |
| H | 3-Cl | 4-Cl | Me | 4-$NH_2$ | H |
| Me | 2-Cl | 4-Cl | Et | 3-$NH_2$ | H |
| Me | 2-Cl | 5-Cl | Et | 4-$NH_2$ | H |
| Me | 3-Cl | 4-Cl | n-Pr | 4-$NMe_2$ | H |
| Et | 2-Cl | 4-Cl | n-Pr | 4-Me | H |
| Et | 2-Cl | 5-Cl | n-Pr | 4-Et | H |
| Et | 3-Cl | 4-Cl | n-Pr | 4-n-Pr | H |
| H | 2-MeO | 4-MeO | n-Pr | 4-Cl | H |
| H | 3-MeO | 5-MeO | n-Pr | 4-F | H |
| H | 3-MeO | 4-MeO | n-Pr | 4-Br | H |
| Me | 2-MeO | 4-MeO | n-Pr | 4-MeO | H |
| Me | 3-MeO | 5-MeO | n-Pr | 4-EtO | H |
| Me | 3-MeO | 4-MeO | n-Pr | 4-CF3 | H |

$R^2$ is H; $R^3$ is H; $R^4$ is H; $R^7$ is H; $R^{18}$ is H

| $R^1$ | $R^5$ | $R^6$ | $R^1$ | $R^5$ | $R^6$ |
|---|---|---|---|---|---|
| n-Pr | 4-$CF_3CH_2O$ | H | n-Bu | 3-n-Pr | H |
| n-Pr | 3-$NMe_2$ | H | n-Bu | 3-Cl | H |
| n-Pr | 3-Me | H | n-Bu | 3-F | H |
| n-Pr | 3-Et | H | n-Bu | 3-MeO | H |
| n-Pr | 3-n-Pr | H | n-Bu | 3-EtO | H |
| n-Pr | 3-Cl | H | n-Bu | 3-$CF_3$ | H |
| n-Pr | 3-F | H | n-Bu | 3-$CF_3CH_2O$ | H |
| n-Pr | 3-Br | H | i-Pr | H | H |
| n-Pr | 3-MeO | H | i-Pr | 4-Me | H |
| n-Pr | 3-EtO | H | i-Pr | 4-Et | H |
| n-Pr | 3-$CF_3$ | H | i-Pr | 4-n-Pr | H |
| n-Pr | 3-$CF_3CH_2O$ | H | i-Pr | 4-i-Pr | H |
| n-Pr | 3-Me | 4-Me | i-Pr | 4-Cl | H |
| n-Pr | 3-Me | 5-Me | i-Pr | 4-F | H |
| n-Pr | 3-Cl | 4-Cl | i-Pr | 4-Br | H |
| n-Pr | 3-MeO | 4-MeO | i-Pr | 4-MeO | H |
| n-Pr | 3-MeO | 5-MeO | i-Pr | 4-EtO | H |
| n-Pr | H | H | i-Pr | 4-$CF_3$ | H |
| n-Bu | H | H | i-Pr | 4-$CF_3CH_2O$ | H |
| n-Bu | 4-Me | H | i-Pr | 3-Me | 4-Me |
| n-Bu | 4-Et | H | i-Pr | 3-Me | 5-Me |
| n-Bu | 4-n-Pr | H | i-Pr | 3-Cl | 4-Cl |
| n-Bu | 4-i-Pr | H | i-Pr | 3-MeO | 4-MeO |
| n-Bu | 4-Cl | H | i-Pr | 3-MeO | 5-MeO |
| n-Bu | 4-F | H | H | 4-TMS | H |
| n-Bu | 4-Br | H | H | 4-I | H |
| n-Bu | 4-MeO | H | H | 4-t-BuO | H |
| n-Bu | 4-EtO | H | H | 4-$CF_3(CH_2)_3O$ | H |
| n-Bu | 4-$CF_3$ | H | H | 4-$CF_3(CF_2)_2$ | H |
| n-Bu | 4-$CF_3CH_2O$ | H | H | 4-$(CF_3)_2CH$ | H |
| n-Bu | 3-Me | H | H | 4-$CH_3CHClCH$ | H |
| n-Bu | 3-Et | H | Me | 4-TMS | H |

$R^2$ is H; $R^3$ is H; $R^4$ is H; $R^7$ is H; $R^{18}$ is H

| $R^1$ | $R^5$ | $R^6$ | $R^1$ | $R^5$ | $R^6$ |
|---|---|---|---|---|---|
| Me | 4-I | H | H | 4-MeS(O)$_2$ | H |
| Me | 4-*t*-BuO | H | H | 4-$CH_2$=CH | H |
| Me | 4-$CF_3(CH_2)_3O$ | H | H | 4-$CH_2$=C($CH_3$)$CH_2$) | H |
| H | 4-MeS | H | H | 4-$CH_2$=$CHCH_2O$ | H |
| H | 4-EtS | H | H | 4-$MeOCH_2CH_2$ | H |
| H | 4-MeS(O) | H | H | 4-$MeOCH_2O$ | H |
| H | 4-*i*-PrS(O) | H | | | |

$R^3$ is H; $R^4$ is H; $R^7$ is H; $R^{18}$ is H

| $R^1$ | $R^2$ | $R^5$ | $R^6$ | $R^1$ | $R^2$ | $R^5$ | $R^6$ |
|---|---|---|---|---|---|---|---|
| Me | 4-Me | H | H | Me | 4-Et | 4-Et | H |
| Me | 4-Me | 4-Me | H | Me | 4-Et | 4-*i*-Pr | H |
| Me | 4-Me | 4-Cl | H | Me | 4-Et | 3-Me | H |
| Me | 4-Me | 4-MeO | H | Me | 4-Et | 3-Cl | H |
| Me | 4-Me | 4-EtO | H | Me | 4-Et | 3-MeO | H |
| Me | 4-Me | 4-Et | H | Me | 4-Et | 3-EtO | H |
| Me | 4-Me | 4-*i*-Pr | H | Me | 4-Et | 3-Et | H |
| Me | 4-Me | 3-Me | H | Me | 4-Et | 3-*i*-Pr | H |
| Me | 4-Me | 3-Cl | H | Et | 4-Et | H | H |
| Me | 4-Me | 3-MeO | H | Et | 4-Et | 4-Me | H |
| Me | 4-Me | 3-EtO | H | Et | 4-Et | 4-Cl | H |
| Me | 4-Me | 3-Et | H | Et | 4-Et | 4-MeO | H |
| Me | 4-Me | 3-*i*-Pr | H | Et | 4-Et | 4-EtO | H |
| Me | 4-Et | H | H | Et | 4-Et | 4-Et | H |
| Me | 4-Et | 4-Me | H | Et | 4-Et | 4-*i*-Pr | H |
| Me | 4-Et | 4-Cl | H | Me | 4-Me | 3-Me | 4-Me |
| Me | 4-Et | 4-MeO | H | Me | 4-Me | 3-Me | 5-Me |
| Me | 4-Et | 4-EtO | H | Me | 4-Me | 3-Cl | 4-Cl |

$R^3$ is H; $R^4$ is H; $R^7$ is H; $R^{18}$ is H

| $R^1$ | $R^2$ | $R^5$ | $R^6$ | $R^1$ | $R^2$ | $R^5$ | $R^6$ |
|---|---|---|---|---|---|---|---|
| Me | 4-Me | 3-Cl | 5-Cl | H | 6-OH | 4-Me | H |
| Me | 4-Me | 3-MeO | 4-MeO | H | 6-OMe | 3-Me | H |
| Me | 4-Me | 3-MeO | 5-MeO | H | 6-OMe | 3-Me | 4-Me |
| H | 6-OH | H | H | H | 6-OEt | 4-Cl | H |
| H | 6-OMe | H | H | H | 5-OMe | 4-F | H |
| H | 6-OEt | H | H | H | 5-OMe | 3-Cl | H |
| H | 6-OC(O)Me | H | H | H | 5-OMe | 4-Cl | H |
| H | 5-OH | H | H | H | 5-Br | 4-Cl | H |
| H | 5-OMe | H | H | Me | 6-OH | H | H |
| H | 5-OEt | H | H | Me | 6-OMe | H | H |
| H | 5-Br | H | H | Me | 4-n-Pr | H | H |
| H | 5-Me | H | H | Et | 4-n-Pr | H | H |
| H | 6-Me | H | H | | | | |

$R^3$ is Me; $R^4$ is H; $R^7$ is H; $R^{18}$ is H

| $R^1$ | $R^2$ | $R^5$ | $R^6$ | $R^1$ | $R^2$ | $R^5$ | $R^6$ |
|---|---|---|---|---|---|---|---|
| Me | 4-Me | H | H | Me | 4-Et | 4-Me | H |
| Me | 4-Me | 4-Me | H | Me | 4-Et | 4-Cl | H |
| Me | 4-Me | 4-Cl | H | Me | 4-Et | 4-MeO | H |
| Me | 4-Me | 4-MeO | H | Me | 4-Et | 4-EtO | H |
| Me | 4-Me | 4-EtO | H | Me | 4-Et | 4-Et | H |
| Me | 4-Me | 4-Et | H | Me | 4-Et | 4-i-Pr | H |
| Me | 4-Me | 4-i-Pr | H | Me | 4-Et | 3-Me | H |
| Me | 4-Me | 3-Me | H | Me | 4-Et | 3-Cl | H |
| Me | 4-Me | 3-Cl | H | Me | 4-Et | 3-MeO | H |
| Me | 4-Me | 3-MeO | H | Me | 4-Et | 3-EtO | H |
| Me | 4-Me | 3-EtO | H | Me | 4-Et | 3-Et | H |
| Me | 4-Me | 3-Et | H | Me | 4-Et | 3-i-Pr | H |
| Me | 4-Me | 3-i-Pr | H | Et | 4-Et | H | H |
| Me | 4-Et | H | H | Et | 4-Et | 4-Me | H |

$R^3$ is Me; $R^4$ is H; $R^7$ is H; $R^{18}$ is H

| $R^1$ | $R^2$ | $R^5$ | $R^6$ | $R^1$ | $R^2$ | $R^5$ | $R^6$ |
|---|---|---|---|---|---|---|---|
| Et | 4-Et | 4-Cl | H | H | 5-OEt | H | H |
| Et | 4-Et | 4-MeO | H | H | 5-Br | H | H |
| Et | 4-Et | 4-EtO | H | H | 5-Me | H | H |
| Et | 4-Et | 4-Et | H | H | 6-Me | H | H |
| Et | 4-Et | 4-i-Pr | H | H | 6-OH | 4-Me | H |
| Me | 4-Me | 3-Me | 4-Me | H | 6-OMe | 3-Me | H |
| Me | 4-Me | 3-Me | 5-Me | H | 6-OMe | 3-Me | 4-Me |
| Me | 4-Me | 3-Cl | 4-Cl | H | 6-OEt | 4-Cl | H |
| Me | 4-Me | 3-Cl | 5-Cl | H | 5-OMe | 4-F | H |
| Me | 4-Me | 3-MeO | 4-MeO | H | 5-OMe | 3-Cl | H |
| Me | 4-Me | 3-MeO | 5-MeO | H | 5-OMe | 4-Cl | H |
| H | 6-OH | H | H | H | 5-Br | 4-Cl | H |
| H | 6-OMe | H | H | Me | 6-OH | H | H |
| H | 6-OEt | H | H | Me | 6-OMe | H | H |
| H | 6-OC(O)Me | H | H | Me | 4-n-Pr | H | H |
| H | 5-OH | H | H | Et | 4-n-Pr | H | H |
| H | 5-OMe | H | H | | | | |

$R^3$ is Me; $R^4$ is Me; $R^7$ is H; $R^{18}$ is H

| $R^1$ | $R^2$ | $R^5$ | $R^6$ | $R^1$ | $R^2$ | $R^5$ | $R^6$ |
|---|---|---|---|---|---|---|---|
| Me | 4-Me | H | H | Me | 4-Me | 3-EtO | H |
| Me | 4-Me | 4-Me | H | Me | 4-Me | 3-Et | H |
| Me | 4-Me | 4-Cl | H | Me | 4-Me | 3-i-Pr | H |
| Me | 4-Me | 4-MeO | H | Me | 4-Et | H | H |
| Me | 4-Me | 4-EtO | H | Me | 4-Et | 4-Me | H |
| Me | 4-Me | 4-Et | H | Me | 4-Et | 4-Cl | H |
| Me | 4-Me | 4-i-Pr | H | Me | 4-Et | 4-MeO | H |
| Me | 4-Me | 3-Me | H | Me | 4-Et | 4-EtO | H |
| Me | 4-Me | 3-Cl | H | Me | 4-Et | 4-Et | H |
| Me | 4-Me | 3-MeO | H | Me | 4-Et | 4-i-Pr | H |

$R^3$ is Me; $R^4$ is Me; $R^7$ is H; $R^{18}$ is H

| $R^1$ | $R^2$ | $R^5$ | $R^6$ |
|---|---|---|---|
| Me | 4-Et | 3-Me | H |
| Me | 4-Et | 3-Cl | H |
| Me | 4-Et | 3-MeO | H |
| Me | 4-Et | 3-EtO | H |
| Me | 4-Et | 3-Et | H |
| Me | 4-Et | 3-i-Pr | H |
| Et | 4-Et | H | H |
| Et | 4-Et | 4-Me | H |
| Et | 4-Et | 4-Cl | H |
| Et | 4-Et | 4-MeO | H |
| Et | 4-Et | 4-EtO | H |
| Et | 4-Et | 4-Et | H |
| Et | 4-Et | 4-i-Pr | H |
| Me | 4-Me | 3-Me | 4-Me |
| Me | 4-Me | 3-Me | 5-Me |
| Me | 4-Me | 3-Cl | 4-Cl |
| Me | 4-Me | 3-Cl | 5-Cl |
| Me | 4-Me | 3-MeO | 4-MeO |
| Me | 4-Me | 3-MeO | 5-MeO |
| H | 6-OH | H | H |
| H | 6-OMe | H | H |

| $R^1$ | $R^2$ | $R^5$ | $R^6$ |
|---|---|---|---|
| H | 6-OEt | H | H |
| H | 6-OC(O)Me | H | H |
| H | 5-OH | H | H |
| H | 5-OMe | H | H |
| H | 5-OEt | H | H |
| H | 5-Br | H | H |
| H | 5-Me | H | H |
| H | 6-Me | H | H |
| H | 6-OH | 4-Me | H |
| H | 6-OMe | 3-Me | H |
| H | 6-OMe | 3-Me | 4-Me |
| H | 6-OEt | 4-Cl | H |
| H | 5-OMe | 4-F | H |
| H | 5-OMe | 3-Cl | H |
| H | 5-OMe | 4-Cl | H |
| H | 5-Br | 4-Cl | H |
| Me | 6-OH | H | H |
| Me | 6-OMe | H | H |
| Me | 4-n-Pr | H | H |
| Et | 4-n-Pr | H | H |

$R^2$ is H; $R^3$ is Me; $R^4$ is Me; $R^{18}$ is H

| $R^1$ | $R^5$ | $R^6$ | $R^7$ |
|---|---|---|---|
| H | 3-Me | 4-Me | 5-Me |
| H | 3-Br | 4-Me | 5-Br |
| H | 3-Cl | 4-MeO | 5-Cl |
| H | 3-MeO | 4-MeO | 5-MeO |

$R^2$ is H; $R^3$ is Me; $R^4$ is H; $R^{18}$ is H

| $R^1$ | $R^5$ | $R^6$ | $R^7$ |
|---|---|---|---|
| Me | 3-Me | 4-Me | 5-Me |
| Me | 3-Br | 4-Me | 5-Br |
| Me | 3-Cl | 4-MeO | 5-Cl |
| Me | 3-MeO | 4-MeO | 5-MeO |

$R^2$ is H; $R^3$ is Me; $R^4$ is Me; $R^{18}$ is H

| $R^1$ | $R^5$ | $R^6$ | $R^7$ |
|---|---|---|---|
| H | 4-TMS | H | H |
| Me | 4-TMS | H | H |
| Et | 4-TMS | H | H |
| Et | 3-Me | 4-Me | 5-Me |
| Et | 3-MeO | 4-MeO | 5-MeO |
| H | 2-Cl | 5-Br | H |
| Me | 2-Cl | 5-Br | H |
| H | 3-Me | 4-Me | 5-Me |
| H | 3-Br | 4-Me | 5-Br |
| H | 3-Cl | 4-MeO | 5-Cl |
| H | 3-MeO | 4-MeO | 5-MeO |

$R^2$ is H; $R^3$ is Me; $R^4$ is H; $R^{18}$ is H

| $R^1$ | $R^5$ | $R^6$ | $R^7$ |
|---|---|---|---|
| Me | 3-Me | 4-Me | 5-Me |
| Me | 3-Br | 4-Me | 5-Br |
| Me | 3-Cl | 4-Me | 5-Cl |
| Me | 3-MeO | 4-MeO | 5-MeO |
| H | 4-TMS | H | H |
| Me | 4-TMS | H | H |
| Et | 4-TMS | H | H |
| Et | 3-Me | 4-Me | 5-Me |
| Et | 3-Me | 4-MeO | 5-MeO |
| H | 2-Cl | 5-Br | H |
| Me | 2-Cl | 5-Br | H |

$R^4$ is Me; $R^6$, $R^2$, $R^{18}$ and $R^7$ are H

| $R^1$ | $R^3$ | $R^5$ |
|---|---|---|
| Me | MeC≡C | 4-Me |
| Me | MeC≡C | 4-$CF_3CH_2O$ |
| Me | Cl | 3-$CF_3$ |
| Me | $CF_2Cl$ | 4-MeO |
| i-Pr | $CF_3$ | H |
| i-Pr | sec-Bu | 2-F |
| i-Pr | $CF_3$ | 3-Cl |
| i-Pr | $CF_3$ | 3-Me |
| i-Pr | $CF_3$ | 4-$CF_3CH_2O$ |
| i-Pr | Et | 3-$CF_3$ |

$R^2$, $R^{18}$, $R^1$, $R^6$ and $R^7$ are H

| $R^3$ | $R^4$ | $R^5$ |
|---|---|---|
| i-Pr | MeO | 4-MeO |
| Et | c-Pr | H |
| Et | MeC≡C | 3-F |
| Et | $CH_2F$ | 4-Cl |
| Et | $CF_3CH_2O$ | 4-Me |
| Et | i-Pr | 4-$CF_3CH_2O$ |
| Et | n-Bu | 3-$CF_3$ |
| Et | HC≡$CCH_2O$ | 4-MeO |
| t-Bu | Br | 4-Cl |
| Ph | $CF_3(CF_2)_3$ | 4-Me |

$R^4$ is Me; $R^6$, $R^2$, $R^{18}$ and $R^7$ are H

| $R^1$ | $R^3$ | $R^5$ |
|---|---|---|
| Bzl | 4-*sec*-BuS | 4-$CF_3CH_2O$ |
| H | $NH_2$ | 4-Me |
| 4-*c*-Pr | $CH_3OCH_2$ | 4-Me |
| 4-*c*-Pr | $CF_3CH_2O$ | 4-$CF_3CH_2O$ |
| 4-*c*-Pr | MeS | 4-$CF_3$ |
| 4-*c*-Pr | $CH_2$=C(Et) | 4-MeO |
| 4-*c*-Pr | $CH_2$=$CHCH_2$ | H |
| 4-*c*-Pr | *t*-BuO | 4-F |
| 4-*c*-Pr | $HCF_2O$ | 2-Cl |
| 4-*c*-Pr | $CH_2$=$CHCH_2O$ | 4-Me |
| 4-*c*-Pr | MeC≡$CCH_2O$ | 4-$CF_3CH_2O$ |
| 4-*c*-Pr | $NMe_2$ | 3-$CF_3$ |
| 4-*c*-Pr | NHEt | 4-MeO |

$R^2$ is H; $R^4$ is Me; $R^6$, $R^{18}$ and $R^7$ are H

| $R^1$ | $R^3$ | $R^5$ |
|---|---|---|
| Me | MeC≡C | H |
| Me | MeC≡C | F |
| Me | MeC≡C | Cl |

$R^2$, $R^{18}$, $R^1$, $R^6$ and $R^7$ are H

| $R^3$ | $R^4$ | $R^5$ |
|---|---|---|
| Cl | Cl | H |
| Cl | Cl | 2-F |
| Cl | Cl | 3-Cl |
| Cl | Cl | 4-Me |
| $CH_3C$≡C | Cl | 4-$CF_3CH_2O$ |
| $CH_3C$≡C | F | 3-$CF_3$ |
| $CH_3C$≡C | $CH_3OCH_2$ | 4-MeO |
| $OCF_3$ | *sec*-Bu | 4-Cl |
| $OCF_3$ | Br | 4-Me |
| $OCF_3$ | *i*-Pr | 4-$CF_3CH_2O$ |
| NH2 | NH2 | 4-Me |
| NH2 | NH2 | 4-Cl |
| NHMe | NHMe | 4-MeO |

$R^{18}$, $R^2$, $R^1$, $R^6$ and $R^7$ are H

| $R^3$ | $R^4$ | $R^5$ |
|---|---|---|
| *c*-Pr | *c*-Pr | H |
| *c*-Pr | *c*-Pr | 4-F |
| *c*-Pr | *c*-Pr | 4-Cl |
| *c*-Pr | *c*-Pr | 4-Me |
| *c*-Pr | $CH_3C$≡C | 4-$CF_3CH_2O$ |
| *c*-Pr | $CH_3C$≡C | 3-CF3 |
| *c*-Pr | $CH_3C$≡C | 3-MeO |
| *c*-Pr | $CF_3$ | H |
| *c*-Pr | $CF_3$ | 2-F |
| *c*-Pr | $CF_3$ | 3-Cl |

TABLE 6

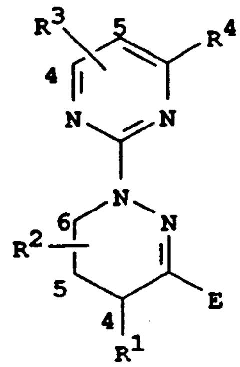

| $R^1$, $R^2$, and $R^3$ are H; $R^4$ is Me | $R^1$ and $R^2$ are H; $R^3$ is 4-Me; $R^4$ is Me |
|---|---|
| E | E |
| 1-naphthalenyl | 1-naphthalenyl |
| 2-furanyl | 2-furanyl |
| 2-naphthalenyl | 2-naphthalenyl |
| 3-thienyl | 3-thienyl |
| 2,5-dimethyl-3-furanyl | 2,5-dimethyl-3-furanyl |
| 2,5-dimethyl-3-thienyl | 2,5-dimethyl-3-thienyl |
| 4-methylphenoxy | 4-methylphenoxy |
| 2-chlorophenoxy | 2-chlorophenoxy |
| 2,6-dimethylphenoxy | 2,6-dimethylphenoxy |
| 3-methylphenylthio | 4-cyanophenylthio |
| phenylamino | 4-methylphenylamino |
| benzyl | Cl |
| Et | n-hex |
| sec-Bu | Me |
| c-propyl | c-hexyl |
| cis-2-methylcycloheptyl | $CF_3CH_2CH_2$ |
| sec-butylthio | n-butoxy |
| $CF_3CH_2O$ | $Cl(CH_2)_5O$ |
| 5-methyl-2-thienyl | 4-methyl-3-furanyl |
| 5-methyl-2-pyridyl | 2-methyl-3-pyridyl |

$R^1$, $R^2$ and $R^3$ are H; $R^4$ is Me

| E |
|---|
| 4-pyridyl |
| 2-indanyl |
| 2-tetrahydronaphthalenyl |
| 6-Me-3-pyridyl |
| 2-pyridyl |

$R^1$, $R^2$, $R^3$ and $R^4$ are H

| E |
|---|
| 1-naphthalenyl |
| 2-furanyl |
| 3-thienyl |
| 3-pyridyl |

$R^3$ is 4-Me; $R^4$ is Me

| $R^1$ | $R^2$ | E |
|---|---|---|
| H | 5-Me | Ph |
| H | 5-*i*-Pr | 2-Me-Ph |
| H | 5-*n*-Bu | 2-Cl-Ph |
| H | 5-CN | 2-MeO-Ph |
| H | 5-$CF_3$ | $CF_3CH_2O$-Ph |
| H | 6-$CF_3CH_2$ | 1-naphthalenyl |
| *i*-Pr | 5-Me | Ph |
| *i*-Pr | 5-Me | 2-Me-Ph |

$R^1$ and $R^2$ are H; $R^3$ is 4-Me; $R^4$ is Me

| E |
|---|
| 4-chloro-3-pyridyl |
| 2-indanyl |
| 2-tetrahydronaphthalenyl |
| 6-Me-3-pyridyl |
| 2-pyridyl |

$R^1$ and $R^4$ are Me; $R^3$ is 4-Me; $R^2$ is H

| E |
|---|
| 1-naphthalenyl |
| 2-furanyl |
| 3-thienyl |
| 3-pyridyl |

$R^3$ is 4-Me; $R^4$ is Me

| $R^1$ | $R^2$ | E |
|---|---|---|
| H | 5-Et | Ph |
| H | 5-*sec*-Bu | 2-Me-Ph |
| H | 5-$CF_3(CF_2)_3$ | 2-Cl-Ph |
| H | 5-*t*-Bu | 2-MeO-Ph |
| H | 5-$FCH_2$ | 2-$CF_3CH_2O$-Ph |
| H | 6-*n*-Pr | 1-naphthalenyl |
| Me | 4-Me | Ph |
| Me | 4-Me | 2-Me-Ph |

$R^3$ is 4-Me; $R^4$ is Me

| $R^1$ | $R^2$ | E |
|---|---|---|
| *i*-Pr | 5-Me | 2-Cl-Ph |
| *i*-Pr | 5-Me | 2-MeO-Ph |
| *i*-Pr | 6-Me | 2-$CF_3CH_2O$-Ph |
| Cl | H | Ph |
| F | H | 4-Me-Ph |
| $CF_3CF_2$ | H | 4-Cl-Ph |
| $CH_2$=$CHCH_2$ | H | 4-MeO-Ph |

$R^3$ is 4-Me; $R^4$ is Me

| $R^1$ | $R^2$ | E |
|---|---|---|
| $CO_2Me$ | H | 2-$CF_3CH_2O$-Ph |
| 2-Me-Ph | H | Me |
| Bzl | H | Ph |
| 2-naphthalenyl | H | *n*-Bu |
| 3-thienyl | H | $CF_3CF_2$ |
| 3-pyridyl | H | Me |
| CN | 5-Me | Ph |
| *t*-Bu | 5-Me | 2-Me-Ph |
| $ClCH_2$ | 5-Me | 2-Cl-Ph |
| Et | 5-Me | 2-MeO-Ph |
| *n*-Pr | 5-Me | 2-$CF_3CH_2O$-Ph |
| Me | 4-Me | 2-$CF_3$-Ph |
| *i*-Pr | 4-Me | 2-$CF_3$-Ph |
| $CF_3$ | 4-$CF_3$ | 2-$CF_3$-Ph |

$R^3$ is 4-Me; $R^4$ is Me

| $R^1$ | $R^2$ | E |
|---|---|---|
| Me | 4-Me | 2-TMS-Ph |
| Me | 4-Me | 2-Cl-Ph |
| Me | 4-Me | 2-MeO-Ph |
| Me | 4-Me | 2-$CF_3CH_2O$-Ph |
| Br | H | Ph |
| CN | H | 4-Me-Ph |
| Ac | H | 4-Cl-Ph |
| $CH_3C{\equiv}CCH_2$ | H | 4-MeO-Ph |

$R^3$ is 4-Me; $R^4$ is Me

| $R^1$ | $R^2$ | E |
|---|---|---|
| $CO_2Et$ | H | 2-$CF_3CH_2O$-Ph |
| 4-Cl-Ph | H | Ph |
| 5-Me-3-furyl | H | i-Pr |
| EtCO | H | 2-Cl-Ph |
| 2-furyl | 4-Me | $CF_3$ |
| Ph | 5-Me | Me |
| CN | 4-Me | Ph |
| t-Bu | 4-Me | 2-Me-Ph |
| $FCH_2$ | 4-Me | 2-Cl-Ph |
| Et | 4-Me | 2-MeO-Ph |
| $Cl(CH_2)_4$ | 4-Me | 2-$CF_3CH_2O$-Ph |
| Me | 4-Me | 2-$CF_3$-Ph |
| i-Pr | 5-CN | 2-$CF_3$-Ph |
| $CF_3$ | 5-Me | 2-$CF_3$-Ph |
| i-Pr | 4-Me | 2-TMS-Ph |

TABLE 7

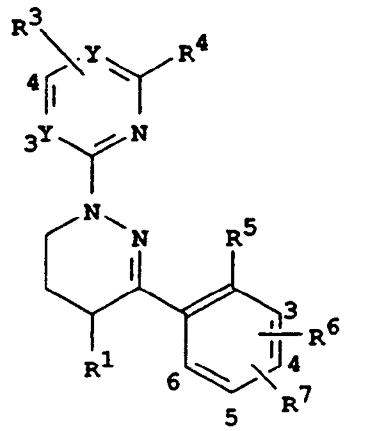

$R^7$ is H; $R^3$ is H; $R^4$ is H; Y is CH

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |

$R^7$ is H; $R^3$ is H; $R^4$ is H; Y is CH

| $R^1$ | $R^5$ | $R^6$ | $R^1$ | $R^5$ | $R^6$ |
|---|---|---|---|---|---|
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $HCF_2O$ | H | H | $HCF_2O$ | 6-$HCF_2O$ |
| H | Br | H | H | I | H |
| H | t-BuO | H | H | EtO | H |

$R^7$ is H; $R^3$ is H; $R^4$ is Me; Y is CH

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |

$R^7$ is H; $R^3$ is H; $R^4$ is Me; Y is CH

| $R^1$ | $R^5$ | $R^6$ | $R^1$ | $R^5$ | $R^6$ |
|---|---|---|---|---|---|
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $HCF_2O$ | H | H | $HCF_2O$ | 6-$HCF_2O$ |
| H | Br | H | Me | I | H |
| H | tBuO | H | Me | EtO | H |

$R^7$ is H; $R^3$ is 4-Me; $R^4$ is Me; Y is N

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |

$R^7$ is H; $R^3$ is 4-Me; $R^4$ is Me; Y is N

| $R^1$ | $R^5$ | $R^6$ |
|---|---|---|
| Me | $CF_3CH_2O$ | 4-F |
| Me | $CF_3$ | 4-F |
| Me | MeO | 4-F |
| H | H | 3-$CF_3$ |
| H | F | 6-F |
| H | Cl | 6-Cl |
| H | Me | 6-Me |
| H | $CF_3CH_2O$ | 6-Me |
| H | $CF_3$ | 6-Me |
| H | MeO | 6-MeO |
| H | H | 4-Br |
| Me | F | 6-F |
| Me | Cl | 6-Cl |
| Me | Me | 6-Me |
| n-Pr | $CF_3CH_2O$ | H |
| t-Bu | $CF_3$ | H |
| sec-Bu | MeO | H |
| H | $HCF_2O$ | H |
| H | Br | H |
| H | t-BuO | H |

| $R^1$ | $R^5$ | $R^6$ |
|---|---|---|
| Me | $CF_3CH_2O$ | H |
| Me | $CF_3$ | H |
| Me | MeO | H |
| Et | H | H |
| Et | F | H |
| Et | Cl | H |
| Et | Me | H |
| Et | $CF_3CH_2O$ | H |
| Et | $CF_3$ | H |
| Et | MeO | H |
| i-Pr | H | H |
| i-Pr | F | H |
| i-Pr | Cl | H |
| i-Pr | Me | H |
| i-Pr | $CF_3CH_2O$ | H |
| i-Pr | $CF_3$ | H |
| i-Pr | MeO | H |
| H | $HCF_2O$ | 6-$HCF_2O$ |
| H | I | H |
| H | EtO | H |

$R^4$ is Me; $R^6$ and $R^7$ are H

Y is CH

| $R^1$ | $R^3$ | $R^5$ |
|---|---|---|
| H | 4-c-Pr | H |
| H | 4-c-Pr | F |
| H | 4-c-Pr | Cl |
| H | 4-c-Pr | Me |
| H | 4-c-Pr | $CF_3CH_2O$ |
| H | 4-c-Pr | $CF_3$ |
| H | 4-c-Pr | MeO |

$R^1$, $R^6$, and $R^7$ are H; Y is N

| $R^3$ | $R^4$ | $R^5$ |
|---|---|---|
| 4-c-Pr | c-Pr | H |
| 4-c-Pr | c-Pr | F |
| 4-c-Pr | c-Pr | Cl |
| 4-c-Pr | c-Pr | Me |
| 4-c-Pr | $CH_3C{\equiv}C$ | $CF_3CH_2O$ |
| 4-c-Pr | $CH_3C{\equiv}C$ | $CF_3$ |
| 4-c-Pr | $CH_3C{\equiv}C$ | MeO. |

$R^4$ is Me; $R^6$ and $R^7$ are H
Y is CH

| $R^1$ | $R^3$ | $R^5$ |
|---|---|---|
| Me | 4-MeC≡C | H |
| Me | 4-MeC≡C | F |
| Me | 4-MeC≡C | Cl |
| Me | 4-MeC≡C | Me |
| Me | 4-MeC≡C | $CF_3CH_2O$ |
| Me | 5-Cl | $CF_3$ |
| Me | 4-$CF_2Cl$ | MeO |
| i-Pr | 5-$CF_3$ | H |
| i-Pr | 4-sec-Bu | F |
| i-Pr | 4-$CF_3$ | Cl |
| i-Pr | 4-$CF_3$ | Me |
| i-Pr | 4-$CF_3$ | $CF_3CH_2O$ |
| i-Pr | 5-Et | $CF_3$ |
| i-Pr | 4-MeO | MeO |
| Et | 4-c-Pr | H |
| Et | 3-MeC≡C | F |
| Et | 4-$CH_2F$ | Cl |
| Et | 4-$CF_3CH_2O$ | Me |
| Et | 4-i-Pr | $CF_3CH_2O$ |
| Et | 4-n-Bu | $CF_3$ |
| Et | 4-HC≡$CCH_2O$ | MeO |
| t-Bu | 3-Br | Cl |
| Ph | 4-$CF_3(CF_2)_3$ | Me |
| Bzl | 4-sec-BuS | $CF_3CH_2O$ |

$R^1$, $R^6$, and $R^7$ are H; Y is N

| $R^3$ | $R^4$ | $R^5$ |
|---|---|---|
| 4-c-Pr | $CF_3$ | H |
| 4-c-Pr | $CF_3$ | F |
| 4-c-Pr | $CF_3$ | Cl |
| 4-c-Pr | $CH_3OCH_2$ | Me |
| 4-c-Pr | $CF_3CH_2O$ | $CF_3CH_2O$ |
| 4-c-Pr | MeS | $CF_3$ |
| 4-c-Pr | $CH_2$=C(Et) | MeO |
| 4-c-Pr | $CH_2$=$CHCH_2$ | H |
| 4-c-Pr | t-BuO | F |
| 4-c-Pr | $HCF_2O$ | Cl |
| 4-c-Pr | $CH_2$=$CHCH_2O$ | Me |
| 4-c-Pr | MeC≡$CCH_2O$ | $CF_3CH_2O$ |
| 4-c-Pr | $NMe_2$ | $CF_3$ |
| 4-c-Pr | NHEt | MeO |
| 4-Cl | Cl | H |
| 4-Cl | Cl | F |
| 4-Cl | Cl | Cl |
| 4-Cl | Cl | Me |
| 4-$CH_3$C≡C | Cl | $CF_3CH_2O$ |
| 4-$CH_3$C≡C | F | $CF_3$ |
| 4-$CH_3$C≡C | $CH_3OCH_2$ | MeO |
| 4-$OCF_3$ | sec-Bu | Cl |
| 4-$OCF_3$ | Br | Me |
| 4-$OCF_3$ | i-Pr | $CF_3CH_2O$ |

TABLE 8

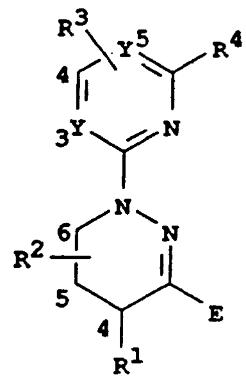

| $R^1$, $R^2$ and $R^3$ are H; $R^4$ is Me; Y is CH | $R^1$ and $R^2$ are H; $R^3$ is 4-Me; $R^4$ is Me; Y is N |
|---|---|
| E | E |
| 1-naphthalenyl | 1-naphthalenyl |
| 2-furanyl | 2-furanyl |
| 2-naphthalenyl | 2-naphthalenyl |
| 3-thienyl | 3-thienyl |
| 2,5-dimethyl-3-furanyl | 2,5-dimethyl-3-furanyl |
| 2,5-dimethyl-3-thienyl | 2,5-dimethyl-3-thienyl |
| 4-methylphenoxy | 4-methylphenoxy |
| 2-chlorophenoxy | 2-chlorophenoxy |
| 2,6-dimethylphenoxy | 2,6-dimethylphenoxy |
| 3-methylphenylthio | 4-cyanophenylthio |
| phenylamino | 4-methylphenylamino |
| benzyl | Cl |
| Et | n-hex |
| sec-Bu | Me |
| c-propyl | c-hexyl |
| cis-2-methylcycloheptyl | $CF_3CH_2CH_2$ |
| sec-butylthio | n-BuO |
| $CF_3CH_2O$ | $Cl(CH_2)_5O$ |
| 5-methyl-2-thienyl | 4-methyl-3-furanyl |
| 5-methyl-2-pyridyl | 2-methyl-3-pyridyl |

$R^1$, $R^2$, $R^3$ and $R^4$ are H;
Y is CH

**E**

4-pyridyl
2-indanyl
2-tetrahydronaphthalenyl

$R^1$, $R^2$, $R^3$ and $R^4$ are H;
Y is CH

**E**

1-naphthalenyl
2-furanyl
3-thienyl
3-pyridyl

$R^1$ and $R^2$ are H; $R^3$ is 4-Me;
$R^4$ is Me; Y is N

**E**

4-chloro-3-pyridyl
2-indanyl
2-tetrahydronaphthalenyl

$R^1$ and $R^4$ are Me; $R^3$ is 4-Me;
$R^2$ is H; Y is N

**E**

1-naphthalenyl
2-furanyl
3-thienyl
3-pyridyl

TABLE 9

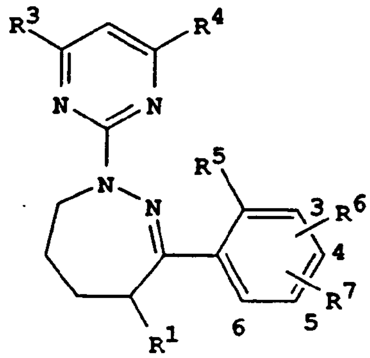

$R^7$ is H; $R^3$ is Me; $R^4$ is Me

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |

$R^7$ is H; $R^3$ is Me; $R^4$ is Me

| $R^1$ | $R^5$ | $R^6$ |
|---|---|---|
| H | $CF_3CH_2O$ | 6-Me |
| H | $CF_3$ | 6-Me |
| H | MeO | 6-MeO |
| H | H | 4-Br |
| Me | F | 6-F |
| Me | Cl | 6-Cl |
| Me | Me | 6-Me |
| n-Pr | $CF_3CH_2O$ | H |
| t-Bu | $CF_3$ | H |
| sec-Bu | MeO | H |
| H | $HCF_2O$ | H |
| H | Br | H |
| H | t-BuO | H |

| $R^1$ | $R^5$ | $R^6$ |
|---|---|---|
| Et | $CF_3CH_2O$ | H |
| Et | $CF_3$ | H |
| Et | MeO | H |
| i-Pr | H | H |
| i-Pr | F | H |
| i-Pr | Cl | H |
| i-Pr | Me | H |
| i-Pr | $CF_3CH_2O$ | H |
| i-Pr | $CF_3$ | H |
| i-Pr | MeO | H |
| H | $HCF_2O$ | 6-$HCF_2O$ |
| H | I | H |
| H | EtO | H |

$R^1$ is H; $R^3$ and $R^4$ are Me

| $R^5$ | $R^6$ | $R^7$ |
|---|---|---|
| H | 4-Cl | 5-Cl |
| H | 4-F | 6-sec-Bu |
| H | 4-Et | 5-I |
| H | 3-F | 6-$CF_3CH_2O$ |
| H | 4-Me | 6-$CF_3CF_2$ |
| H | 4-Br | 6-n-BuO |
| Me | 4-Me | 6-Me |
| Me | 4-F | 6-Me |
| Me | 4-t-Bu | 6-t-Bu |
| Me | 4-$CF_3$ | 6-Cl |
| Me | 3-Me | 5-Br |
| Me | 5-i-Pr | 6-MeO |

$R^1$ is H; $R^3$ and $R^4$ are Me

| $R^5$ | $R^6$ | $R^7$ |
|---|---|---|
| Cl | 4-Cl | 6-Cl |
| Cl | 4-Cl | 6-MeO |
| Cl | 3-Me | 4-Cl |
| Cl | 3-$CF_3$ | 5-$CF_3$ |
| Cl | 4-MeO | 5-t-BuO |
| Cl | 3-n-Bu | 4-Me |
| TMS | H | H |
| TMS | H | 4-F |
| TMS | H | 6-Me |
| TMS | H | 6-MeO |
| TMS | H | 6-Cl |
| TMS | H | 6-$HCF_2O$ |

$R^1$ is H; $R^3$ and $R^4$ are Me

| $R^5$ | $R^6$ | $R^7$ |
|---|---|---|
| t-Bu | 6-t-Bu | H |
| t-Bu | 4-t-BuO | H |
| t-Bu | H | H |
| $CF_3(CH_2)_3O$ | H | H |
| $CF_3(CF_2)_2$ | H | H |
| $(CF_3)_2CH$ | H | H |
| sec-BuS | H | H |
| MeS | 6-MeS | H |
| EtS | 4-F | H |
| MeS(O) | H | H |
| i-PrS(O) | H | H |
| t-BuS$(O)_2$ | H | H |
| MeS$(O)_2$ | H | H |
| $CH_2$=CH | H | H |
| $CH_2$=C$(CH_3)CH_2$ | H | H |
| $CH_2$=$CHCH_2O$ | H | H |
| $MeOCH_2CH_2$ | H | H |
| $MeO_2C$ | H | H |
| $MeOCH_2O$ | H | H |

$R^1$, $R^3$ and $R^4$ are Me

| $R^5$ | $R^6$ | $R^7$ |
|---|---|---|
| Br | 6-Br | H |
| $NMe_2$ | H | H |
| CONHEt | H | H |
| CN | H | H |
| 4-F-Ph | H | H |
| 2-Me-Ph | H | H |
| $NO_2$ | 6-Me | H |
| 4-Me-PhO | H | H |
| PhS | H | H |
| $CO_2H$ | 3-MeO | H |
| $CO_2H$ | H | H |
| HC≡C | H | H |
| MeC≡C | H | H |
| MeC≡$CCH_2O$ | 4-F | H |
| t-BuO | H | H |
| n-PrO | H | H |
| EtO | 5-EtO | H |
| Ac | H | H |
| sec-BuCO | H | H |

$R^4$ is Me; $R^6$ and $R^7$ are H

| $R^1$ | $R^3$ | $R^5$ |
|---|---|---|
| H | c-Pr | H |
| H | c-Pr | F |
| H | c-Pr | Cl |
| H | c-Pr | Me |
| H | c-Pr | $CF_3CH_2O$ |
| H | c-Pr | $CF_3$ |

$R^1$, $R^6$ and $R^7$ are H

| $R^3$ | $R^4$ | $R^5$ |
|---|---|---|
| c-Pr | c-Pr | H |
| c-Pr | c-Pr | F |
| c-Pr | c-Pr | Cl |
| c-Pr | c-Pr | Me |
| c-Pr | $CH_3C$≡C | $CF_3CH_2O$ |
| c-Pr | $CH_3C$≡C | $CF_3$ |

$R^4$ is Me; $R^6$ and $R^7$ are H

| $R^1$ | $R^3$ | $R^5$ |
|---|---|---|
| H | c-Pr | MeO |
| Me | MeC≡C | H |
| Me | MeC≡C | F |
| Me | MeC≡C | Cl |
| Me | MeC≡C | Me |
| Me | MeC≡C | $CF_3CH_2O$ |
| Me | Cl | $CF_3$ |
| Me | $CF_2Cl$ | MeO |
| i-Pr | $CF_3$ | H |
| i-Pr | sec-Bu | F |
| i-Pr | $CF_3$ | Cl |
| i-Pr | $CF_3$ | Me |
| i-Pr | $CF_3$ | $CF_3CH_2O$ |
| i-Pr | Et | $CF_3$ |
| i-Pr | MeO | MeO |
| Et | c-Pr | H |
| Et | MeC≡C | F |
| Et | $CH_2F$ | Cl |
| Et | $CF_3CH_2O$ | Me |
| Et | i-Pr | $CF_3CH_2O$ |
| Et | n-Bu | $CF_3$ |
| Et | HC≡$CCH_2O$ | MeO |
| t-Bu | Br | Cl |
| Ph | $CF_3(CF_2)_3$ | Me |
| Bzl | sec-BuS | $CF_3CH_2O$ |

$R^1$, $R^6$ and $R^7$ are H

| $R^3$ | $R^4$ | $R^5$ |
|---|---|---|
| c-Pr | $CH_3C$≡C | MeO |
| c-Pr | $CF_3$ | H |
| c-Pr | $CF_3$ | F |
| c-Pr | $CF_3$ | Cl |
| c-Pr | $CH_3OCH_2$ | Me |
| c-Pr | $CF_3CH_2O$ | $CF_3CH_2O$ |
| c-Pr | MeS | $CF_3$ |
| c-Pr | $CH_2$=C(Et) | MeO |
| c-Pr | $CH_2$=$CHCH_2$ | H |
| c-Pr | t-BuO | F |
| c-Pr | $HCF_2O$ | Cl |
| c-Pr | $CH_2$=$CHCH_2O$ | Me |
| c-Pr | MeC≡$CCH_2O$ | $CF_3CH_2O$ |
| c-Pr | $NMe_2$ | $CF_3$ |
| c-Pr | NHEt | MeO |
| Cl | Cl | H |
| Cl | Cl | F |
| Cl | Cl | Cl |
| Cl | Cl | Me |
| $CH_3C$≡C | Cl | $CF_3CH_2O$ |
| $CH_3C$≡C | F | $CF_3$ |
| $CH_3C$≡C | $CH_3OCH_2$ | MeO |
| $OCF_3$ | sec-Bu | Cl |
| $OCF_3$ | Br | Me |
| $OCF_3$ | i-Pr | $CF_3CH_2O$ |

TABLE 10

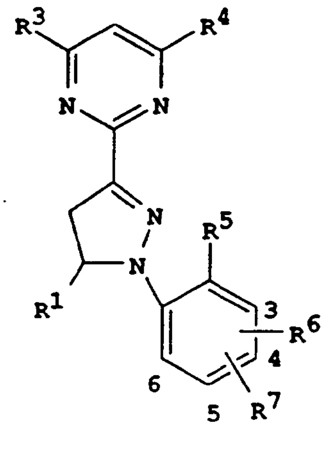

$R^7$ is H; $R^3$ is Me; $R^4$ is Me

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |

$R^7$ is H; $R^3$ is Me; $R^4$ is Me

| $R^1$ | $R^5$ | $R^6$ | $R^1$ | $R^5$ | $R^6$ |
|---|---|---|---|---|---|
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | *i*-Pr | H | H |
| Me | F | 6-F | *i*-Pr | F | H |
| Me | Cl | 6-Cl | *i*-Pr | Cl | H |
| Me | Me | 6-Me | *i*-Pr | Me | H |
| *n*-Pr | $CF_3CH_2O$ | H | *i*-Pr | $CF_3CH_2O$ | H |
| *t*-Bu | $CF_3$ | H | *i*-Pr | $CF_3$ | H |
| *sec*-Bu | MeO | H | *i*-Pr | MeO | H |
| H | $HCF_2O$ | H | H | $HCF_2O$ | 6-$HCF_2O$ |
| H | Br | H | H | I | H |
| H | *t*-BuO | H | H | EtO | H |

$R^7$ is H; $R^3$ is Me; $R^4$ is H

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |

$R^7$ is H; $R^3$ is Me; $R^4$ is H

| $R^1$ | $R^5$ | $R^6$ | $R^1$ | $R^5$ | $R^6$ |
|---|---|---|---|---|---|
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $HCF_2O$ | H | H | $HCF_2O$ | 6-$HCF_2O$ |
| H | Br | H | H | I | H |
| H | t-BuO | H | H | EtO | H |

$R^4$ is Me; $R^6$ and $R^7$ are H

| $R^1$ | $R^3$ | $R^5$ |
|---|---|---|
| H | c-Pr | H |
| H | c-Pr | F |
| H | c-Pr | Cl |
| H | c-Pr | Me |
| H | c-Pr | $CF_3CH_2O$ |
| H | c-Pr | $CF_3$ |

$R^1$, $R^6$ and $R^7$ are H

| $R^3$ | $R^4$ | $R^5$ |
|---|---|---|
| c-Pr | c-Pr | H |
| c-Pr | c-Pr | F |
| c-Pr | c-Pr | Cl |
| c-Pr | c-Pr | Me |
| c-Pr | $CH_3C{\equiv}C$ | $CF_3CH_2O$ |
| c-Pr | $CH_3C{\equiv}C$ | $CF_3$ |

| $R^4$ is Me; $R^6$ and $R^7$ are H | | | $R^1$, $R^6$ and $R^7$ are H | | |
|---|---|---|---|---|---|
| $R^1$ | $R^3$ | $R^5$ | $R^3$ | $R^4$ | $R^5$ |
| H | c-Pr | MeO | c-Pr | $CH_3C≡C$ | MeO |
| Me | MeC≡C | H | c-Pr | $CF_3$ | H |
| Me | MeC≡C | F | c-Pr | $CF_3$ | F |
| Me | MeC≡C | Cl | c-Pr | $CF_3$ | Cl |
| Me | MeC≡C | Me | c-Pr | $CH_3OCH_2$ | Me |
| Me | MeC≡C | $CF_3CH_2O$ | c-Pr | $CF_3CH_2O$ | $CF_3CH_2O$ |
| Me | Cl | $CF_3$ | c-Pr | MeS | $CF_3$ |
| Me | $CF_2Cl$ | MeO | c-Pr | $CH_2=C(Et)$ | MeO |
| i-Pr | $CF_3$ | H | c-Pr | $CH_2=CHCH_2$ | H |
| i-Pr | sec-Bu | F | c-Pr | t-BuO | F |
| i-Pr | $CF_3$ | Cl | c-Pr | $HCF_2O$ | Cl |
| i-Pr | $CF_3$ | Me | c-Pr | $CH_2=CHCH_2O$ | Me |
| i-Pr | $CF_3$ | $CF_3CH_2O$ | c-Pr | $MeC≡CCH_2O$ | $CF_3CH_2O$ |
| i-Pr | Et | $CF_3$ | c-Pr | $NMe_2$ | $CF_3$ |
| i-Pr | MeO | MeO | c-Pr | NHEt | MeO |
| Et | c-Pr | H | Cl | Cl | H |
| Et | MeC≡C | F | Cl | Cl | F |
| Et | $CH_2F$ | Cl | Cl | Cl | Cl |
| Et | $CF_3CH_2O$ | Me | Cl | Cl | Me |
| Et | i-Pr | $CF_3CH_2O$ | $CH_3C≡C$ | Cl | $CF_3CH_2O$ |
| Et | n-Bu | $CF_3$ | $CH_3C≡C$ | F | $CF_3$ |
| Et | $HC≡CCH_2O$ | MeO | $CH_3C≡C$ | $CH_3OCH_2$ | MeO |
| t-Bu | Br | Cl | $OCF_3$ | sec-Bu | Cl |
| Ph | $CF_3(CF_2)_3$ | Me | $OCF_3$ | Br | Me |
| Bzl | sec-BuS | $CF_3CH_2O$ | $OCF_3$ | i-Pr | $CF_3CH_2O$ |

TABLE 11

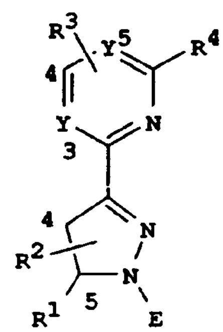

$R^3$ is 4-Me; $R^4$ is Me; Y is N

| $R^1$ | $R^2$ | E |
|---|---|---|
| H | 4-Me | Ph |
| H | 4-*i*-Pr | 2-Me-Ph |
| H | 4-*n*-Bu | 2-Cl-Ph |
| H | 4-CN | 2-MeO-Ph |
| H | 4-$CF_3$ | 2-$CF_3CH_2O$-Ph |
| H | 4-$CF_3CH_2$ | 1-naphthalenyl |
| *i*-Pr | 4-Me | Ph |
| *i*-Pr | 4-Me | 2-Me-Ph |
| *i*-Pr | 4-Me | 2-Cl-Ph |
| *i*-Pr | 4-Me | 2-MeO-Ph |
| *i*-Pr | 4-Me | 2-$CF_3CH_2O$-Ph |
| Cl | H | Ph |
| F | H | 2-Me-Ph |
| $CF_3CF_2$ | H | 2-Cl-Ph |

$R^3$ is H; $R^4$ is Me; Y is CH

| $R^1$ | $R^2$ | E |
|---|---|---|
| H | 4-Et | Ph |
| H | 4-*sec*-Bu | 2-Me-Ph |
| H | 4-$CF_3(CF_2)_3$ | 2-Cl-Ph |
| H | 4-*t*-Bu | 2-MeO-Ph |
| H | 4-$FCH_2$ | 2-$CF_3CH_2O$-Ph |
| H | 4-*n*-Pr | 1-naphthalenyl |
| Me | 5-Me | Ph |
| Me | 5-Me | 2-Me-Ph |
| Me | 5-Me | 2-Cl-Ph |
| Me | 5-Me | 2-MeO-Ph |
| Me | 5-Me | 2-$CF_3CH_2O$-Ph |
| Br | H | Ph |
| CN | H | 2-Me-Ph |
| Ac | H | 2-Cl-Ph |

$R^3$ is 4-Me; $R^4$ is Me; Y is N

| $R^1$ | $R^2$ | E |
|---|---|---|
| $CH_2$=$CHCH_2$ | H | 2-MeO-Ph |
| $CO_2Me$ | H | 2-$CF_3CH_2O$-Ph |
| 2-Me-Ph | H | Me |
| Bzl | H | Ph |
| 2-naphthalenyl | H | n-Bu |
| 3-thienyl | H | $CF_3CF_2$ |
| 3-pyridyl | H | Me |
| CN | 4-Me | Ph |
| t-Bu | 4-Me | 2-Me-Ph |
| $ClCH_2$ | 4-Me | 2-Cl-Ph |
| Et | 4-Me | 2-MeO-Ph |
| n-Pr | 4-Me | 2-$CF_3CH_2O$-Ph |
| Me | 5-Me | 2-$CF_3$-Ph |
| i-Pr | 5-Me | 2-$CF_3$-Ph |
| $CF_3$ | 5-$CF_3$ | 2-$CF_3$-Ph |
| Me | 5-Me | 2-TMS-Ph |

$R^3$ is H; $R^4$ is Me; Y is CH

| $R^1$ | $R^2$ | E |
|---|---|---|
| $CH_3C{\equiv}CCH_2$ | H | 2-MeO-Ph |
| $CO_2Et$ | H | 2-$CF_3CH_2O$-Ph |
| 4-Cl-Ph | H | Ph |
| 5-Me-3-furyl | H | i-Pr |
| EtCO | H | 2-Cl-Ph |
| 2-furyl | 5-Me | $CF_3$ |
| Ph | 4-Me | Me |
| CN | 5-Me | Ph |
| t-Bu | 5-Me | 2-Me-Ph |
| $FCH_2$ | 5-Me | 2-Cl-Ph |
| Et | 5-Me | 2-MeO-Ph |
| $Cl(CH_2)_4$ | 5-Me | 2-$CF_3CH_2O$-Ph |
| Me | 5-Me | 2-$CF_3$-Ph |
| i-Pr | 4-CN | 2-$CF_3$-Ph |
| $CF_3$ | 4-Me | 2-$CF_3$-Ph |
| i-Pr | 5-Me | 2-TMS-Ph |

TABLE 12

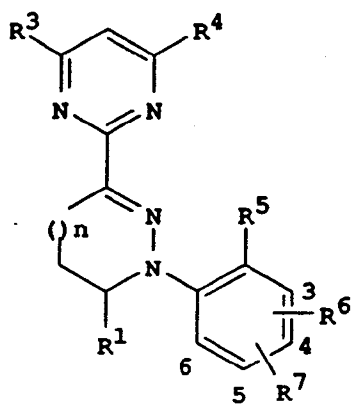

$R^7$ is H; $R^3$ is Me; $R^4$ is Me; n is 1

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |

$R^7$ is H; $R^3$ is Me; $R^4$ is Me; n is 1

| $R^1$ | $R^5$ | $R^6$ | $R^1$ | $R^5$ | $R^6$ |
|---|---|---|---|---|---|
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $HCF_2O$ | H | H | i-Pr | H |
| H | Br | H | H | I | H |
| H | t-BuO | H | H | EtO | H |

$R^7$ is H; $R^3$ is Me; $R^4$ is H; n is 1

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |

$R^7$ is H; $R^3$ is Me; $R^4$ is H; n is 1

| $R^1$ | $R^5$ | $R^6$ | $R^1$ | $R^5$ | $R^6$ |
|---|---|---|---|---|---|
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $NO_2$ | 6-Cl | Me | CN | 6-CN |
| H | Br | 6-Br | Me | $MeS(O)_2$ | 4-F |
| H | $HCF_2O$ | 4-MeO | Me | i-Pr | H |

$R^4$ is Me; $R^6$ and $R^7$ are H; n is 1

| $R^1$ | $R^3$ | $R^5$ |
|---|---|---|
| H | c-Pr | H |
| H | c-Pr | F |
| H | c-Pr | Cl |
| H | c-Pr | Me |
| H | c-Pr | $CF_3CH_2O$ |
| H | c-Pr | $CF_3$ |

$R^1$, $R^6$ and $R^7$ are H; n is 1

| $R^3$ | $R^4$ | $R^5$ |
|---|---|---|
| c-Pr | c-Pr | H |
| c-Pr | c-Pr | F |
| c-Pr | c-Pr | Cl |
| c-Pr | c-Pr | Me |
| c-Pr | $CH_3C{\equiv}C$ | $CF_3CH_2O$ |
| c-Pr | $CH_3C{\equiv}C$ | $CF_3$ |

| $R^4$ is Me; $R^6$ and $R^7$ are H; n is 1 | | | $R^1$, $R^6$ and $R^7$ are H; n is 1 | | |
|---|---|---|---|---|---|
| $R^1$ | $R^3$ | $R^5$ | $R^3$ | $R^4$ | $R^5$ |
| H | c-Pr | MeO | c-Pr | $CH_3C{\equiv}C$ | MeO |
| Me | $MeC{\equiv}C$ | H | c-Pr | $CF_3$ | H |
| Me | $MeC{\equiv}C$ | F | c-Pr | $CF_3$ | F |
| Me | $MeC{\equiv}C$ | Cl | c-Pr | $CF_3$ | Cl |
| Me | $MeC{\equiv}C$ | Me | c-Pr | $CH_3OCH_2$ | Me |
| Me | $MeC{\equiv}C$ | $CF_3CH_2O$ | c-Pr | $CF_3CH_2O$ | $CF_3CH_2O$ |
| Me | Cl | $CF_3$ | c-Pr | MeS | $CF_3$ |
| Me | $CF_2Cl$ | MeO | c-Pr | $CH_2{=}C(Et)$ | MeO |
| i-Pr | $CF_3$ | H | c-Pr | $CH_2{=}CHCH_2$ | H |
| i-Pr | sec-Bu | F | c-Pr | t-BuO | F |
| i-Pr | $CF_3$ | Cl | c-Pr | $HCF_2O$ | Cl |
| i-Pr | $CF_3$ | Me | c-Pr | $CH_2{=}CHCH_2O$ | Me |
| i-Pr | $CF_3$ | $CF_3CH_2O$ | c-Pr | $MeC{\equiv}CCH_2O$ | $CF_3CH_2O$ |
| i-Pr | Et | $CF_3$ | c-Pr | $NMe_2$ | $CF_3$ |
| i-Pr | MeO | MeO | c-Pr | NHEt | MeO |
| Et | c-Pr | H | Cl | Cl | H |
| Et | $MeC{\equiv}C$ | F | Cl | Cl | F |
| Et | $CH_2F$ | Cl | Cl | Cl | Cl |
| Et | $CF_3CH_2O$ | Me | Cl | Cl | Me |
| Et | i-Pr | $CF_3CH_2O$ | $CH_3C{\equiv}C$ | Cl | $CF_3CH_2O$ |
| Et | n-Bu | $CF_3$ | $CH_3C{\equiv}C$ | F | $CF_3$ |
| Et | $HC{\equiv}CCH_2O$ | MeO | $CH_3C{\equiv}C$ | $CH_3OCH_2$ | MeO |
| t-Bu | Br | Cl | $OCF_3$ | sec-Bu | Cl |
| Ph | $CF_3(CF_2)_3$ | Me | $OCF_3$ | Br | Me |
| Bzl | sec-BuS | $CF_3CH_2O$ | $OCF_3$ | i-Pr | $CF_3CH_2O$ |

TABLE 13

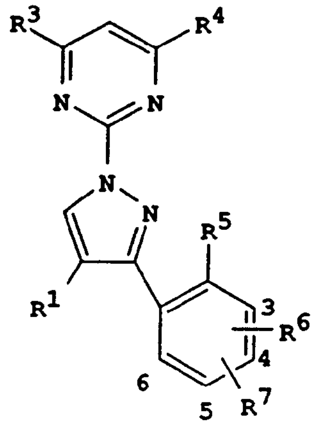

$R^7$ is H; $R^3$ is Me; $R^4$ is Me

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |

R7 is H; R3 is Me; R4 is Me

| $R^1$ | $R^5$ | $R^6$ | $R^1$ | $R^5$ | $R^6$ |
|---|---|---|---|---|---|
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $HCF_2O$ | H | H | $HCF_2O$ | 6-$HCF_2O$ |
| H | Br | H | H | I | H |
| H | t-BuO | H | H | EtO | H |

R7 is H; R3 is Me; R4 is H

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |

$R^7$ is H; $R^3$ is Me; $R^4$ is H

| $R^1$ | $R^5$ | $R^6$ |
|---|---|---|
| H | H | 3-$CF_3$ |
| H | F | 6-F |
| H | Cl | 6-Cl |
| H | Me | 6-Me |
| H | $CF_3CH_2O$ | 6-Me |
| H | $CF_3$ | 6-Me |
| H | MeO | 6-MeO |
| H | H | 4-Br |
| Me | F | 6-F |
| Me | Cl | 6-Cl |
| Me | Me | 6-Me |
| n-Pr | $CF_3CH_2O$ | H |
| t-Bu | $CF_3$ | H |
| sec-Bu | MeO | H |
| H | $HCF_2O$ | H |
| H | Br | H |
| H | t-BuO | H |

| $R^1$ | $R^5$ | $R^6$ |
|---|---|---|
| Et | H | H |
| Et | F | H |
| Et | Cl | H |
| Et | Me | H |
| Et | $CF_3CH_2O$ | H |
| Et | $CF_3$ | H |
| Et | MeO | H |
| i-Pr | H | H |
| i-Pr | F | H |
| i-Pr | Cl | H |
| i-Pr | Me | H |
| i-Pr | $CF_3CH_2O$ | H |
| i-Pr | $CF_3$ | H |
| i-Pr | MeO | H |
| H | $HCF_2O$ | 6-$HCF_2O$ |
| H | I | H |
| H | EtO | H |

$R^4$ is Me; $R^6$ and $R^7$ are H;

| $R^1$ | $R^3$ | $R^5$ |
|---|---|---|
| H | c-Pr | H |
| H | c-Pr | F |
| H | c-Pr | Cl |
| H | c-Pr | Me |
| H | c-Pr | $CF_3CH_2O$ |
| H | c-Pr | $CF_3$ |

$R^1$, $R^6$ and $R^7$ are H;

| $R^3$ | $R^4$ | $R^5$ |
|---|---|---|
| c-Pr | c-Pr | H |
| c-Pr | c-Pr | F |
| c-Pr | c-Pr | Cl |
| c-Pr | c-Pr | Me |
| c-Pr | $CH_3C{\equiv}C$ | $CF_3CH_2O$ |
| c-Pr | $CH_3C{\equiv}C$ | $CF_3$ |

$R^4$ is Me; $R^6$ and $R^7$ are H

| $R^1$ | $R^3$ | $R^5$ |
|---|---|---|
| H | c-Pr | MeO |
| Me | MeC≡C | H |
| Me | MeC≡C | F |
| Me | MeC≡C | Cl |
| Me | MeC≡C | Me |
| Me | MeC≡C | $CF_3CH_2O$ |
| Me | Cl | $CF_3$ |
| Me | $CF_2Cl$ | MeO |
| i-Pr | $CF_3$ | H |
| i-Pr | sec-Bu | F |
| i-Pr | $CF_3$ | Cl |
| i-Pr | $CF_3$ | Me |
| i-Pr | $CF_3$ | $CF_3CH_2O$ |
| i-Pr | Et | $CF_3$ |
| i-Pr | MeO | MeO |
| Et | c-Pr | H |
| Et | MeC≡C | F |
| Et | $CH_2F$ | Cl |
| Et | $CF_3CH_2O$ | Me |
| Et | i-Pr | $CF_3CH_2O$ |
| Et | n-Bu | $CF_3$ |
| Et | HC≡$CCH_2O$ | MeO |
| t-Bu | Br | Cl |
| Ph | $CF_3(CF_2)_3$ | Me |
| Bzl | sec-BuS | $CF_3CH_2O$ |

$R^1$, $R^6$ and $R^7$ are H

| $R^3$ | $R^4$ | $R^5$ |
|---|---|---|
| c-Pr | $CH_3C$≡C | MeO |
| c-Pr | $CF_3$ | H |
| c-Pr | $CF_3$ | F |
| c-Pr | $CF_3$ | Cl |
| c-Pr | $CH_3OCH_2$ | Me |
| c-Pr | $CF_3CH_2O$ | $CF_3CH_2O$ |
| c-Pr | MeS | $CF_3$ |
| c-Pr | $CH_2$=C(Et) | MeO |
| c-Pr | $CH_2$=$CHCH_2$ | H |
| c-Pr | t-BuO | F |
| c-Pr | $HCF_2O$ | Cl |
| c-Pr | $CH_2$=$CHCH_2O$ | Me |
| c-Pr | MeC≡$CCH_2O$ | $CF_3CH_2O$ |
| c-Pr | $NMe_2$ | $CF_3$ |
| c-Pr | NHEt | MeO |
| Cl | Cl | H |
| Cl | Cl | F |
| Cl | Cl | Cl |
| Cl | Cl | Me |
| $CH_3C$≡C | Cl | $CF_3CH_2O$ |
| $CH_3C$≡C | F | $CF_3$ |
| $CH_3C$≡C | $CH_3OCH_2$ | MeO |
| $OCF_3$ | sec-Bu | Cl |
| $OCF_3$ | Br | Me |
| $OCF_3$ | i-Pr | $CF_3CH_2O$ |

TABLE 14

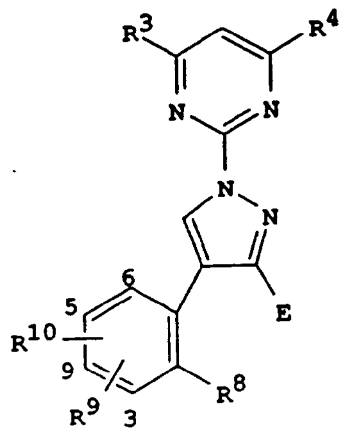

$R^3$ and $R^4$ are Me; $R^{10}$ is H

| E | $R^8$ | $R^9$ | E | $R^8$ | $R^9$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |

$R^3$ and $R^4$ are Me; $R^{10}$ is H

| E | $R^8$ | $R^9$ | E | $R^8$ | $R^9$ |
|---|---|---|---|---|---|
| H | $CF_3$ | 6-Me | Et | $CF_3$ | H |
| H | MeO | 6-MeO | Et | MeO | H |
| H | H | 4-Br | *i*-Pr | H | H |
| Me | F | 6-F | *i*-Pr | F | H |
| Me | Cl | 6-Cl | *i*-Pr | Cl | H |
| Me | Me | 6-Me | *i*-Pr | Me | H |
| *n*-Pr | $CF_3CH_2O$ | H | *i*-Pr | $CF_3CH_2O$ | H |
| *t*-Bu | $CF_3$ | H | *i*-Pr | $CF_3$ | H |
| *sec*-Bu | MeO | H | *i*-Pr | MeO | H |
| Ph | $HCF_2O$ | H | H | $HCF_2O$ | 6-$HCF_2O$ |
| H | Br | H | Ph | I | H |
| H | *t*-BuO | H | H | EtO | H |

$R^3$ is Me; $R^4$ and $R^{10}$ are H

| E | $R^8$ | $R^9$ | E | $R^8$ | $R^9$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |

$R^3$ is Me; $R^4$ and $R^{10}$ are H

| E | $R^8$ | $R^9$ |
|---|---|---|
| H | H | 3-$CF_3$ |
| H | F | 6-F |
| H | Cl | 6-Cl |
| H | Me | 6-Me |
| H | $CF_3CH_2O$ | 6-Me |
| H | $CF_3$ | 6-Me |
| H | MeO | 6-MeO |
| H | H | 4-Br |
| Me | F | 6-F |
| Me | Cl | 6-Cl |
| Me | Me | 6-Me |
| n-Pr | $CF_3CH_2O$ | H |
| t-Bu | $CF_3$ | H |
| sec-Bu | MeO | H |
| Ph | $HCF_2O$ | 4-MeO |
| H | Br | H |
| H | t-BuO | H |
| Et | H | H |
| Et | F | H |
| Et | Cl | H |
| Et | Me | H |
| Et | $CF_3CH_2O$ | H |
| Et | $CF_3$ | H |
| Et | MeO | H |
| i-Pr | H | H |
| i-Pr | F | H |
| i-Pr | Cl | H |
| i-Pr | Me | H |
| i-Pr | $CF_3CH_2O$ | H |
| i-Pr | $CF_3$ | H |
| i-Pr | MeO | H |
| H | $HCF_2O$ | 6-$HCF_2O$ |
| Ph | I | H |
| H | EtO | H |

$R^4$ is Me; $R^9$ and $R^{10}$ are H

| E | $R^3$ | $R^8$ |
|---|---|---|
| H | c-Pr | H |
| H | c-Pr | F |
| H | c-Pr | Cl |
| H | c-Pr | Me |
| H | c-Pr | $CF_3CH_2O$ |
| H | c-Pr | $CF_3$ |

$R^9$ and $R^{10}$ are H; E is Me

| $R^3$ | $R^4$ | $R^8$ |
|---|---|---|
| c-Pr | c-Pr | H |
| c-Pr | c-Pr | F |
| c-Pr | c-Pr | Cl |
| c-Pr | c-Pr | Me |
| c-Pr | $CH_3C{\equiv}C$ | $CF_3CH_2O$ |
| c-Pr | $CH_3C{\equiv}C$ | $CF_3$ |

$R^4$ is Me; $R^9$ and $R^{10}$ are H

| E | $R^3$ | $R^8$ |
|---|---|---|
| H | *c*-Pr | MeO |
| Me | MeC≡C | H |
| Me | MeC≡C | F |
| Me | MeC≡C | Cl |
| Me | MeC≡C | Me |
| Me | MeC≡C | $CF_3CH_2O$ |
| Me | Cl | $CF_3$ |
| Me | $CF_2Cl$ | MeO |
| *i*-Pr | $CF_3$ | H |
| *i*-Pr | *sec*-Bu | F |
| *i*-Pr | $CF_3$ | Cl |
| *i*-Pr | $CF_3$ | Me |
| *i*-Pr | $CF_3$ | $CF_3CH_2O$ |
| *i*-Pr | Et | $CF_3$ |
| *i*-Pr | MeO | MeO |
| Et | *c*-Pr | H |
| Et | MeC≡C | F |
| Et | $CH_2F$ | Cl |
| Et | $CF_3CH_2O$ | Me |
| Et | *i*-Pr | $CF_3CH_2O$ |
| Et | *n*-Bu | $CF_3$ |
| Et | HC≡C$CH_2O$ | MeO |
| *t*-Bu | Br | Cl |
| Ph | $CF_3(CF_2)_3$ | Me |
| Bzl | *sec*-BuS | $CF_3CH_2O$ |

$R^9$ and $R^{10}$ are H; E is Me

| $R^3$ | $R^4$ | $R^8$ |
|---|---|---|
| *c*-Pr | $CH_3$C≡C | MeO |
| *c*-Pr | $CF_3$ | H |
| *c*-Pr | $CF_3$ | F |
| *c*-Pr | $CF_3$ | Cl |
| *c*-Pr | $CH_3OCH_2$ | Me |
| *c*-Pr | $CF_3CH_2O$ | $CF_3CH_2O$ |
| *c*-Pr | MeS | $CF_3$ |
| *c*-Pr | $CH_2$=C(Et) | MeO |
| *c*-Pr | $CH_2$=$CHCH_2$ | H |
| *c*-Pr | *t*-BuO | F |
| *c*-Pr | $HCF_2O$ | Cl |
| *c*-Pr | $CH_2$=$CHCH_2O$ | Me |
| *c*-Pr | MeC≡C$CH_2O$ | $CF_3CH_2O$ |
| *c*-Pr | $NMe_2$ | $CF_3$ |
| *c*-Pr | NHEt | MeO |
| Cl | Cl | H |
| Cl | Cl | F |
| Cl | Cl | Cl |
| Cl | Cl | Me |
| $CH_3$C≡C | Cl | $CF_3CH_2O$ |
| $CH_3$C≡C | F | $CF_3$ |
| $CH_3$C≡C | $CH_3OCH_2$ | MeO |
| $OCF_3$ | *sec*-Bu | Cl |
| $OCF_3$ | Br | Me |
| $OCF_3$ | *i*-Pr | $CF_3CH_2O$ |

TABLE 15

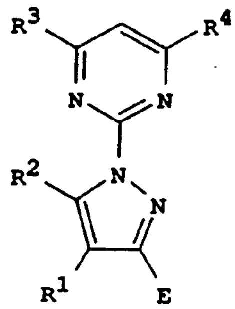

R³ is Me; R⁴ is Me

| $R^1$ | $R^2$ | E |
|---|---|---|
| H | Me | Ph |
| H | i-Pr | 2-Me-Ph |
| H | n-Bu | 2-Cl-Ph |
| H | CN | 2-MeO-Ph |
| H | $CF_3$ | $CF_3CH_2O$-Ph |
| H | $CF_3CH_2$ | 1-naphthalenyl |
| i-Pr | Me | Ph |
| i-Pr | Me | 2-Me-Ph |
| i-Pr | Me | 2-Cl-Ph |
| i-Pr | Me | 2-MeO-Ph |
| i-Pr | Me | 2-$CF_3CH_2O$-Ph |
| Cl | H | Ph |
| F | H | 2-Me-Ph |
| $CF_3CF_2$ | H | 2-Cl-Ph |

$R^3$ is H; $R^4$ is Me

| $R^1$ | $R^2$ | E |
|---|---|---|
| H | Et | Ph |
| H | sec-Bu | 2-Me-Ph |
| H | $CF_3(CF_2)_3$ | 2-Cl-Ph |
| H | t-Bu | 2-MeO-Ph |
| H | $FCH_2$ | 2-$CF_3CH_2O$-Ph |
| H | n-Pr | 1-naphthalenyl |
| Me | Me | Ph |
| Me | Me | 2-Me-Ph |
| Me | Me | 2-Cl-Ph |
| Me | Me | 2-MeO-Ph |
| Me | Me | 2-$CF_3CH_2O$-Ph |
| Br | H | Ph |
| CN | H | 2-Me-Ph |
| Ac | H | 2-Cl-Ph |

$R^3$ is Me; $R^4$ is Me

| $R^1$ | $R^2$ | E |
|---|---|---|
| $CH_2{=}CHCH_2$ | H | 2-MeO-Ph |
| $CO_2Me$ | H | 2-$CF_3CH_2O$-Ph |
| 2-Me-Ph | H | Me |
| Bzl | H | Ph |
| 2-naphthalenyl | H | n-Bu |
| 3-thienyl | H | $CF_3CF_2$ |
| 3-pyridyl | H | Me |
| Ph | Me | H |
| 2-Me-Ph | Me | H |
| 2-Cl-Ph | Me | H |
| 2-MeO-Ph | Me | H |
| 2-$CF_3CH_2O$-Ph | Me | H |
| Ph | Me | n-Pr |
| Ph | Me | $CF_3$ |
| Ph | Me | i-Pr |
| Ph | Me | sec-Bu |

$R^3$ is H; $R^4$ is Me

| $R^1$ | $R^2$ | E |
|---|---|---|
| $CH_3C{\equiv}CCH_2$ | H | 2-MeO-Ph |
| $CO_2Et$ | H | 2-$CF_3CH_2O$-Ph |
| 4-Cl-Ph | H | Ph |
| 5-Me-3-furyl | H | Ph |
| EtCO | H | i-Pr |
| 2-furyl | H | 2-Cl-Ph |
| Ph | Me | $CF_3$ |
| Ph | Me | H |
| 2-Me-Ph | Me | H |
| 2-Cl-Ph | Me | H |
| 2-MeO-Ph | Me | H |
| 2-$CF_3CH_2O$-Ph | Me | H |
| Ph | i-Pr | Me |
| Ph | CF3 | Me |
| Ph | Et | Me |
| Ph | n-Bu | Me |

TABLE 16

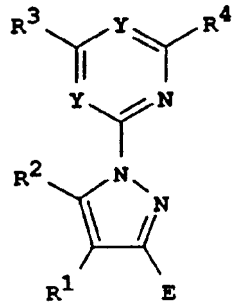

$R^3$ and $R^4$ is Me; Y is N

| $R^1$ | $R^2$ | E |
|---|---|---|
| H | Me | Ph |
| H | i-Pr | 2-Me-Ph |
| H | n-Bu | 2-Cl-Ph |
| H | CN | 2-MeO-Ph |
| H | $CF_3$ | $CF_3CH_2O$-Ph |
| H | $CF_3CH_2$ | 1-naphthalenyl |
| i-Pr | Me | Ph |
| i-Pr | Me | 2-Me-Ph |
| i-Pr | Me | 2-Cl-Ph |
| i-Pr | Me | 2-MeO-Ph |
| i-Pr | Me | 2-$CF_3CH_2O$-Ph |
| Cl | H | Ph |
| F | H | 2-Me-Ph |
| $CF_3CF_2$ | H | 2-Cl-Ph |

$R^3$ is H; $R^4$ is Me; Y is CH

| $R^1$ | $R^2$ | E |
|---|---|---|
| H | Et | Ph |
| H | sec-Bu | 2-Me-Ph |
| H | $CF_3(CF_2)_3$ | 2-Cl-Ph |
| H | t-Bu | 2-MeO-Ph |
| H | $FCH_2$ | 2-$CF_3CH_2O$-Ph |
| H | n-Pr | 1-naphthalenyl |
| Me | Me | Ph |
| Me | Me | 2-Me-Ph |
| Me | Me | 2-Cl-Ph |
| Me | Me | 2-MeO-Ph |
| Me | Me | 2-$CF_3CH_2O$-Ph |
| Br | H | Ph |
| CN | H | 2-Me-Ph |
| Ac | H | 2-Cl-Ph |

| $R^3$ and $R^4$ are Me; Y is N | | | $R^3$ is H; $R^4$ is Me; Y is CH | | |
|---|---|---|---|---|---|
| $R^1$ | $R^2$ | E | $R^1$ | $R^2$ | E |
| $CH_2$=$CHCH_2$ | H | 2-MeO-Ph | $CH_3C{\equiv}CCH_2$ | H | 2-MeO-Ph |
| $CO_2Me$ | H | 2-$CF_3CH_2O$-Ph | $CO_2Et$ | H | 2-$CF_3CH_2O$-Ph |
| 2-Me-Ph | H | Me | 4-Cl-Ph | H | Ph |
| Bzl | H | Ph | 5-Me-3-furyl | H | i-Pr |
| 2-naphthalenyl | H | n-Bu | EtCO | H | 2-Cl-Ph |
| 3-thienyl | H | $CF_3CF_2$ | 2-furyl | H | $CF_3$ |
| 3-pyridyl | H | Me | Ph | Me | Me |
| Ph | Me | H | Ph | Me | H |
| 2-Me-Ph | Me | H | 2-Me-Ph | Me | H |
| 2-Cl-Ph | Me | H | 2-Cl-Ph | Me | H |
| 2-MeO-Ph | Me | H | 2-MeO-Ph | Me | H |
| 2-$CF_3CH_2O$-Ph | Me | H | 2-$CF_3CH_2O$-Ph | Me | H |
| Ph | Me | n-Pr | Ph | i-Pr | Me |
| Ph | Me | $CF_3$ | Ph | CF3 | Me |
| Ph | Me | i-Pr | Ph | Et | Me |
| Ph | Me | sec-Bu | Ph | n-Bu | Me |

TABLE 17

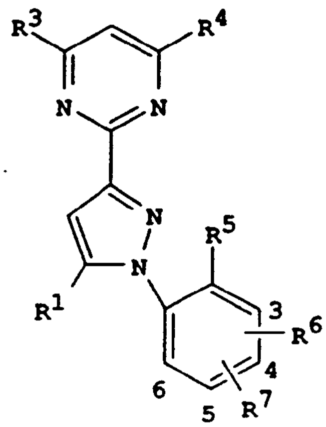

$R^3$ and $R^4$ are Me; $R^7$ is H

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |

$R^3$ and $R^4$ are Me; $R^7$ is H

| $R^1$ | $R^5$ | $R^6$ | $R^1$ | $R^5$ | $R^6$ |
|---|---|---|---|---|---|
| H | $CF_3$ | 6-Me | Et | $CF_3$ | H |
| H | MeO | 6-MeO | Et | MeO | H |
| H | H | 4-Br | *i*-Pr | H | H |
| Me | F | 6-F | *i*-Pr | F | H |
| Me | Cl | 6-Cl | *i*-Pr | Cl | H |
| Me | Me | 6-Me | *i*-Pr | Me | H |
| *n*-Pr | $CF_3CH_2O$ | H | *i*-Pr | $CF_3CH_2O$ | H |
| *t*-Bu | $CF_3$ | H | *i*-Pr | $CF_3$ | H |
| *sec*-Bu | MeO | H | *i*-Pr | MeO | H |
| H | $HCF_2O$ | H | H | $HCF_2O$ | 6-$HCF_2O$ |
| H | Br | H | H | I | H |
| H | *t*-BuO | H | H | EtO | H |

$R^3$ is Me; $R^4$ and $R^7$ are H

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |

$R^3$ is Me; $R^4$ and $R^7$ are H

| $R^1$ | $R^5$ | $R^6$ |
|---|---|---|
| H | H | 3-$CF_3$ |
| H | F | 6-F |
| H | Cl | 6-Cl |
| H | Me | 6-Me |
| H | $CF_3CH_2O$ | 6-Me |
| H | $CF_3$ | 6-Me |
| H | MeO | 6-MeO |
| H | H | 4-Br |
| Me | F | 6-F |
| Me | Cl | 6-Cl |
| Me | Me | 6-Me |
| n-Pr | $CF_3CH_2O$ | H |
| t-Bu | $CF_3$ | H |
| sec-Bu | MeO | H |
| H | $HCF_2O$ | H |
| H | Br | H |
| H | t-BuO | H |

| $R^1$ | $R^5$ | $R^6$ |
|---|---|---|
| Et | H | H |
| Et | F | H |
| Et | Cl | H |
| Et | Me | H |
| Et | $CF_3CH_2O$ | H |
| Et | $CF_3$ | H |
| Et | MeO | H |
| i-Pr | H | H |
| i-Pr | F | H |
| i-Pr | Cl | H |
| i-Pr | Me | H |
| i-Pr | $CF_3CH_2O$ | H |
| i-Pr | $CF_3$ | H |
| i-Pr | MeO | H |
| H | $HCF_2O$ | 6-$HCF_2O$ |
| H | I | H |
| H | EtO | H |

$R^4$ is Me; $R^6$ and $R^7$ are H

| $R^1$ | $R^3$ | $R^5$ |
|---|---|---|
| H | c-Pr | H |
| H | c-Pr | F |
| H | c-Pr | Cl |
| H | c-Pr | Me |
| H | c-Pr | $CF_3CH_2O$ |
| H | c-Pr | $CF_3$ |

$R^1$, $R^6$ and $R^7$ are H

| $R^3$ | $R^4$ | $R^5$ |
|---|---|---|
| c-Pr | c-Pr | H |
| c-Pr | c-Pr | F |
| c-Pr | c-Pr | Cl |
| c-Pr | c-Pr | Me |
| c-Pr | $CH_3C{\equiv}C$ | $CF_3CH_2O$ |
| c-Pr | $CH_3C{\equiv}C$ | $CF_3$ |

$R^4$ is Me; $R^6$ and $R^7$ are H

| $R^1$ | $R^3$ | $R^5$ |
|---|---|---|
| H | c-Pr | MeO |
| Me | $MeC\equiv C$ | H |
| Me | $MeC\equiv C$ | F |
| Me | $MeC\equiv C$ | Cl |
| Me | $MeC\equiv C$ | Me |
| Me | $MeC\equiv C$ | $CF_3CH_2O$ |
| Me | Cl | $CF_3$ |
| Me | $CF_2Cl$ | MeO |
| i-Pr | $CF_3$ | H |
| i-Pr | sec-Bu | F |
| i-Pr | $CF_3$ | Cl |
| i-Pr | $CF_3$ | Me |
| i-Pr | $CF_3$ | $CF_3CH_2O$ |
| i-Pr | Et | $CF_3$ |
| i-Pr | MeO | MeO |
| Et | c-Pr | H |
| Et | $MeC\equiv C$ | F |
| Et | $CH_2F$ | Cl |
| Et | $CF_3CH_2O$ | Me |
| Et | i-Pr | $CF_3CH_2O$ |
| Et | n-Bu | $CF_3$ |
| Et | $HC\equiv CCH_2O$ | MeO |
| t-Bu | Br | Cl |
| Ph | $CF_3(CF_2)_3$ | Me |
| Bzl | sec-BuS | $CF_3CH_2O$ |

$R^1$, $R^6$ and $R^7$ are H

| $R^3$ | $R^4$ | $R^5$ |
|---|---|---|
| c-Pr | $CH_3C\equiv C$ | MeO |
| c-Pr | $CF_3$ | H |
| c-Pr | $CF_3$ | F |
| c-Pr | $CF_3$ | Cl |
| c-Pr | $CH_3OCH_2$ | Me |
| c-Pr | $CF_3CH_2O$ | $CF_3CH_2O$ |
| c-Pr | MeS | $CF_3$ |
| c-Pr | $CH_2{=}C(Et)$ | MeO |
| c-Pr | $CH_2{=}CHCH_2$ | H |
| c-Pr | t-BuO | F |
| c-Pr | $HCF_2O$ | Cl |
| c-Pr | $CH_2{=}CHCH_2O$ | Me |
| c-Pr | $MeC\equiv CCH_2O$ | $CF_3CH_2O$ |
| c-Pr | $NMe_2$ | $CF_3$ |
| c-Pr | NHEt | MeO |
| Cl | Cl | H |
| Cl | Cl | F |
| Cl | Cl | Cl |
| Cl | Cl | Me |
| $CH_3C\equiv C$ | Cl | $CF_3CH_2O$ |
| $CH_3C\equiv C$ | F | $CF_3$ |
| $CH_3C\equiv C$ | $CH_3OCH_2$ | MeO |
| $OCF_3$ | sec-Bu | Cl |
| $OCF_3$ | Br | Me |
| $OCF_3$ | i-Pr | $CF_3CH_2O$ |

TABLE 18

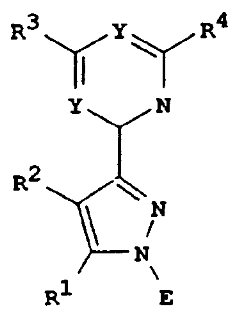

$R^3$ and $R^4$ are Me; Y is N

| $R^1$ | $R^2$ | E |
|---|---|---|
| H | Me | Ph |
| H | *i*-Pr | 2-Me-Ph |
| H | *n*-Bu | 2-Cl-Ph |
| H | CN | 2-MeO-Ph |
| H | $CF_3$ | $CF_3CH_2O$-Ph |
| H | $CF_3CH_2$ | 1-naphthalenyl |
| *i*-Pr | Me | Ph |
| *i*-Pr | Me | 2-Me-Ph |
| *i*-Pr | Me | 2-Cl-Ph |
| *i*-Pr | Me | 2-MeO-Ph |
| *i*-Pr | Me | 2-$CF_3CH_2O$-Ph |
| Cl | H | Ph |
| F | H | 2-Me-Ph |
| $CF_3CF_2$ | H | 2-Cl-Ph |

$R^3$ is H; $R^4$ is Me; Y is CH

| $R^1$ | $R^2$ | E |
|---|---|---|
| H | Et | Ph |
| H | *sec*-Bu | 2-Me-Ph |
| H | $CF_3(CF_2)_3$ | 2-Cl-Ph |
| H | *t*-Bu | 2-MeO-Ph |
| H | $FCH_2$ | 2-$CF_3CH_2O$-Ph |
| H | *n*-Pr | 1-naphthalenyl |
| Me | Me | Ph |
| Me | Me | 2-Me-Ph |
| Me | Me | 2-Cl-Ph |
| Me | Me | 2-MeO-Ph |
| Me | Me | 2-$CF_3CH_2O$-Ph |
| Br | H | Ph |
| CN | H | 2-Me-Ph |
| Ac | H | 2-Cl-Ph |

$R^3$ and $R^4$ are Me; Y is N

| $R^1$ | $R^2$ | E |
|---|---|---|
| $CH_2$=$CHCH_2$ | H | 2-MeO-Ph |
| $CO_2Me$ | H | 2-$CF_3CH_2O$-Ph |
| 2-Me-Ph | H | Me |
| Bzl | H | Ph |
| 2-naphthalenyl | H | n-Bu |
| 3-thienyl | H | $CF_3CF_2$ |
| 3-pyridyl | H | Me |
| Ph | Me | H |
| 2-Me-Ph | Me | H |
| 2-Cl-Ph | Me | H |
| 2-MeO-Ph | Me | H |
| 2-$CF_3CH_2O$-Ph | Me | H |
| Ph | Me | n-Pr |
| Ph | Me | $CF_3$ |
| Ph | Me | i-Pr |
| Ph | Me | sec-Bu |

$R^3$ is H; $R^4$ is Me; Y is CH

| $R^1$ | $R^2$ | E |
|---|---|---|
| $CH_3C{\equiv}CCH_2$ | H | 2-MeO-Ph |
| $CO_2Et$ | H | 2-$CF_3CH_2O$-Ph |
| 4-Cl-Ph | H | Ph |
| 5-Me-3-furyl | H | Ph |
| EtCO | H | i-Pr |
| 2-furyl | H | 2-Cl-Ph |
| Ph | Me | $CF_3$ |
| Ph | Me | Ph |
| 2-Me-Ph | Me | H |
| 2-Cl-Ph | Me | H |
| 2-MeO-Ph | Me | H |
| 2-$CF_3CH_2O$-Ph | Me | H |
| Ph | i-Pr | Me |
| Ph | CF3 | Me |
| Ph | Et | Me |
| Ph | n-Bu | Me |

TABLE 19

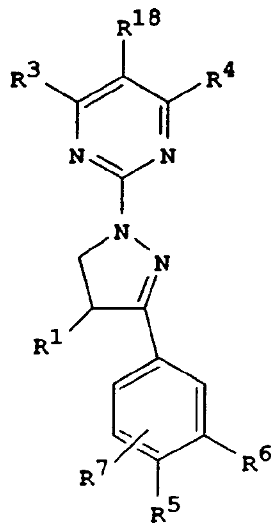

| $R^3$ is Me | | | | | | $R^3$ is Me; $R^4$ and $R^{18}$ together forms $-(CH_2)_3-$ | | | |
|---|---|---|---|---|---|---|---|---|---|
| $R^1$ | $R^4$ | $R^{18}$ | $R^5$ | $R^6$ | $R^7$ | $R^1$ | $R^5$ | $R^6$ | $R^7$ |
| H | OH | n-Bu | Cl | H | H | H | Cl | H | H |
| H | OH | n-Pr | Me | H | H | H | Me | H | H |
| H | OH | Et | Me | Me | H | H | Et | H | H |
| Me | OH | n-Bu | Cl | H | 2-Cl | Me | i-Pr | H | H |
| Et | OH | n-Bu | H | MeO | H | Et | H | Me | H |
| H | Ph | H | Cl | H | H | H | Cl | H | 2-Cl |
| H | Ph | H | Me | H | H | H | Me | Me | H |
| H | Ph | H | Me | Me | H | H | Et | Et | H |
| Me | Ph | H | Cl | H | 2-Cl | Me | Me | Me | H |
| Et | Ph | H | H | MeO | H | Et | Me | H | H |
| H | $TMS-CH_2$ | H | Cl | H | H | H | F | H | H |
| H | $TMS-CH_2$ | H | Me | H | H | H | H | Br | H |
| H | $TMS-CH_2$ | H | Me | Me | H | H | MeO | H | H |
| Me | $TMS-CH_2$ | H | Cl | H | 2-Cl | Me | H | MeO | H |
| Et | $TMS-CH_2$ | H | H | MeO | H | Et | Cl | H | H |

$R^3$ is Me

| $R^1$ | $R^4$ | $R^{18}$ | $R^5$ | $R^6$ | $R^7$ |
|---|---|---|---|---|---|
| H | Me | Cl | Me | Me | H |
| H | Me | Br | Cl | H | H |
| H | Me | Cl | Cl | H | H |
| Me | Me | Br | F | H | H |
| Et | Me | Cl | $CF_3$ | H | H |

$R^3$ is Me; $R^4$ and $R^{18}$ together forms $-(CH_2)_3-$

| $R^1$ | $R^5$ | $R^6$ | $R^7$ |
|---|---|---|---|
| H | H | Cl | H |
| H | H | $CF_3$ | H |
| H | H | F | H |
| Me | H | Cl | H |

$R^3$ is Me; $R^4$ and $R^{18}$ together forms $-(CH_2)_4-$

| $R^1$ | $R^5$ | $R^6$ | $R^7$ |
|---|---|---|---|
| H | Cl | H | H |
| H | Me | H | H |
| H | Et | H | H |
| Me | i-Pr | H | H |
| Et | H | Me | H |
| H | Cl | H | 2-Cl |
| H | Me | Me | H |
| H | Et | Et | H |
| Me | Me | Me | H |
| Et | Me | H | H |
| H | MeO | H | H |
| H | H | MeO | H |

| $R^1$ | $R^5$ | $R^6$ | $R^7$ |
|---|---|---|---|
| H | MeO | MeO | H |
| Me | MeO | H | 2-MeO |
| Et | F | H | H |
| H | $CF_3$ | H | H |
| H | $CF_3CH_2O$ | H | H |
| H | $HCF_2O$ | H | H |
| Me | EtO | H | H |
| Et | H | EtO | H |
| H | H | Cl | H |
| H | H | $CF_3$ | H |
| H | H | F | H |
| Me | H | Cl | H |

TABLE 20

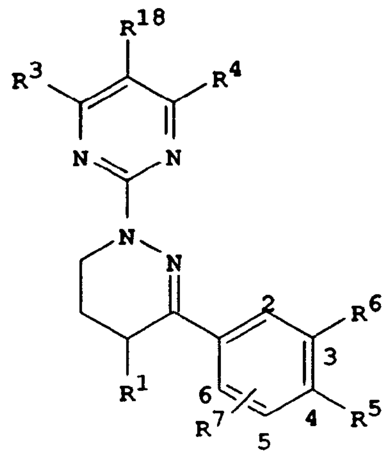

| $R^3$ is Me | | | | | |
|---|---|---|---|---|---|
| $R^1$ | $R^4$ | $R^{18}$ | $R^5$ | $R^6$ | $R^7$ |
| H | OH | n-Bu | Cl | H | H |
| H | OH | n-Pr | Me | H | H |
| H | OH | Et | Me | Me | H |
| Me | OH | n-Bu | Cl | H | 2-Cl |
| Et | OH | n-Bu | H | MeO | H |
| H | Ph | H | Cl | H | H |
| H | Ph | H | Me | H | H |
| H | Ph | H | Me | Me | H |
| Me | Ph | H | Cl | H | 2-Cl |
| Et | Ph | H | H | MeO | H |
| H | TMS-$CH_2$ | H | Cl | H | H |
| H | TMS-$CH_2$ | H | Me | H | H |
| H | TMS-$CH_2$ | H | Me | Me | H |
| Me | TMS-$CH_2$ | H | Cl | H | 2-Cl |
| Et | TMS-$CH_2$ | H | H | MeO | H |

| $R^3$ is Me; $R^4$ and $R^{18}$ together forms $-(CH_2)_3-$ | | | |
|---|---|---|---|
| $R^1$ | $R^5$ | $R^6$ | $R^7$ |
| H | Cl | H | H |
| H | Me | H | H |
| H | Et | H | H |
| Me | i-Pr | H | H |
| Et | H | Me | H |
| H | Cl | H | 2-Cl |
| H | Me | Me | H |
| H | Et | Et | H |
| Me | Me | Me | H |
| Et | Me | H | H |
| H | F | H | H |
| H | H | Br | H |
| H | MeO | H | H |
| Me | H | MeO | H |

$R^3$ is Me

| $R^1$ | $R^4$ | $R^{18}$ | $R^5$ | $R^6$ | $R^7$ |
|---|---|---|---|---|---|
| H | Me | Cl | Me | Me | H |
| H | Me | Br | Cl | H | H |
| H | Me | Cl | Cl | H | H |
| Me | Me | Br | F | H | H |
| Et | Me | Cl | $CF_3$ | H | H |

$R^3$ is Me; $R^4$ and $R^{18}$ together forms $-(CH_2)_3-$

| $R^1$ | $R^5$ | $R^6$ | $R^7$ |
|---|---|---|---|
| Et | Cl | H | H |
| H | H | Cl | H |
| H | H | $CF_3$ | H |
| H | H | F | H |
| Me | H | Cl | H |

$R^3$ is Me; $R^4$ and $R^{18}$ together forms $-(CH_2)_4-$

| $R^1$ | $R^5$ | $R^6$ | $R^7$ |
|---|---|---|---|
| H | Cl | H | H |
| H | Me | H | H |
| H | Et | H | H |
| Me | i-Pr | H | H |
| Et | H | Me | H |
| H | Cl | H | 2-Cl |
| H | Me | Me | H |
| H | Et | Et | H |
| Me | Me | Me | H |
| Et | Me | H | H |
| H | MeO | H | H |
| H | H | MeO | H |

| $R^1$ | $R^5$ | $R^6$ | $R^7$ |
|---|---|---|---|
| Me | EtO | H | H |
| Et | H | EtO | H |
| H | H | Cl | H |
| H | H | $CF_3$ | H |
| H | H | F | H |
| H | MeO | MeO | H |
| Me | MeO | H | 2-MeO |
| Et | F | H | H |
| H | $CF_3$ | H | H |
| H | $CF_3CH_2O$ | H | H |
| H | $HCF_2O$ | H | H |
| Me | H | Cl | H |

TABLE 21

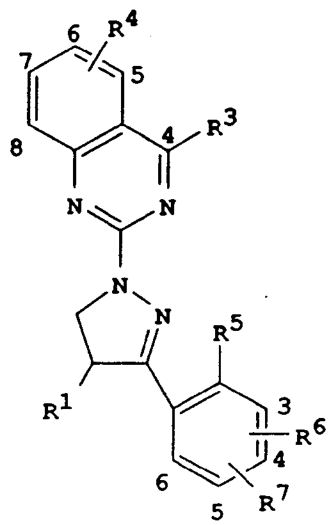

$R^7$ is H; $R^3$ is Me; $R^4$ is 6-Me

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |

$R^7$ is H; $R^3$ is Me; $R^4$ is 6-Me

| $R^1$ | $R^5$ | $R^6$ | $R^1$ | $R^5$ | $R^6$ |
|---|---|---|---|---|---|
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | *i*-Pr | H | H |
| Me | F | 6-F | *i*-Pr | F | H |
| Me | Cl | 6-Cl | *i*-Pr | Cl | H |
| Me | Me | 6-Me | *i*-Pr | Me | H |
| *n*-Pr | $CF_3CH_2O$ | H | *i*-Pr | $CF_3CH_2O$ | H |
| *t*-Bu | $CF_3$ | H | *i*-Pr | $CF_3$ | H |
| *sec*-Bu | MeO | H | *i*-Pr | MeO | H |
| H | $HCF_2O$ | H | H | $HCF_2O$ | 6-$HCF_2O$ |
| H | Br | H | H | I | H |
| H | *t*-BuO | H | H | EtO | H |
| H | H | 4-$NMe_2$ | Me | H | 4-$NEt_2$ |
| H | H | 4-piperidino | Me | H | 4-pyrolidino |

$R^7$ is H; $R^3$ is Me; $R^4$ is H

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |

$R^7$ is H; $R^3$ is Me; $R^4$ is H

| $R^1$ | $R^5$ | $R^6$ | $R^1$ | $R^5$ | $R^6$ |
|---|---|---|---|---|---|
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $NO_2$ | 6-Cl | Me | CN | 6-CN |
| H | Br | 6-Br | Me | $MeS(O)_2$ | 4-F |
| H | $HCF_2O$ | 4-MeO | Me | i-Pr | H |

$R^7$ is H; $R^3$ is H; $R^4$ is H

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| H | F | 5-F | Me | F | H |
| H | Cl | 5-Cl | Me | Cl | H |
| H | Me | 4-F | Me | Me | H |

$R^7$ is H; $R^3$ is H; $R^4$ is H

| $R^1$ | $R^5$ | $R^6$ | $R^1$ | $R^5$ | $R^6$ |
|---|---|---|---|---|---|
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |
| Me | $CF_3$ | 6-Me | Et | $CF_3$ | H |
| Me | MeO | 6-MeO | Et | MeO | H |
| H | H | 4-Br | i-Pr | H | H |
| H | F | 6-F | i-Pr | F | H |
| H | Cl | 6-Cl | i-Pr | Cl | H |
| H | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| Me | t-Bu | 4-MeO | H | TMS | 6-Me |
| Me | i-PrO | H | H | TMS | 4-F |
| Me | $CF_3CF_2CF_2$ | H | H | TMS | 5-$CF_3$ |

$R^1$ is H; $R^3$ is Et; $R^4$ is H

| $R^5$ | $R^6$ | $R^7$ |
|---|---|---|
| H | 4-Cl | 5-Cl |
| H | 4-F | 6-sec-Bu |
| H | 4-Et | 5-I |
| H | 3-F | 6-$CF_3CH_2O$ |
| H | 4-Me | 6-$CF_3CF_2$ |
| H | 4-Br | 6-n-BuO |

$R^1$, $R^3$ is Et; $R^4$ is H

| $R^5$ | $R^6$ | $R^7$ |
|---|---|---|
| Cl | 4-Cl | 6-Cl |
| Cl | 4-Cl | 6-MeO |
| Cl | 3-Me | 4-Cl |
| Cl | 3-$CF_3$ | 5-$CF_3$ |
| Cl | 4-MeO | 5-t-BuO |
| Cl | 3-n-Bu | 4-Me |

| $R^1$ is H; $R^3$ is Et; $R^4$ is H | | | $R^1$, $R^3$ is Et; $R^4$ is H | | |
|---|---|---|---|---|---|
| $R^5$ | $R^6$ | $R^7$ | $R^5$ | $R^6$ | $R^7$ |
| Me | 4-Me | 6-Me | TMS | H | H |
| Me | 4-F | 6-Me | TMS | H | 4-F |
| Me | 4-*t*-Bu | 6-*t*-Bu | TMS | H | 6-Me |
| Me | 4-$CF_3$ | 6-Cl | TMS | H | 6-MeO |
| Me | 3-Me | 5-Br | TMS | H | 6-Cl |
| Me | 5-*i*-Pr | 6-MeO | TMS | H | 6-$HCF_2O$ |
| *t*-Bu | 6-*t*-Bu | H | Br | 6-Br | H |
| *t*-Bu | 4-*t*-BuO | H | $NMe_2$ | H | H |
| *t*-Bu | H | H | CONHEt | H | H |
| $CF_3(CH_2)_3O$ | H | H | CN | H | H |
| $CF_3(CF_2)_2$ | H | H | 4-F-Ph | H | H |
| $(CF_3)_2CH$ | H | H | 2-MePh | H | H |
| *sec*-BuS | H | H | $NO_2$ | 6-Me | H |
| MeS | 6-MeS | H | 4-Me-PhO | H | H |
| EtS | 4-F | H | PhS | H | H |
| MeS(O) | H | H | $CO_2H$ | 3-MeO | H |
| *i*-PrS(O) | H | H | $CO_2H$ | H | H |
| *t*-BuS$(O)_2$ | H | H | HC≡C | H | H |
| Me$S(O)_2$ | H | H | MeC≡C | H | H |
| $CH_2$=CH | H | H | MeC≡$CCH_2O$ | 4-F | H |
| $CH_2=C(CH_3)CH_2$ | H | H | *t*-BuO | H | H |
| $CH_2=CHCH_2O$ | H | H | *n*-PrO | H | H |
| $MeOCH_2CH_2$ | H | H | EtO | 5-EtO | H |
| $MeO_2C$ | H | H | Ac | H | H |
| $MeOCH_2O$ | H | H | *sec*-BuCO | H | H |

TABLE 22

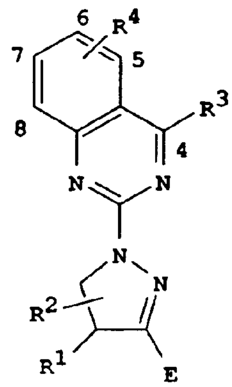

| $R^1$, $R^2$ and $R^4$ are H; $R^3$ is Me | $R^1$ and $R^2$ are H; $R^3$ is Me; $R^4$ is 6-Me |
|---|---|
| E | E |
| 1-naphthalenyl | 1-naphthalenyl |
| 2-furanyl | 2-furanyl |
| 2-naphthalenyl | 2-naphthalenyl |
| 3-thienyl | 3-thienyl |
| 2,5-dimethyl-3-furanyl | 2,5-dimethyl-3-furanyl |
| 2,5-dimethyl-3-thienyl | 2,5-dimethyl-3-thienyl |
| 4-methylphenoxy | 4-methylphenoxy |
| 2-chlorophenoxy | 2-chlorophenoxy |
| 2,6-dimethylphenoxy | 2,6-dimethylphenoxy |
| 3-methylphenylthio | 4-cyanophenylthio |
| phenylamino | 4-methylphenylamino |
| benzyl | Cl |
| Et | n-hex |
| sec-Bu | Me |
| c-propyl | c-hexyl |
| cis-2-methylcycloheptyl | $CF_3CH_2CH_2$ |
| sec-butylthio | n-butoxy |
| $CF_3CH_2O$ | $Cl(CH_2)_5O$ |
| 5-methyl-2-thienyl | 4-methyl-3-furanyl |
| 5-methyl-2-pyridyl | 2-methyl-3-pyridyl |

$R^1$, $R^2$ and $R^4$ are H;
$R^3$ is Me

| E |
|---|
| 2-indanyl |
| 2-tetrahydronaphthalenyl |

$R^1$ and $R^2$ are H; $R^3$ is Me;
$R^4$ is 6-Me

| E |
|---|
| 2-indanyl |
| 2-tetrahydronaphthalenyl |

$R^1$, $R^2$, $R^3$ and $R^4$ are H

| E |
|---|
| 1-naphthalenyl |
| 2-furanyl |
| 3-thienyl |
| 3-pyridyl |

$R^1$ and $R^3$ are Me; $R^2$ and $R^4$ are H;

| E |
|---|
| 1-naphthalenyl |
| 2-furanyl |
| 3-thienyl |
| 3-pyridyl |

$R^3$ is Me; $R^4$ is H

| $R^1$ | $R^2$ | E |
|---|---|---|
| H | 5-Me | Ph |
| H | 5-*i*-Pr | 2-Me-Ph |
| H | 5-*n*-Bu | 2-Cl-Ph |
| H | 5-CN | 2-MeO-Ph |
| H | 5-$CF_3$ | $CF_3CH_2O$-Ph |
| H | 5-$CF_3CH_2$ | 1-naphthalenyl |
| *i*-Pr | 5-Me | Ph |
| *i*-Pr | 5-Me | 2-Me-Ph |
| *i*-Pr | 5-Me | 2-Cl-Ph |
| *i*-Pr | 5-Me | 2-MeO-Ph |

$R^3$ is Et; $R^4$ is H

| $R^1$ | $R^2$ | E |
|---|---|---|
| H | 5-Et | Ph |
| H | 5-*sec*-Bu | 2-Me-Ph |
| H | 5-$CF_3(CF_2)_3$ | 2-Cl-Ph |
| H | 5-*t*-Bu | 2-MeO-Ph |
| H | 5-$FCH_2$ | 2-$CF_3CH_2O$-Ph |
| H | 5-*n*-Pr | 1-naphthalenyl |
| Me | 4-Me | Ph |
| Me | 4-Me | 2-Me-Ph |
| Me | 4-Me | 2-Cl-Ph |
| Me | 4-Me | 2-MeO-Ph |

$R^3$ is Me; $R^4$ is H

| $R^1$ | $R^2$ | E |
|---|---|---|
| i-Pr | 5-Me | 2-$CF_3CH_2O$-Ph |
| Cl | H | Ph |
| F | H | 2-Me-Ph |
| $CF_3CF_2$ | H | 2-Cl-Ph |
| $CH_2$=$CHCH_2$ | H | 2-MeO-Ph |
| $CO_2Me$ | H | 2-$CF_3CH_2O$-Ph |
| 2-Me-Ph | H | Me |
| Bzl | H | Ph |
| 2-naphthalenyl | H | n-Bu |
| 3-thienyl | H | $CF_3CF_2$ |
| 3-pyridyl | H | Me |
| CN | 5-Me | Ph |
| t-Bu | 5-Me | 2-Me-Ph |
| $ClCH_2$ | 5-Me | 2-Cl-Ph |
| Et | 5-Me | 2-MeO-Ph |
| n-Pr | 5-Me | 2-$CF_3CH_2O$-Ph |
| Me | 4-Me | 2-$CF_3$-Ph |
| i-Pr | 4-Me | 2-$CF_3$-Ph |
| $CF_3$ | 4-$CF_3$ | 2-$CF_3$-Ph |
| Me | 4-Me | 2-TMS-Ph |
| H | 5-OH | Ph |
| H | 5-MeO | 4-Me-Ph |
| H | 5-OC(O)Me | 4-Cl-Ph |
| H | 5-OC(O)NHMe | Ph |

$R^3$ is Me; $R^4$ is Me

| $R^1$ | $R^2$ | E |
|---|---|---|
| i-Pr | 5-Me | 2-Cl-Ph |
| i-Pr | 5-Me | 2-MeO-Ph |
| i-Pr | 6-Me | 2-$CF_3CH_2O$-Ph |
| Cl | H | Ph |
| F | H | 4-Me-Ph |
| $CF_3CF_2$ | H | 4-Cl-Ph |
| $CH_2$=$CHCH_2$ | H | 4-MeO-Ph |

$R^3$ is Me; $R^4$ is H

| $R^1$ | $R^2$ | E |
|---|---|---|
| $CO_2Me$ | H | 2-$CF_3CH_2O$-Ph |
| 2-Me-Ph | H | Me |
| Bzl | H | Ph |
| 2-naphthalenyl | H | n-Bu |
| 3-thienyl | H | $CF_3CF_2$ |
| 3-pyridyl | H | Me |
| CN | 5-Me | Ph |
| t-Bu | 5-Me | 2-Me-Ph |
| $ClCH_2$ | 5-Me | 2-Cl-Ph |
| Et | 5-Me | 2-MeO-Ph |
| n-Pr | 6-Me | 2-$CF_3CH_2O$-Ph |
| Me | 4-Me | 2-$CF_3$-Ph |
| i-Pr | 4-Me | 2-$CF_3$-Ph |
| $CF_3$ | 4-$CF_3$ | 2-$CF_3$-Ph |

$R^3$ is Et; $R^4$ is H

| $R^1$ | $R^2$ | E |
|---|---|---|
| Me | 4-Me | 2-TMS-Ph |
| Me | 4-Me | 2-Cl-Ph |
| Me | 4-Me | 2-MeO-Ph |
| Me | 4-Me | 2-$CF_3CH_2O$-Ph |
| Br | H | Ph |
| CN | H | 4-Me-Ph |
| Ac | H | 4-Cl-Ph |
| $CH_3C{\equiv}CCH_2$ | H | 4-MeO-Ph |

$R^3$ is 4-Me; $R^4$ is Me

| $R^1$ | $R^2$ | E |
|---|---|---|
| $CO_2Et$ | H | 2-$CF_3CH_2O$-Ph |
| 4-Cl-Ph | H | Ph |
| 5-Me-3-furyl | H | i-Pr |
| EtCO | H | 2-Cl-Ph |
| 2-furyl | 4-Me | $CF_3$ |
| Ph | 5-Me | Me |
| CN | 4-Me | Ph |
| t-Bu | 4-Me | 2-Me-Ph |
| $FCH_2$ | 4-Me | 2-Cl-Ph |
| Et | 4-Me | 2-MeO-Ph |
| $Cl(CH_2)_4$ | 4-Me | 2-$CF_3CH_2O$-Ph |
| Me | 4-Me | 2-$CF_3$-Ph |
| i-Pr | 5-CN | 2-$CF_3$-Ph |
| $CF_3$ | 5-Me | 2-$CF_3$-Ph |
| i-Pr | 4-Me | 2-TMS-Ph |

TABLE 23

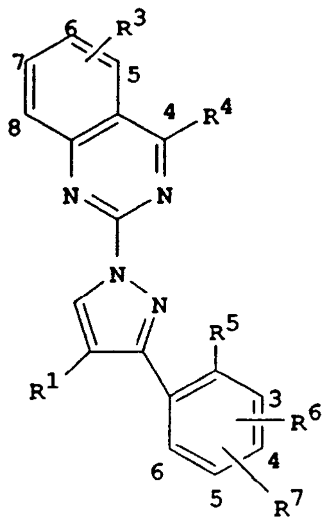

$R^7$ is H; $R^3$ is H; $R^4$ is Me

| $R^1$ | $R^5$ | $R^6$ | $R^1$ | $R^5$ | $R^6$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |

$R^7$ is H; $R^3$ is H; $R^4$ is Me

| $R^1$ | $R^5$ | $R^6$ | $R^1$ | $R^5$ | $R^6$ |
|---|---|---|---|---|---|
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| H | $HCF_2O$ | H | H | $HCF_2O$ | 6-$HCF_2O$ |
| H | Br | H | H | I | H |
| H | t-BuO | H | H | EtO | H |

TABLE 24

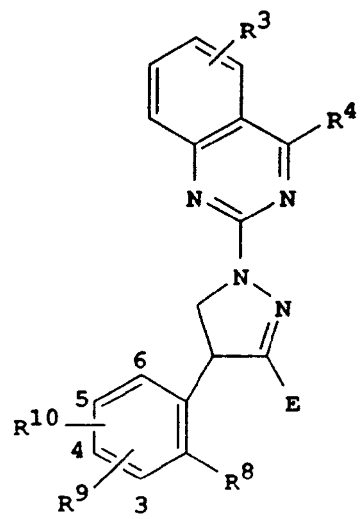

$R^3$ is H; $R^4$ is Me; $R^{10}$ is H

| E | $R^8$ | $R^9$ | E | $R^8$ | $R^9$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |
| H | H | 3-$CF_3$ | Et | H | H |
| H | F | 6-F | Et | F | H |
| H | Cl | 6-Cl | Et | Cl | H |
| H | Me | 6-Me | Et | Me | H |

$R^3$ is H; $R^4$ is Me; $R^{10}$ is H

| E | $R^8$ | $R^9$ | E | $R^8$ | $R^9$ |
|---|---|---|---|---|---|
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| Ph | $HCF_2O$ | H | H | $HCF_2O$ | 6-$HCF_2O$ |
| H | Br | H | Ph | I | H |
| H | t-BuO | H | H | EtO | H |

$R^4$ is Et; $R^3$ and $R^{10}$ are H

| E | $R^8$ | $R^9$ | E | $R^8$ | $R^9$ |
|---|---|---|---|---|---|
| H | H | H | H | H | 4-F |
| H | F | H | H | F | 4-F |
| H | Cl | H | H | Cl | 4-F |
| H | Me | H | H | Me | 4-F |
| H | $CF_3CH_2O$ | H | H | $CF_3CH_2O$ | 4-F |
| H | $CF_3$ | H | H | $CF_3$ | 4-F |
| H | MeO | H | H | MeO | 4-F |
| H | H | 4-Cl | Me | H | H |
| Me | F | 5-F | Me | F | H |
| Me | Cl | 5-Cl | Me | Cl | H |
| Me | Me | 4-F | Me | Me | H |
| Me | $CF_3CH_2O$ | 4-F | Me | $CF_3CH_2O$ | H |
| Me | $CF_3$ | 4-F | Me | $CF_3$ | H |
| Me | MeO | 4-F | Me | MeO | H |

$R^4$ is Et; $R^3$ and $R^{10}$ are H

| E | $R^8$ | $R^9$ | E | $R^8$ | $R^9$ |
|---|---|---|---|---|---|
| H | H | 3-$CF_3$ | Et | H | H |
| H | F | 6-F | Et | F | H |
| H | Cl | 6-Cl | Et | Cl | H |
| H | Me | 6-Me | Et | Me | H |
| H | $CF_3CH_2O$ | 6-Me | Et | $CF_3CH_2O$ | H |
| H | $CF_3$ | 6-Me | Et | $CF_3$ | H |
| H | MeO | 6-MeO | Et | MeO | H |
| H | H | 4-Br | i-Pr | H | H |
| Me | F | 6-F | i-Pr | F | H |
| Me | Cl | 6-Cl | i-Pr | Cl | H |
| Me | Me | 6-Me | i-Pr | Me | H |
| n-Pr | $CF_3CH_2O$ | H | i-Pr | $CF_3CH_2O$ | H |
| t-Bu | $CF_3$ | H | i-Pr | $CF_3$ | H |
| sec-Bu | MeO | H | i-Pr | MeO | H |
| Ph | $HCF_2O$ | 4-MeO | H | $HCF_2O$ | 6-$HCF_2O$ |
| H | Br | H | Ph | I | H |
| H | t-BuO | H | H | EtO | H |

TABLE 25

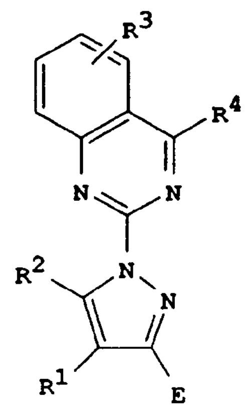

| $R^3$ is H; $R^4$ is Et | | | $R^3$ is H; $R^4$ is Me | | |
|---|---|---|---|---|---|
| $R^1$ | $R^2$ | E | $R^1$ | $R^2$ | E |
| H | Me | Ph | H | Et | Ph |
| H | i-Pr | 2-Me-Ph | H | sec-Bu | 2-Me-Ph |
| H | n-Bu | 2-Cl-Ph | H | $CF_3(CF_2)_3$ | 2-Cl-Ph |
| H | CN | 2-MeO-Ph | H | t-Bu | 2-MeO-Ph |
| H | $CF_3$ | $CF_3CH_2O$-Ph | H | $FCH_2$ | 2-$CF_3CH_2O$-Ph |
| H | $CF_3CH_2$ | 1-naphthalenyl | H | n-Pr | 1-naphthalenyl |
| i-Pr | Me | Ph | Me | Me | Ph |
| i-Pr | Me | 2-Me-Ph | Me | Me | 2-Me-Ph |
| i-Pr | Me | 2-Cl-Ph | Me | Me | 2-Cl-Ph |
| i-Pr | Me | 2-MeO-Ph | Me | Me | 2-MeO-Ph |
| i-Pr | Me | 2-$CF_3CH_2O$-Ph | Me | Me | 2-$CF_3CH_2O$-Ph |
| Cl | H | Ph | Br | H | Ph |
| F | H | 2-Me-Ph | CN | H | 2-Me-Ph |
| $CF_3CF_2$ | H | 2-Cl-Ph | Ac | H | 2-Cl-Ph |

$R^3$ is H; $R^4$ is Et

| $R^1$ | $R^2$ | E |
|---|---|---|
| $CH_2{=}CHCH_2$ | H | 2-MeO-Ph |
| $CO_2Me$ | H | 2-$CF_3CH_2O$-Ph |
| 2-Me-Ph | H | Me |
| Bzl | H | Ph |
| 2-naphthalenyl | H | *n*-Bu |
| 3-thienyl | H | $CF_3CF_2$ |
| 3-pyridyl | H | Me |
| Ph | Me | H |
| 2-Me-Ph | Me | H |
| 2-Cl-Ph | Me | H |
| 2-MeO-Ph | Me | H |
| 2-$CF_3CH_2O$-Ph | Me | H |
| Ph | Me | *n*-Pr |
| Ph | Me | $CF_3$ |
| Ph | Me | *i*-Pr |
| Ph | Me | *sec*-Bu |

$R^3$ is H; $R^4$ is Me

| $R^1$ | $R^2$ | E |
|---|---|---|
| $CH_3C{\equiv}CCH_2$ | H | 2-MeO-Ph |
| $CO_2Et$ | H | 2-$CF_3CH_2O$-Ph |
| 4-Cl-Ph | H | Ph |
| 5-Me-3-furyl | H | Ph |
| EtCO | H | *i*-Pr |
| 2-furyl | H | 2-Cl-Ph |
| Ph | Me | $CF_3$ |
| Ph | Me | H |
| 2-Me-Ph | Me | H |
| 2-Cl-Ph | Me | H |
| 2-MeO-Ph | Me | H |
| 2-$CF_3CH_2O$-Ph | Me | H |
| Ph | *i*-Pr | Me |
| Ph | CF3 | Me |
| Ph | Et | Me |
| Ph | *n*-Bu | Me |

TABLE 26

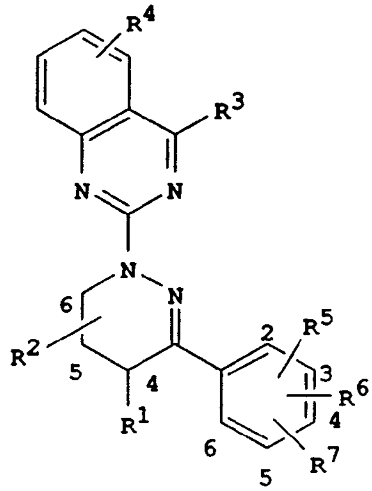

$R^2$, $R^4$ and $R^7$ are H; $R^3$ is Me

| $R^1$ | $R^5$ | $R^6$ | $R^1$ | $R^5$ | $R^6$ |
|---|---|---|---|---|---|
| H | H | H | Me | 4-Me | H |
| H | 4-NMe$_2$ | H | Me | 4-Et | H |
| H | 4-Me | H | Me | 4-i-Pr | H |
| H | 4-Et | H | Me | 4-Cl | H |
| H | 4-n-Pr | H | Me | 4-MeO | H |
| H | 4-i-Pr | H | Me | 4-EtO | H |
| H | 4-n-Bu | H | Me | 4-CF$_3$ | H |
| H | 4-sec-Bu | H | Et | H | H |
| H | 4-i-Bu | H | H | 3-NMe$_2$ | H |
| H | 4-t-Bu | H | H | 3-Me | H |
| H | 4-Cl | H | H | 3-Et | H |
| H | 4-Br | H | H | 3-n-Pr | H |
| H | 4-F | H | H | 3-i-Pr | H |
| H | 4-OH | H | H | 3-n-Bu | H |
| H | 4-MeO | H | H | 3-Cl | H |
| H | 4-EtO | H | H | 3-Br | H |
| H | 4-CF$_3$ | H | H | 3-F | H |
| H | 4-CF$_3$CH$_2$O | H | H | 3-OH | H |
| Me | H | H | H | 3-MeO | H |

$R^2$, $R^4$ and $R^7$ are H; $R^3$ is Me

| $R^1$ | $R^5$ | $R^6$ | $R^1$ | $R^5$ | $R^6$ |
|---|---|---|---|---|---|
| H | 3-EtO | H | H | 3-Me | 4-Me |
| H | 3-$CF_3$ | H | H | 2-Et | 4-Et |
| H | 3-$CF_3CH_2O$ | H | H | 2-Et | 5-Et |
| Me | 3-Me | H | H | 3-Et | 4-Et |
| Me | 3-Et | H | H | 2-Me | 5-t-Bu |
| Me | 3-i-Pr | H | H | 2-Cl | 4-Cl |
| Me | 3-Cl | H | H | 2-Cl | 5-Cl |
| Me | 3-MeO | H | Et | 3-MeO | H |
| Me | 3-EtO | H | Et | 3-EtO | H |
| Me | 3-$CF_3$ | H | Et | 3-$CF_3$ | H |
| Et | 3-Me | H | Me | 2-Me | 4-Me |
| Et | 3-Et | H | Me | 2-Me | 5-Me |
| Et | 3-i-Pr | H | Me | 3-Me | 4-Me |
| Et | 3-Cl | H | Me | 2-Et | 4-Et |
| Et | 4-Me | H | Me | 2-Et | 5-Et |
| Et | 4-Et | H | Me | 3-Et | 4-Et |
| Et | 4-i-Pr | H | Me | 2-Me | 5-t-Bu |
| Et | 4-Cl | H | Et | 2-Me | 4-Me |
| Et | 4-MeO | H | Et | 2-Me | 5-Me |
| Et | 4-EtO | H | Et | 3-Me | 4-Me |
| Et | 4-$CF_3$ | H | Et | 2-Et | 4-Et |
| H | 2-Me | H | Et | 2-Et | 5-Et |
| H | 2-Et | H | Et | 3-Et | 4-Et |
| H | 2-Cl | H | H | 4-Ph | H |
| H | 2-F | H | H | 4-PhO | H |
| H | 2-OH | H | H | 4-c-Hex | H |
| Me | 2-Me | H | H | 4-Hex | H |
| Me | 2-Cl | H | H | 4-n-Amyl | H |
| Me | 2-F | H | Me | 4-Ph | H |
| Et | 2-Me | H | Me | 4-PhO | H |
| Et | 2-Cl | H | Me | 4-c-Hex | H |
| Et | 2-F | H | Me | 4-Hex | H |
| H | 2-Me | 4-Me | Me | 4-n-Amyl | H |
| H | 2-Me | 5-Me | Me | 4-Ph | H |

$R^2$, $R^4$ and $R^7$ are H; $R^3$ is Me

| $R^1$ | $R^5$ | $R^6$ | $R^1$ | $R^5$ | $R^6$ |
|---|---|---|---|---|---|
| Me | 4-PhO | H | Et | 3-$NMe_2$ | H |
| Me | 4-c-Hex | H | H | 3-$NH_2$ | H |
| Me | 4-n-Amyl | H | H | 4-$NH_2$ | H |
| Me | 3-Cl | 4-Cl | Me | 3-$NH_2$ | H |
| Me | 2-Cl | 4-Cl | Me | 4-$NH_2$ | H |
| Me | 2-Cl | 5-Cl | Et | 3-$NH_2$ | H |
| Me | 3-Cl | 4-Cl | Et | 4-$NH_2$ | H |
| Et | 2-Cl | 4-Cl | n-Pr | 4-$NMe_2$ | H |
| Et | 2-Cl | 5-Cl | n-Pr | 4-Me | H |
| Et | 3-Cl | 4-Cl | n-Pr | 4-Et | H |
| H | 2-MeO | 4-MeO | n-Pr | 4-n-Pr | H |
| H | 3-MeO | 5-MeO | n-Pr | 4-Cl | H |
| H | 3-MeO | 4-MeO | n-Pr | 4-F | H |
| Me | 2-MeO | 4-MeO | n-Pr | 4-Br | H |
| Me | 3-MeO | 5-MeO | n-Pr | 4-MeO | H |
| Me | 3-MeO | 4-MeO | n-Pr | 4-EtO | H |
| Et | 2-MeO | 4-MeO | n-Pr | 4-$CF_3$ | H |
| Et | 3-MeO | 5-MeO | n-Pr | 4-$CF_3CH_2O$ | H |
| Et | 3-MeO | 4-MeO | n-Pr | 3-$NMe_2$ | H |
| H | 3-Br | 5-Br | n-Pr | 3-Me | H |
| Me | 3-Br | 5-Br | n-Pr | 3-Et | H |
| Et | 3-Br | 5-Br | n-Pr | 3-n-Pr | H |
| H | 3-Me | 5-Me | n-Pr | 3-Cl | H |
| Me | 3-Me | 5-Me | n-Pr | 3-F | H |
| Et | 3-Me | 5-Me | n-Pr | 3-Br | H |
| H | 3-Cl | 4-MeO | n-Pr | 3-MeO | H |
| Me | 3-Cl | 4-MeO | n-Pr | 3-EtO | H |
| Et | 3-Cl | 4-MeO | n-Pr | 3-$CF_3$ | H |
| Me | 4-$NMe_2$ | H | n-Pr | 3-$CF_3CH_2O$ | H |
| Me | 3-$NMe_2$ | H | n-Pr | 3-Me | 4-Me |
| Et | 4-$NMe_2$ | H | n-Pr | 3-Me | 5-Me |

$R^2$, $R^4$ and $R^7$ are H; $R^3$ is Me

| $R^1$ | $R^5$ | $R^6$ | $R^1$ | $R^5$ | $R^6$ |
|---|---|---|---|---|---|
| n-Pr | 3-Cl | 4-Cl | i-Pr | 4-Cl | H |
| n-Pr | 3-MeO | 4-MeO | i-Pr | 4-F | H |
| n-Pr | 3-MeO | 5-MeO | i-Pr | 4-Br | H |
| n-Pr | H | H | i-Pr | 4-MeO | H |
| n-Bu | H | H | i-Pr | 4-EtO | H |
| n-Bu | 4-Me | H | i-Pr | 4-$CF_3$ | H |
| n-Bu | 4-Et | H | i-Pr | 4-$CF_3CH_2O$ | H |
| n-Bu | 4-n-Pr | H | i-Pr | 3-Me | 4-Me |
| n-Bu | 4-i-Pr | H | i-Pr | 3-Me | 5-Me |
| n-Bu | 4-Cl | H | i-Pr | 3-Cl | 4-Cl |
| n-Bu | 4-F | H | i-Pr | 3-MeO | 4-MeO |
| n-Bu | 4-Br | H | i-Pr | 3-MeO | 5-MeO |
| n-Bu | 4-MeO | H | H | 4-TMS | H |
| n-Bu | 4-EtO | H | H | 4-I | H |
| n-Bu | 4-$CF_3$ | H | H | 4-t-BuO | H |
| n-Bu | 4-$CF_3CH_2O$ | H | H | 4-$CF_3(CH_2)_3O$ | H |
| n-Bu | 3-Me | H | H | 4-$CF_3(CF_2)_2$ | H |
| n-Bu | 3-Et | H | H | 4-$(CF_3)_2CH$ | H |
| n-Bu | 3-n-Pr | H | H | 4-$CH_3CHClCH$ | H |
| n-Bu | 3-Cl | H | Me | 4-TMS | H |
| n-Bu | 3-F | H | Me | 4-I | H |
| n-Bu | 3-MeO | H | Me | 4-t-BuO | H |
| n-Bu | 3-EtO | H | Me | 4-$CF_3(CH_2)_3O$ | H |
| n-Bu | 3-$CF_3$ | H | H | 4-MeS | H |
| n-Bu | 3-$CF_3CH_2O$ | H | H | 4-EtS | H |
| i-Pr | H | H | H | 4-MeS(O) | H |
| i-Pr | 4-Me | H | H | 4-i-PrS(O) | H |
| i-Pr | 4-Et | H | H | 4-MeS$(O)_2$ | H |
| i-Pr | 4-n-Pr | H | H | 4-$CH_2$=CH | H |
| i-Pr | 4-i-Pr | H | H | 4-$CH_2$=$C(CH_3)CH_2$ | H |

$R^2$, $R^4$ and $R^7$ are H, $R^3$ is Et

| $R^1$ | $R^5$ | $R^6$ | $R^1$ | $R^5$ | $R^6$ |
|---|---|---|---|---|---|
| H | 4-$CH_2$=$CHCH_2O$ | H | H | 3-Me | H |
| H | 4-$MeOCH_2CH_2$ | H | H | 3-Et | H |
| H | 4-$MeOCH_2O$ | H | H | 3-n-Pr | H |
| H | H | H | H | 3-i-Pr | H |
| H | 4-$NMe_2$ | H | H | 3-n-Bu | H |
| H | 4-Me | H | H | 3-Cl | H |
| H | 4-Et | H | H | 3-Br | H |
| H | 4-n-Pr | H | H | 3-F | H |
| H | 4-i-Pr | H | H | 3-OH | H |
| H | 4-n-Bu | H | H | 3-MeO | H |
| H | 4-sec-Bu | H | H | 3-EtO | H |
| H | 4-i-Bu | H | H | 3-$CF_3$ | H |
| H | 4-t-Bu | H | H | 3-$CF_3CH_2O$ | H |
| H | 4-Cl | H | Me | 3-Me | H |
| H | 4-Br | H | Me | 3-Et | H |
| H | 4-F | H | Me | 3-i-Pr | H |
| H | 4-OH | H | Me | 3-Cl | H |
| H | 4-MeO | H | Me | 3-MeO | H |
| H | 4-EtO | H | Me | 3-EtO | H |
| H | 4-$CF_3$ | H | Me | 3-$CF_3$ | H |
| H | 4-$CF_3CH_2O$ | H | Et | 3-Me | H |
| Me | H | H | Et | 3-Et | H |
| Me | 4-Me | H | Et | 3-i-Pr | H |
| Me | 4-Et | H | Et | 3-Cl | H |
| Me | 4-i-Pr | H | Et | 4-Me | H |
| Me | 4-Cl | H | Et | 4-Et | H |
| Me | 4-MeO | H | Et | 4-i-Pr | H |
| Me | 4-EtO | H | Et | 4-Cl | H |
| Me | 4-$CF_3$ | H | Et | 4-MeO | H |
| Et | H | H | Et | 4-EtO | H |
| H | 3-$NMe_2$ | H | Et | 4-$CF_3$ | H |

$R^2$, $R^4$ and $R^7$ are H, $R^3$ is Et

| $R^1$ | $R^5$ | $R^6$ | $R^1$ | $R^5$ | $R^6$ |
|---|---|---|---|---|---|
| H | 2-Me | H | Et | 3-Me | 4-Me |
| H | 2-Et | H | Et | 2-Et | 4-Et |
| H | 2-Cl | H | Et | 2-Et | 5-Et |
| H | 2-F | H | Et | 3-Et | 4-Et |
| H | 2-OH | H | H | 4-Ph | H |
| Me | 2-Me | H | H | 4-PhO | H |
| Me | 2-Cl | H | H | 4-c-Hex | H |
| Me | 2-F | H | H | 4-Hex | H |
| Et | 2-Me | H | H | 4-n-Amyl | H |
| Et | 2-Cl | H | Me | 4-Ph | H |
| Et | 2-F | H | Me | 4-PhO | H |
| H | 2-Me | 4-Me | Me | 4-c-Hex | H |
| H | 2-Me | 5-Me | Me | 4-Hex | H |
| H | 3-Me | 4-Me | Me | 4-n-Amyl | H |
| H | 2-Et | 4-Et | H | 3-Cl | 4-Cl |
| H | 2-Et | 5-Et | Me | 2-Cl | 4-Cl |
| H | 3-Et | 4-Et | Me | 2-Cl | 5-Cl |
| H | 2-Me | 5-t-Bu | Me | 3-Cl | 4-Cl |
| H | 2-Cl | 4-Cl | Et | 2-Cl | 4-Cl |
| H | 2-Cl | 5-Cl | Et | 2-Cl | 5-Cl |
| Et | 3-MeO | H | Et | 3-Cl | 4-Cl |
| Et | 3-EtO | H | H | 2-MeO | 4-MeO |
| Et | 3-$CF_3$ | H | H | 3-MeO | 5-MeO |
| Me | 2-Me | 4-Me | H | 3-MeO | 4-MeO |
| Me | 2-Me | 5-Me | Me | 2-MeO | 4-MeO |
| Me | 3-Me | 4-Me | Me | 3-MeO | 5-MeO |
| Me | 2-Et | 4-Et | Me | 3-MeO | 4-MeO |
| Me | 2-Et | 5-Et | Et | 2-MeO | 4-MeO |
| Me | 3-Et | 4-Et | Et | 3-MeO | 5-MeO |
| Me | 2-Me | 5-t-Bu | Et | 3-MeO | 4-MeO |
| Et | 2-Me | 4-Me | H | 3-Br | 5-Br |
| Et | 2-Me | 5-Me | Me | 3-Br | 5-Br |

$R^2$, $R^4$ and $R^7$ are H, $R^3$ is Et

| $R^1$ | $R^5$ | $R^6$ | $R^1$ | $R^5$ | $R^6$ |
|---|---|---|---|---|---|
| Et | 3-Br | 5-Br | n-Pr | 3-n-Pr | H |
| H | 3-Me | 5-Me | n-Pr | 3-Cl | H |
| Me | 3-Me | 5-Me | n-Pr | 3-F | H |
| Et | 3-Me | 5-Me | n-Pr | 3-Br | H |
| H | 3-Cl | 4-MeO | n-Pr | 3-MeO | H |
| Me | 3-Cl | 4-MeO | n-Pr | 3-EtO | H |
| Et | 3-Cl | 4-MeO | n-Pr | 3-$CF_3$ | H |
| Me | 4-$NMe_2$ | H | n-Pr | 3-$CF_3CH_2O$ | H |
| Me | 3-$NMe_2$ | H | n-Pr | 3-Me | 4-Me |
| Et | 4-$NMe_2$ | H | n-Pr | 3-Me | 5-Me |
| Et | 3-$NMe_2$ | H | n-Pr | 3-Cl | 4-Cl |
| H | 3-$NH_2$ | H | n-Pr | 3-MeO | 4-MeO |
| H | 4-$NH_2$ | H | n-Pr | 3-MeO | 5-MeO |
| Me | 3-$NH_2$ | H | n-Pr | H | H |
| Me | 4-$NH_2$ | H | n-Bu | H | H |
| Et | 3-$NH_2$ | H | n-Bu | 4-Me | H |
| Et | 4-$NH_2$ | H | n-Bu | 4-Et | H |
| n-Pr | 4-$NMe_2$ | H | n-Bu | 4-n-Pr | H |
| n-Pr | 4-Me | H | n-Bu | 4-i-Pr | H |
| n-Pr | 4-Et | H | n-Bu | 4-Cl | H |
| n-Pr | 4-n-Pr | H | n-Bu | 4-F | H |
| n-Pr | 4-Cl | H | n-Bu | 4-Br | H |
| n-Pr | 4-F | H | n-Bu | 4-MeO | H |
| n-Pr | 4-Br | H | n-Bu | 4-EtO | H |
| n-Pr | 4-MeO | H | n-Bu | 4-$CF_3$ | H |
| n-Pr | 4-EtO | H | n-Bu | 4-$CF_3CH_2O$ | H |
| n-Pr | 4-$CF_3$ | H | n-Bu | 3-Me | H |
| n-Pr | 4-$CF_3CH_2O$ | H | n-Bu | 3-Et | H |
| n-Pr | 3-$NMe_2$ | H | n-Bu | 3-n-Pr | H |
| n-Pr | 3-Me | H | n-Bu | 3-Cl | H |
| n-Pr | 3-Et | H | n-Bu | 3-F | H |

$R^2$, $R^4$ and $R^7$ are H, $R^3$ is Et

| $R^1$ | $R^5$ | $R^6$ | $R^1$ | $R^5$ | $R^6$ |
|---|---|---|---|---|---|
| n-Bu | 3-MeO | H | H | 4-TMS | H |
| n-Bu | 3-EtO | H | H | 4-I | H |
| n-Bu | $3-CF_3$ | H | H | 4-t-BuO | H |
| n-Bu | $3-CF_3CH_2O$ | H | H | $4-CF_3(CH_2)_3O$ | H |
| i-Pr | H | H | H | $4-CF_3(CF_2)_2$ | H |
| i-Pr | 4-Me | H | H | $4-(CF_3)_2CH$ | H |
| i-Pr | 4-Et | H | H | $4-CH_3CHClCH$ | H |
| i-Pr | 4-n-Pr | H | Me | 4-TMS | H |
| i-Pr | 4-i-Pr | H | Me | 4-I | H |
| i-Pr | 4-Cl | H | Me | 4-t-BuO | H |
| i-Pr | 4-F | H | Me | $4-CF_3(CH_2)_3O$ | H |
| i-Pr | 4-Br | H | H | 4-MeS | H |
| i-Pr | 4-MeO | H | H | 4-EtS | H |
| i-Pr | 4-EtO | H | H | 4-MeS(O) | H |
| i-Pr | $4-CF_3$ | H | H | 4-i-PrS(O) | H |
| i-Pr | $4-CF_3CH_2O$ | H | H | $4-MeS(O)_2$ | H |
| i-Pr | 3-Me | 4-Me | H | $4-CH_2=CH$ | H |
| i-Pr | 3-Me | 5-Me | H | $4-CH_2=C(CH_3)CH_2$ | H |
| i-Pr | 3-Cl | 4-Cl | H | $4-CH_2=CHCH_2O$ | H |
| i-Pr | 3-MeO | 4-MeO | H | $4-MeOCH_2CH_2$ | H |
| i-Pr | 3-MeO | 5-MeO | H | $4-MeOCH_2O$ | H |

$R^2$ is H; $R^3$ is Me; $R^4$ is H

| $R^1$ | $R^5$ | $R^6$ | $R^7$ |
|---|---|---|---|
| H | 3-Me | 4-Me | 5-Me |
| H | 3-Br | 4-Me | 5-Br |
| H | 3-Cl | 4-MeO | 5-Cl |
| H | 3-MeO | 4-MeO | 5-MeO |

$R^2$ is H; $R^3$ is Et; $R^4$ is H;

| $R^1$ | $R^5$ | $R^6$ | $R^7$ |
|---|---|---|---|
| H | 3-Me | 4-Me | 5-Me |
| H | 3-Br | 4-Me | 5-Br |
| H | 3-Cl | 4-MeO | 5-Cl |
| H | 3-MeO | 4-MeO | 5-MeO |

| $R^2$ is H; $R^3$ is Me; $R^4$ is H | | | | $R^2$ is H; $R^3$ is Et; $R^4$ is H | | | |
|---|---|---|---|---|---|---|---|
| $R^1$ | $R^5$ | $R^6$ | $R^7$ | $R^1$ | $R^5$ | $R^6$ | $R^7$ |
| Me | 3-Me | 4-Me | 5-Me | Me | 3-Me | 4-Me | 5-Me |
| Me | 3-Br | 4-Me | 5-Br | Me | 3-Br | 4-Me | 5-Br |
| Me | 3-Cl | 4-MeO | 5-Cl | Me | 3-Cl | 4-Me | 5-Cl |
| Me | 3-MeO | 6-MeO | 5-MeO | Me | 3-MeO | 4-MeO | 5-MeO |
| H | 4-TMS | H | H | H | 4-TMS | H | H |
| Me | 4-TMS | H | H | Me | 4-TMS | H | H |
| Et | 4-TMS | H | H | Et | 4-TMS | H | H |
| Et | 3-Me | 4-Me | 5-Me | Et | 3-Me | 4-Me | 5-Me |
| Et | 3-MeO | 4-MeO | 5-MeO | Et | 3-Me | 4-MeO | 5-MeO |
| H | 2-Cl | 5-Br | H | H | 2-Cl | 5-Br | H |
| Me | 2-Cl | 5-Br | H | Me | 2-Cl | 5-Br | H |

$R^3$ is Me; $R^4$ is H; $R^7$ is H

| $R^1$ | $R^2$ | $R^5$ | $R^6$ | $R^1$ | $R^2$ | $R^5$ | $R^6$ |
|---|---|---|---|---|---|---|---|
| Me | 4-Me | H | H | Me | 4-Me | 3-Me | H |
| Me | 4-Me | 4-Me | H | Me | 4-Me | 3-Cl | H |
| Me | 4-Me | 4-Cl | H | Me | 4-Me | 3-MeO | H |
| Me | 4-Me | 4-MeO | H | Me | 4-Me | 3-EtO | H |
| Me | 4-Me | 4-EtO | H | Me | 4-Me | 3-Et | H |
| Me | 4-Me | 4-Et | H | Me | 4-Me | 3-*i*-Pr | H |
| Me | 4-Me | 4-*i*-Pr | H | Me | 4-Et | H | H |

$R^3$ is Me; $R^4$ is H; $R^7$ is H

| $R^1$ | $R^2$ | $R^5$ | $R^6$ | $R^1$ | $R^2$ | $R^5$ | $R^6$ |
|---|---|---|---|---|---|---|---|
| Me | 4-Et | 4-Me | H | Me | 4-Me | 3-MeO | 5-MeO |
| Me | 4-Et | 4-Cl | H | H | 6-OH | H | H |
| Me | 4-Et | 4-MeO | H | H | 6-OMe | H | H |
| Me | 4-Et | 4-EtO | H | H | 6-OEt | H | H |
| Me | 4-Et | 4-Et | H | H | 6-OC(O)Me | H | H |
| Me | 4-Et | 4-i-Pr | H | H | 5-OH | H | H |
| Me | 4-Et | 3-Me | H | H | 5-OMe | H | H |
| Me | 4-Et | 3-Cl | H | H | 5-OEt | H | H |
| Me | 4-Et | 3-MeO | H | H | 5-Br | H | H |
| Me | 4-Et | 3-EtO | H | H | 5-Me | H | H |
| Me | 4-Et | 3-Et | H | H | 6-Me | H | H |
| Me | 4-Et | 3-i-Pr | H | H | 6-OH | 4-Me | H |
| Et | 4-Et | H | H | H | 6-OMe | 3-Me | H |
| Et | 4-Et | 4-Me | H | H | 6-OMe | 3-Me | 4-Me |
| Et | 4-Et | 4-Cl | H | H | 6-OEt | 4-Cl | H |
| Et | 4-Et | 4-MeO | H | H | 5-OMe | 4-F | H |
| Et | 4-Et | 4-EtO | H | H | 5-OMe | 3-Cl | H |
| Et | 4-Et | 4-Et | H | H | 5-OMe | 4-Cl | H |
| Et | 4-Et | 4-i-Pr | H | H | 5-Br | 4-Cl | H |
| Me | 4-Me | 3-Me | 4-Me | Me | 6-OH | H | H |
| Me | 4-Me | 3-Me | 5-Me | Me | 6-OMe | H | H |
| Me | 4-Me | 3-Cl | 4-Cl | Me | 4-n-Pr | H | H |
| Me | 4-Me | 3-Cl | 5-Cl | Et | 4-n-Pr | H | H |
| Me | 4-Me | 3-MeO | 4-MeO | | | | |

TABLE 27

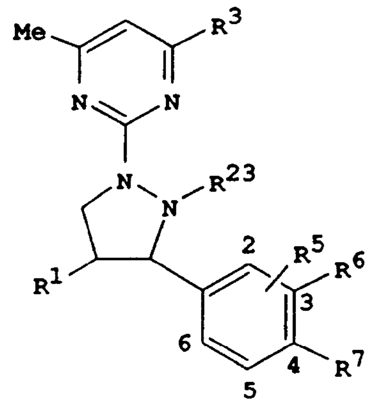

| R1 | R3 | R5 | R6 | R7 | R23 |
|---|---|---|---|---|---|
| H | Me | H | H | H | H |
| H | Me | H | H | Me | H |
| H | Me | H | H | Et | H |
| H | Me | H | H | i-Pr | H |
| H | Me | H | H | Cl | H |
| H | Me | H | H | OMe | H |
| H | Me | H | Me | Me | H |
| H | Me | H | Et | Et | H |
| H | Me | H | H | Me | H |
| H | Me | H | H | H | C(O)OMe |
| H | Me | H | H | Me | C(O)NHPh |
| H | H | H | H | H | H |
| H | H | H | H | Me | H |
| H | H | H | H | OMe | H |
| H | H | H | H | Et | C(O)OMe |
| H | H | H | H | Cl | C(O)NHPh |
| H | Et | H | H | H | H |
| H | Et | H | H | Me | H |
| Me | H | H | H | H | H |
| Me | H | H | H | Me | H |
| Me | H | H | H | Cl | H |
| Me | H | H | H | OMe | H |

| $R^1$ | $R^3$ | $R^5$ | $R^6$ | $R^7$ | $R^{23}$ |
|---|---|---|---|---|---|
| Et | H | H | H | H | H |
| Et | H | H | H | Me | H |
| Et | H | H | H | Cl | H |
| Et | H | H | H | OMe | H |
| i-Pr | H | H | H | Me | H |
| i-Pr | H | H | H | Cl | H |
| i-Pr | H | H | H | OMe | H |
| i-Pr | H | H | H | H | H |
| Me | Me | H | H | H | H |
| Me | Me | H | H | Me | H |
| Me | Me | H | H | Cl | H |
| Me | Me | H | H | OMe | H |
| Et | Me | H | H | Me | H |
| Et | Me | H | H | Cl | H |
| Et | Me | H | H | OMe | H |
| i-Pr | Me | H | H | Me | H |
| i-Pr | Me | H | H | Cl | H |
| i-Pr | Me | H | H | OMe | H |
| H | Me | 2-Me | H | H | H |
| H | Me | 2-Cl | H | H | H |
| Et | Me | H | H | H | C(S)NHPh |
| Et | Me | H | H | H | S(O)Ph |
| Et | Me | H | H | H | $S(O)_2Me$ |
| Et | Me | H | H | H | $S(O)_2NMe_2$ |
| Et | Me | H | H | H | $P(O)(OEt)_2$ |
| i-Pr | Me | H | H | H | $P(S)(OEt)_2$ |
| i-Pr | Me | H | H | H | Me |
| i-Pr | Me | H | H | H | $CH_2Ph$ |

TABLE 28

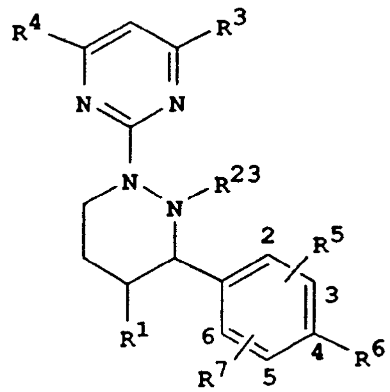

$R^3$ and $R^4$ are Me

| $R^1$ | $R^5$ | $R^6$ | $R^7$ | $R^{23}$ | $R^1$ | $R^5$ | $R^6$ | $R^7$ | $R^{23}$ |
|---|---|---|---|---|---|---|---|---|---|
| H | H | Me | H | H | Me | H | n-Pr | H | H |
| H | H | Et | H | H | Me | H | i-Pr | H | H |
| H | H | i-Pr | H | H | Me | H | Cl | H | H |
| H | H | OMe | H | H | Me | H | OMe | H | H |
| H | H | n-Pr | H | H | Me | 3-Me | Me | H | H |
| H | H | Cl | H | H | Me | 3-Et | Et | H | H |
| H | 3-Me | Me | H | H | Et | H | H | H | H |
| H | 3-Et | Et | H | H | Et | H | Me | H | H |
| H | 2-Et | Et | H | H | Et | H | Et | H | H |
| H | 2-Me | Me | H | H | Et | 3-Me | Me | H | H |
| H | 2-Me | H | 5-Me | H | Et | H | Cl | H | H |
| H | 3-Cl | H | H | H | Et | H | OMe | H | H |
| H | 3-Me | H | H | H | H | H | Me | H | C(O)OMe |
| H | 3-$CF_3$ | H | H | H | H | H | Et | H | C(O)OMe |
| H | 3-OMe | H | H | H | H | H | i-Pr | H | C(O)OMe |
| H | 2-Me | H | H | H | H | 3-Me | Me | H | C(O)OMe |
| H | H | H | H | H | Me | H | Me | H | C(O)NHPh |
| Me | H | H | H | H | Me | H | Et | H | C(O)NHMe |
| Me | H | Me | H | H | Me | 3-Me | Me | H | C(O)NHPh |
| Me | H | Et | H | H | Me | H | OMe | H | Me |

$R^3$ is Me, $R^4$ is H

| $R^1$ | $R^5$ | $R^6$ | $R^7$ | $R^{23}$ | $R^1$ | $R^5$ | $R^6$ | $R^7$ | $R^{23}$ |
|---|---|---|---|---|---|---|---|---|---|
| H | H | Me | H | H | Me | H | n-Pr | H | H |
| H | H | Et | H | H | Me | H | i-Pr | H | H |
| H | H | i-Pr | H | H | Me | H | Cl | H | H |
| H | H | OMe | H | H | Me | H | OMe | H | H |
| H | H | n-Pr | H | H | Me | 3-Me | Me | H | H |
| H | H | Cl | H | H | Me | 3-Et | Et | H | H |
| H | 3-Me | Me | H | H | Et | H | H | H | H |
| H | 3-Et | Et | H | H | Et | H | Me | H | H |
| H | 2-Et | Et | H | H | Et | H | Et | H | H |
| H | 2-Me | Me | H | H | Et | 3-Me | Me | H | H |
| H | 2-Me | H | 5-Me | H | Et | H | Cl | H | H |
| H | 3-Cl | H | H | H | Et | H | OMe | H | H |
| H | 3-Me | H | H | H | H | H | Me | H | C(O)OMe |
| H | 3-$CF_3$ | H | H | H | H | H | Et | H | C(O)OMe |
| H | 3-OMe | H | H | H | H | H | i-Pr | H | C(O)OMe |
| H | 2-Me | H | H | H | H | 3-Me | Me | H | C(O)OMe |
| H | H | H | H | H | Me | H | Me | H | C(O)NHPh |
| Me | H | H | H | H | Me | H | Et | H | C(O)NHMe |
| Me | H | Me | H | H | Me | 3-Me | Me | H | C(O)NHPh |
| Me | H | Et | H | H | Me | H | OMe | H | Me |

$R^3$ is Me, $R^4$ is Et

| $R^1$ | $R^5$ | $R^6$ | $R^7$ | $R^{23}$ | $R^1$ | $R^5$ | $R^6$ | $R^7$ | $R^{23}$ |
|---|---|---|---|---|---|---|---|---|---|
| H | H | Me | H | H | Me | H | n-Pr | H | H |
| H | H | Et | H | H | Me | H | i-Pr | H | H |
| H | H | i-Pr | H | H | Me | H | Cl | H | H |
| H | H | OMe | H | H | Me | H | OMe | H | H |
| H | H | n-Pr | H | H | Me | 3-Me | Me | H | H |
| H | H | Cl | H | H | Me | 3-Et | Et | H | H |
| H | 3-Me | Me | H | H | Et | H | H | H | H |

R³ is Me, R⁴ is Et

| $R^1$ | $R^5$ | $R^6$ | $R^7$ | $R^{23}$ | $R^1$ | $R^5$ | $R^6$ | $R^7$ | $R^{23}$ |
|---|---|---|---|---|---|---|---|---|---|
| H | 3-Et | Et | H | H | Et | H | Me | H | H |
| H | 2-Et | Et | H | H | Et | H | Et | H | H |
| H | 2-Me | Me | H | H | Et | 3-Me | Me | H | H |
| H | 2-Me | H | 5-Me | H | Et | H | Cl | H | H |
| H | 3-Cl | H | H | H | Et | H | OMe | H | H |
| H | 3-Me | H | H | H | H | H | Me | H | C(O)OMe |
| H | 3-$CF_3$ | H | H | H | H | H | Et | H | C(O)OMe |
| H | 3-OMe | H | H | H | H | H | i-Pr | H | C(O)OMe |
| H | 2-Me | H | H | H | H | 3-Me | Me | H | C(O)OMe |
| H | H | H | H | H | Me | H | Me | H | C(O)NHPh |
| Me | H | H | H | H | Me | H | Et | H | C(O)NHMe |
| Me | H | Me | H | H | Me | 3-Me | Me | H | C(O)NHPh |
| Me | H | Et | H | H | Me | H | OMe | H | Me |

TABLE 29

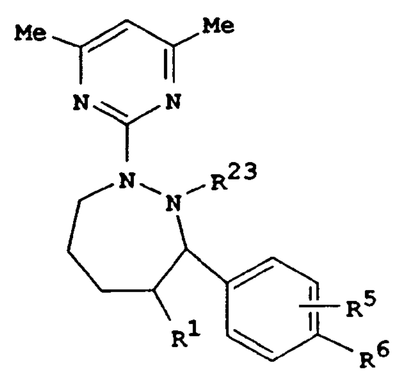

| $R^1$ | $R^5$ | $R^6$ | $R^{23}$ | $R^1$ | $R^5$ | $R^6$ | $R^{23}$ |
|---|---|---|---|---|---|---|---|
| H | H | H | H | Me | H | Et | H |
| H | H | Me | H | Me | H | OMe | H |
| H | H | Et | H | Me | H | Cl | H |
| H | H | i-Pr | H | H | H | H | (CO)OMe |
| H | 3-Me | Me | H | H | H | H | C(O)NHPh |
| H | H | Cl | H | H | H | H | Me |
| Me | H | H | H | Me | H | Me | C(O)OMe |
| Me | H | Me | H | Me | H | Et | C(O)NHPh |
| | | | | Me | H | Me | C(O)NHMe |

TABLE 30

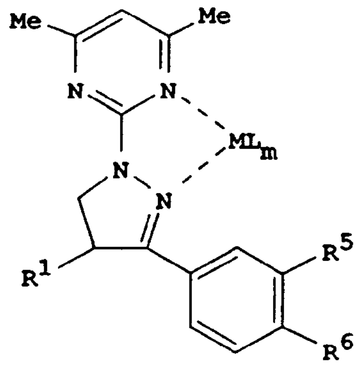

| $R^1$ | $R^5$ | $R^6$ | $ML_m$ |
|---|---|---|---|
| H | H | H | $ZnCl_2$ |
| H | H | H | $CuCl_2$ |
| H | H | H | $FeCl_3$ |
| Me | H | H | $ZnCl_2$ |
| Me | H | H | $CuCl_2$ |
| Me | H | H | $FeCl_3$ |
| Me | H | H | $MnCl_2$ |
| Me | H | H | $MgCl_2$ |
| Et | H | H | $ZnCl_2$ |
| Et | H | H | $CuCl_2$ |
| Et | H | H | $MnCl_2$ |
| i-Pr | H | H | $ZnCl_2$ |
| i-Pr | H | H | $FeCl_3$ |
| Me | H | Me | $ZnCl_2$ |
| Me | H | Me | $CuCl_2$ |
| Me | H | Me | $FeCl_3$ |
| i-Pr | H | Me | $MnCl_2$ |
| Et | H | Me | $MgCl_2$ |
| H | Me | Me | $ZnCl_2$ |

TABLE 31

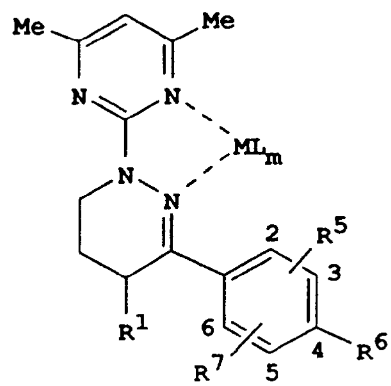

$ML_m$ is $ZnCl_2$

| $R^1$ | $R^5$ | $R^6$ | $R^7$ | $R^1$ | $R^5$ | $R^6$ | $R^7$ |
|---|---|---|---|---|---|---|---|
| H | H | Me | H | Me | H | H | H |
| H | H | Et | H | Me | H | Me | H |
| H | H | i-Pr | H | Me | H | Et | H |
| H | H | OMe | H | Me | H | n-Pr | H |
| H | H | n-Pr | H | Me | H | i-Pr | H |
| H | H | Cl | H | Me | H | Cl | H |
| H | 3-Me | Me | H | Me | H | OMe | H |
| H | 3-Et | Et | H | Me | 3-Me | Me | H |
| H | 2-Et | Et | H | Me | 3-Et | Et | H |
| H | 2-Me | Me | H | Et | H | H | H |
| H | 2-Me | H | 5-Me | Et | H | Me | H |
| H | 3-Cl | H | H | Et | H | Et | H |
| H | 3-Me | H | H | Et | 3-Me | Me | H |
| H | $3-CF_3$ | H | H | Et | H | Cl | H |
| H | 3-OMe | H | H | Et | H | OMe | H |
| H | 2-Me | H | H | Me | 3-Cl | H | H |
| H | H | H | H | | | | |

$ML_m$ is $FeCl_3$

| $R^1$ | $R^5$ | $R^6$ | $R^7$ | $R^1$ | $R^5$ | $R^6$ | $R^7$ |
|---|---|---|---|---|---|---|---|
| H | H | Me | H | Me | H | H | H |
| H | H | Et | H | Me | H | Me | H |
| H | H | i-Pr | H | Me | H | Et | H |
| H | H | OMe | H | Me | H | n-Pr | H |
| H | H | n-Pr | H | Me | H | i-Pr | H |
| H | H | Cl | H | Me | H | Cl | H |
| H | 3-Me | Me | H | Me | H | OMe | H |
| H | 3-Et | Et | H | Me | 3-Me | Me | H |
| H | 2-Et | Et | H | Me | 3-Et | Et | H |
| H | 2-Me | Me | H | Et | H | H | H |
| H | 2-Me | H | 5-Me | Et | H | Me | H |
| H | 3-Cl | H | H | Et | H | Et | H |
| H | 3-Me | H | H | Et | 3-Me | Me | H |
| H | 3-$CF_3$ | H | H | Et | H | Cl | H |
| H | 3-OMe | H | H | Et | H | OMe | H |
| H | 2-Me | H | H | Me | 3-Cl | H | H |
| H | H | H | H | | | | |

$ML_m$ is $CuCl_2$

| $R^1$ | $R^5$ | $R^6$ | $R^7$ | $R^1$ | $R^5$ | $R^6$ | $R^7$ |
|---|---|---|---|---|---|---|---|
| H | H | Me | H | Me | H | H | H |
| H | H | Et | H | Me | H | Me | H |
| H | H | i-Pr | H | Me | H | Et | H |
| H | H | OMe | H | Me | H | n-Pr | H |
| H | H | n-Pr | H | Me | H | i-Pr | H |
| H | H | Cl | H | Me | H | Cl | H |
| H | 3-Me | Me | H | Me | H | OMe | H |
| H | 3-Et | Et | H | Me | 3-Me | Me | H |
| H | 2-Et | Et | H | Me | 3-Et | Et | H |
| H | 2-Me | Me | H | Et | H | H | H |
| H | 2-Me | H | 5-Me | Et | H | Me | H |
| H | 3-Cl | H | H | Et | H | Et | H |

$ML_m$ is $CuCl_2$

| $R^1$ | $R^5$ | $R^6$ | $R^7$ | $R^1$ | $R^5$ | $R^6$ | $R^7$ |
|---|---|---|---|---|---|---|---|
| H | 3-Me | H | H | Et | 3-Me | Me | H |
| H | 3-$CF_3$ | H | H | Et | H | Cl | H |
| H | 3-OMe | H | H | Et | H | OMe | H |
| H | 2-Me | H | H | Me | 3-Cl | H | H |
| H | H | H | H | | | | |

$ML_m$ is $MnCl_2$

| $R^1$ | $R^5$ | $R^6$ | $R^7$ | $R^1$ | $R^5$ | $R^6$ | $R^7$ |
|---|---|---|---|---|---|---|---|
| H | H | Me | H | H | H | H | H |
| H | H | Et | H | Me | H | H | H |
| H | H | i-Pr | H | Me | H | Me | H |
| H | H | OMe | H | Me | H | Et | H |
| H | H | n-Pr | H | Me | H | n-Pr | H |
| H | H | Cl | H | Me | H | i-Pr | H |
| H | 3-Me | Me | H | Me | H | Cl | H |
| H | 3-Et | Et | H | Me | H | OMe | H |
| H | 2-Et | Et | H | Me | 3-Me | Me | H |
| H | 2-Me | Me | H | Me | 3-Et | Et | H |
| H | 2-Me | H | 5-Me | Et | H | H | H |
| H | 3-Cl | H | H | Et | H | Me | H |
| H | 3-Me | H | H | Et | H | Et | H |
| H | 3-$CF_3$ | H | H | Et | 3-Me | Me | H |
| H | 3-OMe | H | H | Et | H | Cl | H |
| H | 2-Me | H | H | Et | H | OMe | H |

$ML_m$ is $MgCl_2$

| $R^1$ | $R^5$ | $R^6$ | $R^7$ | $R^1$ | $R^5$ | $R^6$ | $R^7$ |
|---|---|---|---|---|---|---|---|
| H | H | Me | H | H | H | H | H |
| H | H | Et | H | Me | H | H | H |
| H | H | i-Pr | H | Me | H | Me | H |
| H | H | OMe | H | Me | H | Et | H |
| H | H | n-Pr | H | Me | H | n-Pr | H |
| H | H | Cl | H | Me | H | i-Pr | H |
| H | 3-Me | Me | H | Me | H | Cl | H |
| H | 3-Et | Et | H | Me | H | OMe | H |
| H | 2-Et | Et | H | Me | 3-Me | Me | H |
| H | 2-Me | Me | H | Me | 3-Et | Et | H |
| H | 2-Me | H | 5-Me | Et | H | H | H |
| H | 3-Cl | H | H | Et | H | Me | H |
| H | 3-Me | H | H | Et | H | Et | H |
| H | $3-CF_3$ | H | H | Et | 3-Me | Me | H |
| H | 3-OMe | H | H | Et | H | Cl | H |
| H | 2-Me | H | H | Et | H | OMe | H |

TABLE 32

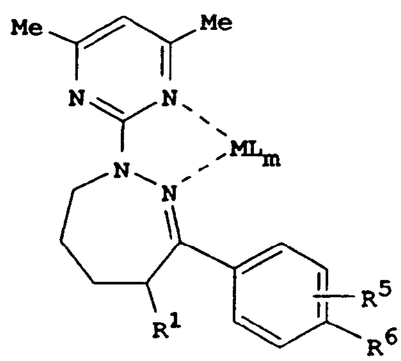

| $R^1$ | $R^5$ | $R^6$ | $R^7$ | $R^1$ | $R^5$ | $R^6$ | $R^7$ |
|---|---|---|---|---|---|---|---|
| H | H | H | $ZnCl_2$ | Me | H | H | $ZnCl_2$ |
| H | H | Me | $FeCl_3$ | Me | H | Me | $CuCl_2$ |
| H | H | Et | $CuCl_2$ | Me | H | Et | $MnCl_2$ |
| H | H | i-Pr | $MnCl_2$ | Me | H | OMe | $MgCl_2$ |
| H | 3-Me | Me | $MgCl_2$ | Me | H | Cl | $ZnCl_2$ |
| H | H | Cl | $FeCl_3$ | | | | |

TABLE 33

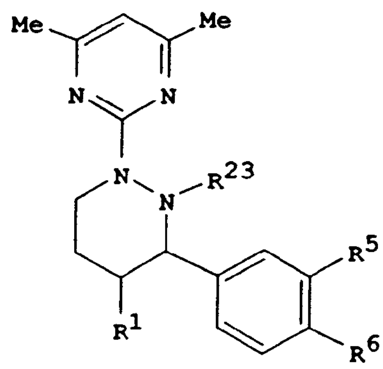

| $R^1$ is Me, $R^5$ is H, $R^6$ is H | $R^1$ is H, $R^5$ is Me, $R^6$ is H |
|---|---|
| $R^{23}$ | $R^{23}$ |
| Me | Me |
| $CH_2Ph$ | $CH_2Ph$ |
| $CH_2CH{=}CH_2$ | $CH_2CH{=}CH_2$ |
| $CH_2C{\equiv}CH$ | $CH_2C{\equiv}CH$ |
| C(=O)Me | C(=O)Me |
| C(=O)Ph | C(=O)Ph |
| C(=O)OMe | C(=O)OMe |
| C(=O)OPh | C(=O)OPh |
| S(=O)Me | S(=O)Me |
| C(=O)Ph | C(=O)Ph |
| $S(=O)_2Me$ | $S(=O)_2Me$ |
| $S(=O)_2Ph$ | $S(=O)_2Ph$ |
| C(=O)NHMe | C(=O)NHMe |
| C(=O)NHPh | C(=O)NHPh |
| $C(=O)NMe_2$ | $C(=O)NMe_2$ |
| C(=S)NHMe | C(=S)NHMe |
| C(=S)NHPh | C(=S)NHPh |
| $P(=S)(OEt)_2$ | $P(=S)(OEt)_2$ |
| $P(=O)(OEt)_2$ | $P(=O)(OEt)_2$ |
| $S(=O)_2NEt_2$ | $S(=O)_2NEt_2$ |

$R^1$ is Me, $R^5$ is H, $R^6$ is Me

$R^{23}$

Me

$CH_2Ph$

$CH_2CH{=}CH_2$

$CH_2C{\equiv}CH$

C(=O)Me

C(=O)Ph

C(=O)OMe

C(=O)OPh

S(=O)Me

C(=O)Ph

$S(=O)_2Me$

$S(=O)_2Ph$

C(=O)NHMe

C(=O)NHPh

$C(=O)NMe_2$

C(=S)NHMe

C(=S)NHPh

$P(=S)(OEt)_2$

$P(=O)(OEt)_2$

$S(=O)_2NEt_2$

$R^1$ is H, $R^5$ is Me, $R^6$ is Me

$R^{23}$

Me

$CH_2Ph$

$CH_2CH{=}CH_2$

$CH_2C{\equiv}CH$

C(=O)Me

C(=O)Ph

C(=O)OMe

C(=O)OPh

S(=O)Me

C(=O)Ph

$S(=O)_2Me$

$S(=O)_2Ph$

C(=O)NHMe

C(=O)NHPh

$C(=O)NMe_2$

C(=S)NHMe

C(=S)NHPh

$P(=S)(OEt)_2$

$P(=O)(OEt)_2$

$S(=O)_2NEt_2$

$R^1$ is Me, $R^5$ is H, $R^6$ is OMe

$R^{23}$

Me

$CH_2Ph$

$CH_2CH{=}CH_2$

$CH_2C{\equiv}CH$

C(=O)Me

C(=O)Ph

C(=O)OMe

$R^1$ is Me, $R^5$ is Me, $R^6$ is Me

$R^{23}$

Me

$CH_2Ph$

$CH_2CH{=}CH_2$

$CH_2C{\equiv}CH$

C(=O)Me

C(=O)Ph

C(=O)OMe

$R^1$ is Me, $R^5$ is H, $R^6$ is OMe

$R^{23}$

C(=O)OPh

S(=O)Me

C(=O)Ph

S(=O)$_2$Me

S(=O)$_2$Ph

C(=O)NHMe

C(=O)NHPh

C(=O)NMe$_2$

C(=S)NHMe

C(=S)NHPh

P(=S)(OEt)$_2$

P(=O)(OEt)$_2$

S(=O)$_2$NEt$_2$

$R^1$ is Me, $R^5$ is Me, $R^6$ is Me

$R^{23}$

C(=O)OPhS

(=O)Me

C(=O)Ph

S(=O)$_2$Me

S(=O)$_2$Ph

C(=O)NHMe

C(=O)NHPh

C(=O)NMe$_2$

C(=S)NHMe

C(=S)NHPh

P(=S)(OEt)$_2$

P(=O)(OEt)$_2$

S(=O)$_2$NEt$_2$

$R^1$ is H, $R^5$ is Cl, $R^6$ is H

$R^{23}$

Me

CH$_2$Ph

CH$_2$CH=CH$_2$

CH$_2$C≡CH

C(=O)Me

C(=O)Ph

C(=O)OMe

C(=O)OPh

S(=O)Me

C(=O)Ph

S(=O)$_2$Me

S(=O)$_2$Ph

C(=O)NHMe

$R^1$ is Et, $R^5$ is H, $R^6$ is H

$R^{23}$

Me

CH$_2$Ph

CH$_2$CH=CH$_2$

CH$_2$C≡CH

C(=O)Me

C(=O)Ph

C(=O)OMe

C(=O)OPh

S(=O)Me

C(=O)Ph

S(=O)$_2$Me

S(=O)$_2$Ph

C(=O)NHMe

$R^1$ is Et, $R^5$ is Cl, $R^6$ is H

$R^{23}$

C(=O)NHPh

C(=O)NMe$_2$

C(=S)NHMe

C(=S)NHPh

P(=S)(OEt)$_2$

P(=O)(OEt)$_2$

S(=O)$_2$NEt$_2$

$R^1$ is H, $R^5$ is H, $R^6$ is Me

$R^{23}$

Me

CH$_2$Ph

CH$_2$CH=CH$_2$

CH$_2$C≡CH

C(=O)Me

C(=O)Ph

C(=O)OMe

C(=O)OPh

S(=O)Me

C(=O)Ph

S(=O)$_2$Me

S(=O)$_2$Ph

C(=O)NHMe

C(=O)NHPh

C(=O)NMe$_2$

C(=S)NHMe

C(=S)NHPh

P(=S)(OEt)$_2$

P(=O)(OEt)$_2$

S(=O)$_2$NEt$_2$

$R^1$ is H, $R^5$ is H, $R^6$ is H

$R^{23}$

C(=O)NHPh

C(=O)NMe$_2$

C(=O)NPh$_2$

C(=S)NHMe

C(=S)NHPh

P(=S)(OEt)$_2$

P(=O)(OEt)$_2$

S(=O)$_2$NEt$_2$

$R^1$ is H, $R^5$ is H, $R^6$ is OMe

$R^{23}$

Me

CH$_2$Ph

CH$_2$CH=CH$_2$

CH$_2$C≡CH

C(=O)Me

C(=O)Ph

C(=O)OMe

C(=O)OPh

S(=O)Me

C(=O)Ph

S(=O)$_2$Me

S(=O)$_2$Ph

C(=O)NHMe

C(=O)NHPh

C(=O)NMe$_2$

C(=O)NPh$_2$

C(=S)NHMe

C(=S)NHPh

P(=S)(OEt)$_2$

P(=O)(OEt)$_2$

S(=O)$_2$NEt$_2$

TABLE 34

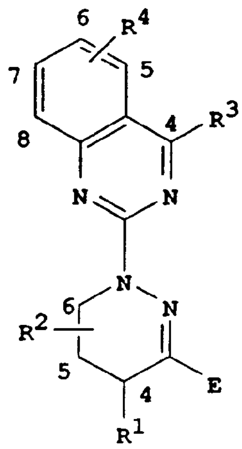

| $R^1$, $R^2$ and $R^3$ are H; $R^4$ is 6-Et | $R^1$ and $R^2$ are H; $R^3$ is Me; $R^4$ is H |
|---|---|
| E | E |
| 1-naphthalenyl | 1-naphthalenyl |
| 2-furanyl | 2-furanyl |
| 2-naphthalenyl | 2-naphthalenyl |
| 3-thienyl | 3-thienyl |
| 2,5-dimethyl-3-furanyl | 2,5-dimethyl-3-furanyl |
| 2,5-dimethyl-3-thienyl | 2,5-dimethyl-3-thienyl |
| 4-methylphenoxy | 4-methylphenoxy |
| 2-chlorophenoxy | 2-chlorophenoxy |
| 2,6-dimethylphenoxy | 2,6-dimethylphenoxy |
| 3-methylphenylthio | 4-cyanophenylthio |
| phenylamino | 4-methylphenylamino |
| benzyl | Cl |
| Et | n-hex |
| sec-Bu | Me |
| c-propyl | c-hexyl |
| cis-2-methylcycloheptyl | $CF_3CH_2CH_2$ |
| sec-butylthio | n-butoxy |

$R^1$, $R^2$ and $R^3$ are H; $R^4$ is 6-Et

| E |
|---|
| $CF_3CH_2O$ |
| 5-methyl-2-thienyl |
| 5-methyl-2-pyridyl |

$R^1$ and $R^2$ are H; $R^3$ is Me; $R^4$ is H

| E |
|---|
| $Cl(CH_2)_5O$ |
| 4-methyl-3-furanyl |
| 2-methyl-3-pyridyl |

$R^1$ and $R^3$ are Me; $R^2$ is 5-Me; $R^4$ is H

| E |
|---|
| 5-methyl-2-pyridyl |
| 4-pyridyl |
| 2-indanyl |
| 2-tetrahydronaphthalenyl |
| 6-Me-3-pyridyl |
| 2-pyridyl |

$R^1$, $R^2$, $R^3$ and $R^4$ are H

| E |
|---|
| 1-naphthalenyl |
| 2-furanyl |
| 3-thienyl |
| 3-pyridyl |

$R^1$ and $R^3$ are Me; $R^2$ and $R^4$ are H;

| E |
|---|
| 2-methyl-3-pyridyl |
| 4-chloro-3-pyridyl |
| 2-indanyl |
| 2-tetrahydronaphthalenyl |
| 6-Me-3-pyridyl |
| 2-pyridyl |
| 1-naphthalenyl |
| 2-furanyl |
| 3-thienyl |
| 3-pyridyl |

$R^3$ is Me; $R^4$ is 6-Me

| $R^1$ | $R^2$ | E |
|---|---|---|
| H | 5-Me | Ph |
| H | 5-*i*-Pr | 2-Me-Ph |
| H | 5-*n*-Bu | 2-Cl-Ph |
| H | 5-CN | 2-MeO-Ph |
| H | 5-$CF_3$ | $CF_3CH_2O$-Ph |
| H | 6-$CF_3CH_2$ | 1-naphthalenyl |
| *i*-Pr | 5-Me | Ph |
| *i*-Pr | 5-Me | 2-Me-Ph |

$R^3$ is Me; $R^4$ is 6-Me

| $R^1$ | $R^2$ | E |
|---|---|---|
| H | 5-Et | Ph |
| H | 5-*sec*-Bu | 2-Me-Ph |
| H | 5-$CF_3(CF_2)_3$ | 2-Cl-Ph |
| H | 5-*t*-Bu | 2-MeO-Ph |
| H | 5-$FCH_2$ | 2-$CF_3CH_2O$-Ph |
| H | 6-*n*-Pr | 1-naphthalenyl |
| Me | 4-Me | Ph |
| Me | 4-Me | 2-Me-Ph |

$R^3$ is Me; $R^4$ is 6-Me

| $R^1$ | $R^2$ | E |
|---|---|---|
| *i*-Pr | 5-Me | 2-Cl-Ph |
| *i*-Pr | 5-Me | 2-MeO-Ph |
| *i*-Pr | 6-Me | 2-$CF_3CH_2O$-Ph |
| Cl | H | Ph |
| F | H | 4-Me-Ph |
| $CF_3CF_2$ | H | 4-Cl-Ph |
| $CH_2$=$CHCH_2$ | H | 4-MeO-Ph |

$R^3$ is Me; $R^4$ is H

| $R^1$ | $R^2$ | E |
|---|---|---|
| $CO_2Me$ | H | 2-$CF_3CH_2O$-Ph |
| 2-Me-Ph | H | Me |
| Bzl | H | Ph |
| 2-naphthalenyl | H | *n*-Bu |
| 3-thienyl | H | $CF_3CF_2$ |
| 3-pyridyl | H | Me |
| CN | 5-Me | Ph |
| *t*-Bu | 5-Me | 2-Me-Ph |
| $ClCH_2$ | 5-Me | 2-Cl-Ph |
| Et | 5-Me | 2-MeO-Ph |
| *n*-Pr | 5-Me | 2-$CF_3CH_2O$-Ph |
| Me | 4-Me | 2-$CF_3$-Ph |
| *i*-Pr | 4-Me | 2-$CF_3$-Ph |
| $CF_3$ | 4-$CF_3$ | 2-$CF_3$-Ph |

$R^3$ is Et; $R^4$ is H

| $R^1$ | $R^2$ | E |
|---|---|---|
| Me | 4-Me | 2-TMS-Ph |
| Me | 4-Me | 2-Cl-Ph |
| Me | 4-Me | 2-MeO-Ph |
| Me | 4-Me | 2-$CF_3CH_2O$-Ph |
| Br | H | Ph |
| CN | H | 4-Me-Ph |
| Ac | H | 4-Cl-Ph |
| $CH_3C{\equiv}CCH_2$ | H | 4-MeO-Ph |

$R^3$ is Me; $R^4$ is 6-Me

| $R^1$ | $R^2$ | E |
|---|---|---|
| $CO_2Et$ | H | 2-$CF_3CH_2O$-Ph |
| 4-Cl-Ph | H | Ph |
| 5-Me-3-furyl | H | i-Pr |
| EtCO | H | 2-Cl-Ph |
| 2-furyl | 4-Me | $CF_3$ |
| Ph | 5-Me | Me |
| CN | 4-Me | Ph |
| t-Bu | 4-Me | 2-Me-Ph |
| $FCH_2$ | 4-Me | 2-Cl-Ph |
| Et | 4-Me | 2-MeO-Ph |
| $Cl(CH_2)_4$ | 4-Me | 2-$CF_3CH_2O$-Ph |
| Me | 4-Me | 2-$CF_3$-Ph |
| i-Pr | 5-CN | 2-$CF_3$-Ph |
| $CF_3$ | 5-Me | 2-$CF_3$-Ph |
| i-Pr | 4-Me | 2-TMS-Ph |

TABLE 35

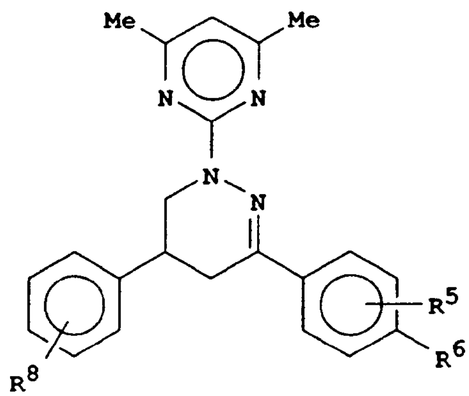

| $R^5$ and $R^6$ are H | $R^5$ is H, $R^6$ is Me | $R^5$ is H, $R^6$ is MeO |
|---|---|---|
| $R^8$ | $R^8$ | $R^8$ |
| H | H | H |
| 2-Me | 2-Me | 2-Me |
| 2-Cl | 2-Cl | 2-Cl |
| 2-Br | 2-Br | 2-Br |
| 2-MeO | 2-MeO | 2-MeO |
| 3-Me | 3-Me | 3-Me |
| 3-Cl | 3-Cl | 3-Cl |
| 3-Br | 3-Br | 3-Br |
| 3-MeO | 3-MeO | 3-MeO |
| 4-Me | 4-Me | 4-Me |
| 4-Cl | 4-Cl | 4-Cl |
| 4-Br | 4-Br | 4-Br |
| 4-MeO | 4-MeO | 4-MeO |
| $3-CF_3$ | $3-CF_3$ | $3-CF_3$ |
| $4-CF_3$ | $4-CF_3$ | $4-CF_3$ |

| $R^5$ is 3-Me, $R^6$ is Me | $R^5$ is H, $R^6$ is Cl | $R^5$ is 2-Me, $R^6$ is H |
|---|---|---|
| $R^8$ | $R^8$ | $R^8$ |
| H | H | H |
| 2-Me | 2-Me | 2-Me |
| 2-Cl | 2-Cl | 2-Cl |

| $R^5$ is 3-Me, $R^6$ is Me | $R^5$ is H, $R^6$ is Cl | $R^5$ is 2-Me, $R^6$ is H |
|---|---|---|
| $R^8$ | $R^8$ | $R^8$ |
| 2-Br | 2-Br | 2-Br |
| 2-MeO | 2-MeO | 2-MeO |
| 3-Me | 3-Me | 3-Me |
| 3-Cl | 3-Cl | 3-Cl |
| 3-Br | 3-Br | 3-Br |
| 3-MeO | 3-MeO | 3-MeO |
| 4-Me | 4-Me | 4-Me |
| 4-Cl | 4-Cl | 4-Cl |
| 4-Br | 4-Br | 4-Br |
| 4-MeO | 4-MeO | 4-MeO |
| 3-$CF_3$ | 3-$CF_3$ | 3-$CF_3$ |
| 4-$CF_3$ | 4-$CF_3$ | 4-$CF_3$ |

## Formulations

Useful formulations of the compounds of Formulae I-VI can be prepared in conventional ways in the form of dusts, granules, pellets, solutions, suspensions, emulsions, wettable powders, emulsifiable concentrates and the like. Many of these formulations may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they may contain these ingredients in the following approximate proportions:

| Formulation | Weight Percent* Ingredient | Diluent(s) | Surfactant(s) |
|---|---|---|---|
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

*Active ingredients plus at least one of a surfactant or a diluent equals 100 weight percent.

Lower or higher levels of active ingredient-can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., NewYork, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, U.S. Patent 3,060,084). Granules and pellets may be made by spraying the active material upon preformed granular carriers or by agglomeration techniques. See J.E.Browning, "Agglomeration", Chemical Engineering, December 4, 1967, pp. 147ff and "Perry's Chemical Engineer's Handbook", 5th Ed., McGraw-Hill, New York, 1973, pp. 8-59ff.

For further information regarding the art of formulation, see for example:

U.S. Patent 3,235,361;

U.S. Patent 3,309,192;

U.S. Patent 2,891,855; and

G. C. Klingman, "Weed Control as a Science", John Wiley & Sons, Inc., New York, 1961, pp. 81-96; and

J. D. Fryer et al., "Weed Control Handbook", 5th Ed., Blackwell Scientific Publications, Oxford, 1968, pp. 101-103.

In the following examples of formulations, all parts are by weight unless otherwise indicated.

## EXAMPLE A

**Wettable Powder**

| | |
|---|---|
| 1-(4,6-dimethyl-2-pyrimidinyl)-1,4,5,6-tetrahydro-3-phenylpyridazine | 50% |
| sodium alkylnaphthalenesulfonate | 2% |
| sodium liginsulfonate | 5% |
| diatomaceous earth | 43% |

The ingredients are blended, coarsely hammer-milled and then air-milled to produce particles essentially all below 10 microns in diameter. The product is reblended before packaging.

## EXAMPLE B

**Granule**

| | |
|---|---|
| Oily active ingredient | 5% |
| attapulgite granules (U.S.S. 20-40 mesh; 0.84-0.42 mm) | 95% |

An oily active ingredient is sprayed on the surface of attapulgite granules in a double-cone blender. The granules are dried and packaged.

EXAMPLE C

Oil Suspension

| | |
|---|---|
| 1-(4,6-dimethyl-2-pyrimidinyl)-1,4,5,6-tetra-hydro-3-phenylpyridazine | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspensions may be applied directly, but preferably after being extended with oils or emulsified in water.

EXAMPLE D

Wettable Powder

| | |
|---|---|
| 1-(4,6-dimethyl-2-pyrimidinyl)-1,4,5,6-tetra-hydro-3-phenylpyridazine | 20% |
| sodium alkylnaphthalenesulfonate | 4% |
| sodium liginsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

EXAMPLE E

Low Strength Granule

| | |
|---|---|
| 1-(4,6-dimethyl-2-pyrimidinyl)-1,4,5,6-tetra-hydro-3-phenylpyridazine | 1% |
| methylene chloride | 9% |
| attapulgite granule (U.S.S. 20-40 sieve) | 90% |

The active ingredient is dissolved in the solvent and the solution is sprayed upon dedusted granules in a double cone blender. After spraying of the solution has been completed, the blender is allowed to run for a short period. The product is then gently dried to remove solvent and the granules are packaged.

EXAMPLE F

Aqueous Suspension

| | |
|---|---|
| 1-(4,6-dimethyl-2-pyrimidinyl)-1,4,5,6-tetra-hydro-3-phenylpyridazine | 10% |
| polyacrylic acid thickener | 0.3% |
| dodecylphenol polyethylene glycol ether | 5.0% |
| disodium phosphate | 1% |
| monosodium phosphate | 0.5% |
| polyvinyl alcohol | 1.0% |
| water | 82.2% |

The ingredients are blended and milled together in a homogenizer to produce particles essentially all under 5 microns in size.

EXAMPLE G

Solution

| | |
|---|---|
| 1-(4,6-dimethyl-2-pyrimidinyl)-1,4,5,6-tetra-hydro-3-phenylpyridazine | 5% |
| water | 95% |

The salt is added directly to the water with stirring to produce the solution, which may then be packaged for use.

EXAMPLE H

Low Strength Granule

| | |
|---|---|
| 1-(4,6-dimethyl-2-pyrimidinyl)-1,4,5,6-tetra-hydro-3-phenylpyridazine | 0.1% |
| attapulgite granules (U.S.S. 20-40 mesh) | 99.9% |

The active ingredient is dissolved in a solvent and the solution is sprayed upon dedusted granules in a double cone blender. After spraying of the solution has been completed, the material is warmed to evaporate the solvent. The material is allowed to cool and then packaged.

EXAMPLE I

Emulsion Concentrate

| | |
|---|---|
| 1-(4,6-dimethyl-2-pyrimidinyl)-1,4,5,6-tetra-hydro-3-phenylpyridazine | 35% |
| blend of polyalcohol carboxylic esters and oil soluble petroleum sulfonates | 6% |
| xylene | 59% |

The ingredients are combined and stirred together to produce a solution. The product can be extended with oils, or emulsified in water.

EXAMPLE J

Emulsifiable Concentrate

| | |
|---|---|
| 1-(4,6-dimethyl-2-pyrimidinyl)-1,4,5,6-tetrahydro-3-phenylpyridazine | 20% |
| chlorobenzene | 74% |
| sorbitan monostearate and polyoxyethylene condensates thereof | 6% |

The ingredients are combined and stirred to produce a solution which can be emulsified in water for application.

Utility

The compounds of this invention are useful as plant disease control agents. They provide control of diseases caused by a broad spectrum of fungal plant pathogens in the Basidiomycete, Ascomycete and Oomycete classes. They are effective in controlling a broad spectrum of plant diseases, particularly foliar pathogens of ornamental, vegetable, field, cereal, and fruit crops. These pathogens include, Venturia inaequalis, Cercosporidium personatum, Cercospora arachidicola, Cercospora beticola, Pseudocercosporella herpotrichoides, Erysiphe graminis, Uncinula necatur, Podosphaera leucotricha, Puccinia recondita, Puccinia gramminis, Hemileia vastatrix, Puccinia striiformis, Puccinia arachidis, Pyricularia oryzae, Phytophthora infestans, Plasmopara viticola, Peronospora tabacina, Pseudoperonospora cubensis, Pythium aphanidermatum, Botrytis cinerea, Monilinia fructicola, Alternaria brassicae, Septoria nodorum, and other species closely related to these pathogens. They also control seed pathogens.

The compounds of this invention can be mixed with various fungicides, bactericides, acaricides, nematicides, insecticides or other biologically active compounds in order to achieve desired results with a minimum of expenditure of time, effort and material. Suitable agents of this type are well-known to those skilled in the art. Some of these agents are listed below:

Fungicides

methyl 2-benzimidazolecarbamate (carbendazim)
tetramethylthiuram disulfide (thiuram)
n-dodecylguanidine acetate (dodine)
manganese ethylenebisdithiocarbamate (maneb)
1,4-dichloro-2,5-dimethoxybenzene (chloroneb)
methyl 1-(butylcarbamoyl)-2-benzimidazolecarbamate (benomyl)
2-cyano-N-ethylcarbamoyl-2-methoxyiminoacetamide (cymoxanil)
N-trichloromethylthiotetrahydrophthalamide (captan)
N-trichloromethylthiophthalimide (folpet)
dimethyl 4,4′-(o-phenylene) bis (3-thioallophanate) (thiophanate-methyl)
2-(thiazol-4-yl)benzimidazole (thiabendazole)
aluminum tris(O-ethylphosphonate)(phosethyl aluminum)
tetrachloroisophthalonitrile (chlorothalonil)
2,6-dichloro-4-nitroaniline (dichloran)
N-(2,6-dimethylphenyl)-N-(methoxyacetyl)alanine methyl ester (metalaxyl)
cis-N-[1,1,2,2-tetrachloroethyl)thio]cyclohex-4-ene-1,2-dicarbioximide (captafol)
3-(3,5-dichlorophenyl)-N-(1-methylethyl)-2,4-dioxo-1-imidazolidine carboxamide (iprodione)
3-(3,5-dichlorophenyl)-5-ethenyl-5-methyl-2,4-oxazolidinedione (vinclozolin)
kasugamycin
O-ethyl-S,S-diphenylphosphorodithioate (edifenphos)
4-(3-(4-(1,1-dimethylethyl)phenyl)-2-methyl)propyl-2,6-dimethylmorpholine (fenpropimorph)

4-(3-4(1,1-dimethylethyl)phenyl)-2-methyl)propylpiperidine (fenpropidine)
1-(4-chlorophenoxy)-3,3-dimethyl-1-1H-1,2,4-triazol-1-yl)butanone (triadimefon)
2-(4-chlorophenyl)-2-(1H-1,2,4-triazol-1-ylmethyl)-hexanenitrile (myclobutanil)
1-[2-(4-chlorophenyl)ethyl]-1-(1,1-dimethylethyl)-1-(1H-1,2,4-triazole-1-yl)ethanol (tebuconazol)
3-chloro-4-[4-methyl-2-(1H-1,2,4-triazol)-1-ylmethyl)-1,3-dioxolan-2-yl]phenyl-4-chlorophenyl ether (difenoconazole)
1-[2-(2,4-dichlorophenyl)pentyl]1H-1,2,4-triazole (penconazole)
2,4′-difluoro-1-(1H-1,2,4-triazole-1-ylmethyl)-benzhydryl alcohol (flutriafol)
1-[[[bis(4-fluorophenyl)]methylsilyl]methyl]-1H-1,2,4-triazole (flusilazole)
N-propyl-N-[2-(2,4,6-trichlorophenoxy)ethyl]imidazole-1-carboxamide (prochloraz)
1-[2-(2,4-dichlorophenyl)-4-propyl-1,3-dioxolan-2-ylmethyl]-1H-1,2,4-triazole (propiconazole)
1-(2-chlorophenyl)-1-(4-chlorophenyl)-1-(5-pyrimidin)-methanol (fenarimol)
1-(4-Chlorophenoxy)-3,3-dimethyl-1-(1H-1,2,4-triazole-1-yl)butan-2-ol (triadimenol)
1-(2,4-dichlorophenyl)-4,4-dimethyl-2-(1H-1,2,4-triazol-1-yl)pentan-3-ol (diclobutrazol) copper oxychloride
methyl N-(2,6-dimethylphenyl)-N-(2-furanylcarbonyl)-DL-alaninate (furalaxyl)
1-(4-amino-1,2-dihydro-2-oxopyrimidin-1-yl)-4-[(S)-3-amino-5-(1-methylguanidino)valeramido]-1,2,3,4-tetra deoxy-β-D-*erythro*-hex-2-enopyranuronic acid (blasticidin-S)
6-(3,5-dichloro-4-methylphenyl)-3(2*H*)-pyridazinone (diclomezine)
*O*-ethyl-*S*,*S*-diphenyl-dithiophosphate (edifenphos)
diisopropyl 1,3-dithiolan-2-ylidenemalonate (isoprothiolane)
*O*,*O*-diisopropyl-*S*-benzyl thiophosphate (iprobenfos)
3′-isopropoxy-2-methylbenzanilide (mepronil)
ferric methanearsonate (ferric ammonium salt) (neo-asozin)
*N*-[(4-chlorophenyl)methyl]-*N*-cyclopentyl-*N*′-phenylurea (pencycuron)
3-allyloxy-1,2-benzoisothiazole 1,1-dioxide (probenazole) 1,2,5,6-tetrahydro-pyrrolo[3,2,1-*ij*]quinolin-4-one (pyroquilon)
α,α,α-trifluoro-o-toluanilide (flutolanil) 5-methyl-1,2,4-triazole(3,4-*b*) benzothiazole (tricyclazole)
4,5,6,7-tetrachlorophthalide (tetrachlorophthalide) 1L-(1,3,4/2,6)-2,3-dihydroxy-6-hydroxymethyl-4[(1S, 4R, 5S, 6S)-4,5,6-trihydroxy-3-hydroxymethylcyclohex-2-enylamino]cyclohexyl-ß-D-glucopyranoside (validamycin)
α,α,α-trifluoro-3′-isopropoxy-2-toluanilide (flutolanil)

Bactericides

tribasic copper sulfate
streptomycin sulfate
oxytetracycline

Acaricides

senecioic acid, ester with 2-sec-butyl-4,6-dinitro-phenol (binapacryl)
6-methyl-1,3-dithiolo[2,3-B]quinonolin-2-one (oxythio-quinox)
2,2,2-trichloro-1,1-bis(4-chlorophenyl)ethanol (dicofol)
bis(pentachloro-2,4-cyclopentadien-1-yl)(dienochlor) tricyclohexyltin hydroxide (cyhexatin)
hexakis(2-methyl-2-phenylpropyl)distannoxane (fenbutin oxide)

Nematicides

2-[diethoxyphosphinylimino]-1,3-diethietane (fosthietan)
S-methyl-1-(dimethylcarbamoyl)-N-(methylcarbamoyloxy)-thioformimidate (oxamyl)
S-methyl-1-carbamoyl-N-(methylcarbamoyloxy)thioformimidate
N-isopropylphosphoramidic acid, O-ethyl-O′-[4-(methyl-thio)-m-tolyl]diester (fenamiphos)

Insecticides

3-hydroxy-N-methylcrotonamide(dimethylphosphate)ester (monocrotophos)
methylcarbamic acid, ester with 2,3-dihydro-2,2-dimethyl-7-benzofuranol (carbofuran)
O-[2,4,5-trichloro-a-(chloromethyl)benzyl]phosphoric acid, O′,O′-dimethyl ester (tetrachlorvinphos)

2-mercaptosuccinic acid, diethyl ester, S-ester with thionophosphoric acid, dimethyl ester (malathion)
phosphorothioic acid, O,O-dimethyl, O-p-nitrophenyl ester (methyl parathion)
methylcarbamic acid, ester with alpha-naphthol (carbaryl)
methyl N-[[(methylamino)carbonyl]oxy]ethanimidothioate (methomyl)
N'-(4-chloro-o-tolyl)-N,N-dimethylformamidine (chlordimeform)
O,O-diethyl-O-(2-isopropyl-4-methyl-6-pyrimidyl)-phosphorothioate (diazinon)
octachlorocamphene (toxaphene)
O-ethyl O-p-nitrophenyl phenylphosphonothioate (EPN) cyano(3-phenoxyphenyl)-methyl 4-chloro-alpha-(1-methylethyl)benzeneacetate (fenvalerate)
(3-phenoxyphenyl)methyl 3-(2,2-dichloro-ethenyl)-2,2-dimethylcyclopropanecarboxylate (permethrin)
dimethyl N,N'-[thiobis(N-methylimmo)carbonyloxy]]-bis[ethanimidothioate] (thiodicarb)
phosphorothiolothionic acid, O-ethyl-O-[4-(methyl-thio)phenyl]-S-n-propyl ester (sulprofos)
alpha-cyano-3-phenoxybenzyl 3-(2,2-dichlorovinyl)-2,2-dimethylcyclopropanecarboxylate (cypermethrin)
cyano(3-phenoxyphenyl)methyl 4-(difluoromethoxy)-alpha-(methylethyl)benzeneacetate (flucythrinate) O,O-diethyl-O-(3,5,6-trichloro-2-pyridyl)phosphorothioate (chlorpyrifos)
O,O-dimethyl-S-[(4-oxo-1,2,3-benzotriazin-3-(4H)-yl)-methyl]phosphorodithioate (azinphos-methyl)
5,6-dimethyl-2-dimethylamino-4-pyrimidinyl dimethyl carbamate (pirimicarb)
S-(N-formyl-N-methylcarbamoylmethyl)-O,O-dimethyl phosphorodithioate (formothion)
S-2-(ethylthioethyl)-O,O-dimethyl phosphiorothioate (demeton-S-methyl)
(5)-alpha-cyano-3-phenoxybenzyl (1R,3R)-3-(2,2-dibromovinyl)-2,2-dimethylcyclopropanecarboxylate (deltamethrin)
cyano(3-phenoxyphenyl)methyl ester of N-(2-chloro-4-trifluoromethylphenyl)alanine (fluvalinate)

Application Method

Disease control is ordinarily accomplished by applying an effective amount of the compounds of the invention either pre-infection or post-infection to the portion of the plant to be protected such as the roots, stems, foliage, fruit, seeds, tubers or bulbs, or to the media (soil or sand) in which the plants to be protected are growing. The compound also may be applied to the seed to protect the seed and seedling.

Rates of application for these compounds can be influenced by many factors of the environment and should be determined under actual use conditions. Foliage can normally be protected when treated at a rate of from less than 1 g/ha to 5000 g/h of active ingredient. Plants growing in soil treated at a concentration from 0.1 to about 20 kg/ha can be protected from disease. Seed and seedlings can normally be protected when seed is treated at a rate of from 0.1 to 10 g per kilogram of seed. The efficacy of the compounds for disease control is evaluated according to Tests A - F below.

Test A

The test compounds are dissolved in acetone in an amount equal to 3 % of the final volume and then suspended at a concentration of 200 ppm in purified water containing 250 ppm of the surfactant Trem 014 (polyhydric alcohol esters). This suspension is sprayed to the point of run-off on wheat seedlings. The following day the seedlings are inoculated with a spore dust of Erysiphe graminis f. sp. tritici, (the causal agent of wheat powdery mildew) and incubated in a growth chamber at 20°C for 7 days, after which disease ratings are made.

Test B

The test compounds are dissolved in acetone in an amount equal to 3 % of the final volume and then suspended at a concentration of 200 ppm in purified water containing 250 ppm of the surfactant Trem 014 (polyhydric alcohol esters). This suspension is sprayed to the point of run-off on wheat seedlings. The following day the seedlings are inoculated with a spore suspension of Puccinia recondita (the causal agent of wheat leaf rust) and incubated in a saturated atmosphere at 20°C for 24 h, and then moved to a growth chamber at 20°C for 6 days, after which disease ratings are made.

Test C

The test compounds are dissolved in acetone in an amount equal to 3 % of the final volume and then suspended at a concentration of 200 ppm in purified water containing 250 ppm of the surfactant Trem 014 (polyhydric alcohol esters). This suspension is sprayed to the point of run-off on rice seedlings. The following day

the seedlings are inoculated with a spore suspension of Pyricularia oryzae (the causal agent of rice blast) and incubated in a saturated atmosphere at 27°C for 24 h, and then moved to a growth chamber at 30°C for 5 days, after which disease ratings are made.

Test D

The test compounds are dissolved in acetone in an amount equal to 3 % of the final volume and then suspended at a concentration of 200 ppm in purified water containing 250 ppm of the surfactant Trem 014 (polyhydric alcohol esters). This suspension is sprayed to the point of run-off on tomato seedlings. The following day the seedlings are inoculated with a spore suspension of Phytophthora infestans (the causal agent of potato and tomato late blight) and incubated in a saturated atmosphere at 20°C for 24 h, and then moved to a growth chamber at 20°C for 5 days, after which disease ratings are made.

Test E

The test compounds are dissolved in acetone in an amount equal to 3 % of the final volume and then suspended at a concentration of 200 ppm in purified water containing 250 ppm of the surfactant Trem 014 (polyhydric alcohol esters). This suspension is sprayed to the point of run-off on grape seedlings. The following day the seedlings are inoculated with a spore suspension ofPlasmopara viticola (the causal agent of grape downy mildew) and incubated in a saturated atmosphere at 20 C for 24 h, moved to a growth chamber at 20 C for 6 days,and then incubated in a saturated atmosphere at 20 C for 24 h, after which disease ratings are made.

Test F

The test compounds are dissolved in acetone in an amount equal to 3 % of the final volume and then suspended at a concentration of 200 ppm in purified water containing 250 ppm of the surfactant Trem 014 (polyhydric alcohol esters). This suspension is sprayed to the point of run-off on cucumber seedlings. The following day the seedlings are inoculated with a spore suspension of Botrytis cinerea (the causal agent of gray mold on many crops) and incubated in a saturated atmosphere at 20 C for 48 h, and moved to a growth chamber at 20 C for 5 days, after which disease ratings are made.

INDEX TABLE A

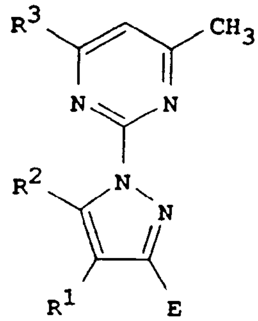

| CMPD. NO. | $R^1$ | $R^2$ | $R^3$ | E | mp(°C)[a] |
|---|---|---|---|---|---|
| 1 | H | Me | Me | Ph | oil |
| 2 | Me | H | H | Ph | 79-81 |
| 3[b] | Ph | H | H | Me | 114-121 |
| 4 | Ph | H | Me | Me | 123-124.5 |
| 5 | Ph | Me | Me | H | 93.5-94 |
| 117 | H | H | $CF_3$ | Ph | 124-127 |
| 148 | H | H | Me | 2-pyridyl | 140-146 |

a $^1H$ NMR data, given for an oil, are given in Index Table O

b 65% compound plus 35% 3-methyl-4-phenyl-1H-pyrazole

INDEX TABLE B

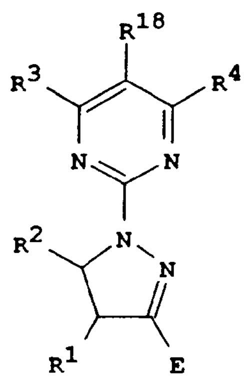

| CMPD. NO. | $R^1$ | $R^2$ | $R^3$ | $R^4$ | $R^{18}$ | E | mp(°C)[a] |
|---|---|---|---|---|---|---|---|
| 6 | H | H | Me | Me | H | Ph | 127-129 |
| 7 | Me | H | H | Me | H | Ph | oil |
| 8 | H | H | H | Me | H | 3-$CF_3$-Ph | 125-130 |
| 9 | H | H | H | Me | H | 1-naphthalenyl | 119-126 |
| 10 | H | H | Me | Me | H | 4-Cl-Ph | 138-143 |
| 11 | H | H | Me | Me | H | 4-F-Ph | 155-160 |
| 12 | H | H | Me | Me | H | 2-Cl-Ph | 116-118 |
| 13 | H | H | H | Me | H | Ph | 142-144 |
| 14 | H | H | H | Me | H | 4-Cl-Ph | 179-181 |
| 15 | H | H | H | Me | H | 4-F-Ph | 158-165 |
| 16 | Me | H | Me | Me | H | Ph | oil |
| 17 | H | H | Me | Me | H | 3-$CF_3$-Ph | 122-127 |
| 18 | H | H | Me | Me | H | 1-naphthalenyl | 199-202 |
| 20 | H | H | H | H | H | 1-naphthalenyl | 152-158 |
| 21 | H | H | H | Me | H | 2-Cl-Ph | oil |
| 22 | H | H | H | Me | H | 2-Me-Ph | 100-105 |
| 23 | H | H | Me | Me | H | 2-Me-Ph | 105-109 |
| 24 | H | H | H | H | H | 4-F-Ph | 169-171 |

a $^1H$ NMR data, given for an oil, are given in Index Table O

| CMPD. NO. | $R^1$ | $R^2$ | $R^3$ | $R^4$ | $R^{18}$ | E | mp(°C)[a] |
|---|---|---|---|---|---|---|---|
| 25 | H | H | H | H | H | Ph | 149-151 |
| 26 | H | H | Me | Me | H | 2-furanyl | 139-141 |
| 27 | H | H | H | Me | H | 2-furanyl | 152 (Dec) |
| 29 | H | H | H | H | H | 2-furanyl | 175 (Dec) |
| 30 | Et | H | H | Me | H | Ph | oil |
| 31 | Et | H | Me | Me | H | Ph | 153-155 |
| 32 | H | H | Me | Me | H | 2-naphthalenyl | 134-137 |
| 33 | H | H | H | Me | H | 2-naphthalenyl | 182-184 |
| 34 | H | H | H | Me | H | 3-thienyl | 90-95 |
| 35 | H | H | Me | Me | H | 3-thienyl | 150-152 |
| 36 | i-Pr | H | Me | Me | H | Ph | 168-170 |
| 37 | i-Pr | H | H | Me | H | Ph | 95-103 |
| 38 | H | H | Me | Me | H | 2,5-dimethyl-3-thienyl | 129-131 |
| 39 | H | H | H | Me | H | 2,5-dimethyl-3-furanyl | 118-122 |
| 40 | H | H | H | Me | H | 2,5-dimethyl-3-thienyl | 119-124 |
| 41 | H | H | Me | Me | H | 2,5-dimethyl-3-furanyl | 111-113 |
| 47 | H | H | Me | Me | H | 2-Br-Ph | 85-92 |
| 48 | H | H | Me | Me | H | 2-i-PrO-Ph | 115-120 |
| 49 | H | H | Me | Me | H | 2,5-di-MeO-Ph | 154-156 |
| 50 | H | H | Me | Me | H | 2,4-diCl-Ph | 103-109 |
| 51 | H | H | Me | Me | H | 3-Me-2-thienyl | 138-140 |
| 52 | H | H | Me | Me | H | 3-F-Ph | 139-141 |
| 53 | H | H | Me | Me | H | 2-fluorenyl | 179-183 |

Dec for mp indicates decomposition.

a $^1H$ NMR data, given for an oil, are given in Index Table O

| CMPD. NO. | $R^1$ | $R^2$ | $R^3$ | $R^4$ | $R^{18}$ | E | mp(°C)[a] |
|---|---|---|---|---|---|---|---|
| 54 | H | H | Me | Me | H | 2-MeO-Ph | 142-150 |
| 55 | H | H | Me | Me | H | 3-Cl-Ph | 144-149 |
| 56 | H | H | Me | Me | H | 4-Me-Ph | 89-92 |
| 57 | Ph | H | Me | Me | H | Ph | 167-170 |
| 58 | H | H | Me | Me | H | 4-Ph-Ph | 220 |
| 61 | H | H | Me | Me | H | 2,4,6-trimethyl-Ph | 110-150 |
| 62 | H | Ph | Me | Me | H | Ph | 179-181 |
| 63 | H | H | Me | Me | H | 3-Me-Ph | 129-131 |
| 64 | H | H | Me | Me | H | t-Bu | 129-131 |
| 65 | H | H | Me | Me | H | 2-pyridyl (85% pure) | 96-105 |
| 70 | H | H | Me | Me | H | 4-n-Pr-Ph | 90-95 |
| 71 | H | H | Me | Me | H | 3,4-dimethyl-Ph | 145-148 |
| 72 | H | H | Me | Me | H | 4-Et-Ph | 106-112 |
| 73 | H | H | Me | Me | H | 4-cyclohexyl-Ph | 164-167 |
| 74 | H | H | Me | Me | H | 2,4,5-trimethyl-Ph | 150-152 |
| 75 | H | H | Me | Me | H | 2,4-dimethyl-Ph | 109-112 |
| 76 | H | H | Me | Me | H | 2,6-di-MeO-Ph | 109-125 |
| 77 | H | H | Me | Me | H | 2,5-dimethyl-Ph | 141-143 |
| 78 | H | H | Me | Me | H | 6-Me-2-naphthalenyl | 186-189 |
| 114 | H | H | Me | $CF_3$ | H | 4-Cl-Ph | 173-175 |
| 115 | H | H | Me | $CF_3$ | H | Ph | 151-152 |
| 116 | H | H | Me | $CF_3$ | H | 4-Me-Ph | gum |
| 118 | H | H | Me | Ph | H | 4-Cl-Ph | 110-113 |
| 119 | H | H | Me | cyclo-propyl | H | 4-Cl-Ph | 167-169 |
| 120 | H | H | Me | OH | n-Bu | 4-Cl-Ph | 228-231 |

a $^1H$ NMR data, given for oils and gums, are given in Index Table O

INDEX TABLE C

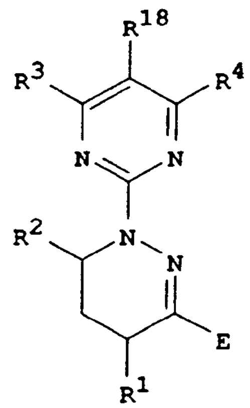

| CMPD. NO. | $R^1$ | $R^2$ | $R^3$ | $R^4$ | $R^{18}$ | E | mp (°C)[a] |
|---|---|---|---|---|---|---|---|
| 42 | H | H | Me | Me | H | Ph | 94-97 |
| 43 | H | H | Me | Me | H | 4-F-Ph | 90-95 |
| 44[b] | H | H | H | Me | H | Ph | oil |
| 45 | H | H | H | Me | H | 4-F-Ph | 134-136 |
| 46 | H | H | Me | Me | H | 4-Br-Ph | 161-164 |
| 59 | H | H | Me | Me | H | 4-OH-Ph | >220°C |
| 60 | H | H | Me | Me | H | 4-t-Bu | 105-115°C |
| 79 | H | H | Me | Me | H | 4-Me-Ph | 102-104 |
| 80 | H | H | Me | Me | H | 4-Cl-Ph | 146-149 |
| 81 | H | H | Me | Me | H | 4-MeO-Ph | 91-94 |
| 82 | H | H | Me | Me | H | 3,4-dimethyl-Ph | 120-121 |
| 86 | H | H | Me | Me | H | 4-n-Pr-Ph | 103-106 |
| 87 | H | H | Me | Me | H | 4-i-Pr-Ph | 90-93 |
| 88 | H | H | Me | Me | H | 4-s-Bu-Ph | 74-77 |
| 89 | H | H | Me | Me | H | 4-Et-Ph | 66-71 |
| 90 | H | H | Me | Me | H | 2,4-dimethyl-Ph | 91-93 |
| 91 | H | H | Me | Me | H | 4-n-Bu-Ph | 55-58 |

a $^1H$ NMR data for oils are given in Index Table O.

b 5:1 ratio of the compound to 4-chlorobutyrophenone.

| CMPD. NO. | $R^1$ | $R^2$ | $R^3$ | $R^4$ | $R^{18}$ | E | mp (°C)[a] |
|---|---|---|---|---|---|---|---|
| 92 | H | OH | Me | Me | H | Ph | 153-155 |
| 93 | H | H | Me | Me | H | 4-cyclohexyl-Ph | 139-141 |
| 94 | H | H | Me | Me | H | 2-Me-Ph | 90-92 |
| 95 | H | H | Me | Et | H | 3,4-dimethyl-Ph | 106-110 |
| 96 | H | H | Me | Me | H | 4-*i*-Bu-Ph | 76-79 |
| 97 | H | H | Me | Me | H | 2-tetrahydro-naphthalenyl | 162-164 |
| 98 | H | H | Me | Me | H | 4-Ph-Ph | 169-171 |
| 99 | H | H | Me | Me | H | 2-indanyl | 140-142 |
| 100 | H | H | Me | Me | H | 4-hexyl-Ph | gum |
| 101 | H | H | Me | Me | H | 3,4-diethyl-Ph | 75-80 |
| 102 | H | H | Me | Me | H | 4-n-pentyl-Ph | 60-63 |
| 103 | H | H | Me | Me | H | 4-PhO | 152-154 |
| 104 | H | H | Me | Me | H | 3-Me-4-Et-Ph(50%) & 3-Et-4-Me-Ph(50%) | 103-106 |
| 105 | Me | H | Me | Me | H | Ph | 109-111 |
| 106 | Et | H | Me | Me | H | Ph | 112-114 |
| 107 | H | H | Me | Me | H | 2,5-dimethyl-Ph | gum |
| 108 | H | H | Me | Me | H | 4-(1-(2-Cl-propyl))-Ph | gum |
| 109 | H | H | Me | Me | H | 3-Cl-Ph | 98-100 |
| 110 | H | H | Me | Me | H | 2-thienyl | 160-162 |
| 111 | H | H | Me | Me | H | 3,4,5-trimethyl-Ph | 154-156 |
| 112 | H | H | Me | Me | H | 2,5-diethyl-Ph | gum, 90% pure |
| 113 | H | MeO | Me | Me | H | Ph | 104-108 |
| 121 | H | H | Me | Me | Cl | 3,4-dimethyl-Ph | 169-173 |

a Dec for mp indicates decomposition.

$^1H$ NMR data for oils and gums are in Index Table O.

| CMPD. NO. | $R^1$ | $R^2$ | $R^3$ | $R^4$ | $R^{18}$ | E | mp(°C)[a] |
|---|---|---|---|---|---|---|---|
| 122 | H | H | Me | $CF_3$ | H | 4-Cl-Ph | 150-152 |
| 123 | H | H | Me | cyclo-propyl | H | 4-Cl-Ph | 123-126 |
| 124 | H | H | Me | Me | Br | 3,4-dimethyl-Ph | 175-179 |
| 125 | H | H | Me | cyclo-propyl | H | 4-Me-Ph | gum |
| 126 | H | H | Me | OH | n-Bu | 4-Cl-Ph | 171-181 |
| 127 | H | H | Me | Me | H | 3,4-dimethyl-Ph | gum |
| 128 | H | H | Me | OH | n-Bu | 3,4-dimethyl-Ph | 140-155 |
| 129 | H | H | Me | i-Bu | H | 3,4-dimethyl-Ph | oil |
| 130 | H | H | Me | i-Bu | H | 4-Cl-Ph | 136-141 |
| 131 | H | H | Me | $CH_2CH_2CH_2$ | | 4-Cl-Ph | 181-184 |
| 132 | H | H | Me | Et | H | 4-Cl-Ph | 95-98 |
| 133 | H | H | Me | cyclo- | H | 4-i-Pr-Ph propyl | 95-99 |
| 134 | H | H | Me | i-Pr | H | 4-Cl-Ph | 96-98 |
| 135 | H | H | Me | cyclo-propyl | H | 4-Et-Ph | gum |
| 136 | H | H | Me | MeO | H | 4-Cl-Ph | 139-143 |
| 137 | H | H | Me | i-Pr | H | 3,4-dimethyl-Ph | oil |
| 138 | H | H | Me | cyclo-propyl | H | 4-n-Pr-Ph | 128-132 |
| 139 | H | H | Me | Ph | H | 4-Cl-Ph | oil, 70% pure |
| 140 | H | H | Me | cyclo-propyl | H | 4-MeO-Ph | oil |
| 141 | H | H | Me | MeO | H | 3,4-dimethyl-Ph | 145-148 |

a $^1H$ NMR data for oils and gums are given in Index Table O.

| CMPD. NO. | $R^1$ | $R^2$ | $R^3$ | $R^4$ | $R^{18}$ | E | mp(°C)[a] |
|---|---|---|---|---|---|---|---|
| 142 | H | H | Me | Me | Me | 4-Cl-Ph | 161-169 |
| 143 | H | H | Me | Et | H | 4-Et-Ph | oil |
| 144 | H | H | Me | Et | H | 4-*i*-Pr-Ph | oil |
| 145 | H | H | Me | Et | H | 4-MeO-Ph | oil |
| 146 | H | H | Me | Et | H | 4-Me-Ph | oil |
| 147 | H | H | Me | *i*-Bu | H | 4-Me-Ph | oil |
| 159 | H | H | Me | Me | H | 2,4-diEt-Ph | 48-51 |
| 160 | H | H | Me | Me | H | 2-Me-4-*t*-Bu-Ph | 130-133, 85% pure |
| 161 | H | H | Me | Me | H | 3-Me-Ph | 128-130 |
| 162 | H | H | Me | Me | H | 3-$CF_3$-Ph | 86-88 |
| 163 | H | H | Me | TMS-$CH_2$ | H | 3,4-diMe-Ph | oil |
| 164 | H | H | Et | Et | H | 4-Cl-Ph | 111-114 |
| 165 | H | H | Et | Et | H | 3,4-diMe-Ph | oil |
| 166 | H | H | Me | *i*-Pr | H | 4-*i*-Pr-Ph | oil |
| 167 | H | H | Et | *i*-Pr | H | 4-Ph-Ph | gum |
| 168 | H | H | Me | *i*-Pr | H | 4-OMe-Ph | oil |
| 169 | H | H | Me | $NMe_2$ | H | 3,4-diMe-Ph | oil |
| 170 | H | H | Et | Et | H | 4-OMe-Ph | oil |
| 171 | H | H | Me | *i*-Pr | H | 4-Et-Ph | gum |
| 172 | H | H | Et | Et | H | 4-*i*-Pr-Ph | oil |

---

$^1H$ NMR data for oils and gums are given in Index Table O.

INDEX TABLE D

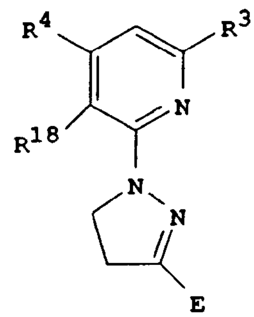

| CMPD. NO. | $R^3$ | $R^4$ | $R^{18}$ | E | mp(°C) |
|---|---|---|---|---|---|
| 19 | H | H | H | Ph | 74-79 |
| 28 | H | H | H | 2-furanyl | 91-93 |
| 66 | Me | Me | CN | Ph | >240 |
| 67 | Me | $CF_3$ | H | Ph | 215-219 |
| 68 | Me | H | H | Ph | 120-121 |
| 69 | Me | H | H | 1-naphthalenyl | 85-90 |

INDEX TABLE E

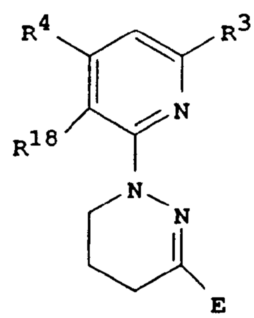

| CMPD. NO. | $R^3$ | $R^4$ | $R^{18}$ | E | mp(°C) |
|---|---|---|---|---|---|
| 83 | Me | $CF_3$ | H | Ph | 75-81 |
| 84 | Me | $CF_3$ | H | 4-*t*-Bu-Ph | 84-90 |
| 85 | Me | H | H | 4-Me-Ph | 82-86 |

INDEX TABLE F

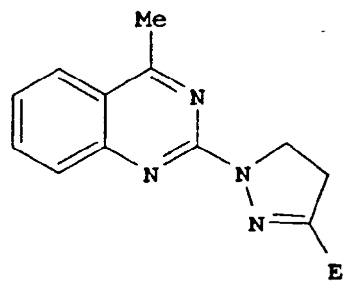

| CMPD. NO. | E | mp(°C) |
|---|---|---|
| 152 | 4-Cl-Ph | 171-180 |

INDEX TABLE G

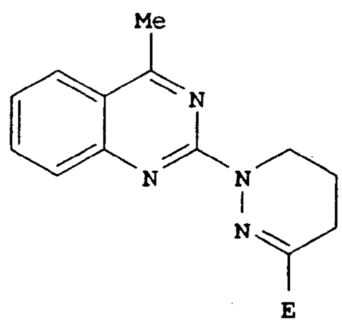

| CMPD. NO. | E | mp(°C) |
|---|---|---|
| 153 | 3,4-dimethyl-Ph | 159-161 |
| 154 | 4-Cl-Ph | 248-252 |
| 155 | 4-*i*-Pr-Ph | 136-142 |
| 173 | 4-MeO-Ph | 150-152 |

INDEX TABLE H

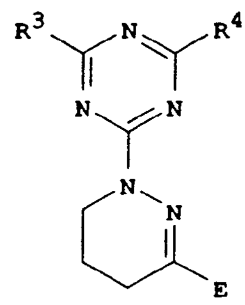

| CMPD. NO. | $R^3$ | $R^4$ | E | mp(°C)[a] |
|---|---|---|---|---|
| 156 | Cl | Cl | 4-Cl-Ph | 181-185 |
| 157 | Me | Cl | 3,4-dimethyl-Ph | oil |

[a] $^1$H NMR data for oils are given in Index Table O.

INDEX TABLE I

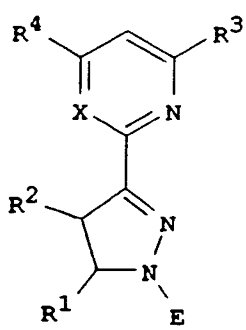

| CMPD. NO. | $R^1$ | $R^2$ | $R^3$ | $R^4$ | X | E | mp(°C) |
|---|---|---|---|---|---|---|---|
| 150 | H | H | H | H | CH | Ph | 77-78 |

## INDEX TABLE J

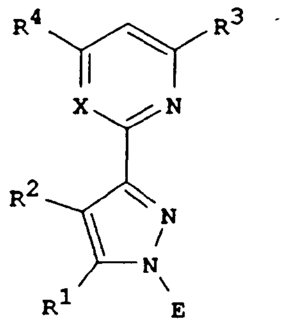

| CMPD. NO. | $R^1$ | $R^2$ | $R^3$ | $R^4$ | X | E | mp(°C)[a] |
|---|---|---|---|---|---|---|---|
| 149 | H | H | Me | Me | N | Ph | oil |

[a] $^1H$ NMR data for oils are given in Index Table O.

## INDEX TABLE K

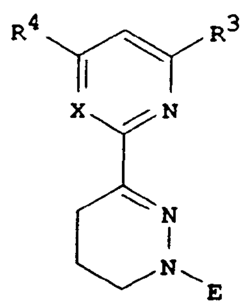

| CMPD. NO. | $R^3$ | $R^4$ | X | E | mp(°C) |
|---|---|---|---|---|---|
| 151 | Me | Me | N | Ph | 134-135 |

INDEX TABLE L

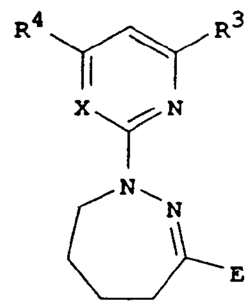

| CMPD. NO. | $R^3$ | $R^4$ | X | E | mp(°C) |
|---|---|---|---|---|---|
| 158 | Me | Me | N | Ph | 97-98 |

INDEX TABLE M

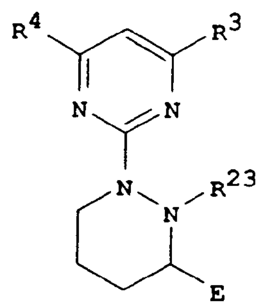

| CMPD. NO. | $R^3$ | $R^4$ | $R^{23}$ | E | mp(°C)[a] |
|---|---|---|---|---|---|
| 174 | Me | Me | H | 3,4-diMe-Ph | oil |

a $^1H$ NMR data for oils are given in Index Table O.

INDEX TABLE N

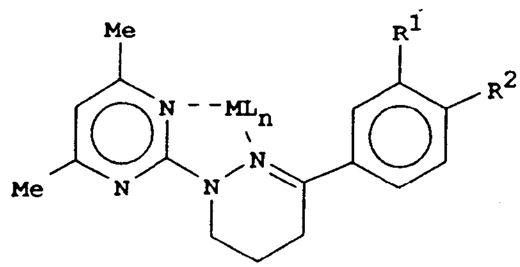

| CMPD. NO. | $ML_n$ | $R^1$ | $R^2$ | mp(°C) |
|---|---|---|---|---|
| 175 | $ZnCl_2$ | H | Cl | 231-232 |
| 176 | $FeCl_3$ | H | Cl | 172-173 |
| 177 | $CuCl_2$ | H | Cl | 135-138 |
| 178 | $CuCl_2$ | $CH_3$ | $CH_3$ | 132-133.5 |
| 179 | $FeCl_3$ | $CH_3$ | $CH_3$ | 150-151 |
| 180 | $MnCl_2$ | $CH_3$ | $CH_3$ | 232-233 |
| 181 | $ZnCl_2$ | $CH_3$ | $CH_3$ | 250-251 |
| 182 | $MgCl_2$ | $CH_3$ | $CH_3$ | 100-101 |

INDEX TABLE O

| CMPD. NO. | $^1$H NMR Data[a] |
| --- | --- |
| 1 | 2.56(s, 6H), 2.72(s, 3H), 6.58(s, 1H), 6.95(s, 1H) |
| 7 | 1.34(d, 3H), 2.46(s, 3H), 6.57(d, 1H), 8.33(d, 1H) |
| 16 | 1.4(d, 3H), 2.4(s, 6H), 4.0(dd, 1H), 4.2(dd, 1H), 6.4(s, 1H) |
| 21 | 2.5(s, 3H), 3.5(t, 2H), 4.2(t, 2H), 6.6(d, 1H), 8.3(d, 1H) |
| 30 | 0.93(t, 3H), 2.4(s, 3H), 6.50(d, 1H), 8.28(d, 1H) |
| 44 | 2.11(m, 2H), 2.46(s, 3H), 2.72(t, 2H), 4.8(t, 2H), 6.60(d, 1H), 7.8(d, 2H), 7.8(d, 2H), 8.40(d, 1H) |
| 116 | 7.75(d, 2H), 7.2(d, 2H), 6.8(s, 1H), 4.2(t, 2H), 3.35(t, 2H), 2.6(s, 3H), 2.4(s, 3H) |
| 100 | 0.88(t, 2H), 1.29(m, 6H), 1.61(m, 2H), 2.15(m, 2H), 2.42(s, 6H), 2.62(t, 2H), 2.70(t, 2H), 4.09(t, 2H), 6.50(s, 1H), 7.18(d, 2H), 7.78(d, 2H) |
| 107 | 2.1(m, 2H), 2.32(s, 3H), 2.38(s, 6H), 2.47(s, 3H), 2.59(t, 2H), 4.10(t, 2H), 6.47(s, 1H), 7.05(d, 1H), 7.16(d, 1H), 7.21(s, 1H) |
| 108 | 1.48(d, 3H), 2.10(m, 2H), 2.41(s, 6H), 2.69(t, 2H), 2.95(dd, 1H), 3.10(dd, 1H), 4.08(t, 2H), 4.1(m, 1H), 6.50(s, 1H), 7.20(d, 2H), 7.81(d, 2H) |
| 112 | 1.2(t, 3H), 1.27(t, 3H), 2.11(m, 2H), 2.36(s, 6H), 2.60(m, 4H), 2.80(q, 2H), 4.10(t, 2H), 6.46(s, 1H), 7.09(d, 1H), 7.16(s, 1H), 7.17(d, 1H) |

[a] $^1$H NMR data are in ppm downfield from tetramethylsilane. Couplings are designated by (s)-singlet, (d)-doublet, (dd)-doublet of doublets, (t)-triplet, (q)-quartet, (m)-multiplet. Samples dissolved in $CDCl_3$ unless otherwise indicated.

| CMPD. NO. | $^1$H NMR Data[a] |
| --- | --- |
| 125 | 7.8(d, 2H), 7.4(d, 2H), 6.4(s, 1H), 4.0(m, 2H), 2.67(t, 2H), 2.42(s, 3H), 2.35(s, 3H), 2.1(m, 2H), 1.9(m, 1H), 1.1(m, 2H), 1.0(m, 2H) |
| 127 | 7.68(s, 1H), 7.55(m, 1H), 7.15(d, 1H), 6.46(s, 1H), 4.01(m, 2H), 2.67(t, 2H), 2.42(s, 3H), 2.27(2s, 6H), 2.15(m, 2H), 1.90(m, 1H), 1.14(m, 2H), 1.00(m, 2H) |
| 129 | 7.7(s, 1H), 7.55(m, 1H), 7.1(d, 1H), 6.45(s, 1H), 4.1(m, 2H), 2.70(t, 2H), 2.50(d, 2H), 2.45(s, 3H), 2.29(s, 3H), 2.27(s, 3H), 2.0-2.2(m, 3H), 0.95(m, 6H) |
| 135 | 7.76(d, 2H), 7.22(d, 2H), 6.47(s, 1H), 4.0(m, 2H), 2.67(m, 4H), 2.41(s, 3H), 2.1(m, 2H), 1.9(m, 1H), 1.24(t, 3H), 1.1(m, 2H), 0.95(m, 2H) |
| 137 | 7.7(s, 1H), 7.59(m, 2H), 7.10(d, 1H), 6.50(s, 1H), 4.1(m, 2H), 2.9(m, 1H), 2.7(t, 2H), 2.45(s, 3H), 2.30(s, 3H), 2.27(s, 3H), 2.1(m, 2H), 1.28(d, 6H) |
| 139 | 8.1(m, 2H), 7.85(m, 2H), 7.85(d, 2H), 7.47(m, 3H), 7.36(d, 2H), 7.11(s, 1H), 4.2(m, 2H), 2.7(t, 2H), 2.56(s, 3H), 2.15(m, 2H) |
| 140 | 7.8(d, 2H), 6.9(d, 2H), 6.46(s, 1H), 4.05(m, 2H), 3.81(s, 3H), 2.65(t, 2H), 2.41(s, 3H), 2.1(m, 2H), 1.9(m, 1H), 1.1(m, 2H), 0.95(m, 2H) |
| 143 | 7.78(d, 2H), 7.2(d, 2H), 6.5(s, 1H), 4.05(m, 2H), 2.7(m, 6H), 2.44(s, 3H), 2.15(m, 2H), 1.30(t, 3H), 1.24(t, 3H) |

---

a $^1$H NMR data are in ppm downfield from tetramethylsilane. Couplings are designated by (s)-singlet, (d)-doublet, (dd)-doublet of doublets, (t)-triplet, (q)-quartet, (m)-multiplet. Samples dissolved in $CDCl_3$ unless otherwise indicated.

| CMPD. NO. | $^1H$ NMR Data[a] |
|---|---|
| 144 | 7.79(d, 2H), 7.22(d, 2H), 6.50(s, 1H), 4.05(m, 2H), 2.9(m, 1H), 2.69(m, 4H), 2.43(s, 3H), 2.05(m, 2H), 1.3(t, 3H), 1.27(d, 6H) |
| 145 | 7.8(d, 2H), 6.9(d, 2H), 6.5(s, 1H), 4.1(m, 2H), 3.83(s, 3H), 2.68(m, 4H), 2.43(s, 3H), 2.1(m, 2H), 1.30(t, 3H) |
| 146 | 7.76(d, 2H), 7.17(d, 2H), 6.5(s, 1H), 4.10(m, 2H), 2.68(m, 4H), 2.43(s, 3H), 2.36(s, 3H), 2.10(m, 2H), 1.30(t, 3H) |
| 147 | 7.75(d, 2H), 7.15(d, 2H), 6.5(s, 1H), 4.1(m, 2H), 2.9(m, 1H), 2.7(t, 2H), 2.45(s, 3H), 2.36(s, 3H), 2.1(m, 2H), 1.28(d, 6H) |
| 149 | 2.60(s, 6H), 6.99(s, 1H), 7.32(m, 2H), 7.46(t, 2H), 7.84(d, 2H), 8.01(d, 1H) |
| 157 | 2.05(s, 3H), 2.1(m, 2H), 2.32(s, 6H), 3.04(t, 2H), 4.20(t, 2H), 7.23(d, 1H), 7.33(m, 3H) |
| 163 | 7.75(m, 1H), 7.6(m, 1H), 7.1(m, 1H), 6.5(s, 1H), 4.1(m, 2H), 2.7(m, 2H), 2.44(s, 5H), 2.3(s, 3H), 2.27(s, 3H), 2.1(m, 2H), 0.15(s, 9H) |
| 165 | 7.7(s, 1H), 7.55(d, 1H), 7.1(d, 1H), 6.51(s, 1H), 4.1(m, 2H), 2.70(m, 6H), 2.30(s, 3H), 2.27(s, 3H), 2.1(m, 2H), 1.32(t, 6H) |
| 166 | 7.8(d, 2H), 7.2(d, 2H), 6.5(s, 1H), 4.1(m, 2H), 2.9(m, 2H), 2.7(t, 2H), 2.45(s, 3H), 2.1(m, 2H), 1.27(m, 12H) |

[a] $^1H$ NMR data are in ppm downfield from tetramethylsilane. Couplings are designated by (s)-singlet, (d)-doublet, (dd)-doublet of doublets, (t)-triplet, (q)-quartet, (m)-multiplet. Samples dissolved in $CDCl_3$ unless otherwise indicated.

| CMPD. NO. | $^1$H NMR Data[a] |
|---|---|
| 167 | 7.95(d, 2H), 7.62(m, 2H), 7.44(m, 2H), 7.30(m, 1H), 6.52(m, 1H), 4.10(m, 2H), 2.9(m, 1H), 2.73(t, 2H), 2.47(s, 3H), 2.15(m, 2H), 1.29(d, 6H) |
| 168 | 7.82(d, 2H), 6.90(d, 2H), 6.49(s, 1H), 4.09(m, 2H), 3.83(s, 3H), 2.90(m, 1H), 2.68(m, 2H), 2.45(s, 3H), 2.10(m, 2H), 1.28(d, 6H) |
| 169 | 7.7(s, 1H), 7.58(m, 1H), 7.10(d, 1H), 5.85(s, 1H), 4.05(m, 2H), 3.12(s, 6H), 2.65(t, 2H), 2.34(s, 3H), 2.29(s, 3H), 2.26(s, 3H), 2.10(m, 2H) |
| 170 | 7.82(d, 2H), 6.90(d, 2H), 6.50(s, 1H), 4.10(m, 2H), 3.83(s, 3H), 2.7(m, 6H), 2.1(m, 2H), 1.31(t, 6H) |
| 171 | 7.8(d, 2H), 7.2(d, 2H), 6.5(s, 1H), 4.1(m, 2H), 2.9(m, 1H), 2.7(m, 4H), 2.45(s, 3H), 2.1(m, 2H), 1.28(d, 6H), 1.24(t, 3H) |
| 172 | 7.79(d, 2H), 7.22(d, 2H), 6.50(s, 1H), 4.1(m, 2H), 2.9(m, 1H), 2.7(m, 6H), 2.1(m, 2H), 1.3(m, 12H) |
| 174 | 7.25(s, 1H), 7.17(m, 2H), 6.4(brS, 1H), 6.22(s, 1H), 4.8(m, 1H), 3.7(m, 1H), 3.2(m, 1H), 2.38(s, 3H), 2.27(s, 9H), 1.9(m, 2H), 1.8(m, 1H), 1.7(m, 1H) |

[a] $^1$H NMR data are in ppm downfield from tetramethylsilane. Couplings are designated by (s)-singlet, (d)-doublet, (dd)-doublet of doublets, (t)-triplet, (q)-quartet, (m)-multiplet. Samples dissolved in $CDCl_3$ unless otherwise indicated.

(brS) = broad singlet

Results for Tests A to F are given in Table 1. In the table, a rating of 100 indicates 100% disease control and a rating of 0 indicates no disease control (relative to the carrier sprayed controls). NT indicates that no test was performed.

TABLE 1

| Cmpd No. | Test A | Test B | Test C | Test D | Test E | Test F |
|---|---|---|---|---|---|---|
| 1 | 97 | NT | 97 | 0 | NT | 0 |
| 2 | 95 | 97 | 14 | 25 | 47* | 0 |
| 3 | 0 | NT | 24 | 0 | NT | 0 |
| 4 | 80 | 96 | 7 | 0 | NT | 67 |
| 5 | 98 | 100 | 24 | 0 | NT | 81 |
| 6 | 61 | 89 | 7 | 91 | 79 | 96 |
| 7 | 91 | 99 | 27 | 0 | 0 | 0 |
| 8 | 74 | 53 | 0 | 29 | 90 | 6 |
| 9 | 0 | 14 | 67 | 0 | 37 | 45 |
| 10 | 61 | 66 | 0 | 14 | 26 | 0 |
| 11 | 61 | 62 | 0 | 21 | 9 | 0 |
| 12 | 81 | 87 | 67 | 36 | 80 | 89 |
| 13 | 79 | 97 | 0 | 34 | 0 | 0 |
| 14 | 61 | 90 | 16 | 21 | 0 | 46 |
| 15 | 68 | 73 | 0 | 42 | 0 | 0 |
| 16 | 98 | 100 | 0 | 19 | 100 | 89 |
| 17 | 82 | 0 | 0 | 19 | 0 | 0 |
| 18 | 0 | 14 | 0 | 0 | 11 | 0 |
| 19 | 63 | 14 | 0 | 0 | 37 | 45 |
| 20 | 0 | 14 | 0 | 0 | 11 | 4 |
| 21 | 86 | 62 | 0 | 46 | 37 | 0 |
| 22 | 86 | 62 | 0 | 0 | 11 | 4 |

| Cmpd No. | Test A | Test B | Test C | Test D | Test E | Test F |
|---|---|---|---|---|---|---|
| 23 | 95 | 62 | 97 | 46 | 11 | 94 |
| 24 | 84 | 0 | 0 | 26 | 48 | 0 |
| 25 | 73 | 49 | 0 | 47 | 48 | 0 |
| 26 | 56 | 49 | 0 | 47 | 66 | 0 |
| 27 | 56 | 49 | 0 | 26 | 48 | 0 |
| 28 | 27 | 0 | 0 | 0 | 24 | 0 |
| 29 | 27 | 0 | 0 | 26 | 0 | 0 |
| 30 | 98 | 97 | 88 | 62 | 73 | 0 |
| 31 | 90 | 97 | 95 | 0 | 92 | 94 |
| 32 | 83 | 92 | 18 | 44 | 15 | 0 |
| 33 | 24 | 16 | 0 | 0 | 39 | 0 |
| 34 | 24 | 16 | 18 | 76 | 100 | 0 |
| 35 | 54 | 62 | 18 | 76 | 15 | 10 |
| 36 | 83 | 98 | 88 | 22 | 96 | 0 |
| 37 | 54 | 81 | 74 | 92 | 100 | 0 |
| 38 | 57 | 65 | 84 | 0 | 42 | 97 |
| 39 | 0 | 65 | 0 | 25 | 19 | 0 |
| 40 | 28 | 65 | 47 | 47 | 92 | 0 |
| 41 | 0 | 21 | 23 | 0 | 19 | 46 |
| 42 | 96 | 99 | 60 | 99 | 35 | 82 |
| 43 | 57 | 89 | 61 | 82 | 35 | 89 |
| 44 | 88 | 100 | 16 | 68 | 49 | 94 |
| 45 | 94 | 89 | 84 | 45 | 38 | 69 |
| 46 | 60 | 58 | 100 | 0 | 91 | 98 |
| 61 | 58 | 89 | 97 | 0 | 96 | 48 |
| 62 | 91 | 93 | 82 | 0 | 37 | 0 |
| 63 | 20 | 53 | 79 | 76 | 100 | 97 |
| 64 | 37 | 21 | 30 | 47 | 0 | 0 |
| 65 | 30 | 54 | 0 | 0 | 0 | 18 |
| 66 | 37 | 0 | 0 | 0 | 0 | 0 |
| 67 | 86 | 0 | 0 | 0 | 0 | 0 |

| Cmpd No. | Test A | Test B | Test C | Test D | Test E | Test F |
|---|---|---|---|---|---|---|
| 68 | 0 | 21 | 8 | 21 | 0 | 0 |
| 69 | 11 | 0 | 11 | 0 | 0 | 0 |
| 70 | 75 | 61 | 76 | 75 | 92 | 0 |
| 71 | 32 | 41 | 39 | 47 | 92 | 98 |
| 72 | 59 | 86 | 29 | 26 | 58 | 47 |
| 73 | 0 | 41 | 0 | 0 | 15 | 0 |
| 74 | 11 | 0 | 16 | 0 | 0 | 0 |
| 75 | 41 | 0 | 2 | 92 | 75 | 10 |
| 76 | 60 | 27 | 75 | 92 | 0 | 0 |
| 77 | 52 | 46 | 96 | 84 | 42 | 94 |
| 78 | 2 | 19 | 2 | 0 | 0 | 6 |
| 79 | 89 | 100 | 100 | 47 | 91 | 98 |
| 80 | 91 | 100 | 100 | 25 | 91 | 98 |
| 81 | 66 | 98 | 99 | 97 | 75 | 48 |
| 82 | 81 | 98 | 97 | 47 | 97 | 88 |
| 83 | 25 | 0 | 10 | 46 | 0 | 0 |
| 84 | 46 | 0 | 0 | 46 | 0 | 0 |
| 85 | 20 | 0 | 20 | 0 | 21 | 0 |
| 86 | 99 | 100 | 99 | 0 | 100 | 97 |
| 87 | 99 | 100 | 99 | 25 | 99 | 82 |
| 88 | 99 | 99 | 97 | 25 | 100 | 46 |
| 89 | 97 | 100 | 99 | 0 | 93 | 97 |
| 90 | 98 | 100 | 100 | 46 | 86 | 94 |
| 91 | 98 | 100 | 97 | 0 | 100 | 46 |
| 92 | 71 | 93 | 96 | 0 | 0 | 90 |
| 93 | 38 | 0 | 8 | 0 | 85 | 0 |
| 94 | 80 | 41 | 0 | 21 | 20 | 0 |
| 95 | 91 | 98 | 90 | 63 | 63 | 90 |
| 96 | 94 | 99 | 90 | 0 | 92 | 69 |
| 97 | 85 | 100 | 90 | 0 | 99 | 90 |
| 98 | 66 | 67 | 90 | 0 | 41 | 0 |
| 99 | 88 | 99 | 91 | 0 | 100 | 99 |

| Cmpd No. | Test A | Test B | Test C | Test D | Test E | Test F |
|---|---|---|---|---|---|---|
| 100 | 63 | 28 | 43 | NT | 92 | 8 |
| 101 | 95 | 98 | 86 | NT | 100 | 94 |
| 102 | 85 | 96 | 82 | NT | 100 | 0 |
| 103 | 72 | 86 | 90 | NT | 43 | 0 |
| 104 | 98 | 100 | 99 | 23 | 100 | 99 |
| 105 | 99 | 100 | 99 | 64 | 92 | 78 |
| 106 | 99 | 100 | 100 | 0 | 100 | 96 |
| 107 | 100 | 92 | 99 | 82 | 100 | 3 |
| 108 | 98 | 100 | 99 | 70 | 92 | 89 |
| 109 | 84 | 100 | 99 | 53 | 100 | 98 |
| 110 | 46 | 67 | 57 | 72 | 0 | 68 |
| 111 | 71* | 44* | 86* | NT | 77* | NT |
| 112 | 99 | 100 | 99 | 57 | 99 | 81 |
| 113 | 95 | 84 | 97 | 37 | 83 | 67 |
| 114 | 45 | 27 | 0 | 58 | 100 | 0 |
| 115 | 18 | 97 | 0 | 58 | 0 | 0 |
| 116 | 76 | 66 | 0 | 73 | 42 | 0 |
| 117 | 0 | 12 | 0 | 0 | 19 | 0 |
| 118 | 61 | 12 | 0 | 22 | 0 | 0 |
| 119 | 86 | 61 | 25 | 0 | 19 | 0 |
| 120 | 0 | 24 | 0 | 0 | 0 | 0 |
| 121 | 52 | 12 | 92 | 0 | 42 | 0 |
| 122 | 0 | 12 | 0 | 22 | 42 | 0 |
| 123 | 71 | 12 | 95 | 62 | 92 | 0 |
| 124 | 41 | 0 | 25 | 0 | 19 | 0 |
| 125 | 62 | 84 | 78 | 0 | 97 | 0 |
| 126 | 0 | 0 | 0 | 0 | 0 | 0 |
| 127 | 54 | 9 | 8 | 0 | 100 | 0 |
| 128 | 0 | 24 | 0 | 0 | 0 | 0 |
| 129 | 83 | 64 | 93 | 23 | 97 | 10 |
| 130 | 61 | 25 | 72 | NT | 75 | 0 |

| Cmpd No. | Test A | Test B | Test C | Test D | Test E | Test F |
|---|---|---|---|---|---|---|
| 131 | 61 | 66 | 93 | NT | 97 | 99 |
| 132 | 100 | 100 | 99 | NT | 100 | 99 |
| 133 | 100 | 99 | 91 | 0 | 100 | 88 |
| 134 | 91 | 52 | 91 | 0 | 92 | 93 |
| 135 | 96 | 85 | 80 | 0 | 100 | 88 |
| 136 | 89 | 26 | 32 | 0 | 100 | 88 |
| 137 | 98 | 67 | 91 | 0 | 100 | 93 |
| 138 | 0 | 65 | 14 | 0 | 39 | 62 |
| 139 | 26 | 65 | 14 | 0 | 14 | 0 |
| 140 | 97 | 96 | 92 | 0 | 100 | 96 |
| 141 | 29 | 5 | 0 | 0 | 0 | 3 |
| 142 | 46 | 67 | 96 | 37 | 0 | 99 |
| 143 | 98 | 99 | 98 | 37 | 64 | 96 |
| 144 | 98 | 100 | 96 | 57 | 64 | 68 |
| 145 | 98 | 100 | 99 | 57 | 91 | 94 |
| 146 | 97 | 100 | 93 | 84 | 92 | 99 |
| 147 | 95 | 100 | 99 | 74 | 92 | 92 |
| 149 | 74 | 79 | 0 | 0 | 0 | 0 |
| 150 | 0 | 24 | 0 | 43 | 0 | 0 |
| 151 | 31** | 0** | 0** | NT | 0** | NT |
| 152 | 55 | 22 | 19 | 0 | 37 | 0 |
| 153 | 86 | 93 | 90 | 0 | 91 | 95 |
| 154 | 80 | 67 | 84 | 12 | 93 | 98 |
| 155 | 73 | 0 | 83 | 0 | 75 | 0 |
| 156 | 0 | 84 | 10 | 0 | 83 | 0 |
| 157 | 10 | 0 | 21 | 12 | 0 | 0 |
| 158 | 76 | 11 | 100 | 63 | 21 | 90 |

| Cmpd No. | Test A | Test B | Test C | Test D | Test E | Test F |
|---|---|---|---|---|---|---|
| 159 | 96* | 85* | 90* | NT | 28* | 79 |
| 160 | 0 | 0 | 8 | 0 | 0 | 0 |
| 161 | 99 | 100 | 99 | 74 | 91 | 99 |
| 162 | 98 | 100 | 99 | 42 | 41 | 93 |
| 163 | 92 | 100 | 97 | 84 | 75 | 96 |
| 164 | 82* | 64 | 89* | 17 | 66* | 97 |
| 165 | 31* | 85 | 93 | 17 | 88 | 38* |
| 166 | 92* | 8* | 35* | 16 | 20* | 64 |
| 167 | 53 | 0 | 0 | 0 | 18 | 0 |
| 168 | 83 | 99 | 96 | 40 | 92 | 99 |
| 169 | 85 | 0 | 23 | 85 | 91 | 46 |
| 170 | 98 | 96 | 99 | 41 | 73 | 99 |
| 171 | 0*** | 11*** | 4*** | NT | NT | NT |
| 172 | 98 | 98 | 90 | 8 | 100 | 0 |
| 173 | 53 | 93 | 61 | 16 | 99 | 99 |
| 174 | 99 | 100 | 98 | 73 | 100 | 93 |
| 175 | 31* | 8* | 89* | 40 | 45* | 99 |
| 176 | 42* | 8* | 82* | 73 | 27* | 99 |
| 177 | 36* | 93 | 86* | 96 | 84* | 99 |
| 178 | 33 | 61 | 84 | 92 | 96 | 98 |
| 179 | 85 | 98 | 99 | 74 | 91 | 98 |
| 180 | 95 | 100 | 99 | 74 | 96 | 99 |
| 181 | 85 | 100 | 100 | 74 | 96 | 99 |
| 182 | 91 | 100 | 99 | 74 | 96 | 94 |

* = Plants were sprayed at a concentration of 40 ppm.
** = Plants were sprayed at a concentration of 20 ppm.
*** = Plants were sprayed at a concentration of 10 ppm.

## Claims

1. A fungicidal compound selected from the group of either Formulae I, II, III, IV, V or VI, including all geometric and stereoisomers, their salts, metal complexes thereof

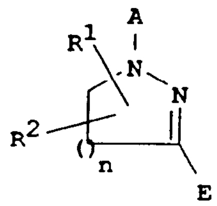

I

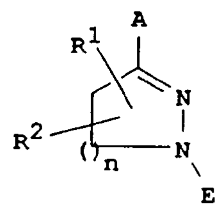

II

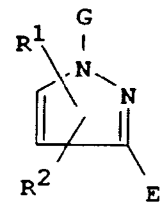

III

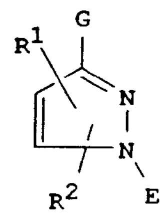

IV

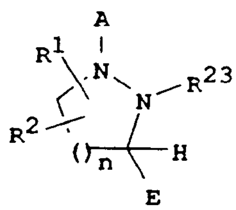

V

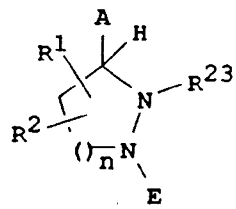

VI

wherein:

A is 2-pyrimidinyl, 2-pyridyl, 2-quinolinyl, 2-quinazolinyl, 1-isoquinolinyl or 3 isoquinolinyl each optionally substituted with $R^3$, $R^4$ and $R^{18}$; or s-triazinyl optionally substituted with $R^3$ and $R^4$; provided that $R^3$, $R^4$ and $R^{18}$ only substitute carbon atoms of the heterocycles;

G is 2-quinazolinyl optionally substituted with $R^3$, $R^4$ and $R^{18}$;

E is H; halogen; $C_1$-$C_6$ alkyl; $C_3$-$C_7$ cycloalkyl optionally substituted with 1-2 methyl; $C_1$-$C_6$ haloalkyl; $C_1$-$C_6$ alkylthio; $C_1$-$C_6$ alkoxy; $C_1$-$C_6$

haloalkoxy; or phenyl, phenoxy, phenylthio, phenylamino, phenylmethyl, indanyl, tetrahydronaphthalenyl, 1-naphthalenyl, 2-naphthalenyl, thienyl, furanyl or pyridyl each optionally substituted with $R^5$, $R^6$ and $R^7$;

n is 1, 2 or 3;

$R^1$ is H; halogen; cyano; hydroxy, $C_1$-$C_4$ alkoxy, -OC(=O)$R^{19}$, -OC(=O)NH$R^{20}$ $C_1$-$C_4$ alkyl; $C_1$-$C_4$ haloalkyl; $C_2$-$C_3$ alkylcarbonyl; $C_2$-$C_4$ alkenyl; $C_2$-$C_6$ alkoxyalkyl; $C_2$-$C_4$ alkynyl; $C_2$-$C_3$ alkoxycarbonyl; or phenyl, phenylmethyl, 1-naphthalenyl, 2-naphthalenyl, thienyl, furanyl or pyridyl each optionally substituted with $R^8$, $R^9$ and $R^{10}$;

$R^2$ is H, cyano, $C_1$-$C_4$ alkyl or $C_1$-$C_4$ haloalkyl;

$R^3$, $R^4$ and $R^{18}$ are independently halogen; cyano; hydroxy; ($C_1$-$C_4$ alkyl)$_3$silylmethyl; phenyl optionally substituted with $R^{21}$; $C_1$-$C_6$ alkyl; cyclopropyl; $C_1$-$C_6$ haloalkyl; $C_1$-$C_6$ alkylthio; $C_2$-$C_4$ alkenyl; $C_2$-$C_4$ alkynyl; $C_1$-$C_4$ alkoxy; $C_1$-$C_4$ haloalkoxy; $C_2$-$C_4$ alkenyloxy; $C_2$-$C_4$ alkynyloxy; $C_2$-$C_4$ alkoxyalkyl; $NR^{11}R^{12}$; or when $R^3$ and $R^4$, $R^3$ and $R^{18}$ or $R^4$ and $R^{18}$ substitute adjacent carbon atoms, then $R^3$ and $R^4$, $R^3$ and $R^{18}$ or $R^4$ and $R^{18}$ may together be $-(CH_2)_3-$ or $-(CH_2)_4-$ each optionally substituted with 1-2 methyl;

$R^5$ and $R^8$ are independently halogen; cyano; nitro; hydroxy, hydroxycarbonyl; $C_1$-$C_6$ alkyl; $C_3$ -$C_6$ cycloalkyl, $C_1$-$C_6$ haloalkyl; $C_1$-$C_4$ alkylthio; $C_1$-$C_4$ alkylsulfinyl; $C_1$-$C_4$ alkylsulfonyl; ($C_1$-$C_4$ alkyl)$_3$silyl; $C_2$-$C_5$ alkylcarbonyl; $C_2$-$C_4$ alkenyl; $C_2$-$C_4$ alkenyloxy; $C_2$-$C_4$ alkynyl; $C_2$-$C_4$ alkynyloxy; $C_1$-$C_4$ alkoxy; $C_1$-$C_4$ haloalkoxy; $C_2$-$C_4$ alkoxyalkyl; $C_2$-$C_5$ alkoxycarbonyl; $C_2$-$C_4$ alkoxyalkoxy; $NR^{13}R^{14}$; C(=O)$NR^{15}R^{16}$; or phenyl, phenoxy or phenylthio each optionally substituted with $R^{17}$;

$R^6$, $R^7$, $R^9$, $R^{10}$, $R^{17}$, $R^{21}$, $R^{22}$, and $R^{24}$ are independently halogen, $C_1$-$C_4$ alkyl, $C_1$-$C_4$ haloalkyl, $C_1$-$C_4$ alkoxy or $C_1$-$C_4$ haloalkoxy;

$R^{11}$, $R^{12}$, $R^{13}$, $R^{14}$, $R^{15}$, $R^{16}$ are independently H; $C_1$-$C_2$ alkyl; or $R^{11}$ and $R^{12}$, $R^{13}$ and $R^{14}$ or $R^{15}$ and $R^{16}$ can be taken together with the nitrogen to which they attached to form a morpholino, pyrrolidino or piperidino group.

$R^{19}$ and $R^{25}$ are H or $C_1$-$C_3$ alkyl;

$R^{20}$ and $R^{26}$ are $C_1$-$C_4$ alkyl; or phenyl optionally substituted with $R^{22}$;

$R^{23}$ is H, $C_1$-$C_4$ alkyl, $C_1$-$C_4$ haloalkyl, $C_2$-$C_5$ alkylcarbonyl, phenylcarbonyl optionally substituted with $R^{24}$, $C_3$-$C_4$ alkenyl, $C_3$-$C_4$ alkynyl, phenylmethyl optionally substituted with $R^{24}$ on the phenyl ring. $C_1$-$C_4$ alkylsulfinyl, $C_1$-$C_4$ alkylsulfonyl, phenylsulfinyl, $C_1$-$C_4$ alkylsulfonyl, phenylsulfinyl optionally substituted with $R^{24}$, phenylsulfonyl optionally substituted with $R^{24}$, $C_2$-$C_4$ alkoxycarbonyl, phenoxycarbonyl option-

ally substituted with $R^{24}$, $C(=O)NR^{25}R^{26}$, $C(=S)NHR^{26}$ $P(=S)(OR^{26})_2$, $P(=O)(OR^{26})_2$, or $S(=O)_2NR^{25}R^{26}$;

provided that

i) when E is halogen, $C_1$-$C_6$ alkylthio, $C_1$-$C_6$ alkoxy, $C_1$-$C_6$ haloalkoxy, phenoxy, phenylthio or phenylamino, then E may only substitute compounds of Formula I and III;

ii) for compounds of Formula I, when A is 2-pyridyl, n is 2, and $R^1$ and $R^2$ are H, then E is not phenyl substituted with 1 to 2 fluorine, chlorine, trifluoromethyl, $C_1$-$C_4$ alkyl, $C_1$-$C_4$ alkoxy, or E is not thienyl or furanyl;

iii) for compounds of Formula III, either E is phenyl, phenoxy, phenylthio, phenylmethyl, 1-naphthalenyl, 2-naphthalenyl, thienyl, furanyl, pyridyl each optionally substituted with $R^5$, $R^6$ and $R^7$; or $R^1$ is phenyl, phenylmethyl, 1-naphthalenyl, 2-naphthalenyl, thienyl, furanyl or pyridyl each optionally substituted with $R^8$, $R^9$ and $R^{10}$; and $R^1$ must be in the 4-position;

iv) for compounds of Formula III, $R^5$ is not $NR^{13}R^{14}$;

v) for compounds of Formulae I and II, when n is 1, $R^1$ and $R^2$ do not occupy the 5-position of the pyrazoline ring;

vi) for compounds of Formula I, when A is s-triazinyl, then $R^3$ or $R^4$ are not $NH_2$;

vii) for compounds of Formula I, when A is 2-pyridyl optionally substituted with $R^3$, $R^{18}$ and $R^4$, and n is 1, then E is not phenylamino optionally substituted with $R^5$, $R^6$ and $R^7$;

viii) for compounds of Formulae I and III, when A is 2-pyridyl, n is 1, and $R^1$ and $R^2$ are H, then E is not phenyl, 4-bromophenyl, 4-methoxyphenyl, 4-nitrophenyl or 4-hydroxyphenyl;

ix) for compounds of Formula II, when n is 3, E is not H or $C_1$-$C_5$ alkyl;

x) for compounds of Formula II, when n is 1, then E is not H;

xi) for compounds of Formula I, when n is 1, and A is 6-methoxypyridine, then E is not 4-*N,N*-diethylaminophenyl;

xii) for compounds of Formula II, when A is 2-pyridyl, n is 2, and $R^1$ and $R^2$ are H, then E is not $C_1$-$C_4$ alkyl or pyridyl.

2. A fungicidal compound selected from the group of either Formulae I or II, including all geometric and stereoisomers, their salts, metal complexes thereof

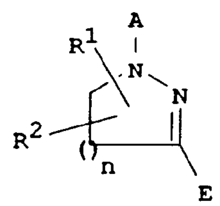

I

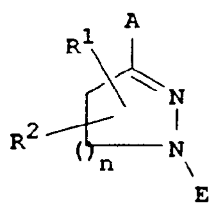

II

wherein:

A is 2-pyrimidinyl or 2-pyridyl, each optionally substituted with $R^3$ and $R^4$; or s-triazinyl optionally substituted with $R^3$ and $R^4$; provided that $R^3$ and $R^4$ only substitute carbon atoms of the heterocycles;

E is H; halogen; $C_1$-$C_6$ alkyl; $C_3$-$C_7$ cycloalkyl optionally substituted with 1-2 methyl; $C_1$-$C_6$ haloalkyl; $C_1$-$C_6$ alkylthio; $C_1$-$C_6$ alkoxy; $C_1$-$C_6$ haloalkoxy; or phenyl, phenoxy, phenylthio, phenylamino, phenylmethyl, 1-naphthalenyl, 2-naphthalenyl, thienyl, furanyl or pyridyl each optionally substituted with $R^5$, $R^6$ and $R^7$;

n is 1, 2 or 3;

$R^1$ is H; halogen; cyano; $C_1$-$C_4$ alkyl; $C_1$-$C_4$ haloalkyl; $C_2$-$C_3$ alkylcarbonyl; $C_2$-$C_4$ alkenyl; $C_2$-$C_6$ alkoxyalkyl; $C_2$-$C_4$ alkynyl; $C_2$-$C_3$ alkoxycarbonyl; or phenyl, phenylmethyl, 1-naphthalenyl, 2-naphthalenyl, thienyl, furanyl or pyridyl each optionally substituted with $R^8$, $R^9$ and $R^{10}$;

$R^2$ is H, cyano, $C_1$-$C_4$ alkyl or $C_1$-$C_4$ haloalkyl;

$R^3$ and $R^4$ are independently halogen; cyano; $C_1$-$C_4$ alkyl; cyclopropyl; $C_1$-$C_4$ haloalkyl; $C_1$-$C_4$ alkylthio; $C_2$-$C_4$ alkenyl; $C_2$-$C_4$ alkynyl; $C_1$-$C_4$ alkoxy; $C_1$-$C_4$ haloalkoxy; $C_2$-$C_4$ alkenyloxy; $C_2$-$C_4$ alkynyloxy; $C_2$-$C_4$ alkoxyalkyl; or $NR^{11}R^{12}$;

$R^5$ and $R^8$ are independently halogen; cyano; nitro; hydroxy, hydroxycarbonyl; $C_1$-$C_4$ alkyl; $C_1$-$C_4$ haloalkyl; $C_1$-$C_4$ alkylthio; $C_1$-$C_4$ alkylsulfinyl; $C_1$-$C_4$ alkylsulfonyl; $(C_1$-$C_4$ alkyl$)_3$silyl; $C_2$-$C_5$ alkylcarbonyl; $C_2$-$C_4$ alkenyl; $C_2$-$C_4$ alkenyloxy; $C_2$-$C_4$ alkynyl; $C_2$-$C_4$ alkynyloxy; $C_1$-$C_4$ alkoxy; $C_1$-$C_4$ haloalkoxy; $C_2$-$C_4$

alkoxyalkyl; $C_2$-$C_5$ alkoxycarbonyl; $C_2$-$C_4$ alkoxyalkoxy; $NR^{13}R^{14}$; $C(=O)NR^{15}R^{16}$; or phenyl, phenoxy or phenylthio each optionally substituted with $R^{17}$;

$R^6$, $R^7$, $R^9$, $R^{10}$ and $R^{17}$ are independently halogen, $C_1$-$C_4$ alkyl, $C_1$-$C_4$ haloalkyl, $C_1$-$C_4$ alkoxy or $C_1$-$C_4$ haloalkoxy;

$R^{11}$, $R^{12}$, $R^{13}$, $R^{14}$, $R^{15}$, $R^{16}$ are independently H or $C_1$-$C_2$ alkyl;

provided that

i) when E is halogen, $C_1$-$C_6$ alkylthio, $C_1$-$C_6$ alkoxy, $C_1$-$C_6$ haloalkoxy, phenoxy, phenylthio or phenylamino, then E may only substitute compounds of Formula I;

ii) for compounds of Formula I, when A is 2-pyridyl, n is 2, and $R^1$ and $R^2$ are H, then E is not phenyl substituted with 1 to 2 fluorine, chlorine, trifluoromethyl, $C_1$-$C_4$ alkyl, $C_1$-$C_4$ alkoxy, or E is not thienyl or furanyl;

iii) for compounds of Formulae I and II, when n is 1, $R^1$ and $R^2$ do not occupy the 5-position of the pyrazoline ring;

iv) for compounds of Formula I, when A is s-triazinyl, then $R^3$ or $R^4$ are not $NH_2$;

v) for compounds of Formula I, when A is 2-pyridyl optionally substituted with $R^3$, $R^{18}$ and $R^4$, and n is 1, then E is not phenylamino optionally substituted with $R^5$, $R^6$ and $R^7$;

vi) for compounds of Formula I, when A is 2-pyridyl, n is 1, and $R^1$ and $R^2$ are H, then E is not phenyl, 4-bromophenyl, 4-methoxyphenyl, 4-nitrophenyl or 4-hydroxyphenyl;

vii) for compounds of Formula II, when n is 3, E is not H or $C_1$-$C_5$ alkyl;

viii) for compounds of Formula II, when n is 1, then E is not H;

ix) for compounds of Formula I, when n is 1, and A is 6-methoxypyridine, then E is not 4-*N,N*-diethylaminophenyl;

x) for compounds of Formula II, when A is 2-pyridyl, n is 2, and $R^1$ and $R^2$ are H, then E is not $C_1$-$C_4$ alkyl or pyridyl.

3. A Compound of Claim 1 of Formula I or V wherein:

A is 2-pyrimidinyl or 2-quinazolinyl optionally substituted with $R^3$, $R^4$ and $R^{18}$; and

$R^1$ is H; hydroxy, $C_1$-$C_4$ alkoxy, $C_1$-$C_4$ alkyl; $C_1$-$C_4$ haloalkyl; $C_2$-$C_3$ alkylcarbonyl; $C_2$-$C_4$ alkenyl; $C_2$-$C_4$ alkynyl; $C_2$-$C_3$ alkoxycarbonyl; or phenyl, phenylmethyl, 1-naphthalenyl, 2-naphthalenyl, thienyl, furanyl or pyridyl each optionally substituted with $R^8$, $R^9$ and $R^{10}$;

$R^3$, $R^4$ and $R^{18}$ are independently halogen, $C_1$-$C_4$ alkyl, cyclopropyl, $C_1$-$C_4$ haloalkyl, allyl, $C_2$-$C_3$ alkynyl, $C_1$-$C_4$ alkoxy or $C_1$-$C_4$ haloalkoxy;

$R^{23}$ is H, $C(=O)NHR^{26}$, or $C_2$-$C_4$ alkoxycarbonyl; and metal complexes thereof.

4. A compound of Claim 3 and metal complexes thereof, wherein:

A is 2-pyrimidinyl optionally substituted with $R^3$, $R^4$ and $R^{18}$;

n is 1 or 2;

E is phenyl, indanyl, tetrahydronaphthalenyl, 1-naphthalenyl, thienyl, or pyridyl each optionally substituted with $R^5$, $R^6$ and $R^7$;

$R^1$ is H; hydroxy, $C_1$-$C_4$ alkoxy, or $C_1$-$C_4$ alkyl;

$R^5$ is halogen; cyano; $C_1$-$C_4$ alkyl; $C_1$-$C_4$ haloalkyl; allyl; propargyl; $C_1$-$C_4$ alkoxy; $C_1$-$C_4$ haloalkoxy; or phenyl or phenoxy each optionally substituted with $R^{17}$; and

$R^6$, $R^7$, $R^9$, $R^{10}$ and $R^{17}$ are independently H, F, Cl, methyl, trifluoromethyl, methoxy or trifluoromethoxy.

5. A compound of Claim 4 and metal complexes thereof, wherein

E is phenyl, indanyl or tetrahydronaphthalenyl, each optionally substituted with $R^5$, $R^6$ or $R^7$; and

$R^2$ is H or $C_1$-$C_4$ alkyl.

6. The compound of Claim 1 selected from the group consisting of

1-(4,6-dimethyl-2-pyrimidinyl)-3-(3,4-dimethylphenyl)-1,4,5,6-tetrahydropyridazine;

1-(4,6-dimethyl-2-pyrimidinyl)-3-(4-ethylphenyl)-1,4,5,6-tetrahydropyridazine;

1-(4,6-dimethyl-2-pyrimidinyl)-1,4,5,6-tetrahydro-3-(4-methylphenyl)pyridazine;

1-(4,6-dimethyl-2-pyrimidinyl)-1,4,5,6-tetrahydro-3-(4-(1-methylethyl)phenyl)pyridazine;

1-(4,6-dimethyl-2-pyrimidinyl)-4-ethyl-1,4,5,6-tetrahydro-3-phenylpyridazine; and

1-(4,6-dimethyl-2-pyrimidinyl)-1,4,5,6-tetrahydro-4-methyl-3-phenylpyridazine.

7. A fungicidal composition comprising a fungicidally effective amount of any of the compounds of Claims 1,

2, 3, 4, 5 or 6 and an inert diluent or surfactant.

**8.** A method for controlling fungus disease in plants comprising applying to the locus to be protected an effective amount of a compound of Formulae I, II, III, IV, V or VI

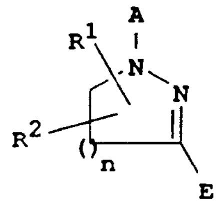
I

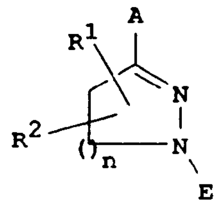
II

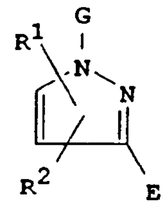
III

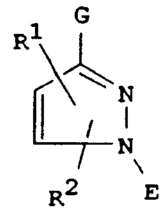
IV

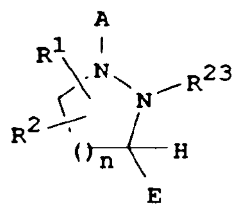
V

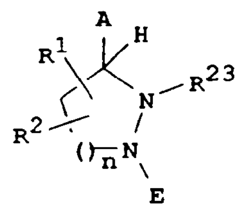
VI

wherein:

A and G are 2-pyrimidinyl, 2-pyridyl, 2-quinolinyl, 2-quinazolinyl, 1-isoquinolinyl or 3 isoquinolinyl each optionally substituted with $R^3$, $R^4$ and $R^{18}$; or s-triazinyl optionally substituted with $R^3$ and $R^4$; provided that $R^3$, $R^4$ and $R^{18}$ only substitute carbon atoms of the heterocycles;

E is H; halogen; $C_1$-$C_6$ alkyl; $C_3$-$C_7$ cycloalkyl optionally substituted with 1-2 methyl; $C_1$-$C_6$ haloalkyl; $C_1$-$C_6$ alkylthio; $C_1$-$C_6$ alkoxy; $C_1$-$C_6$ haloalkoxy; or phenyl, phenoxy, phenylthio, phenylamino, phenylmethyl, indanyl, tetrahydronaphthalenyl, 1-naphthalenyl,,2-naphthalenyl, thienyl, furanyl or pyridyl each optionally substituted with $R^5$, $R^6$ and $R^7$;

n is 1, 2 or 3;

$R^1$ is H; halogen; cyano; hydroxy, $C_1$-$C_4$ alkoxy, -OC(=O)$R^{19}$, -OC(=O)NH$R^{20}$ $C_1$-$C_4$ alkyl; $C_1$-$C_4$ haloalkyl; $C_2$-$C_3$ alkylcarbonyl; $C_2$-$C_4$ alkenyl; $C_2$-$C_6$ alkoxyalkyl; $C_2$-$C_4$ alkynyl; $C_2$-$C_3$ alkoxycarbonyl; or phenyl, phenylmethyl, 1-naphthalenyl, 2-naphthalenyl, thienyl, furanyl or pyridyl each optionally substituted with $R^8$, $R^9$ and $R^{10}$;

$R^2$ is H, cyano, $C_1$-$C_4$ alkyl or $C_1$-$C_4$ haloalkyl;

$R^3$, $R^4$ and $R^{18}$ are independently halogen; cyano; hydroxy; ($C_1$-$C_4$ alkyl)$_3$silylmethyl; phenyl optionally substituted with $R^{21}$; $C_1$-$C_6$ alkyl; cyclopropyl; $C_1$-$C_6$ haloalkyl; $C_1$-$C_6$ alkylthio; $C_2$-$C_4$ alkenyl; $C_2$-$C_4$ alkynyl; $C_1$-$C_4$ alkoxy; $C_1$-$C_4$ haloalkoxy; $C_2$-$C_4$ alkenyloxy; $C_2$-$C_4$ alkynyloxy; $C_2$-$C_4$ alkoxyalkyl; $NR^{11}R^{12}$; or when $R^3$ and $R^4$, $R^3$ and $R^{18}$ or $R^4$ and $R^{18}$ substitute adjacent carbon atoms, then $R^3$ and $R^4$, $R^3$ and $R^{18}$ or $R^4$ and $R^{18}$ may together be $-(CH_2)_3-$ or $-(CH_2)_4-$ each optionally substituted with 1-2 methyl;

$R^5$ and $R^8$ are independently halogen; cyano; nitro; hydroxy, hydroxycarbonyl; $C_1$-$C_6$ alkyl; $C_3$-$C_6$ cycloalkyl, $C_1$-$C_6$ haloalkyl; $C_1$-$C_4$ alkylthio; $C_1$-$C_4$ alkylsulfinyl; $C_1$ -$C_4$ alkylsulfonyl; ($C_1$-$C_4$ alkyl)$_3$silyl; $C_2$-$C_5$ alkylcarbonyl; $C_2$-$C_4$ alkenyl; $C_2$-$C_4$ alkenyloxy; $C_2$-$C_4$ alkynyl; $C_2$-$C_4$ alkynyloxy; $C_1$-$C_4$ alkoxy; $C_1$-$C_4$ haloalkoxy; $C_2$-$C_4$ alkoxyalkyl; $C_2$-$C_5$ alkoxycarbonyl; $C_2$-$C_4$ alkoxyalkoxy; $NR^{13}R^{14}$; C(=O)$NR^{15}R^{16}$; or phenyl, phenoxy or phenylthio each optionally substituted with $R^{17}$;

$R^6$, $R^7$, $R^9$, $R^{10}$, $R^{17}$, $R^{21}$, $R^{22}$, and $R^{24}$ are independently halogen, $C_1$-$C_4$ alkyl, $C_1$-$C_4$ haloalkyl, $C_1$-$C_4$ alkoxy or $C_1$-$C_4$ haloalkoxy;

$R^{11}$, $R^{12}$, $R^{13}$, $R^{14}$, $R^{15}$, $R^{16}$ are independently H; $C_1$-$C_2$ alkyl; or $R^{11}$ and $R^{12}$, $R^{13}$ and $R^{14}$ or $R^{15}$ and $R^{16}$ can be taken together with the nitrogen to which they attached to form a morpholino, pyrrolidino or piperidino group.

$R^{19}$ and $R^{25}$ are H or $C_1$-$C_3$ alkyl;

$R^{20}$ and $R^{26}$ are $C_1$-$C_4$ alkyl; or phenyl optionally substituted with $R^{22}$;

$R^{23}$ is H, $C_1$-$C_4$ alkyl, $C_1$-$C_4$ haloalkyl, $C_2$-$C_5$ alkylcarbonyl, phenylcarbonyl optionally substituted

with $R^{24}$, $C_3$-$C_4$ alkenyl, $C_3$-$C_4$ alkynyl, phenylmethyl optionally substituted with $R^{24}$ on the phenyl ring. $C_1$-$C_4$ alkylsulfinyl, $C_1$-$C_4$ alkylsulfonyl, phenylsulfinyl, $C_1$-$C_4$ alkylsulfonyl, phenylsulfinyl optionally substituted with $R^{24}$, phenylsulfonyl optionally substituted with $R^{24}$, $C_2$-$C_4$ alkoxycarbonyl, phenoxycarbonyl optionally substituted with $R^{24}$, $C(=O)NR^{25}R^{26}$, $C(=S)NHR^{26}$ $P(=S)(OR^{26})_2$, $P(=O)(OR^{26})_2$, or $S(=O)_2NR^{25}R^{26}$;

or their agriculturally suitable salts or metal complexes thereof;

provided that

i) when E is halogen, $C_1$-$C_6$ alkylthio, $C_1$-$C_6$ alkoxy, $C_1$-$C_6$ haloalkoxy, phenoxy, phenylthio or phenylamino, then E may only substitute compounds of Formula I and III;

ii) for compounds of Formula III, either E is phenyl, phenoxy, phenylthio, phenylamino, phenylmethyl, 1-naphthalenyl, 2-naphthalenyl, thienyl, furanyl, pyridyl each optionally substituted with $R^5$, $R^6$ and $R^7$; or $R^1$ is phenyl, benzyl, 1-naphthalenyl, 2-naphthalenyl, thienyl, furanyl or pyridyl each optionally substituted with $R^8$, $R^9$ and $R^{10}$; and $R^1$ must be in the 4-position;

iii) for compounds of Formula I, when E is H, n is 1, $R^1$ is 5-methyl, and $R^2$ is H, then A is not s-triazinyl optionally substituted with $R^3$ and $R^4$.

**9.** A method of Claim 8 employing compounds of Formulae I and V wherein:

A and G are 2-pyrimidinyl or 2-quinazolinyl optionally substituted with $R^3$, $R^4$ and $R^{18}$; and

$R^1$ is H; hydroxy, $C_1$-$C_4$ alkoxy, $C_1$-$C_4$ alkyl; $C_1$-$C_4$ haloalkyl; $C_2$-$C_3$ alkylcarbonyl; $C_2$-$C_4$ alkenyl; $C_2$-$C_4$ alkynyl; $C_2$-$C_3$ alkoxycarbonyl; or phenyl, phenylmethyl, 1-naphthalenyl, 2-naphthalenyl, thienyl, furanyl or pyridyl each optionally substituted with $R^8$, $R^9$ and $R^{10}$;

$R^3$, $R^4$ and $R^{13}$ are independently halogen, $C_1$-$C_4$ alkyl, cyclopropyl, $C_1$-$C_4$ haloalkyl, allyl, $C_2$-$C_3$ alkynyl, $C_1$-$C_4$ alkoxy or $C_1$-$C_4$ haloalkoxy;

$R^{23}$ is H, $C(=O)NHR^{26}$, or $C_2$-$C_4$ alkoxycarbonyl; and metal complexes thereof.

**10.** A method according to Claim 9 wherein:

A is 2-pyrimidinyl optionally substituted with $R^3$, $R^4$ and $R^{18}$;

n is 1 or 2;

E is phenyl, indanyl, tetrahydronaphthalenyl, 1-naphthalenyl, thienyl, or pyridyl each optionally substituted with $R^5$, $R^6$ and $R^7$;

$R^1$ is H; hydroxy, $C_1$-$C_4$ alkoxy, or $C_1$-$C_4$ alkyl;

$R^5$ is halogen; cyano; $C_1$-$C_4$ alkyl; $C_1$-$C_4$ haloalkyl; allyl; propargyl; $C_1$-$C_4$ alkoxy; $C_1$-$C_4$ haloalkoxy; or phenyl or phenoxy each optionally substituted with $R^{17}$; and

$R^6$, $R^7$, $R^9$, $R^{10}$ and $R^{17}$ are independently H, F, Cl, methyl, trifluoromethyl, methoxy or trifluoromethoxy;

and metal complexes thereof.

**11.** A method according to Claim 10 wherein

E is phenyl, indanyl or tetrahydronaphthalenyl each optionally substituted with $R^5$, $R^6$ and $R^7$; and

$R^2$ is H or $C_1$-$C_4$ alkyl.

**12.** The method of Claim 11 wherein the compound is selected from the group consisting of

1-(4,6-dimethyl-2-pyrimidinyl)-3-(3,4-dimethylphenyl)-1,4,5,6-tetrahydropyridazine;

1-(4,6-dimethyl-2-pyrimidinyl)-3-(4-ethylphenyl)-1,4,5,6-tetrahydropyridazine;

1-(4,6-dimethyl-2-pyrimidinyl)-1,4,5,6-tetrahydro-3-(4-methylphenyl)pyridazine;

1-(4,6-dimethyl-2-pyrimidinyl)-1,4,5,6-tetrahydro-3-(4-(1-methylethyl)phenyl)pyridazine;

1-(4,6-dimethyl-2-pyrimidinyl)-4-ethyl-1,4,5,6-tetrahydro-3-phenylpyridazine; and

1-(4,6-dimethyl-2-pyrimidinyl)-1,4,5,6-tetrahydro-4-methyl-3-phenylpyridazine.